# EUROPÄISCHE PATENTSCHRIFT

(11) **EP 0 509 398 B1**
(45) Veröffentlichungstag und Bekanntmachung des Hinweises auf die Patenterteilung: **19.09.2001**
(21) Anmeldenummer: 92106158.6
(22) Anmeldetag: 09.04.1992
(51) Int. Cl.: C07D 241/44, C07D 241/38, C07D 241/52, C07D 401/04, C07D 409/06, C07D 487/04, A61K 31/495

(54) **Chinoxaline, Verfahren zu ihrer Herstellung und ihre Verwendung**
Quinoxalines, process for their preparation and their use
Quinoxalines, procédé pour leur préparation et leur utilisation

(30) Priorität: 15.04.1991 DE 4112234; 20.12.1991 DE 4142322
(43) Veröffentlichungstag der Anmeldung: 21.10.1992
(73) Patentinhaber: Aventis Pharma Deutschland GmbH, 65929 Frankfurt am Main (DE)
(72) Erfinder: Billhardt, Uta-Maria, Dr., W-6242 Kronberg/Taunus (DE); Rösner, Manfred, Dr., W-6239 Eppstein/Taunus (DE); Riess, Günther, Dr., W-6234 Hattersheim (DE); Winkler, Irvin, Dr., W-6237 Liederbach (DE); Bender, Rudolf, Dr., W-6232 Bad Soden am Taunus (DE)

(56) Entgegenhaltungen:
- EP-A- 0 190 105
- EP-A- 0 266 102
- US-A- 4 032 639
- CHEMICAL ABSTRACTS, vol. 78, no. 28, 1973, Columbus, Ohio, US; abstract no. 136218V, SH. OHMIYA ET AL.: 'SYNTHESIS OF CONDENSED QUINOXALINES. IV.' Seite 379 ;Spalte 2 & CHEMICAL AND PHARMACEUTICAL BULLETIN Bd. 21, Nr. 2, 1973, TOKYO JP Seiten 353-357
- CHEMICAL ABSTRACTS, vol. 116, 1992, Columbus, Ohio, US; abstract no. 83647D, N. KALYANAM: 'STUDIES OF ANTIAMOEBIC COMPOUNDS. PART 2.' Seite 818 ;Spalte 2 & INDIAN J. CHEM. SECT B Bd. 30, Nr. B11, 1991, BOMBAY Seiten 1077-1079
- DATABASE WPI, Woche , Derwent Publications Ltd., London, GB, Klasse , AN 6632543 & BE 706623 A
- HINSBERG: "Ueber hydrirte Oxychinoxaline", ANNALEN DER CHEMIE, , 1888, Band 248, Nr. , Seiten 71 - 84
- BIRD: "The nature of some diazetidinone rarrangements", J.CHEM.SOC., , 1963, Band , Nr. , Seiten 674 - 677

## Beschreibung

Gegenstand der vorliegenden Erfindung sind Chinoxaline, Verfahren zu ihrer Herstellung und ihre Verwendung.
Chinoxaline sind eine altbekannte Verbindungsklasse (O. Hinsberg, J. Liebigs Ann. Chem. 237, 327 (1986)).
In der Patentliteratur sind Chinoxalinderivate für verschiedene medizinische Anwendungen beschrieben.
Die österreichische Patentschrift 28 48 48 (19.12.67) nennt 1-N-Dialkylaminoalkyl-3,4-dihydro-chinoxalin-2(1H)- one als Spasmolytika. Antiinflammatorisch wirkende 4-N-Aroyl-, Arylacyl- und Arylsulfonyl-3,4-dihydro-chinoxalin-2(1H)-one werden in einer Reihe von Patentanmeldungen der japanischen Firma Sumitomo Chem. Co. Ltd. beschrieben (JA 17 137/69 (11.4.66), JA 17 136/69 (8.4.66), JA 7 008/422 (9.8.66), BE 706 623 (16.11.66)). 3,4-Dihydro-chinoxalin-2(1H)-on-3-carboxamide sind in der US-Patentanmeldung US 3 654 275 (4.4.72) enthalten. Sie wirken ebenfalls antiinflammatorisch. Als antihypertensive und antisekretorische Reagenzien werden Pyridinylalkyl- tetrahydro-pyrazino[1,2-a]chinoxalinon-derivate in den US- Anmeldungen US 4 203 987 (21.5.79) und 4 032 639 (22.3.76) von American Home Prod. Corp. beschrieben. Eine europäische Patentanmeldung von Pfizer Inc. (EP 266 102 A (30.10.86)) beinhaltet 4-N-Benzolsulfonyl-3,4-dihydrochinoxalin-2(1H)-on-1-alkylcarbonsäuren als Aldose-Reduktase-Inhibitoren.
Antivirale Aktivität wurde bisher jedoch noch nicht nachgewiesen.

Weiterhin sind bestimmte Chinoxaline in der Landwirtschaft bekannt und zwar als herbizide Mittel (vgl. Ep 0 190 105).
In der Absicht, neue Verbindungen mit antiviraler Wirkung bereitzustellen wurde nun überraschenderweise gefunden, daß Chinoxaline der Formeln I und la 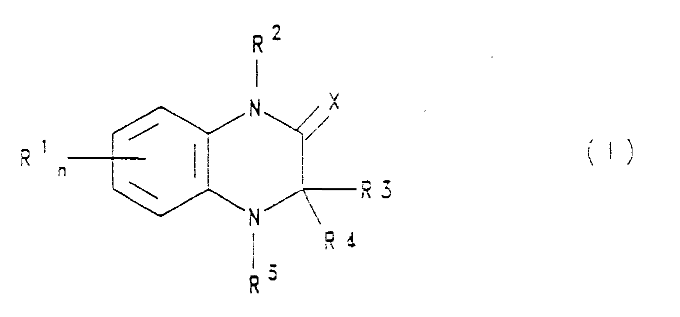 sowie deren tautomere Formen der allgemeinen Formel la 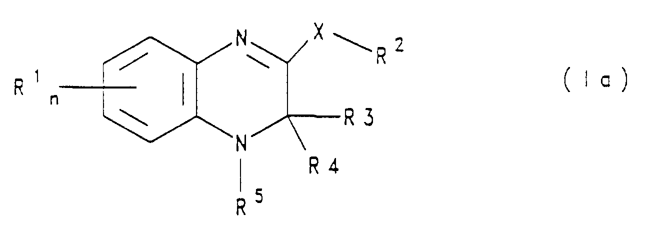 sowie deren physiologisch verträgliche Salze eine antivirale Wirkung insbesondere gegen Retroviren, wie zum Beispiel das 'Human Immunodeficiency Virus' (HIV) aufweisen.

In den erfindungsgemäßen Verbindungen der Formel I bzw. la bedeuten:
1)
   - n: null,
   eins
   oder zwei,
   die einzelnen Substituenten R¹ unabhängig voneinander
   Fluor, Chlor, Brom, Trifluormethyl, Hydroxy, C1 - C4-Alkyl, C1 - C4-Alkoxy, (C1 - C4-Alkoxy)-(C1 - C4-alkoxy), C1- C4-Alkylthio, Nitro, Amino, C1 - C4-Alkylamino, Di(C1 - C4-alkyl)amino, Piperidino, Morpholino, 1-Pyrrolidinyl, 4-Methylpiperazinyl, C1 - C4-Acyl, C1 - C4-Acyloxy, C1 - C4-Acylamino, Cyano, Carbamoyl, Carboxy, (C1 - C4-Alkyl)-oxycarbonyl, Hydroxysulfonyl, Sulfamoyl
   oder
   einen mit bis zu zwei
   voneinander unabhängigen Resten R⁶ substituierten Phenyl-, Phenoxy-, Phenylthio-, Phenylsulfonyl-, Phenoxysulfonyl-, Benzoyl-, 2-Pyridyl-, 3-Pyridyl- oder 4-Pyridylrest,
   wobei R⁶
   Fluor, Chlor, Brom, Cyano, Trifluormethyl, Nitro, Amino, C1 - C4-Alkyl, C1 - C4-Alkoxy, (C1 - C4-Alkyl)-oxycarbonyl, Phenyl, Phenoxy,
   sein kann,
   R² Wasserstoff und R⁵
   C1 - C6-Alkyl,
   gegebenenfalls substituiert mit Fluor,
   Chlor, Hydroxy, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, Oxo, Thioxo, Carboxy, Carbamoyl;
   C2 - C6-Alkenyl,
   gegebenenfalls substituiert mit Fluor,
   Chlor, Hydroxy, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, Oxo, Thioxo, Carboxy, Carbamoyl;
   Allenyl,
   C3 - C8-Alkinyl,
   gegebenenfalls substituiert mit Fluor,
   Chlor, Hydroxy, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, Oxo, Thioxo, Carboxy, Carbamoyl;
   C3 - C8-Cycloalkyl,
   gegebenenfalls substituiert mit Fluor,
   Chlor, Hydroxy, C1 - C4-Alkyl, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, Oxo, Thioxo, Carboxy, Carbamoyl;
   C3 - C8-Cycloalkenyl,
   gegebenenfalls substituiert mit Fluor,
   Chlor, Hydroxy, C1 - C4-Alkyl, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, Oxo, Thioxo, Carboxy, Carbamoyl;
   (C3 - C6-Cycloalkyl)-(C1 - C2-alkyl),
   gegebenenfalls substituiert mit Fluor,
   Chlor, Hydroxy, C1 - C4-Alkyl, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, Oxo, Thioxo, Carboxy, Carbamoyl;
   (C3 - C6-Cycloalkenyl)-(C1 - C2-alkyl),
   gegebenenfalls substituiert mit Fluor,
   Chlor, Hydroxy, C1 - C4-Alkyl, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, Oxo, Thioxo, Carboxy, Carbamoyl;
   C1 - C6-Alkylcarbonyl,
   gegebenenfalls substituiert mit Fluor,
   Chlor, Hydroxy, C1 - C4-Alkyl, C1 - C4-Acyloxy, Benzoyloxy, Benzyloxy, Phenoxy, C1 - C4-Alkoxy, C1 - C4-Alkylamino, C1 - C4-Alkenylamino, Di(C1 - C4-alkyl)amino, C1 - C4-Alkylthio, Oxo, Thioxo, Carboxy, Carbamoyl;
   C2 - C6-Alkenylcarbonyl, gegebenenfalls substituiert durch Fluor, Chlor oder Hydroxy;
   (C3 - C6-Cycloalkyl)carbonyl,
   (C5 - C6-Cycloalkenyl)carbonyl,
   (C3 - C6-Cycloalkyl)-(C1 - C2-alkyl)carbonyl,
   (C5 - C6-Cycloalkenyl)-(C1 - C2-alkyl)carbonyl,
   C1 - C6-Alkyloxycarbonyl, gegebenenfalls substituiert durch Fluor, Chlor, Brom, Hydroxy, C1 - C4-Alkoxy, C1 - C4-Alkylamino, Di(C1 - C4-alkyl)amino, C1 - C4-Alkylthio;
   C2 - C6-Alkenyloxycarbonyl, gegebenenfalls substituiert durch Fluor, Chlor, Hydroxy, C1 - C4-Alkoxy;
   C2 - C6-Alkinyloxycarbonyl, gegebenenfalls substituiert durch Fluor, Chlor, Hydroxy, C1 - C4-Alkoxy;
   C1 - C6-Alkylthiocarbonyl, gegebenenfalls substituiert durch Fluor, Chlor, Hydroxy, C1 - C4-Alkoxy;
   C2 - C6-Alkenylthiocarbonyl, gegebenenfalls substituiert durch Fluor, Chlor, Hydroxy, C1 - C4-Alkoxy;
   C1 - C6-Alkylamino- und Di(C1 - C6-alkyl)aminocarbonyl, gegebenenfalls substituiert durch Fluor, Chlor, Hydroxy, C1 - C4-Alkoxy;
   Pyrrolidin-1-yl, Morpholino-, Piperidino-, Piperazinyl-, oder 4-Methylpiperazin-1-yl-carbonyl;
   C2 - C6-Alkenylamino- und Di(C1 - C6-alkenyl)aminocarbonyl, gegebenenfalls substituiert durch Fluor, Chlor, Hydroxy, C1 - C4-Alkoxy;
   C1 - C4-Alkylsulfonyl, gegebenenfalls substituiert durch Fluor, Chlor, Hydroxy, C1 - C4-Alkoxy;
   C1 - C4-Alkenylsulfonyl;
   oder mit bis zu zwei voneinander unabhängigen Resten R⁶ substituiertes Aryl, Arylcarbonyl, (Arylthio)carbonyl, Aryloxycarbonyl, Arylaminocarbonyl, (Arylamino)thiocarbonyl, Arylsulfonyl, Arylalkylaminocarbonyl, Arylalkyl, Arylalkenyl, Arylalkoxycarbonyl, Aryl(alkylthio)carbonyl, wobei der Alkylrest jeweils 1 bis 3 C-Atome enthalten kann und R⁶ wie oben definiert ist
   oder mit bis zu zwei voneinander unabhängigen Resten R⁶ substituiertes 1- oder 2-Naphthylmethyl, 2-, 3- oder 4-Picolyl, 2- oder 3-Furylmethyl, 2- oder 3-Thienylmethyl, 2- oder 3-Pyrrolylmethyl,
   2-, 3- oder 4-Pyridylcarbonyl, 2- oder 3-Furylcarbonyl, 2- oder 3-Thienylcarbonyl, 2-, 3- oder 4-Picolyloxycarbonyl, 2-oder 3-Furylmethyloxycarbonyl, 2- oder 3-Thienylmethyloxycarbonyl,
   und
   R³ und R⁴ gleich oder verschieden, unabhängig voneinander Wasserstoff, C1 - C4-Alkyl, gegebenenfalls substituiert mit Fluor, Chlor, Hydroxy, Amino, Mercapto, C1 - C4-Acyloxy, Benzoyloxy, Phenoxy, C1 - C4-Alkoxy, C1 - C4-Alkylamino, Di(C1 - C4-alkyl)amino, C1 - C4-Alkylthio, C1 - C4-Alkylsulfonyl, C1 - C4-Alkylsulfinyl, Carboxy, Carbamoyl;
   C2 - C6-Alkenyl, gegebenenfalls substituiert mit Fluor oder Chlor;
   C3 - C6-Cycloalkyl, gegebenenfalls substituiert mit Fluor, Chlor, Hydroxy, Amino, Mercapto, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, C1 - C4-Alkylsulfonyl, C1 - C4-Alkylsulfinyl, Carboxy, Carbamoyl;
   C3 - C8-Cycloalkenyl, gegebenenfalls substituiert mit Fluor oder Chlor;
   mit bis zu zwei voneinander unabhängigen Resten R⁶ substituiertes Aryl, Benzyl, Heteroaryl oder Heteroarylmethyl bedeuten,
   R³ und R⁴ können ferner auch
   Teil eines gesättigten oder ungesättigten carbo- oder heterocyclischen Ringes mit 3 bis 6 C-Atomen sein, der gegebenenfalls mit Fluor, Chlor, Hydroxy, Amino, C1 - C4-Acyloxy, Benzoyloxy, C1 - C4-Alkoxy, Oxo, Thioxo, Carboxy, Carbamoyl substituiert sein kann, und
   die in den vorausgegangenen Definitionen genannten Arylgruppen sind aromatische Gruppen mit 6-14 C-Atomen, die in den vorausgegangenen Definitionen genannten heterocyclischen Ringe bzw. Heteroarylgruppen enthalten 1-13 C-Atome und 1-6 Heteroatome ausgewählt aus der Gruppe O, S und N, wobei im Falle eines an dieser Stelle gesättigten N-haltigen Ringes N - Z vorliegt, worin Z H oder R⁵ mit den jeweiligen oben beschriebenen Definitionen bedeutet,
   X bedeutet Sauerstoff oder Schwefel
   mit Ausnahme der Verbindungen in denen R³ und R⁴ gleichzeitig H bedeuten
   und Verbindungen, in denen R⁵ COCHCl₂ bedeuten und Verbindungen, in denen n 1, R¹, R³ und R⁴ Methyl und R⁵ Acetyl bedeuten und Verbindungen, in denen n Null, R³ und R⁴ Methyl oder Phenyl und R⁵ Ethyloxycarbonyl bedeuten.
   In einer bevorzugten Gruppe von Verbindungen der Formel I bzw. la bedeuten:
2)
   - n: null,
   eins
   oder zwei,
   die einzelnen Substituenten R¹ unabhängig voneinander
   Fluor, Chlor, Brom, Trifluormethyl, Hydroxy, C1 - C4-Alkyl, C1 - C4-Alkoxy, (C1 - C4-Alkoxy)-(C1 - C2-alkoxy), C1- C4-Alkylthio, Nitro, Amino, C1 - C4-Alkylamino, Di(C1 - C4-alkyl)amino, Piperidino, Morpholino, 1-Pyrrolidinyl, 4-Methylpiperazinyl, C1 - C4-Acyl, C1 - C4-Acyloxy, C1 - C4-Acylamino, Cyano, Carbamoyl, Carboxy, (C1 - C4-Alkyl)-oxycarbonyl, Hydroxysulfonyl, Sulfamoyl
   oder
   einen mit bis zu zwei
   voneinander unabhängigen Resten R⁶ substituierten Phenyl-, Phenoxy-, Phenylthio-, Phenylsulfonyl-, Phenoxysulfonyl-, Benzoyl-, 2-Pyridyl-, 3-Pyridyl- oder 4-Pyridylrest,
   wobei R⁶
   Fluor, Chlor, Brom, Cyano, Trifluormethyl, Nitro, Amino, C1 - C4-Alkyl, C1 - C4-Alkoxy, (C1 - C4-Alkyl)-oxycarbonyl, Phenyl, Phenoxy
   sein kann,
   R² Wasserstoff und R⁵
   C1 - C6-Alkyl
   gegebenenfalls substituiert mit C1 - C4-Alkoxy oder C1 - C4-Alkylthio;
   C2 - C6-Alkenyl,
   gegebenenfalls substituiert mit Oxo;
   Allenyl,
   C3 - C8-Alkinyl, insbesondere 2-Butinyl;
   C3 - C6-Cycloalkyl;
   C5 - C6-Cycloalkenyl;
   (C3 - C6-Cycloalkyl)-(C1 - C2-alkyl), insbesondere Cyclopropylmethyl, gegebenenfalls substituiert mit C1 - C4-Alkyl;
   (C3 - C6-Cycloalkenyl)-(C1 - C2-alkyl), insbesondere Cyclohexenylmethyl;
   C1 - C6-Alkylcarbonyl,
   gegebenenfalls substituiert mit Fluor,
   Chlor, Hydroxy, Benzyloxy, Phenoxy, C1 - C4-Alkoxy, C1 - C4-Alkylamino, C1 - C4-Alkenylamino, Di(C1 - C4-alkyl)amino, C1 - C4-Alkylthio;
   C2 - C6-Alkenylcarbonyl;
   C1 - C6-Alkyloxycarbonyl, gegebenenfalls substituiert durch Fluor, Chlor, Brom, Hydroxy, C1 - C4-Alkoxy, C1 - C4-Alkylamino, Di(C1 - C4-alkyl)amino, C1 - C4-Alkylthio;
   C2 - C6-Alkenyloxycarbonyl, insbesondere Vinyloxycarbonyl, Allyloxycarbonyl, Isopropenyloxycarbonyl, Butenyloxycarbonyl, Pentenyloxycarbonyl;
   C2 - C6-Alkinyloxycarbonyl, insbesondere Propinyloxycarbonyl, Butinyloxycarbonyl;
   C1 - C6-Alkylthiocarbonyl;
   C2 - C6-Alkenylthiocarbonyl, insbesondere Allylthiocarbonyl;
   C1 - C6-Alkylamino- und Di(C1 - C6-alkyl)aminocarbonyl;
   Pyrrolidin-1-yl, Morpholino-, Piperidino-, Piperazinyl-, oder 4-Methylpiperazin-1-yl-carbonyl;
   C2 - C6-Alkenylamino- und Di(C1 - C6-alkenyl)aminocarbonyl;
   C1 - C4-Alkylsulfonyl;
   C1 - C4-Alkenylsulfonyl;
   oder mit bis zu zwei voneinander unabhängigen Resten R⁶ substituiertes Aryl, insbesondere Phenyl,
   Arylcarbonyl, insbesondere Benzoyl, (Arylthio)carbonyl, Aryloxycarbonyl, Arylaminocarbonyl, (Arylamino)thiocarbonyl, Arylalkylaminocarbonyl, Arylsulfonyl,
   Arylalkyl, insbesondere Benzyl, Phenylethyl, Arylalkenyl, Arylalkylcarbonyl, Arylalkoxycarbonyl, Aryl(alkylthio)carbonyl, wobei der Alkylrest jeweils 1 bis 3 C-Atome enthalten kann und R⁶ wie oben definiert ist
   oder mit bis zu zwei voneinander unabhängigen Resten R⁶ substituiertes 1- oder 2-Naphthylmethyl, 2-, 3- oder 4-Picolyl, 2- oder 3-Furylmethyl, 2- oder 3-Thienylmethyl, 2- oder 3-Pyrrolylmethyl,
   2-, 3- oder 4-Pyridylcarbonyl, 2- oder 3-Furylcarbonyl, 2- oder 3-Thienylcarbonyl, 2-, 3- oder 4-Picolyloxycarbonyl, 2-oder 3-Furylmethyloxycarbonyl, 2- oder 3-Thienylmethyloxycaronyl,
   und
   R³ und R⁴ gleich oder verschieden, unabhängig voneinander
   Wasserstoff,
   C1 - C4-Alkyl,
   gegebenenfalls substituiert mit Hydroxy, Mercapto,
   C1 - C4-Alkoxy, C1 - C4-Alkylthio, C1 - C4-Alkylsulfonyl, C1 - C4-Alkylsulfinyl, Carboxy, Carbamoyl;
   C2 - C6-Alkenyl,
   mit bis zu zwei voneinander unabhängigen Resten R⁶ substituiertes Aryl, Benzyl, Thienyl oder Thienylmethyl, wobei R⁶ wie oben definiert ist, bedeuten,
   R³ und R⁴ können auch
   Teil eines gesättigten oder ungesättigten carbo- oder heterocyclischen Ringes mit 3 bis 6 C-Atomen sein, der gegebenenfalls mit Oxo oder Thioxo substituiert sein kann und
   X bedeutet Sauerstoff oder Schwefel.
   Eine ganz besondere Bedeutung haben Verbindungen der Formel I oder la wie oben beschrieben, dadurch gekennzeichnet, daß die genannten Substituenten bedeuten:
3)
   - n: null oder
   eins
   die einzelnen Substituenten R¹ unabhängig voneinander
   Fluor, Chlor, Brom, C1 - C2-Alkyl, C1 - C2-Alkoxy, C2 - C4-Acyl, Cyano
   R² Wasserstoff und R⁵
   C2-C6-Alkenyl
   C3 - C8-Alkinyl, insbesondere 2-Butinyl;
   (C3 - C6-Cycloalkyl)-(C1 - C2-alkyl), insbesondere Cyclopropylmethyl, gegebenenfalls substituiert mit C1 - C4-Alkyl;
   (C3 - C6-Cycloalkenyl)-(C1 - C2-alkyl), insbesondere Cyclohexenylmethyl;
   C2 - C6-Alkylcarbonyl,
   C2 - C6-Alkenylcarbonyl;
   C1 - C6-Alkyloxycarbonyl;
   C2 - C6-Alkenyloxycarbonyl, insbesondere Vinyloxycarbonyl, Allyloxycarbonyl, Isopropenyloxycarbonyl, Butenyloxycarbonyl, Pentenyloxycarbonyl;
   C2 - C6-Alkinyloxycarbonyl, insbesondere Propinyloxycarbonyl, Butinyloxycarbonyl;
   C2 - C6-Alkenylthiocarbonyl, insbesondere Allylthiocarbonyl;
   C1 - C4-Alkylsulfonyl;
   C1 - C4-Alkenylsulfonyl;
   oder mit bis zu zwei voneinander unabhängigen Resten R⁶ substituiertes Arylalkyl, insbesondere Benzyl oder Arylalkenyl, wobei der Alkylrest jeweils 1 bis 3 C-Atome und der Alkenylrest 2-3 C-Atome enthalten kann
   oder mit bis zu zwei voneinander unabhängigen Resten R⁶ substituiertes 1-Naphthylmethyl, 2- oder 3-Picolyl, 2-Furylmethyl, 2- oder 3-Thienylmethyl,
   wobei R⁶
   Fluor, Chlor, Brom, Cyano, C1 - C2-Alkyl, C1 - C2-Alkoxy
   und
   R³ und R⁴ gleich oder verschieden, unabhängig voneinander
   Wasserstoff,
   C1 - C4-Alkyl,
   gegebenenfalls substituiert mit Hydroxy, Mercapto, C1 - C4-Alkoxy, C1 - C2-Alkylthio und X bedeutet Sauerstoff oder Schwefel.

Die in den vorangegangenen Definitionen genannten Alkylgruppen können geradkettig oder verzweigt sein. Sofern nicht anders definiert, enthalten sie vorzugsweise 1-8, besonders bevorzugt 1-6, insbesondere 1-4 C-Atome. Beispiele sind die Methyl-, Ethyl-, Propyl-, 1-Methylethyl-, Butyl-, 1-Methylpropyl-, 2-Methylpropyl-, 1,1-Dimethylethylgruppe und ähnliche.

Die in den vorangegangenen Definitionen genannten Alkenylgruppen können geradkettig oder verzweigt sein und enthalten 1 bis 3 Doppelbindungen. Sofern nicht anders definiert, enthalten diese Gruppen vorzugsweise 2-8, insbesondere 2-6 C-Atome. Beispiele sind die 2-Propenyl-, 1-Methylethenyl, 2-Butenyl-, 3-Butenyl-, 2-Methyl-2-propenyl-, 3- Methyl-2-butenyl, 2,3-Dimethyl-2-butenyl, 3,3-Dichlor-2-propenyl, Pentadienylgruppe und ähnliche.

Die in den vorangegangenen Definitionen genannten Alkinylgruppen können geradkettig oder verzweigt sein und enthalten 1 bis 3 Dreifachbindungen. Sofern nicht anders definiert, enthalten sie vorzugsweise 2-8, besonders bevorzugt 3-6 C-Atome. Beispiele sind die 2-Propinyl- und 3-Butinylgruppe und ähnliche.

Die in den vorangegangenen Definitionen genannten Cycloalkyl- und Cycloalkenylgruppen enthalten, sofern nicht anders definiert, vorzugsweise 3-8, besonders bevorzugt 4-6 C-Atome. Beispiele sind die Cyclopropyl-, Cyclobutyl-, Cyclopentyl-, Cyclopentenyl-, Cyclohexyl- oder Cyclohexenylgruppe.

Die in den vorangegangenen Definitionen genannten Acylgruppen können aliphatisch, cycloaliphatisch oder aromatisch sein. Sofern nicht anders definiert, enthalten sie vorzugsweise 1-8, besonders bevorzugt 2-7 C-Atome. Beispielhafte Acylgruppen sind die Formyl-, Acetyl-, Chloracetyl-, Trifluoracetyl-, Hydroxyacetyl-, Propionyl-, Butyryl-, Isobutyryl-, Pivaloyl-, Cyclohexanoyl- oder Benzoylgruppe.

Die in den vorausgegangenen Definitionen genannten Arylgruppen sind vorzugsweise aromatische Gruppen mit 6-14 C-Atomen, insbesondere mit 6-10 C-Atomen wie z.B. Phenyl, Naphthyl.

In den obengenannten heterocyclischen Ringen bzw. Heteroarylgruppen kommen als Heteroatome insbesondere zum Beispiel O, S, N in Betracht, wobei im Falle eines an dieser Stelle gesättigten N-haltigen Ringes N-Z vorliegt, worin Z, H oder R⁵ mit den jeweiligen oben beschriebenen Definitonen bedeutet.

Soweit nicht anders definiert, haben die heterocyclischen Ringe vorzugsweise 1-13 C-Atome und 1-6 Heteroatome, insbesondere 3-9 C-Atome und 1-4 Heteroatome.

Für die in den vorangegangenen Definitionen genannten Heteroarylgruppen kommen beispielsweise heteroaromatische Reste wie 2- oder 3- Thienyl, 2- oder 3-Furyl, 2-, 3- oder 4- Pyridyl, Pyrimidyl, Indolyl, Chinolyl oder Isochinolyl in Frage.

Die in den vorausgegangenen Definitionen aufgeführten Aralkylgruppen sind beispielsweise Benzyl, Phenylethyl, Naphthylmethyl oder Styryl.

Die obengenannten Substituenten R¹ bis R⁵ sind vorzugsweise 3-fach, besonders bevorzugt 2-fach, insbesondere einfach mit den jeweils angegebenen Substituenten substituiert.

Für die jeweiligen zusammengesetzten Substituentendefinitionen (wie z.B. Arylalkoxycarbonyl) sind die zuvor als bevorzugt beschriebenen Bereiche für die einzelnen Substituenten ebenfalls bevorzugt.

In Abhängigkeit von den verschiedenen Substituenten können Verbindungen der Formeln I und la mehrere asymmetrische Kohlenstoffatome besitzen.
Gegenstand der Erfindung sind deshalb sowohl die reinen Stereoisomeren als auch Mischungen derselben, wie z. B. das zugehörige Racemat.
Die reinen Stereoisomeren der Verbindungen der Formeln I und la lassen sich durch bekannte Methoden oder in Analogie zu bekannten Methoden direkt herstellen oder nachträglich trennen.

Die Verbindungen der Formeln I und la lassen sich nach bekannten Methoden oder Modifikationen derselben herstellen (s. z. B. Rodd's Chemistry of Carbon Compounds, S. Coffey, M. F. Ansell (Herausgeber); Elsevier, Amsterdam, 1989; Vol. IV Teil IJ, S. 301 - 311. Heterocyclic Compounds, R. C. Elderfield (Herausgeber); Wiley, New York, 1957; Vol. 6, S. 491 - 495).
Zum Gegenstand der vorliegenden Erfindung gehört weiterhin
ein Verfahren zur Herstellung von Verbindungen der Formel I und la wie oben unter 1) erläutert, dadurch gekennzeichnet, daß
A) zur Herstellung von Verbindungen der Formel I mit X gleich Sauerstoff und den Resten R¹, R², R³, R⁴ und R⁵ wie in unter 1) - 4) definiert eine Verbindung der Formel II, 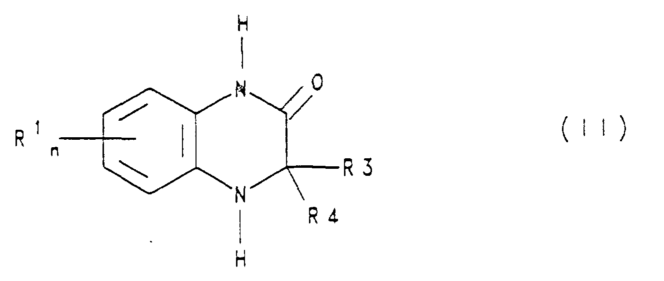 wobei für R¹, R³ und R⁴ die in unter 1) genannten Definitionen gelten, mit einer Verbindung der Formel III,

   R-Z (III)

   wobei R die oben unter 1) genannten Bedeutungen für R⁵ und R² mit Ausnahme von Wasserstoff, Hydroxy, C1 - C6-Alkoxy, Aryloxy, C1 - C6-Acyloxy, Amino, C1 - C6-Alkylamino, Di(C1 - C6-alkyl)amino, Arylamino, C1 - C6-Acylamino hat und Z eine Abgangsgruppe ist umsetzt
   oder
   daß B) Verbindungen der Formel I, mit X gleich Schwefel und R¹, R², R³, R⁴ und R⁵ wie unter 1) definiert hergestellt werden durch Reaktion einer Verbindung der Formel I, wobei X Sauerstoff ist und für R¹, R², R³, R⁴ und R⁵ die unter 1) genannten Definitionen gelten, mit einem Schwefelungsreagenz,
   oder
daß C)
   Verbindungen der Formel la, wobei X und die Reste R¹ bis R⁵ wie unter 1) definiert, hergestellt werden, indem man eine Verbindung der Formel IV, 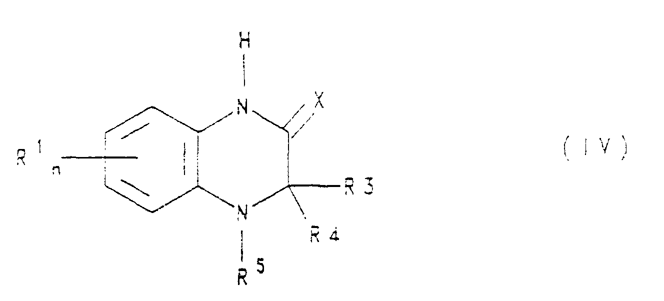 bzw. 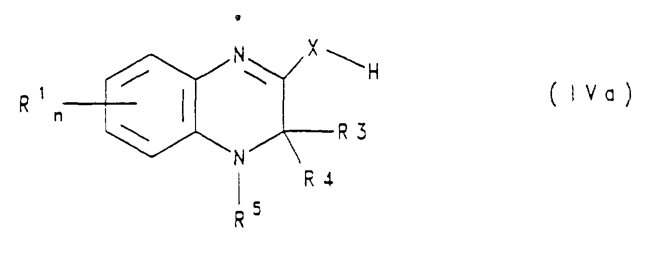 wobei für R¹, R³, R⁴ und R⁵ die unter 1) genannten Definitionen gelten, mit einer Verbindung der Formel III,

   R²-Z (III)

   wobei für R² die unter 1) für Formel I und la beschriebenen Definitionen mit Ausnahme von Wasserstoff, Hydroxy, C1 - C6-Alkoxy, Aryloxy, C1 - C6-Acyloxy, Amino, C1 - C6-Alkylamino, Di(C1 - C6-alkyl)amino, Arylamino, C1 - C6-Acylamino gelten und Z eine Abgangsgruppe ist umsetzt,
   oder
daß D)
   Verbindungen der Formel I mit X gleich Sauerstoff und den Resten R¹ bis R⁵ wie unter 1) definiert durch Cyclisierung einer Verbindung der Formel V 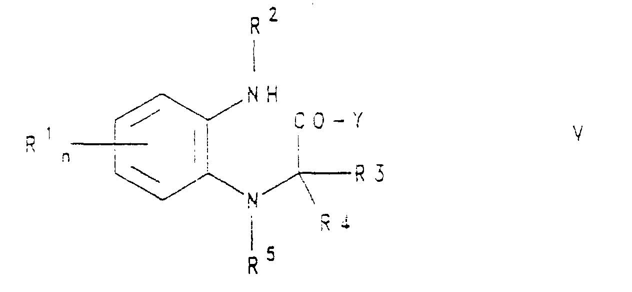 mit R¹ bis R⁵ wie unter 1) definiert und Y gleich Hydroxy, C1 - C4-Alkoxy, ggf. halogeniertes C1 - C4-Acyloxy, Chlor, Brom oder Jod, hergestellt werden,
   oder
daß E)
   Verbindungen der Formel I, wobei X Sauerstoff ist, R⁴ und R⁵ Wasserstoff sind und für R¹ bis R³ die unter 1) genannten Definitionen gelten, aus den Chinoxalinonen der Formel XI, 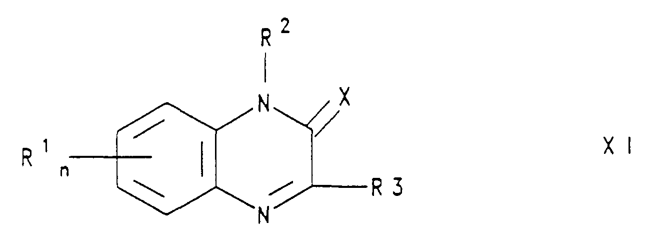 mit R¹ bis R³ wie unter 1) definiert, hergestellt werden, indem man an die C=N- Bindung Wasserstoff anlagert,
   oder
daß F)
   Verbindungen der Formel I, wobei X Sauerstoff und R¹ bis R⁵ wie unter 1) definiert, hergestellt werden aus Verbindungen der Formel VI, 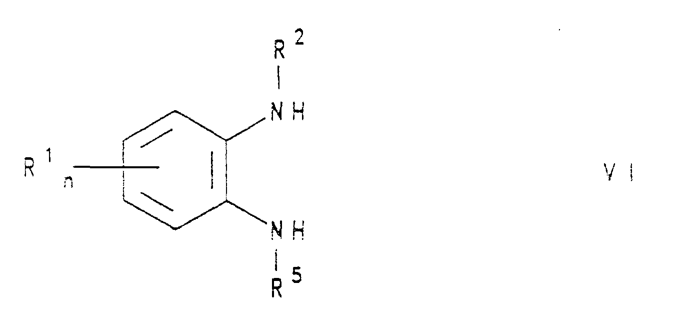 mit R¹, R² und R⁵ wie unter 1) definiert, durch Umsetzung mit Chloroform oder Bromoform und einer Carbonylverbindung der Formel XIII,

   R³-CO-R⁴ (XIII)

   mit R³ und R⁴ wie unter 1) definiert oder mit α-(Trihalogenmethyl)-alkanolen der Formel XIV,

   Hal₃C-C(OH)-R³R⁴ (XIV)

   worin Hal für Cl, Br oder J steht,
   in denen R³ und R⁴ wie unter 1) definiert sind,
   oder
daß G)
   Verbindungen der Formel I, mit X gleich Sauerstoff und R¹, R², R³, R⁴ und R⁵ wie unter 1) definiert, durch Reaktion einer Verbindung der Formel I, wobei X Sauerstoff ist, für R¹, R², R⁵ sowie für R³ und R⁴ die unter 1) genannten Definitionen gelten, außer daß mindestens einer der Reste R³ oder R⁴ Wasserstoff ist, mit einem Alkylierungsreagenz der Formel XV,

   R'-Z (XV)

   wobei R' die oben angegebenen Bedeutungen für R³ und R⁴ mit Ausnahme von Wasserstoff hat und Z eine Abgangsgruppe ist, hergestellt werden,
   oder
daß H)
   Verbindungen der Formel I, mit X gleich Sauerstoff, R¹, R², R³ und R⁴ wie in Anspruch 1 definiert und R⁵ C1 - C6-Alkyl, gegebenenfalls substituiert mit Fluor, Chlor, Hydroxy, C1 - C4-Acyloxy, Benzoyloxy, Phenoxy, C1 - C4-Alkoxy, C1 - C4Alkylamino, Di(C1 - C4-Alkyl)amino, C1 - C4-Alkylthio, Carboxy, Carbamoyl, C3 - C6-Alkenyl, gegebenenfalls substituiert mit Fluor, Chlor, Hydroxy, C1 - C4-Acyloxy, Benzoyloxy, Phenoxy, C1 - C4-Alkoxy, C1 - C4-Alkylamino, Di(C1 - C4-alkyl)amino, C1 - C4-Alkylthio, Carboxy, Carbamoyl, C3 - C8-Alkinyl, gegebenenfalls substituiert mit Fluor, Chlor, Hydroxy, C1 - C4-Acyloxy, Benzoyloxy, Phenoxy, C1 - C4-Alkoxy, C1 - C4-Alkylamino, Di(C1 - C4-alkyl)amino, C1 - C4-Alkylthio, Carboxy, Carbamoyl, C4 - C8-Cycloalkyl, gegebenenfalls substituiert mit Fluor, Chlor, Hydroxy, C1 - C4-Acyloxy, Benzoyloxy, C1 - C4-Alkoxy, C1 - C4-Alkylamino, Di(C1 - C4-alkyl)-amino, C1 - C4-Alkylthio, Carboxy, Carbamoyl, C5 - C8-Cycloalkenyl, gegebenenfalls substituiert mit Fluor, Chlor, Hydroxy, C1 -C4-Acyloxy, Benzoyloxy, Phenoxy, C1 - C4-Alkoxy, C1 - C4-Alkylamino, Di(C1 - C4-alkyl)amino, C1 - C4-Alkylthio, Carboxy, Carbamoyl, durch reduktive Alkylierung einer Verbindung der Formel I, wobei R⁵ Wasserstoff und X Sauerstoff sind und für R¹, R², R³ und R⁴ die unter 1) genannten Definitionen gelten mit einer Carbonylverbindung der Formel XVI,

   R''-C(=O)-R''' (XVI),

   wobei R" und R"' gleich oder verschieden, unabhängig voneinander Wasserstoff, C1 - C5-Alkyl, gegebenenfalls substituiert mit Fluor, Chlor, Hydroxy, C1 - C4-Acyloxy, Benzoyloxy, Phenoxy, C1 - C4-Alkoxy, C1 - C4-Alkylamino, Di(C1 - C4-alkyl)amino, C1 - C4-Alkylthio, Carboxy, Carbamoyl, C3 - C5-Alkenyl, gegebenenfalls substituiert mit Fluor, Chlor, Hydroxy, C1 - C4-Acyloxy, Benzoyloxy, Phenoxy, C1 - C4-Alkoxy, C1 - C4-Alkylamino, Di(C1 - C4-alkyl)amino, C1 - C4-Alkylthio, Carboxy, Carbamoyl, C3 - C7-Alkinyl, gegebenenfalls substituiert mit Fluor, Chlor, Hydroxy, C1 - C4-Acyloxy, Benzoyloxy, Phenoxy, C1 - C4-Alkoxy, C1 - C4-Alkylamino, Di(C1 - C4-alkyl)amino, C1 - C4-Alkylthio, Carboxy, Carbamoyl, C4 - C8-Cycloalkyl, gegebenenfalls substituiert mit Fluor, Chlor, Hydroxy, C1 - C4-Acyloxy, Benzoyloxy, Phenoxy, C1 - C4-Alkoxy, C1 - C4-Alkylamino, Di(C1 - C4-alkyl)amino, C1 - C4-Alkylthio, Carboxy, Carbamoyl, C6-Cycloalkenyl, gegebenenfalls substituiert mit Fluor, Chlor, Hydroxy, C1 - C4-Acyloxy, Benzoyloxy, Phenoxy, C1 - C4-Alkoxy, C1 - C4-Alkylamino, Di(C1 - C4-alkyl)amino, C1 - C4-Alkylthio, Carboxy, und wobei R" und R"' unter Bildung eines 4- bis 8-gliedrigen Ringes miteinander verknüpft sein können, hergestellt werden
oder I)
   Verbindungen der Formel I, mit X gleich Sauerstoff und R¹, R², R³ und R⁴ wie unter 1) definiert und R⁵ C1 - C6-Alkyloxycarbonyl, C1 - C6-Alkylthiocarbonyl, C2 - C6-Alkenyloxycarbonyl, C2 - C6-Alkenylthiocarbonyl, C2 - -C6-Alkinyloxycarbonyl, C1 - C6-Alkylaminocarbonyl, C3 - C6-Alkenylaminocarbonyl, Di(C1 - C6-alkyl)aminocarbonyl, Pyrrolidin-1-yl, Morpholino-, Piperidino-, Piperazinyl-, 4-Methylpiperazin-1-yl-carbonyl gegebenenfalls substituiert mit Fluor, Chlor, Hydroxy, oder mit bis zu zweivoneinander unabhängigen Resten R⁶ substituiertes Aryloxycarbonyl, Arylthio(carbonyl), Arylaminocarbonyl, (Arylalkylthio)carbonyl, Aryalkylaminocarbonyl, wobei der Alkylrest jeweils 1 bis 3 C-Atome enthalten kann, hergestellt werden, indem man eine Verbindung der Formel XVII, 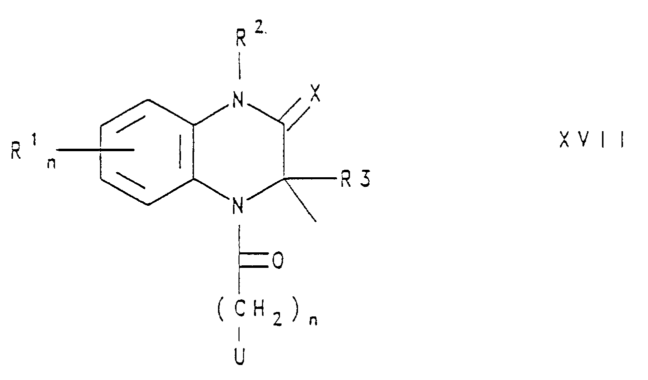 wobei für R¹, R², R³ und R⁴ die unter 1) genannten Definitionen gelten, n gleich 0, 1, 2 oder 3 ist, X gleich Sauerstoff und U eine Abgangsgruppe ist, mit einer Verbindung der Formel XVIII,

   Nu - H (XVIII),

   wobei Nu C1 - C6-Alkyloxy, C2 - C6-Alkenyloxy, C2 - C6-Alkinyloxy, C1 - C6-Alkylthio, C2 - C6-Alkenylthio, C1 - C6-Alkylamino- und Di(C1 - C6-Alkyl)amino, C3 - C6-Alkenylamino- und Di(C1 - C6-alkyl)amino, gegebenenfalls substituiert durch Fluor, Chlor, Hydroxy, C1 - C4-Alkoxy, Pyrrolidin-1-yl, Morpholino-, Piperidino-, Piperazinyl-, oder 4-Methylpiperazin-1-yl-, oder mit bis zu zwei voneinander unabhängigen Resten R⁶ (R⁶ ist wie eingangs definiert) substituiertes Aryloxy, Arylthio, Arylamino, Arylalkyloxy, Arylalkylthio, Aryalkylamino, 2-, 3-, oder 4-Pyridyl-, 2- oder 3-Furyl, 2- oder 3-Thienyl, 2-, 3- oder 4-Picolyloxy, 2- oder 3-Furylmethyloxy, 2- oder 3-Thienylmethyloxy wobei der Alkylrest jeweils 1 bis 3 C-Atome enthalten kann, sein kann, zur Reaktion bringt.

Die obengenannte Methode A läuft vorzugsweise unter folgenden Bedingungen ab:

Der Substituent Z in der Formel III ist eine geeignete Abgangsgruppe, wie z. B. Chlor, Brom oder Jod, ein geeignetes Radikal der Schwefelsäure, ein aliphatischer oder aromatischer Sulfonsäureester oder ggf. halogeniertes Acyloxy.

Die Umsetzung wird zweckmäßigerweise in einem inerten Lösungsmittel durchgeführt. Geeignet sind z. B. aromatische Kohlenwasserstoffe wie Toluol oder Xylol, niedere Akohole wie Methanol, Ethanol oder 1-Butanol, Ether wie Tetrahydrofuran oder Glycoldimethylether, dipolar aprotische Lösungsmittel wie N,N-Dimethylformamid, N-Methyl-2-pyrrolidon, Acetonitril, Nitrobenzol, Dimethylsulfoxid oder Gemische dieser Lösungsmittel. Auch Zweiphasensysteme mit wäßrigen Lösungen von Basen in Gegenwart eines Phasentransferkatalysators, wie z. B. Benzyltriethylammoniumchlorid, sind möglich.
Die Anwesenheit einer geeigneten Base z. B. eines Alkali- oder Erdalkalimetallcarbonats oder -hydrogencarbonats wie Natriumcarbonat, Calciumcarbonat oder Natriumbicarbonat, eines Alkali- oder Erdalkalihydroxids wie Kaliumhydroxid oder Bariumhydroxid, eines Alkoholats wie Natriumethanolat oder Kalium-tert.-butylat, einer lithiumorganischen Verbindung wie Butyllithium oder Lithiumdiisopropylamin, eines Alkali- oder Erdalkalihydrids wie Natriumhydrid oder Calciumhydrid, ein Alkalifluorid wie Kaliumfluorid oder einer organischen Base wie Triethylamin oder Pyridin zum Auffangen der bei der Reaktion freiwerdenden Säure kann nützlich sein.

In manchen Fällen ist der Zusatz eines Jodsalzes, z. B. Kaliumjodid, angebracht. Die Reaktion wird gewöhnlich bei Temperaturen zwischen - 10 und 160 °C durchgeführt, vorzugsweise bei Raumtemperatur.

Für diese Umsetzung müssen etwaige nucleophile Substituenten wie z. B. Hydroxy-, Mercapto- oder Aminogruppen mit Ausnahme der 1- und/oder 4- Position in Verbindungen der Formel II oder III, vor Durchführung der Reaktion in geeigneter Weise derivatisiert oder mit wieder abspaltbaren gebräuchlichen Schutzgruppen wie z. B. Acetyl oder Benzyl versehen werden.

Für die Umsetzung wie zuvor unter B) beschrieben wird vorzugsweise als Schwefelungsreagenz 2,4- Bis(4-methoxyphenyl)-1,3-dithia-2,4-diphosphetan-2,4-disulfid (Lawessons Reagenz), Bis(tricyclohexylzinn)sulfid, Bis(tri-n- butylzinn)sulfid, Bis(triphenylzinn)sulfid, Bis(trimethylsilyl)sulfid oder Phosphorpentasulfid verwendet. Die Reaktion wird zweckmäßigerweise in einem organischen Lösungsmittel oder einem Lösungsmittelgemisch, bei Raumtemperatur oder höher, bevorzugt bei der Siedetemperatur des Reaktionsgemisches und möglichst unter wasserfreien Bedingungen durchgeführt. Geeignet sind z.B. Schwefelkohlenstoff, Toluol, Xylol, Pyridin, 1,2-Dichlorethan. Bei Verwendung der erwähnten Zinn- oder Silylsulfide ist es angebracht, die Schwefelungsreaktion in Gegenwart einer Lewissäure wie Bortrichlorid durchzuführen.

In Gegenwart anderer Carbonylgruppen in einer Verbindung der Formel I, z. B. in einer Verbindung, wo X gleich Sauerstoff und einer oder mehrere Reste R¹ bis R⁶ gleich Acyl sind, ist das Carbonyl vor der Schwefelungsreaktion nach bekannten Methoden durch eine geeignete Schutzgruppe, z. B. durch Acetalisierung, zu schützen; anschließende Schutzgruppenabspaltung führt zur gewünschten Verbindung.

Für die oben unter C beschriebene Umsetzung ist der Substituent Z eine geeignete Abgangsgruppe, vorzugsweise Chlor, Brom oder Jod, ein geeignetes Radikal der Schwefelsäure, ein aliphatischer oder aromatischer Sulfonsäureester oder ggf. halogeniertes Acyloxy.

Die Reaktionsbedingungen für diese Umsetzung entsprechen denjenigen der Methode A.

Die unter D) beschriebene Cyclisierung findet in einem geeigneten Lösungsmittel statt wie Methanol, Ethanol, N,N-Dimethylformamid oder N-Methylpyrrolidon in Gegenwart einer Base; geeignet sind Alkali- oder Erdalkalimetallcarbonate oder - hydrogencarbonate wie Natriumcarbonat, Calciumcarbonat oder Natriumbicarbonat, Alkali- oder Erdalkalihydroxide wie Kaliumhydroxid oder Bariumhydroxid, Alkoholate wie Natriumethanolat oder Kalium- tert.-butylat, lithiumorganische Verbindungen wie Butyllithium oder Lithiumdiisopropylamin, Alkali- oder Erdalkalihydride wie Natriumhydrid oder Calciumhydrid oder eine organische Base wie Triethylamin oder Pyridin - letztere können auch als Lösungsmittel verwendet werden, oder organische oder anorganische Säuren wie Eisessig, Trifluoressigsäure, Salzsäure oder Phosphorsäure. Die Reaktion wird vorzugsweise bei Temperaturen zwischen 20 und 120 °C, besonders bevorzugt bei Raumtemperatur durchgeführt.

Die Verbindungen der allgemeinen Formel V, wobei R¹ bis R⁵ und Y wie unter 1) definiert, können aus Verbindungen der Formel VI, 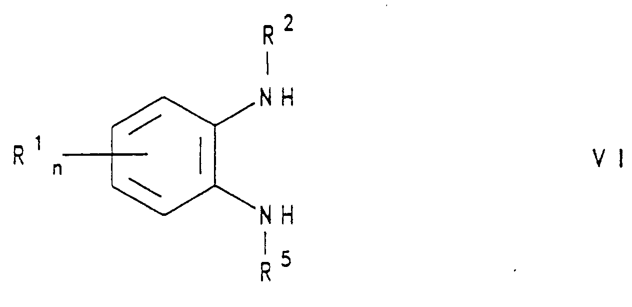 wobei R¹, R² und R⁵ wie unter 1) definiert, durch Alkylierung mit einer Verbindung der Formel VII, 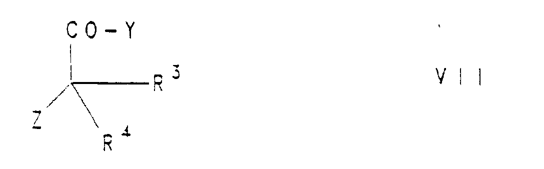 wobei R³, R⁴, Y wie unter 1) und Z wie unter A) definiert, erhalten werden. Die Reaktionsbedingungen für diese Alkylierung entsprechen den zur Methode A gegebenen.
Unter geeigneten Bedingungen findet dabei gleichzeitig der Ringschluß zum Dihydrochinoxalin der allgemeinen Formel I statt.

Verbindungen der allgemeinen Formel V, in denen R¹, R³ bis R⁵ und Y wie unter 1) definiert sind und R² Wasserstoff bedeutet können außerdem auch aus Verbindungen der Formel VIII, 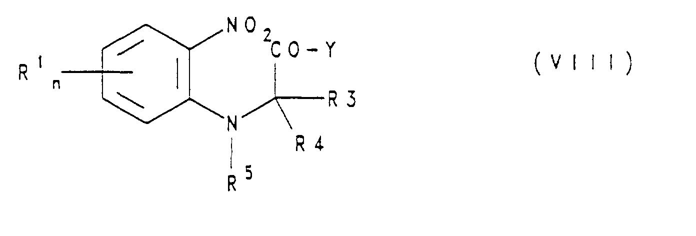 wobei R¹, R³ bis R⁵ und Y wie unter 1) definiert sind, hergestellt werden indem man die Nitrogruppe nach bekannten Verfahren zur Aminogruppe reduziert. Unter geeigneten Bedingungen, z. B. bei Reduktion in Anwesenheit von Säure findet dabei gleichzeitig der Ringschluß zum Dihydrochinoxalin der allgemeinen Formel I statt.
Die Reduktion wird nach Standardmethoden (s. z. B. Methoden der Organischen Chemie (Houben-Weyl), E. Müller (Herausgeber); G. Thieme Verlag, Stuttgart, 1957; Bd. XI/1, S. 360- 490) z. B. mit Zinn(II)chlorid in Eisessig, TiCl₃ in Salzsäure, oder durch katalytische Hydrierung durchgeführt, wobei die Wahl des Reagenzes durch die chemische Stabilität der verschiedenen Substituenten R¹, R³ bis R⁵ bestimmt wird; ist z. B. einer der Reste Alkenyl wird man die erste Methode wählen, um die Doppelbindung zu erhalten.

Die als Ausgangsmaterialien für die beschriebenen Synthesen benötigten Phenylendiamine der allgemeinen Formel VI sind literaturbekannt oder käuflich oder können nach literaturbekannten Methoden synthetisiert werden.

N-Ortho-Nitrophenylaminosäurederivate der generellen Formel VIII, wobei R¹ₙ und R³ bis R⁵ wie unter 1) definiert sind und Y gleich OR⁷ ist, worin R⁷ für Wasserstoff, C1 - C6-Alkyl, ggf. jeweils z. B. Halogen-substituiertes Phenyl, Benzyl oder 9- Fluorenylmethyl steht, bedeutet, können z. B. durch Aminierung von ortho-Halogennitroaromaten der allgemeinen Formel IX, 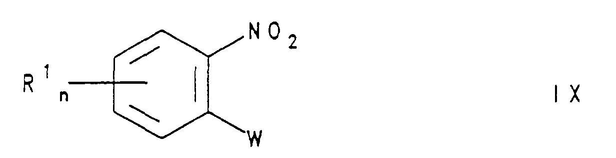 wobei R¹ wie unter 1) definiert ist und W Fluor, Chlor, Brom oder Jod bedeutet, mit Aminosäuren oder ihren Estern der allgemeinen Formel X, 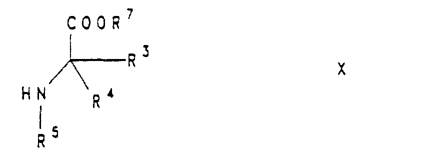 wobei R³, R⁴, R⁵ und R⁷ wie unter 1) definiert sind, erhalten werden.
Die Reaktion kann in Gegenwart einer anorganischen oder organischen Hilfsbase, wie z. B. Natrium- oder Kaliumcarbonat, Natriumhydroxid oder Triethylamin durchgeführt werden. Günstig ist die Verwendung eines inerten Lösungsmittels bei Temperaturen zwischen 0 und 150 °C, vorzugsweise bei Rückflußtemperatur. Geeignete Lösungsmittel sind offenkettige oder cyclische Ether, z. B. Tetrahydrofuran oder Glycoldimethylether, aromatische Kohlenwasserstoffe, z. B. Toluol oder Chlorbenzol, Alkohole, z. B. Ethanol, Isopropanol oder Glycolmonomethylether, dipolar aprotische Lösungsmittel, z. B. N,N-Dimethylformamid, N-Methyl-2-pyrrolidon oder 1,3- Dimethyl-tetrahydro-2(1H)-pyrimidinon.

Die N-ortho-Nitrophenylaminosäuren der Formel VIII mit Y gleich Hydroxy lassen sich falls gewünscht oder erforderlich nach wohlbekannten Standardmethoden in die Säurederivate der Formel VIII mit Y gleich Hydroxy, C1 - C4-Alkoxy, ggf. halogeniertes C1 - C4-Acyloxy, Chlor, Brom oder Jod umwandeln.

Ortho-Halogennitroaromaten der allgemeinen Formel IX und Aminosäuren der allgemeinen Formel X sind literaturbekannt und käuflich oder lassen sich nach literaturbekannten Methoden herstellen.

Die oben unter E) beschriebene Umsetzung erfolgt vorzugsweise durch katalytische Hydrierung (mit Wasserstoff) oder Hydrosilylierung (mit Alkylsilanen, z. B. Diphenylsilan) in Gegenwart eines Hydrierkatalysators, z. B. Raney-Nickel oder Palladium auf Kohle, bei einem Wasserstoffdruck von 1 bis 5 bar oder mittels eines Reduktionsmittels aus der Klasse der komplexen Metallhydride wie Natriumborhydrid oder Natriumcyanoborhydrid oder mit Metallen bzw. Metallsalzen und Säure wie z. B. Zink/Eisessig oder SnCl₂/HCl. Zweckmäßigerweise wird die Reaktion in einem inerten Lösungsmittel wie niederen Alkoholen, z. B. Methanol oder Isopropanol, Ethern wie Tetrahydrofuran oder Glycoldimethylether, dipolar aprotische Lösungsmitteln wie N,N- Dimethylformamid, aromatischen Kohlenwasserstoffen wie Toluol oder Xylol oder Gemischen dieser Lösungsmittel bei Temperaturen zwischen -20 und 100 °C, vorzugsweise bei Raumtemperatur durchgeführt.

Wird bei der beschriebenen Umsetzung ein chiraler Hydrierkatalysator, z. B. Di-*µ*-chloro-bis[(cycloocta-1c,5c- dien)-rhodium(I)]/ (+) oder (-)-4,5-Bis(diphenylphosphino- methyl)-2,2-dimethyl-1,3-dioxolan, oder ein chirales komplexes Metallhydrid, z. B. Natrium-tris-(N-benzyloxycarbonyl-L- prolinoyloxy)-borhydrid, verwendet, so lassen sich selektiv die einzelnen Enantiomeren erzeugen.

In Gegenwart von Substituenten in Verbindungen der Formel Xl, welche unter den beschriebenen Bedingungen hydriert bzw. reduziert werden können, z. B. Oxo, ist die Verwendung eines Intermediates der Formel XI, mit Substituenten, die nicht angegriffen werden, welche aber zu der benötigten Gruppe derivatisiert werden können, z. B. Hydroxy, nötig. Die Substituenten können auch mit einer gebräuchlichen Schutzgruppe, z. B. einer Acetalschutzgruppe, versehen sein, die nach der oben beschriebenen Umsetzung wieder entfernt werden kann.

Chinoxalinone der allgemeinen Formel XI mit R¹ bis R³ wie unter definiert können nach bekannten Verfahren durch Kondensation eines Phenylendiamins der Formel VI, wobei R¹ und R² wie unter 1) definiert sind und R⁵ gleich Wasserstoff ist, mit einer alpha-Ketocarbonsäure der allgemeinen Formel XII,

R³-CO-COOH (XII)

wobei R³ wie unter 1) definiert ist, erhalten werden.
Zweckmäßigerweise wird die Reaktion in einem inerten Lösungsmittel in einem Temperaturbereich zwischen 0 und 150 °C durchgeführt; geeignete Lösungsmittel sind z. B. Alkohole, z. B. Ethanol oder Isopropanol, offenkettige oder cyclische Ether, z. B. Glycoldimethylether oder Tetrahydrofuran oder dipolar aprotische Lösungsmittel, z. B. N,N-Dimethylformamid oder Acetonitril.

Die oben unter F) beschriebene Umsetzung wird zweckmäßigerweise in einem Zweiphasensystem, bestehend aus einem organischen, nicht wassermischbaren Lösungsmittel oder Lösungsmittelgemisch, bestehend aus z. B. halogenierten Kohlenwasserstoffen, z. B. Dichlormethan oder 1,2-Dichlorethan oder aromatischen Kohlenwasserstoffen, z. B. Toluol oder Xylol und einer konzentrierten wäßrigen Lösung eines Alkali- oder Erdalkalimetallhydroxids, z. B. Natrium- oder Bariumhydroxid durchgeführt. Vorteilhaft ist die Anwesenheit eines Phasentransferkatalysators, wie z. B. Benzyltriethylammoniumchlorid oder Tetrabutylammoniumbromid.
Die Reaktion wird gewöhnlich bei Temperaturen zwischen 0 und 50 °C durchgeführt, vorzugsweise bei Raumtemperatur.

Substituenten in Verbindungen der Formeln VI und XIII, bzw. XIV die unter den Reaktionsbedingungen nicht stabil sind, müssen durch solche ersetzt werden, die zu der benötigten Gruppe derivatisiert werden können. Die Substituenten können auch mit einer gebräuchlichen Schutzgruppe versehen sein, die nach der oben beschriebenen Umsetzung wieder entfernt werden kann.

Bei der oben unter G) beschriebenen Reaktion ist Z in Formel XV eine geeignete Abgangsgruppe, wie z. B. Chlor, Brom oder Jod, ein geeignetes Radikal der Schwefelsäure, ein aliphatischer oder aromatischer Sulfonsäureester oder ggf. halogeniertes Acyloxy.

Die Reaktionsbedingungen für diese Umsetzung entsprechen denjenigen der Methode A.

Die unter H) beschriebene Reaktion findet vorzugsweise durch katalytische Hydrierung (mit Wasserstoff) in Gegenwart eines Hydrierkatalysators, z.B. Palladium auf Kohle, bei einem Wasserstoffdruck von 1 bis 5 bar, oder mittels eines Reduktionsmittels aus der Klasse der komplexen Metallhydride, wie Natriumborhydrid, Natriumtriacetoxyborhydrid oder Natriumcyanoborhydrid statt.

Zweckmäßigerweise wird die Umsetzung in einem inerten Lösungsmittel, wie niederen Alkoholen, z.B. Methanol oder Isopropanol, Ethern, z.B. Tetrahydrofuran oder Glycoldimethylether, halogenierten Kohlenwasserstoffen, z.B. Dichlormethan oder Dichlorethan, bei Temperaturen zwischen -20 und 100 °C, vorzugsweise bei Raumtemperatur durchgeführt. Die Anwesenheit einer Säure, wie z.B. Essigsäure oder Trifluoressigsäure, oder einer Lewissäure, wie z.B. Titantetrachlorid, ist vorteilhaft. In Gegenwart von Substituenten in Verbindungen der Formeln I und XVI, welche unter den beschriebenen Bedingungen hydriert bzw. reduziert werden können, z.B. Oxo, ist die Verwendung eines Intermediates der Formeln I und XVI, mit Substituenten, die nicht angegriffen werden, welche aber zu der benötigten Gruppe derivatisiert werden könne, z.B. Hydroxy, nötig. Säurelabile Gruppen, wie z.B. Acetale oder unter den Reaktionsbedingungen reagierende Gruppen, wie z.B. primäre Amine, sind ebenfalls zu vermeiden bzw. mit einer gebräuchlichen Schutzgruppe zu versehen.

Die unter I) beschriebene Umsetzung wird zweckmäßigerweise in einem inerten Lösungsmittel durchgeführt. Geeignet sind z.B. aromatische Kohlenwasserstoffe wie Toluol oder Xylol, niedere Alkohole wie Methanol, Ethanol oder 1-Butanol, Ether wie Tetrahydrofuran oder Glycoldimethylether, dipolar aprotische Lösungsmittel wie N,N-Dimethylformamid, N-Methyl-2-pyrrolidon, Acetonitril, Nitrobenzol, Dimethylsulfoxid oder Gemische dieser Lösungsmittel. Auch Zweiphasensysteme mit wäßrigen Lösungen von Basen in Gegenwart eines Phasentransferkatalysators, wie z.B. Benzyltriethylammoniumchlorid, sind möglich.
Die Anwesenheit einer geeigneten Base z.B. eines Alkali- oder Erdalkalihydroxids wie Kaliumhydroxid oder Bariumhydroxid, eines Alkoholats wie Natriumethanolat oder Kalium-tert.-butylat, einer lithiumorganischen Verbindung wie Butyllithium oder Lithiumdiisopropylamid, eines Alkali- oder Erdalkalihydrids wie Natriumhydrid oder Calciumhydrid, ein Alkalifluorid wie Kaliumfluorid oder einer organischen Base wie Triethylamin oder Pyridin kann nützlich sein. Die Reaktion wird gewöhnlich bei Temperaturen zwischen -10 und 160 °C durchgeführt, vorzugsweise bei Raumtemperatur.

Für diese Umsetzung sind etwaige nucleophile Substituenten wie z.B. Hydroxy-, Mercapto- oder Aminogruppen in den Verbindungen XVII und XVIII, die nicht an der Reaktion beteiligt sind, in geeigneter Weise zu derivatisieren oder mit wieder abspaltbaren gebräuchlichen Schutzgruppen wie z.B. Acetyl oder Benzyl zu versehen.

Die für die genannte Reaktion benötigten Verbindungen XVII, wobei für R¹, R², R³ und R⁴ die unter 1) beschriebenen Definitionen gelten, n gleich 0, 1, 2 oder 3 ist, X gleich Sauerstoff und U eine geeignete Abgangsgruppe, Halogen, wie z.B. Chlor, Brom, Jod, ein halogeniertes aliphatischen oder aromatisches Alkoholat wie z.B. 2,2,2-Trichlorethoxy, Chlorphenoxy oder ein über Stickstoff verknüpfter Heterocyclus wie z.B. Imidazolyl, Triazolyl, Benztriazolyl ist, werden hergestellt, indem man eine Verbindung der Formel I, wobei R⁵ Wasserstoff und X Sauerstoff sind und für R¹, R², R³ und R⁴ die unter 1) beschriebenen Definitionen gelten mit einem geeigneten Kohlensäurederivat, z.B. Phosgen, Diphosgen, Triphosgen, Chlorameisensäuretrichlorethylester oder Carbonyldiimidazol beziehungsweise mit einem geeigneten Halogencarbonsäurehalogenid, z.B. Bromacetylchlorid umsetzt.

Die Umsetzung wird zweckmäßigerweise in einem inerten Lösungsmittel durchgeführt. Geeignet sind z.B. aromatische Kohlenwasserstoffe wie Toluol oder Xylol, Ether wie Tetrahydrofuran oder Glycoldimethylether oder halogenierte Kohlenwasserstoffe wie Dichlormethan oder Dichlorethan.

Die Anwesenheit einer geeigneten Base z.B. eines Alkali- oder Erdalkalihydroxids wie Kaliumhydroxid oder Bariumhydroxid oder einer ogranischen Base wie Triethylamin oder Pyridin kann nützlich sein.

Die Reaktion wird gewöhnlich bei Temperaturen zwischen -30 und 160 °C durchgeführt, vorzugsweise bei Raumtemperatur.

Weiterhin gehören zum Gegenstand der vorliegenden Erfindung die Verbindungen wie unter 1) bis 3) beschrieben als Arzneimittel, vorzugsweise zur Behandlung von Viruserkrankungen, insbesondere von Erkrankungen hervorgerufen durch das HIV.

Des weiteren betrifft die vorliegende Erfindung Arzneimittel mit einem Gehalt an mindestens einer erfindungsgemäßen Verbindung, sowie die Verwendung der genannten Verbindungen zur Herstellung von Arzneimitteln, vorzugsweise zur Behandlung von Viruserkrankungen, insbesondere zur Behandlung von Krankheiten, hervorgerufen durch das HIV.

Weiterhin gehört zum Gegenstand der vorliegenden Erfindung die Verwendung von Verbindungen der obengenannten Formel I bzw. la zur Herstellung von Arzneimitteln zur Behandlung von Viruserkrankungen.

Für diese Verwendung sind die oben unter 1)- 2) genannten und erläuterten Verbindungen bevorzugt.

Die erfindungsgemäßen Arzneimittel können enteral (oral), parenteral (intravenös), rektal, subcutan, intramuskulär oder lokal (topisch) angewendet werden.

Sie können in Form von Lösungen, Pulvern (Tabletten, Kapseln einschließlich Microkapseln), Salben (Cremes oder Gele) oder Suppositorien verabreicht werden. Als Hilfsstoffe für derartige Formulierungen kommen die pharmazeutisch üblichen flüssigen oder festen Füllstoffe und Streckmittel, Lösemittel, Emulgatoren, Gleitstoffe, Geschmackskorrigentien, Farbstoffe und/oder Puffersubstanzen in Frage.

Als zweckmäßige Dosierung werden 0.1 - 10, vorzugsweise 0.2 - 8 mg/kg Körpergewicht ein oder mehrmals täglich verabreicht. Die verwendeten Dosierungseinheiten richten sich zweckmäßigerweise nach der jeweiligen Pharmakokinetik der verwendeten Substanz bzw. der verwendeten galenischen Zubereitung.

Die verwendete Dosierungseinheit der erfindungsgemäßen Verbindungen beträgt z. B. 1 - 1500 mg, vorzugsweise 50 - 500 mg.

Die erfindungsgemäßen Verbindungen können auch in Kombination mit anderen antiviralen Mitteln, wie z. B. Nucleosidanaloga, Proteaseinhibitoren oder Adsorptionsinhibitoren, Immunstimulantien, Interferonen, Interleukinen und Koloniestimulierenden Faktoren (z.B. GM-CSF, G-CSF, M-CSF), verabreicht werden.

### Wirksamkeitstests

### Prüfung von Präparaten gegen HIV in der Zellkultur Methodenbeschreibung

### Medium:

### RMPI pH 6.8

Komplettes Medium enthält zusätzlich 20 % foetales Kälberserum und 40 IU/ml rekombinantes Interleukin 2.

### Zellen:

Aus frischem Spenderblut mittels Ficoll^{R}-Gradienten- Zentrifugation isolierte Lymphozyten werden unter Zusatz von 2 *µ*g/ml Phytohämagglutinin (Wellcome) in komplettem Medium 36 h bei 37 °C unter 5 % CO₂ kultiviert. Die Zellen werden nach Zusatz von 10 % DMSO bei einer Zelldichte von 5 x 10⁶ eingefroren und in flüssigem Stickstoff gelagert. Für den Versuch werden die Zellen aufgetaut, im RPMI-Medium gewaschen und im kompletten Medium 3 - 4 Tage kultiviert.

### Ansatz:

Die Prüfpräparate wurden in einer Konzentration von 16.7 mg/ml in DMSO gelöst und in komplettem Medium auf 1 mg/ml verdünnt. In 24er Multiwell-Schalen wurden 0.4 ml Medium vorgelegt. Nach Zugabe von 0.1 ml des gelösten Präparates in die obere Reihe der Schale wurde durch Übertragung von jeweils 0.1 ml eine geometrische Verdünnungsreihe erzeugt. Präparatfreie Kontrollen enthielten stets 0.4 ml komplettes Medium mit 0.5% DMSO. Lymphozytenkulturen mit einer Zellzahl von 5 x 10⁵ Zellen/ml wurden durch Zugabe 1/50 Volumen Überstand aus HIV-infizierten Lymphozytenkulturen infiziert. Der Titer dieser Kulturüberstände wurde durch Endpunktverdünnung mit 1 - 5 x 10⁶ infektiöse Einheiten/ml bestimmt. Nach 30 min. Inkubation bei 37 °C wurden die infizierten Lymphozyten abzentrifugiert und im gleichen Volumen Medium wieder aufgenommen. Von dieser Zellsuspension wurden jeweils 0.6 ml in alle Vertiefungen der Testplatte gegeben. Die Ansätze wurden 3 Tage bei 37 °C inkubiert.

### Auswertung:

Die infizierten Zellkulturen wurden unter dem Mikroskop auf Anwesenheit von Riesenzellen untersucht, die eine aktive Virusvermehrung in der Kultur anzeigen. Die geringste Präparatekonzentration, bei der keine Riesenzellen auftraten, wurde als Hemmkonzentration gegen HIV bestimmt. Zur Kontrolle wurden die Überstände aus den Kulturplatten mit Hilfe eines HIV- Antigentests entsprechend den Angaben des Herstellers (Organon) auf Anwesenheit von HIV-Antigen bestimmt.

### Ergebnisse:

Die Ergebnisse dieses Tests zeigt Tabelle 1.

| Verbindung von Beispiel Nr. | T-Zellkulturassay MHK (*µ*g/ml) |
|---|---|
| III | 0,8 |
| IV | > 0,8 |
| VI-A | 0,16 |
| VI-B | 20 |
| VI-C | < 0,8 |
| VII | < 0,16 |
| X | 0,8 |
| XII | < 0,8 |
| XIII | < 0,16 |
| XIV | < 0,16 |
| 3-7 | 0,08 |
| 3-21 | 0,16 |
| 3-23 | 0,08 |
| 3-24 | 0,08 |
| 3-25 | 0,4 |
| 3-26 | 0,4 |
| 3-29 | < 0,4 |
| 3-30 | < 0,01 |
| 3-32 | < 0,4 |
| 3-33 | 0,4 |
| 3-36 | < 2,0 |
| 3-44 | < 0,8 |
| 3-48 | < 0,8 |
| 3-49 | < 0,8 |
| 3-52 | > 0,8 |
| 3-53 | > 0,8 |
| 3-57 | < 0,8 |
| 3-62 | < 4,0 |
| 3-64 | > 0,8 |
| 3-66 | > 0,08 |
| 3-67 | < 0,8 |
| 3-73 | > 0,4 |
| 3-75 | < 0,8 |
| 3-76 | < 0,08 |
| 3-80 | 0,4 |
| 3-81 | 0,08 |
| 3-87 | > 0,8 |
| 3-88 | 0,8 |
| XX | < 4,0 |
| 6-1 | 0,4 |
| 6-16 | < 0,8 |
| 6-17 | < 0,8 |
| 6-19 | < 0,8 |
| 6-20 | < 0,8 |
| 6-22 | > 0,8 |
| 6-27 | < 0,4 |
| 6-32 | < 0,08 |
| 6-33 | > 0,8 |
| 6-34 | < 0,4 |
| 6-35 | < 0,08 |
| 6-36 | < 0,8 |
| 6-39 | 0,4 |
| 6-41 | < 20 |
| 6-50 | < 0,01 |
| XXIII | < 0,01 |
| 7-1 | < 0,16 |
| 7-2 | < 0,01 |
| 7-3 | < 0,01 |
| 7-7 | 0,04 |
| 7-10 | < 0,04 |
| 7-11 | < 0,01 |
| 7-12 | < 0,8 |
| 7-13 | < 0,08 |
| 7-14 | < 0,08 |
| 7-16 | 0,4 |
| 7-21 | < 0,01 |
| 7-22 | < 0,01 |
| 7-23 | < 0,01 |
| 10-4 | 0,4 |
| 10-5 | < 0,8 |
| 10-9 | < 0,8 |
| 10-10 | 0,08 |
| 10-13 | 0,08 |
| 10-14 | < 0,8 |
| 10-17 | 0,8 |
| 10-18 | < 0,8 |
| 10-20 | < 0,8 |
| 10-21 | < 0,8 |
| 10-27 | 0,8 |
| 10-28 | < 0,8 |
| 11-1 | < 0,8 |
| 11-2 | > 0,8 |
| 11-3 | < 0,8 |
| 11-4 | 0,8 |
| 11-11 | 0,01 |

### Untersuchung der Substanzen auf Hemmung der HIV-"Reverse Transkriptase"

Die Aktivität der Reversen Transkriptase (RT) wurde mit Hilfe eines "Scintillation Proximity Assay" (SPA) bestimmt.
Das Reagenzkit für den RT-SPA wurde von Amersham/Buchler (Braunschweig) bezogen. Das Enzym RT (aus HIV in E. coli cloniert) stammte von der Firma HT-Biotechnology Ltd, Cambridge, UK.

### Ansatz

Der Test wurden nach dem Methoden-Manual des Herstellers Amersham durchgeführt - mit folgenden Modifikationen:
- dem "Assay"-Puffer wurde Rinderserumalbumin zu der Endkonzentration 0,5 mg/ml zugesetzt.
- der Test wurde in Eppendorf-Reaktionsgefäßen mit 100 *µ*l Ansatzvolumen durchgeführt.
- das RT-Konzentrat des Herstellers (5000 U/ml) wurde in Tris-HCl Puffer 20 mM; pH 7,2; 30 % Glycerin auf eine Aktivität von 15 U pro ml verdünnt.
- die Inkubationszeit für die Ansätze betrug 60 min (37 °C).
- nach Abstoppen der Reaktion und "Entwicklung" mit der Perlen-Suspension wurden 130 *µ* l Ansatz in 4,5 ml Tris-HCl Puffer, 10 mM; pH 7,4; 0,15 M, NaCl transferiert und die Tritium-Aktivität in einem β-Counter gemessen.

### Substanzprüfung

Für eine Vorprüfung der Inhibitoraktivität wurden die Substanzen in DMSO gelöst (Stammlösung c = 1 mg/ml) und in Verdünnung in DMSO 10⁻¹, 10⁻², 10⁻³ usw. getestet.

Zur Bestimmung von IC₅₀-Werten wurden die Inhibitor-Stammlösungen in Tris-HCl Puffer, 50 mM, pH 8 weiterverdünnt und in geeigneten Konzentrationen getestet.

Aus der graphischen Darstellung RT-Aktivität versus log C_{Inh.} wurde die einer 50 %igen Enzymhemmung zugehörige Konzentration ermittelt.

Die Ergebnisse der Untersuchung zeigt Tabelle 1a.

**Tabelle 1a**

| Verbindung von Beispiel Nr. | Reverse Transkriptase Assay IC₅₀ (*µ* g/ml) |
|---|---|
| V | 7,5 |
| VI-A | 0,08 |
| VI-C | 0,8 |
| VII | 0,1 |
| XIII | 0,04 |
| XIV | 0,16 |
| 3-23 | 0,1 - 1 |
| 3-24 | 0,1 - 1 |
| 3-25 | 0,1 - 1 |
| 3-29 | 0,1 - 1 |
| 3-30 | 0,025 |
| 3-32 | ca 0,1 |
| 3-36 | 0,1 - 1 |
| 3-49 | ca 1 |
| 3-57 | ca 1 |
| 3-75 | 0,1 - 1 |
| 3-76 | 0,018 |
| 3-81 | ca 1 |
| 6-1 | ca 1 |
| 6-8 | 0,1 - 1 |
| 6-9 | ca 1 |
| 6-16 | ca 1 |
| 6-17 | 0,1 - 1 |
| 6-27 | ca 1 |
| 6-35 | 0,1 - 1 |
| 6-50 | 0,01-0,1 |
| XXIII | 0,025 |
| 7-1 | 0,08 |
| 7-2 | 0,07 |
| 7-3 | 0,07 |
| 7-7 | 0,1 |
| 7-10 | 0,11 |
| 7-11 | 0,01 |
| 7-12 | ca 1 |
| 7-13 | 0,1 - 1 |
| 7-16 | ca 1 |
| 10-9 | ca 1 |
| 10-10 | ca 1 |
| 10-13 | ca 1 |
| 10-17 | ca 1 |
| 10-18 | 0,1 - 1 |
| 10-20 | 0,1-1 |
| 10-21 | 0,1 - 1 |
| 10-27 | 0,1 - 1 |
| 10-28 | 0,1 - 1 |
| 11-11 | 0,1 - 1 |
| 10-34 | 0,1 - 1 |
| 11-6 | 0,1 - 1 |
| 11-5 | 0,1 - 1 |
| 11-7 | ca 1 |
| 11-13 | ca 1 |
| 7-20 | 0,1 - 1 |
| 7-14 | 0,01 - 0,1 |
| 7-15 | 0,01 - 0,1 |
| 7-17 | 0,01 - 0,1 |
| 7-18 | 0,01 - 0,1 |
| 7-19 | 0,01 - 0,1 |
| 7-21 | 0,01 - 0,1 |
| 7-22 | 0,01 - 0,1 |
| 7-23 | 0,01 - 0,1 |
| 3-34 | 0,1 - 1 |
| 3-35 | 0,1 - 1 |
| 3-37 | 0,1 - 1 |
| 3-7 | 0,08 |
| 3-127 | 0,01 - 0,1 |
| 3-128 | 0,01 - 0,1 |
| 3-129 | 0,01 - 0,1 |
| 7-24 | < 0,01 |
| 7-25 | < 0,01 |
| 7-26 | 0,01 - 0,1 |
| 7-27 | 0,1 - 1 |
| 7-28 | < 0,01 |
| 7-29 | 0,01 - 0,1 |
| 7-30 | < 0,01 |
| 7-31 | < 0,01 |
| IC₅₀ = 0.08 *µ*g/ ml | |

Durch die nachfolgenden Beispiele sowie durch den Inhalt der Patentansprüche wird die vorliegende Erfindung näher erläutert.

### Beispiel I

### (3S)-6-Chlor-3-methyl-3,4-dihydro-chinoxalin-2(1H)-on

### A) (S)-N-(3-Chlor-6-nitrophenyl)-alanin

2,4-Dichlornitrobenzol (21.0 g, 0.109 mol) und 23.0 g (0.258 mol) L-Alanin wurden in 400 ml 2-Methoxyethanol unter Zusatz von 120 ml 2N Natronlauge für 48 h unter Rückfluß erhitzt. Anschließend wurde im Vakuum eingeengt und der Rückstand in wäßriger Natriumhydrogencarbonatlösung aufgenommen. Es wurde dreimal mit Essigsäureethylester extrahiert, dann mit 6N Salzsäure angesäuert und das gelbe Produkt mit Essigsäureethylester extrahiert. Die organische Phase wurde einmal mit gesättigter wäßriger Natriumchloridlösung gewaschen, getrocknet (Magnesiumsulfat) und das Lösungsmittel unter vermindertem Druck entfernt. Zurück blieben 14.7 g (55 %) gelber Feststoff vom Schmelzpunkt 167 - 169 °C (nach Kristallisation aus Essigsäureethylester):
¹H-NMR (270 MHz, d₆-DMSO): δ = 1.47 (d, J = 7 Hz, 3 H), 4.57 (quintett, J = 7 Hz, 1 H), 6.77 (dd, J = 9, 2 Hz, 1 H), 7.11 (d, J = 2 Hz, 1 H), 8.12 (d, J = 9 Hz, 2 H), 8.41 (br. d, J = 7 Hz, 1 H), 13.2 ppm (br., 1 H).
MS: (M + H)⁺ = 245

### B) (3S)-6-Chlor-3-methyl-3,4-dihydro-chinoxalin-2(1H)-on

Das Produkt des Beispiels IA (14.0 g, 0.057 mol) wurde in 400 ml Methanol gelöst und unter Raney-Nickel-Katalyse mit 1 atm Wasserstoff bei Raumtemperatur hydriert. Nach Aufnahme der berechneten Menge Wasserstoff wurde der Katalysator abgesaugt und die Reaktionslösung im Vakuum eingeengt. Der Rückstand wurde durch Chromatographie an Kieselgel mit Essigsäureethylester / Heptan = 1 : 2 und 1 : 1 als Elutionsmittel gereinigt. Die Ausbeute betrug 6.0 g (53 %) eines bräunlichen Feststoffs mit Schmelzpunkt 122 - 123 °C (nach Umkristallisation aus Isopropanol / Heptan).
¹H-NMR (60 MHz, d₆-DMSO): δ = 1.23 (d, J = 11 Hz, 3 H), 3.81 (dq, J = 11, 4 Hz, 1 H), 6.27 (br., 1 H), 6.3 - 6.9 (m, 3 H), 10.3 ppm (br., 1 H).
MS: (M + H)⁺ = 197
[α]_{D}²³ = + 77.3° (c = 1, MeOH)

### C) (3R)-6-Chlor-3-methyl-3,4-dihydro-chinoxalin-2(1H)-on

Die Verbindung wurde nach den unter Beispiel IA und IB beschriebenen Methoden ausgehend von D-Alanin hergestellt. Schmp. 123 - 124 °C (nach Umkristallisation aus Isopropanol / Heptan)
Die NMR-Daten stimmten mit denen der in Beispiel IB beschriebenen Verbindung überein.
[α]_{D}²³ = - 81.0° (c = 1, MeOH)

### D) (3RS)-6-Chlor-3-methyl-3,4-dihydro-chinoxalin-2(1H)-on

Die Verbindung wurde nach den in Beispiel IA und IB beschriebenen Methoden ausgehend von D,L-Alanin hergestellt. Schmp. 110 °C (nach Umkristallisation aus Isopropanol / Heptan)
Die NMR-Daten stimmten mit denen der in Beispiel IB beschriebenen Verbindung überein.

Die folgenden Verbindungen der Formel I wurden unter Verwendung der entsprechenden Halogenaromaten und Aminosäurederivate in analoger Weise synthetisiert:

### Beispiel II

### (3S)-3-Benzyl-7-chlor-3,4-dihydrochinoxalin-2(1H)-on

### A) (S)-N-(4-Chlor-2-nitrophenyl)-phenylalanin

L-Phenylalanin (8,3 g, 0,05 mol) und 4,8 g (0,025 mol) 2,5-Dichlornitrobenzol wurden in 40 ml wasserfreiem Dimethylsulfoxid (DMSO) gelöst und unter Rühren in einer Argonatmosphäre auf 80 °C erwärmt. Kalium-tert.-butylat (4,2 g, 0,025 mol), gelöst in 30 ml DMSO wurden innerhalb von 40 min zugetropft. Man rührte 3 h bei 80 bis 90 °C weiter, ließ abkühlen, saugte unumgesetztes Phenylalanin ab und wusch mit Wasser nach. Die gesammelten alkalischen Filtrate wurden zweimal mit Diethylether extrahiert, zur Entfernung von nicht umgesetzten Dichlornitrobenzol. Anschließend wurde mit Eisessig angesäuert, mehrmals mit Essigsäureethylester extrahiert, über Magnesiumsulfat getrocknet und eingedampft.

Das Produkt fiel als rotes Öl (6,7 g, 84 %) an, das ohne Reinigung weiter umgesetzt wurde.

### B) (3S)-3-Benzyl-7-chlor-3,4-dihydrochinoxalin-2(1H)-on

Das Produkt aus Beispiel IIA (12 g) wurde in 300 ml wasserfreiem Methanol gelöst und unter Palladium/Kohle-Katalyse mit 1 atm Wasserstoff bei Raumtemperatur hydriert. Nach beendeter Reaktion wurde abgesaugt, eingeengt und an Kieselgel mit Diisopropylether als Elutionsmittel chromatographiert. Dabei erhielt man 1,32 g des gewünschten Produkts, das aus Isopropanol kristallisierte, Schmp. 185 °.
¹H-NMR (270 MHz, d₆-DMSO): δ = 2.9 (m, 2 H), 4.08 (m, 1 H), 6.09 (d, 1 H), 6.7 (m, 2 H), 6.78 (m, 1 H), 7.2 (m, 5 H), 10.34 ppm (br. s, 1 H).
MS: (M + H)⁺ = 273, (M - 92)⁺ = 181.

Die Verbindungen der Tabelle 2 wurden wie in den vorausgehenden Beispielen beschrieben hergestellt.

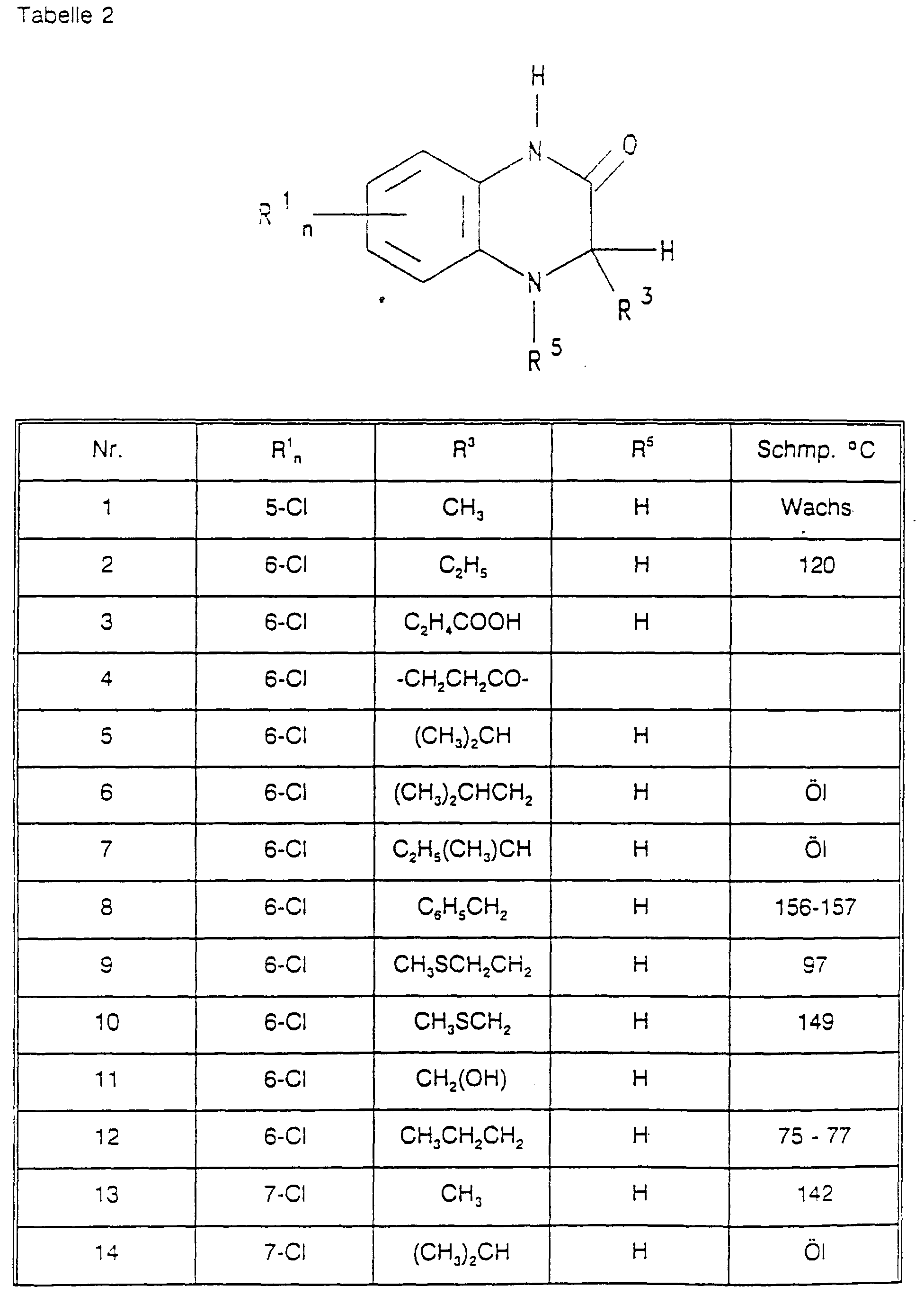

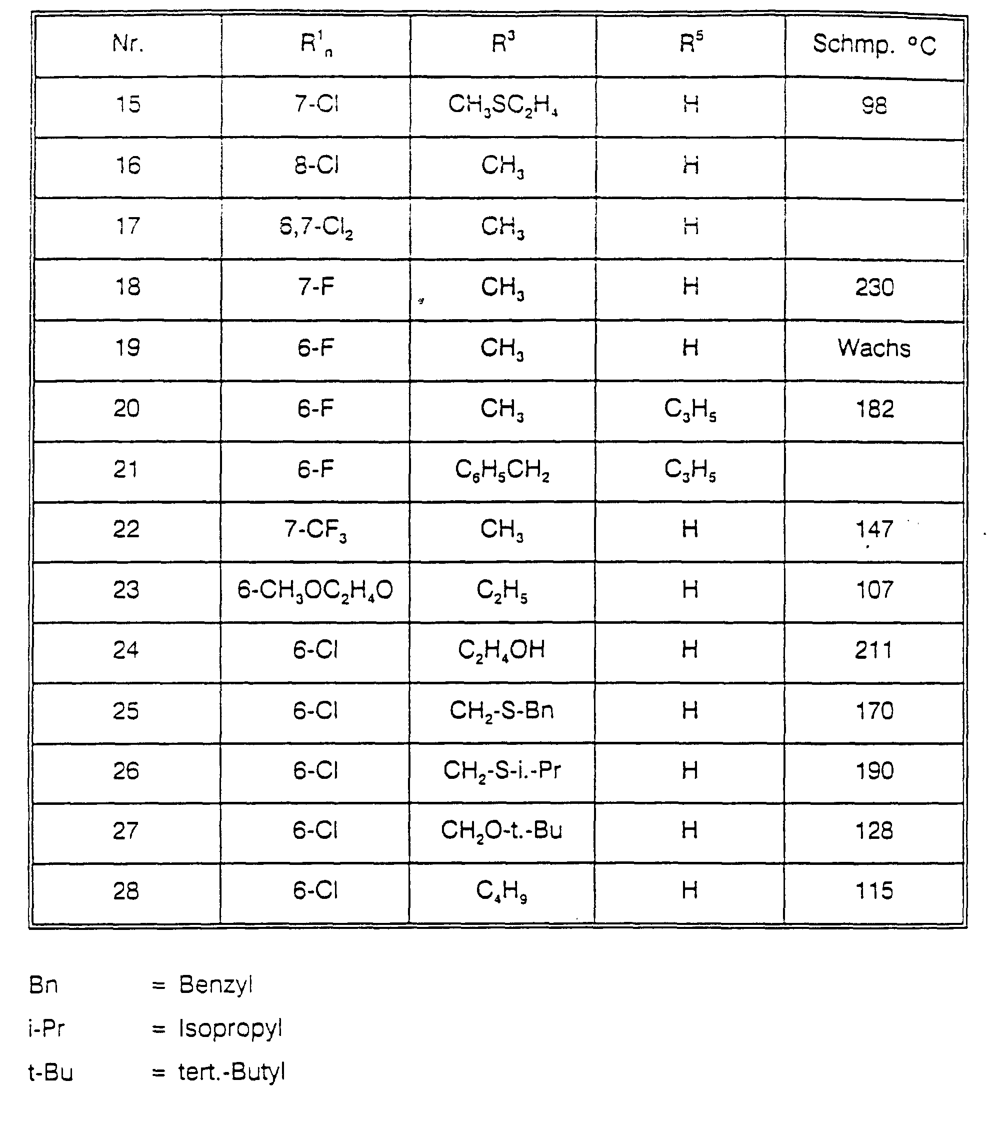

### Beispiel III

### (3S)-4-N-(Benzyloxycarbonyl)-6-chlor-3-methyl-3,4-dihydro-chinoxalin-2(1H)-on

Die Verbindung des Beispiels IB (1.0 g, 5.1 mmol) wurde in 20 ml Dichlormethan gelöst. Es wurden 10 ml 2N wäßrige Natriumhydrogencarbonatlösung zugegeben und unter Eiskühlung und kräftigem Rühren 0.9 ml (90 %; 5.7 mmol) Chlorameisensäurebenzylester zugetropft. Das Zweiphasensystem wurde anschließend bei Raumtemperatur für 60 h gerührt. Nach 30 h wurden nochmals 0.2 ml (1.3 mmol) Chlorameisensäurebenzylester zugegeben. Nach vollständiger Umsetzung wurden die Phasen getrennt, die organische Phase einmal mit Wasser gewaschen, getrocknet (Magnesiumsulfat) und im Vakuum das Lösungsmittel entfernt. Das Produkt wurde durch Chromatographie an Kieselgel mit Methyl-tert.-butylether / Heptan = 1 : 1 als Elutionsmittel gereinigt. Man erhielt 1.65 g (98 %) weißes schaumartiges Produkt.
¹H-NMR (270 MHz, d₆-DMSO): δ = 1.15 (d, J = 7 Hz, 3 H), 4.85 (q, J = 7 Hz, 1 H), 5.20 (d, J = 12 Hz, 1 H), 5.27 (d, J = 12 Hz, 1 H), 6.97 (d, J = 7 Hz, 1 H), 7.19 (dd, J = 8, 2 Hz, 1 H), 7.3 - 7.45 (m, 5 H), 7.67 (d, J = 2 Hz, 1 H), 10.81 ppm (br. s, 1 H).
MS: (M + H)⁺ = 381

### Beispiel IV

### (3S)-4-N-(Benzyloxycarbonyl)-6-chlor-3-methyl-8-nitro-3,4- dihydro-chinoxalin-2(1H)-on

Die Verbindung des Beispiels III (1.5 g, 4.5 mmol) wurde in Eisessig (15 ml) nitriert. Insgesamt 5 ml (124.3 mmol) rauchende Salpetersäure wurden innerhalb von 4 h bei 0 °C bis Raumtemperatur zugetropft. Anschließend wurde auf 100 ml Eiswasser gegossen und das als gelber Feststoff anfallende Produkt abgesaugt, gründlich mit Wasser gewaschen und getrocknet. Schmp. 85 °C (subl.)
¹H-NMR (270 MHz, d₆-DMSO): δ = 1.22 (d, J = 8 Hz, 3 H), 4.89 (q, J = 8 Hz, 1 H), 5.24 (d, J = 12 Hz, 1 H), 5.31 (d, J = 12 Hz, 1 H), 7.35 - 7.5 (m, 5 H), 7.69 (s, 1 H), 8.00 (s, 1 H), 11.11 ppm (br. s, 1 H).
MS: (M + H)⁺ = 376

### Beispiel V

### (3S)-8-Amino-4-N-(benzyloxycarbonyl)-6-chlor-3-methyl-3,4- dihydro-chinoxalin-2(1H)-on

Die Verbindung des Beispiels IV (1.5 g, 4.0 mmol) wurde in 150 ml Methanol gelöst und unter Raney-Nickel-Katalyse mit 1 atm Wasserstoff bei Raumtemperatur hydriert. Nach Aufnahme der berechneten Menge Wasserstoff wurde vom Katalysator abgesaugt und im Vakuum eingeent. Das Produkt wurde durch Chromatographie an Kieselgel mit Essigsäureethylester / Heptan = 2 : 1 als Elutionsmittel gereinigt. Die Ausbeute betrug 0.68 g (49 %) bräunlicher Feststoff vom Schmelzpunkt 152 - 154 °C.
¹H-NMR (270 MHz, d₆-DMSO): δ = 1.11 (d, J = 8 Hz, 3 H), 4.79 (q, J = 8 Hz, 1 H), 5.15 (d, J = 12 Hz, 1 H), 5.24 (d, J = 12 Hz, 1 H), 5.38 (br. s, 2 H), 6.42 (s, 1 H), 7.3 - 7.4 (m, 6 H), 10.59 ppm (br. s, 1 H).
MS: (M + H)⁺ = 346

### Beispiel VI

### A) (3S)-6-Chlor-3-methyl-4-N-(3-methyl-2-buten-1-yl)-3,4- dihydro-chinoxalin-2(1H)-on

Die Verbindung des Beispiels IB (1.0 g, 5.0 mmol) wurde in 20 ml Acetonitril gelöst und in Gegenwart von 1.0 g (7.0 mmol) gepulvertem Kaliumcarbonat mit 3-Methyl-2-buten-1-ylbromid (90 %-ig; 0.92 ml, 7.0 mmol) bei Raumtemperatur alkyliert. Nach 7 h war die Reaktion beendet. Es wurde abgesaugt, im Vakuum eingeent und das Produkt durch Chromatographie an Kieselgel mit Essigsäureethylester / Heptan = 1 : 2 als Elutionsmittel gereinigt. Die Ausbeute betrug 0.97 g (72 %) bräunlicher Feststoff vom Schmelzpunkt 117 - 118 °C (nach Kristallisation aus Methyl-tert.-butylether / Heptan).
¹H-NMR (270 MHz, d₆-DMSO): δ = 1.02 (d J = 8 Hz, 3 H), 1.74 (s, 6 H), 3.69 (dd, J = 14, 8 Hz, 1 H), 3.85 - 3.9 (m, 2 H), 5.19 (m, 1 H), 6.65 - 6.8 (m, 3 H), 10.47 ppm (br. s, 1 H).
MS: (M + H)⁺ = 265
[α]_{D}²³ = + 168.0° (c = 1, MeOH)

### B) (3R)-6-Chlor-3-methyl-4-N-(3-methyl-2-buten-1-yl)-3,4- dihydro-chinoxalin-2(1H)-on

Die Verbindung wurde nach der in Beispiel VIA beschriebenen Methode ausgehend von der Verbindung des Beispiels IC hergestellt. Schmp. 115 - 117 °C (nach Umkristallisation aus Isopropanol / Diethylether)
Die NMR-Daten stimmten mit denen der in Beispiel VIA beschriebenen Verbindung überein.
[α]_{D}²³ = - 172° (c = 1, MeOH)

### C) (3RS)-6-Chlor-3-methyl-4-N-(3-methyl-2-buten-1-yl)-3,4- dihydro-chinoxalin-2(1H)-on

Die Verbindung wurde nach der in Beispiel VIA beschriebenen Methode ausgehend von der Verbindung des Beispiels ID hergestellt. Schmp. 148 - 149 °C (nach Umkristallisation aus Isopropanol / Diethylether)
Die NMR-Daten stimmten mit denen der in Beispiel VIA beschriebenen Verbindung überein.

### Beispiel VII

### (3S)-6-Chlor-3-methyl-4-N-(2-buten-1-yl)-3,4-dihydro-chinoxalin-2(1H)-on

Die Substanz wurde analog zu der in Beispiel VIA beschriebenen Verbindung hergestellt, jedoch mit 2-Buten-1-ylbromid als Alkylierungsmittel. Schmp. 87 - 88 °C (nach Kristallisation aus Diethylether / Heptan)
¹H-NMR (270 MHz, d₆-DMSO): δ = 1.01 (d, J = 8 Hz, 3 H), 1.70 (dd, J = 8, 1 Hz, 3 H), 3.63 (dd, J = 16, 6 Hz, 1 H), 3.85 - 4.0 (m, 2 H), 5.47 (m, 1 H), 5.75 (m, 1 H), 6.65 - 6.8 (m, 3 H), 10.48 ppm (br. s, 1 H).
MS: (M + H)⁺ = 251

### Beispiel VIII

### 4-N-(Isopropenyloxycarbonyl)-3,3,7-trimethyl-3,4-dihydrochinoxalin-2(1H)-on

3,3,7-Trimethyl-3,4-dihydrochinoxalin-2(1H-on) (0,4 g, 2,1 mmol) wurden in 10 ml wasserfreiem Pyridin gelöst und bei Raumtemperatur unter Rühren mit 0,24 ml (2,2 mmol) Chlorameisensäureisopropenylester versetzt. Man ließ 6 h bei Raumtemperatur rühren, versetzte mit Wasser, saugte die entstehende Fällung ab, wusch mit Wasser nach und trocknete. Es wurden 0,4 g (69 %) farblose Kristalle vom Schmelzpunkt 185 °C erhalten.
¹H-NMR (270 MHz, d₆-DMSO): δ = 1.5 (s, 6 H), 1.9 (s, 3 H), 2.25 (s, 3 H), 4.7 (m, 2 H), 6.7 - 6.9 (m, 2 H), 7.15 (d, J = 8 Hz, 1 H), 10.6 ppm (br. s, 1 H).
MS: M⁺ = 274

### Beispiel IX

### (3S)-6-Chlor-4-N-(4-methoxyphenoxycarbonyl)-3-methyl-3,4- dihydro-chinoxalin-2(1H)-on

Die Verbindung des Beispiels IB (0.5 g, 2.55 mmol) wurde in 10 ml wasserfreiem N,N-Dimethylformamid gelöst und 0.41 ml (2.8 mmol) Triethylamin zugegeben. Unter Rühren wurden erst 0.42 ml (2.8 mmol) Chlorameisensäure-4-methoxyphenylester zugetropft und nach 2 h nochmals 0.21 ml (1.9 mmol). Nach vollständigem Umsatz (18 h) wurde das Lösungsmittel unter vermindertem Druck abgezogen, der Rückstand in Essigsäureethylester aufgenommen, mit Wasser gewaschen und getrocknet (Natriumsulfat). Nach dem Einengen blieben 0.48 g (54 %) eines weißen Feststoffs zurück. Schmp. 187 - 190 °C (nach Umkristallisation aus Isopropanol)
¹H-NMR (270 MHz, d₆-DMSO): δ = 1.24 (d, J = 8 Hz, 3 H), 3.77 (s, 3 H), 4.94 (q, J = 8 Hz, 1 H), 6.97 (dd, J = 8, 2 Hz, 1 H), 7.03 (d, J = 8 Hz, 1 H),7.2 - 7.3 (m, 3 H), 7.78 (s, 1 H), 10.89 ppm (br. s, 1 H).
MS: (M + H)⁺ = 347

### Beispiel X

### (3S)-6-Chlor-4-N-(4-fluorphenoxycarbonyl)-3-methyl-3,4-dihydro-chinoxalin-2(1H)-on

Die Verbindung wurde analog zu der in Beispiel VIA beschriebenen Verbindung hergestellt, außer daß Chlorameisensäure-4- fluorphenylester als Acylierungsmittel verwendet wurde. Schmp. 168 - 170 °C (nach Kristallisation aus Isopropanol)
¹H-NMR (270 MHz, d₆-DMSO): δ = 1.24 (d, J = 8 Hz, 3 H), 4.94 (q, J = 8 Hz, 1 H), 7.03 (d, 8 Hz, 1 H), 7.2 - 7.5 (m, 5 H), 7.83 (d, J = 2 Hz, 1 H), 10.90 ppm (br. s, 1 H).
MS: (M + H)⁺ = 335

### Beispiel XI

### (3S)-6-Chlor-4-N-(4-chlorphenoxycarbonyl)-3-methyl-3,4-dihydro-chinoxalin-2(1H)-on

Die Verbindung wurde analog zu der in Beispiel VIA beschriebenen Verbindung hergestellt, außer daß Chlorameisensäure-4- chlorphenylester zur Acylierung eingesetzt wurde. Schmp. 185 - 188 °C (nach Kristallisation aus Isopropanol/Diethylether)
¹H-NMR (270 MHz, d₆-DMSO): δ = 1.25 (d, J = 8 Hz, 3 H), 4.94 (q, J = 8 Hz, 1 H), 7.04 (d, 8 Hz, 1 H), 7.25 (dd, J = 8, 2 Hz, 1 H), 7.35 - 7.6 (m, 4 H), 7.80 (s, 1 H), 10.91 ppm (br. s, 1 H).
MS: (M + H)⁺ = 351

### Beispiel XII

### (3S)-4-N-(2-Bromethyloxycarbonyl)-6-chlor-3-methyl-3,4-dihydrochinoxalin-2(1H)-on

Die Verbindung wurde analog zu der in Beispiel VIA beschriebenen Verbindung hergestellt, außer daß Chlorameisensäure-2- bromethylester zur Acylierung eingesetzt wurde. Schmp. 133 - 136 °C (nach Kristallisation aus Isopropanol)
¹H-NMR (270 MHz, d₆-DMSO): δ = 1.16 (d, J = 8 Hz, 3 H), 3.7 - 3.8 (m, 2 H), 4.4 - 4.6 (m, 2 H), 4.86 (q, J = 8 Hz), 6.99 (d, 8 Hz, 1 H), 7.21 (dd, 8, 2 Hz, 1 H), 7.74 (d, J = 2 Hz, 1 H), 10.84 ppm (br. s, 1 H).
MS: (M + H)⁺ = 348

### Beispiel XIII

### (3S)-6-Chlor-N-(isopropenyloxycarbonyl)-3-methyl-3,4-dihydro-chinoxalin-2(1H)-on

Die Substanz wurde analog zu der in Beispiel VIA beschriebenen Verbindung hergestellt, außer daß Chlorameisensäure- isopropenylester zur Acylierung eingesetzt wurde. Schmp. 158 - 159 °C
¹H-NMR (270 MHz, CDCl₃): δ = 1.33 (d, J = 8 Hz, 3 H), 2.02 (s, 3 H), 4.79 (s, 1 H), 4.83 (s, 1 H), 5.17 (q, J = 8 Hz, 1 H), 6.86 (d, J = 8 Hz, 1 H), 7.12 (dd, J = 8 , 2 Hz, 1 H), 7.74 (br. s, 1 H), 9.28 ppm (br. s, 1 H).
MS: (M + H)⁺ = 281

### Beispiel XIV

### (3S)-6-Chlor-3-methyl-4-N-(vinyloxycarbonyl)-3,4-dihydrochinoxalin-2(1H)-on

Die Substanz wurde analog zu der in Beispiel VIA beschriebenen Verbindung hergestellt, außer daß Chlorameisensäurevinylester zur Acylierung eingesetzt wurde. Schmp. 177 - 179 °C
¹H-NMR (270 MHz, CDCl₃): δ = 1.33 (d, J = 8 Hz, 3 H), 4.96 (dd, J = 14, 2 Hz, 1 H), 5.20 (q, J = 8 Hz, 1 H), 6.83 (d, J = 8 Hz, 1 H), 7.12 (dd, J = 8, 2 Hz, 1 H), 7.2 - 7.3 (m, 2 H), 7.71 (br. s, 1 H), 9.42 ppm (br. s, 1 H). MS: (M + H)⁺ = 267

### Beispiel XV und Beispiel XVI

6-Chlor-3,4-dihydro-chinoxalin-2(1H)-on wurde analog zu dem in Beispiel VIA beschriebenen Verfahren mit 3-Methyl-2-buten-1-ylbromid umgesetzt. Mittels Chromatographie an Kieselgel ließen sich zwei Produkte isolieren. 6-Chlor-4-N-(3-methyl-2-buten-1-yl)-3,4-dihydro-chinoxalin- 2(1H)-on Schmp. 150 - 151 °C (nach Umkristallisation aus Essigsäureethylester)
¹H-NMR (270 MHz, d₆-DMSO): δ = 1.72 (s, 6 H), 3.67 (s, 2 H), 3.80 (d, J = 7 Hz, 2 H), 5.20 (m, 1 H), 6.7 - 6.8 (m, 3 H), 10.49 ppm (br. s, 1 H).
MS: (M + H)⁺ = 251

6-Chlor-4-N-(3-methyl-2-buten-1-yl)-3-(1,1-dimethyl-2-propen-1- yl)-3,4-dihydrochinoxalin-2(1H)-on
Schmp. 110 - 112 °C (nach Kristallisation aus Heptan)

¹H-NMR (270 MHz, d₆-DMSO): δ = 0.94 (s, 3 H), 0.97 (s, 3 H), 1.65 (s, 3 H), 1.66 (s, 3 H), 3.77 (dd, J = 16, 7 Hz, 1 H), 4.23 (dd, J = 16, 7 Hz, 1 H), 4.8 - 4.9 (m, 2 H), 5.02 (m, 1 H), 5.75 (dd, J = 17, 11 Hz, 1 H), 6.6 - 6.7 (m, 3 H), 10.49 ppm (br. s, 1 H).
MS: (M + H)⁺ = 319

Die folgenden Verbindungen der Formel I wurden aus den entsprechenden unsubstituierten Chinoxalinonen in analoger Weise synthetisiert und gegebenenfalls weiter derivatisiert:

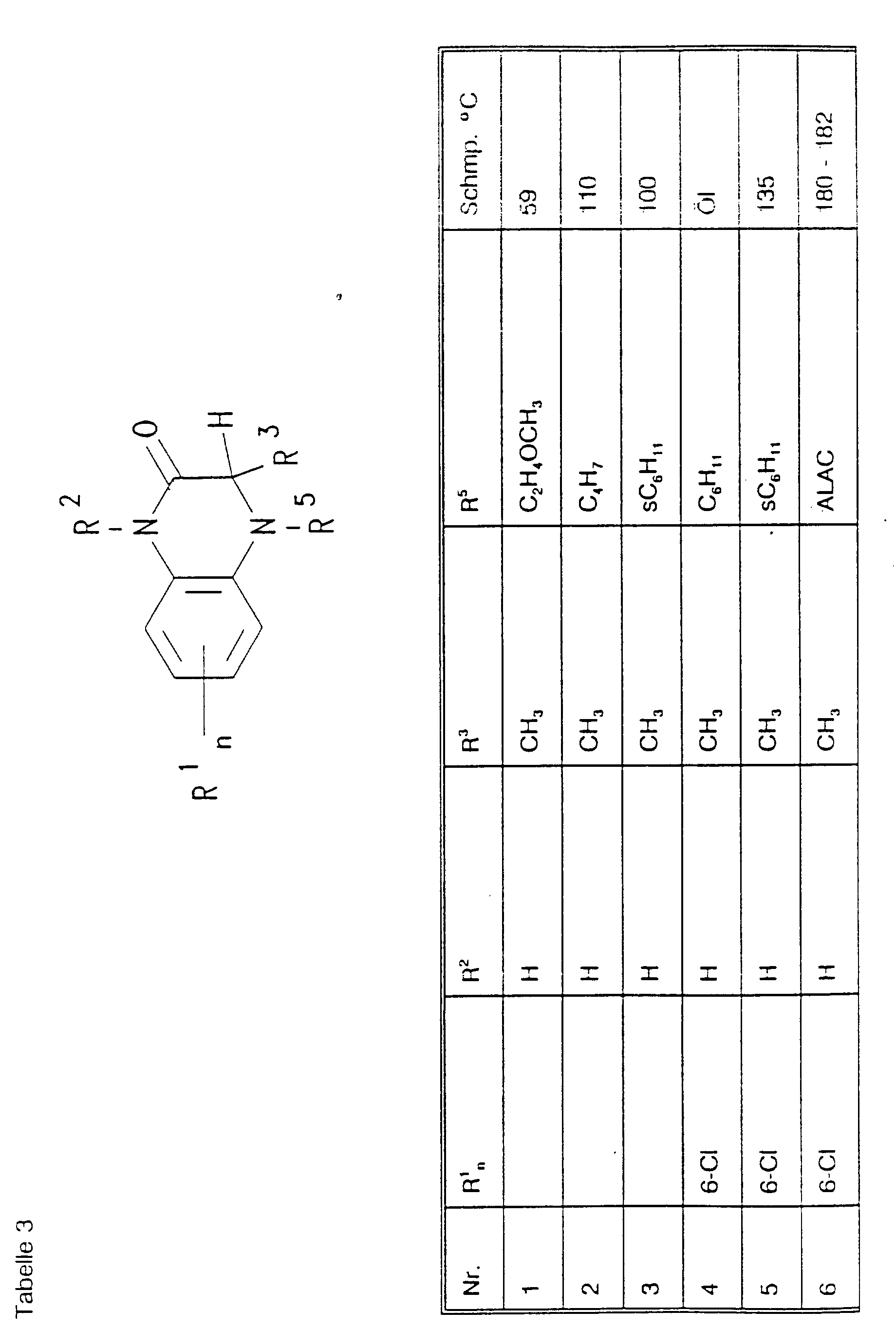

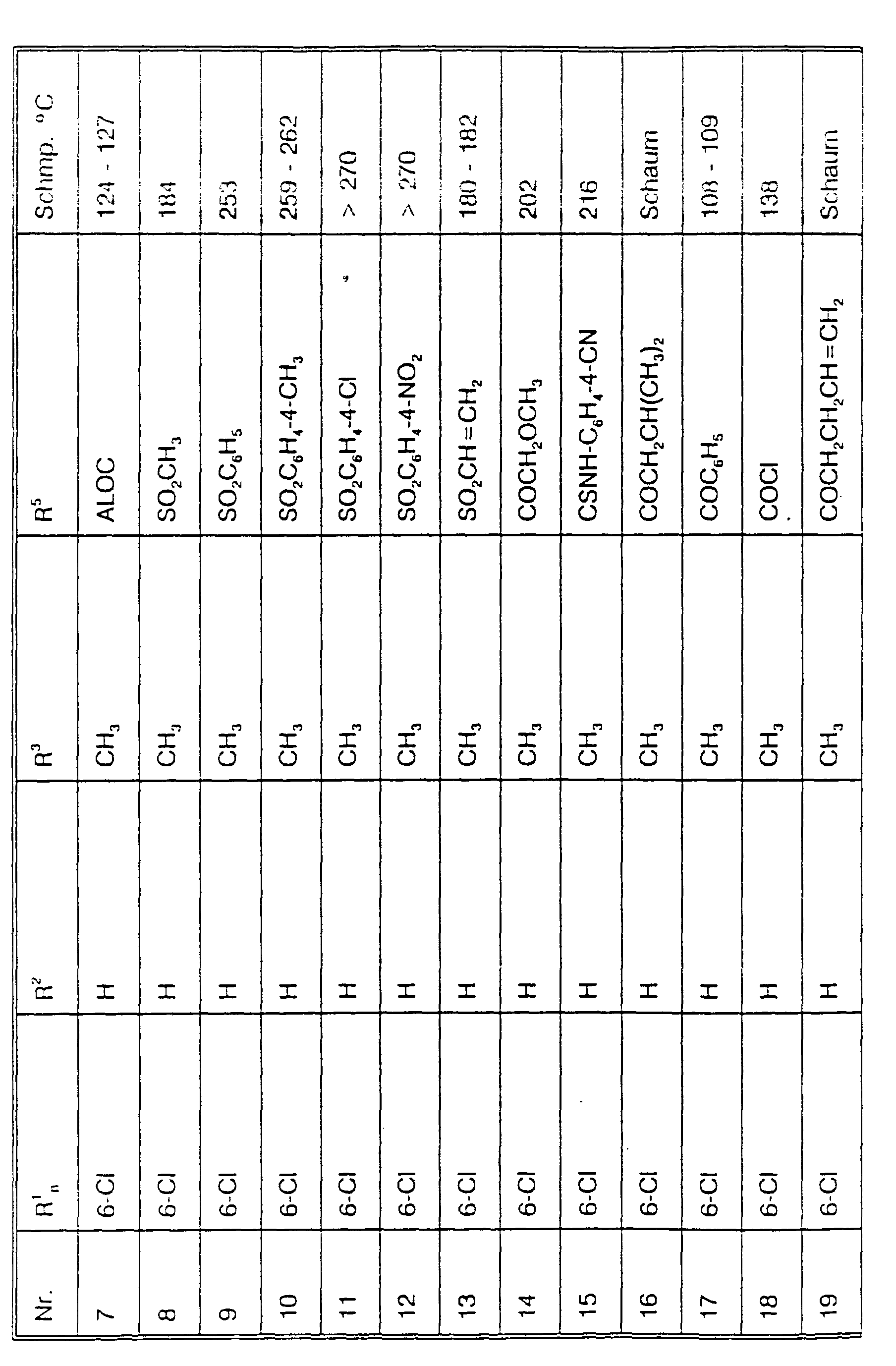

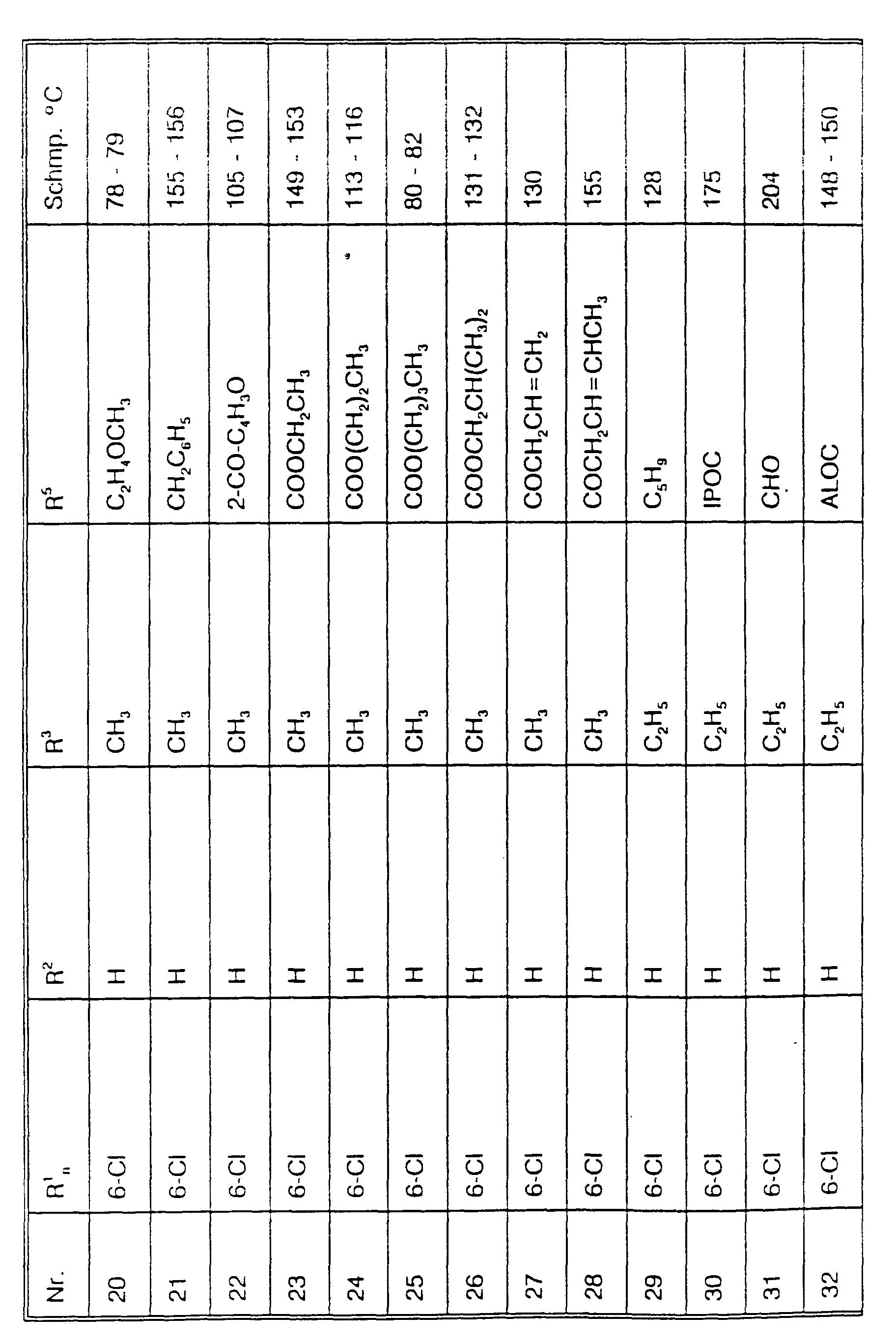

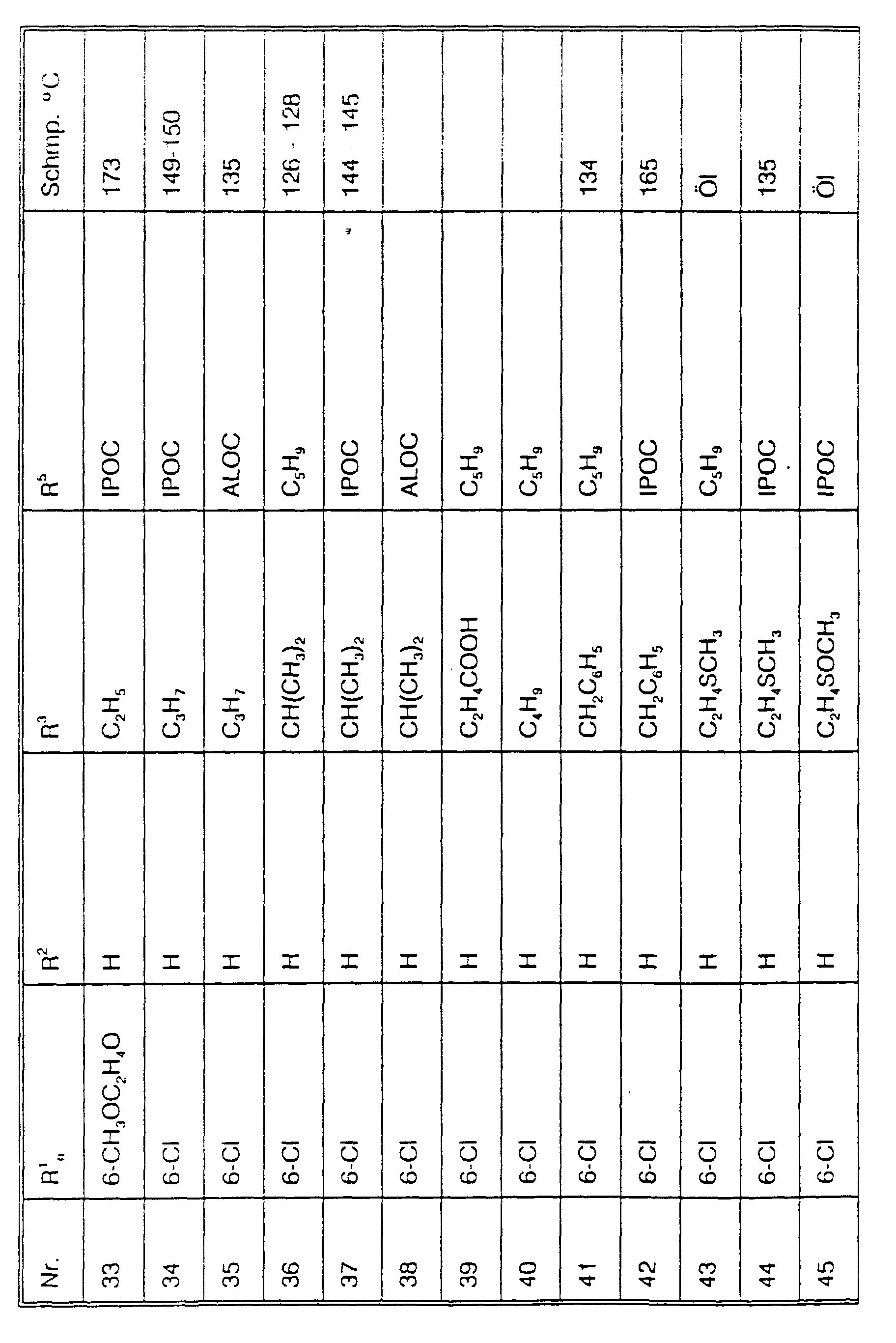

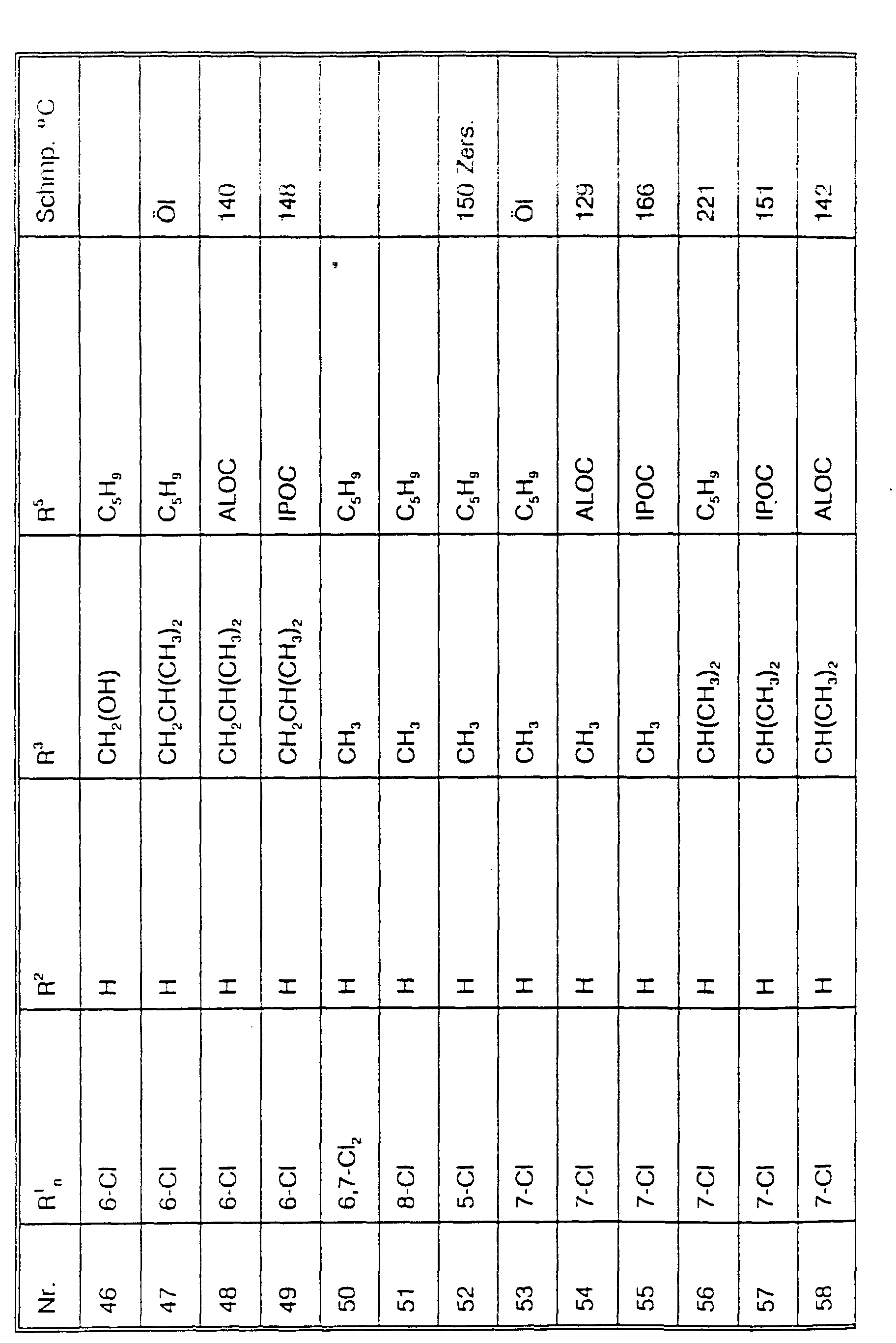

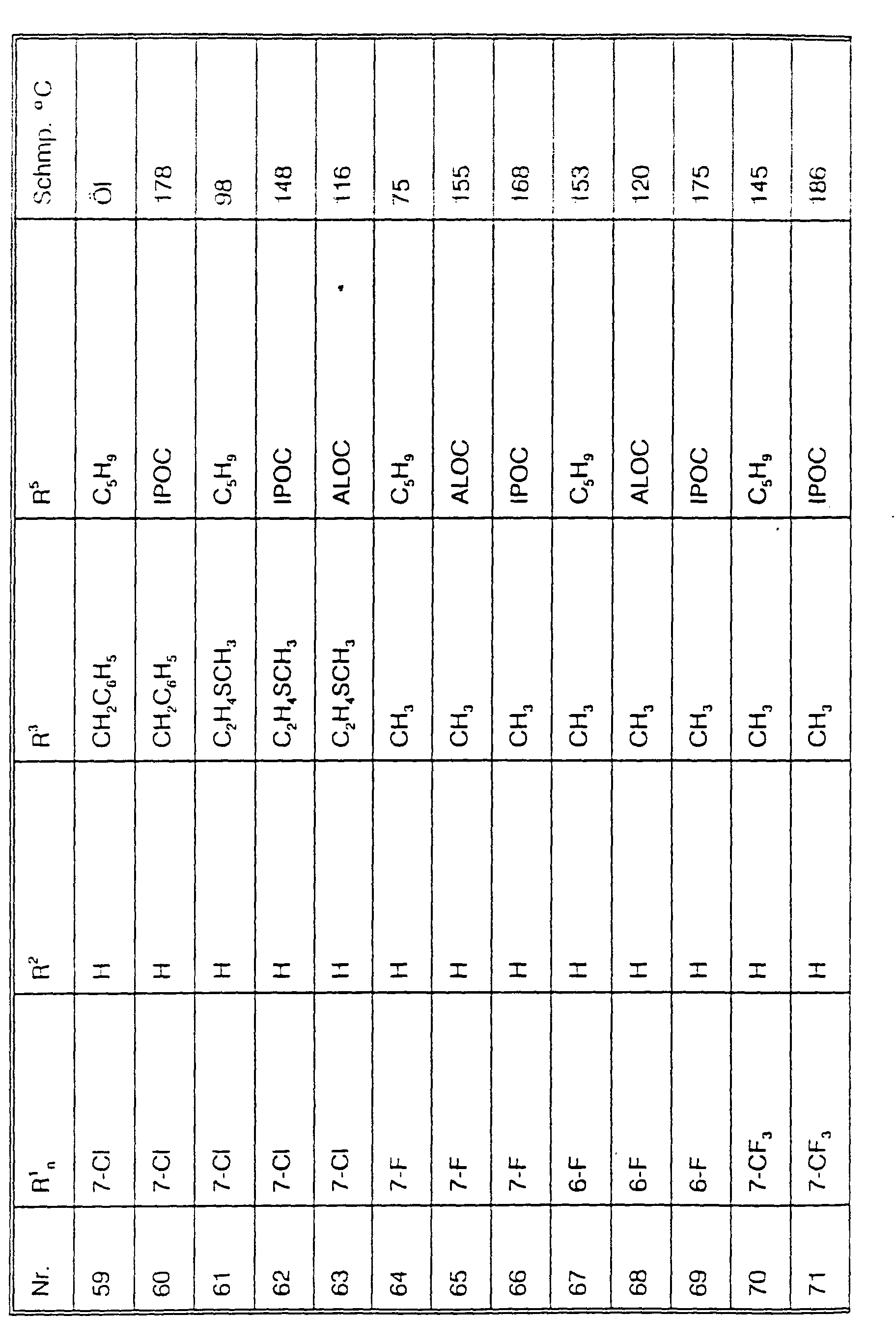

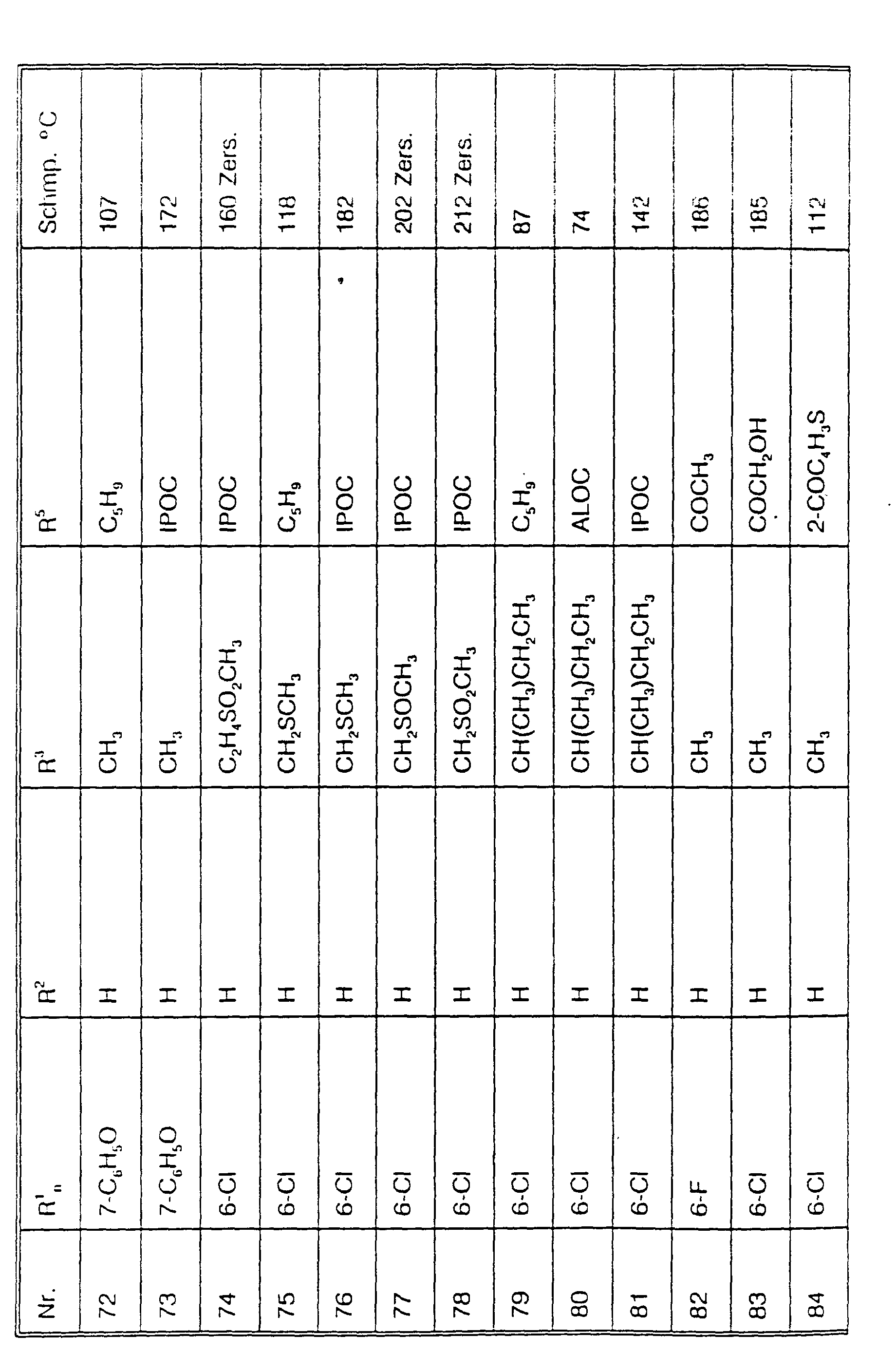

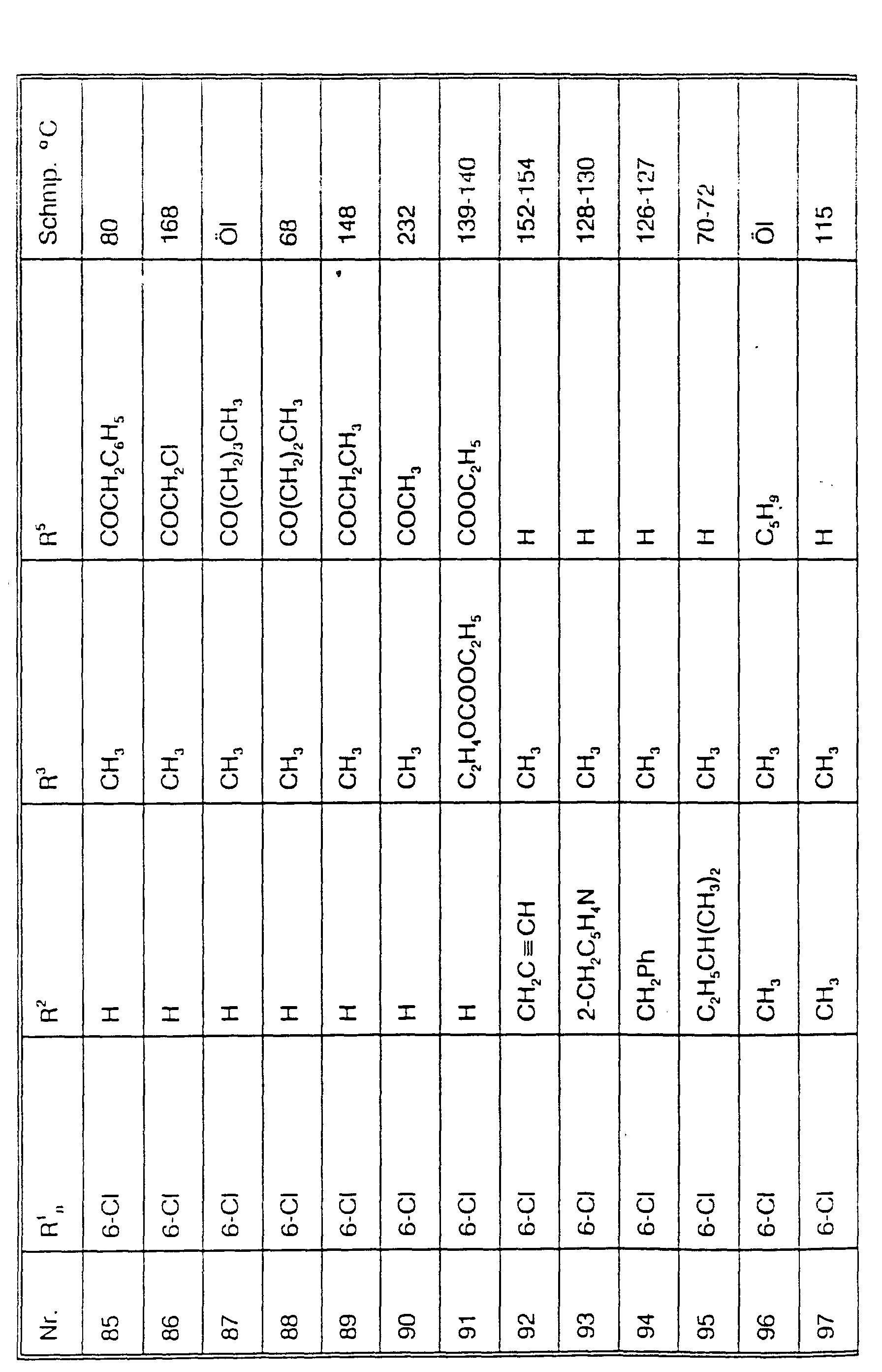

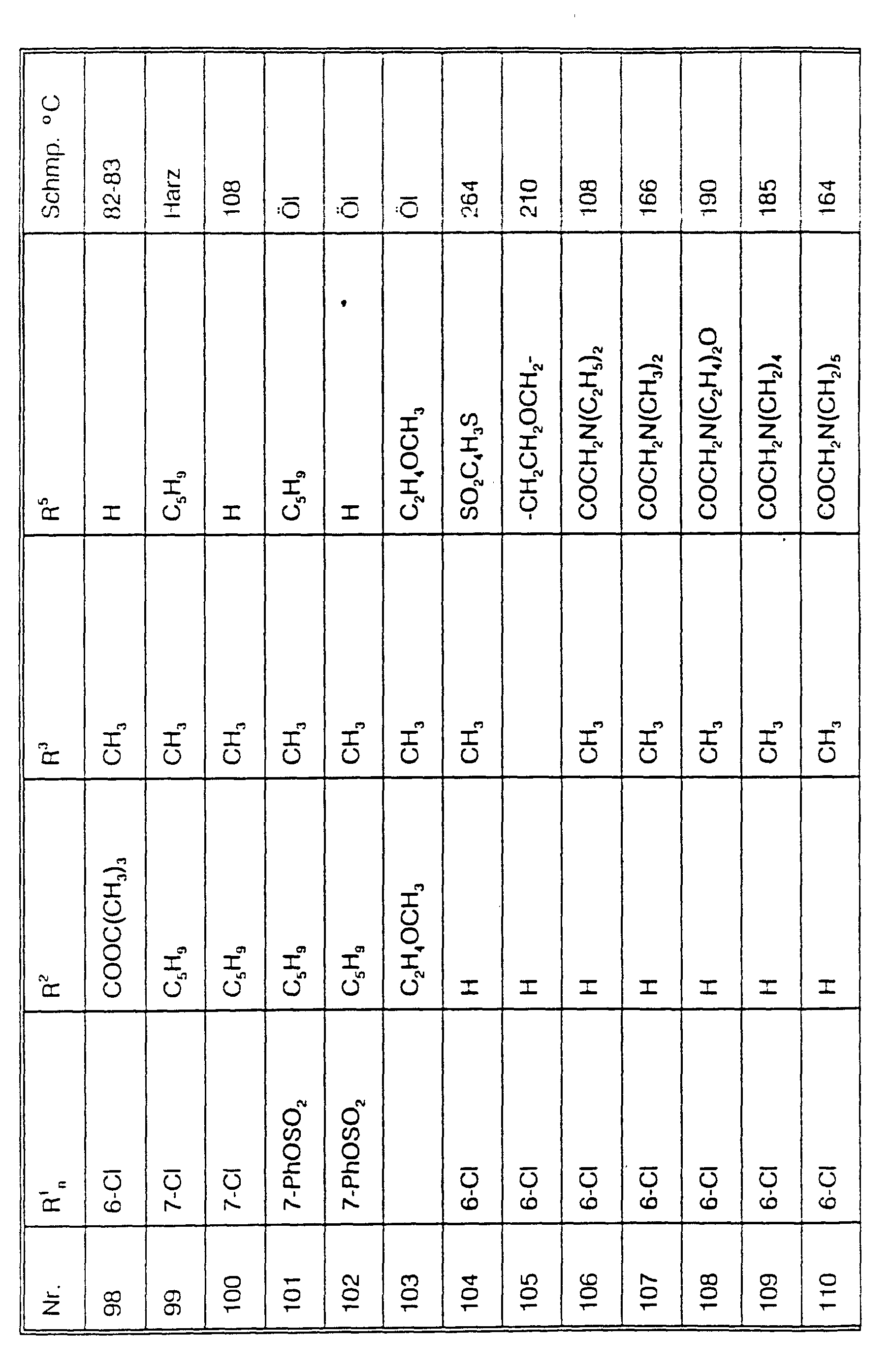

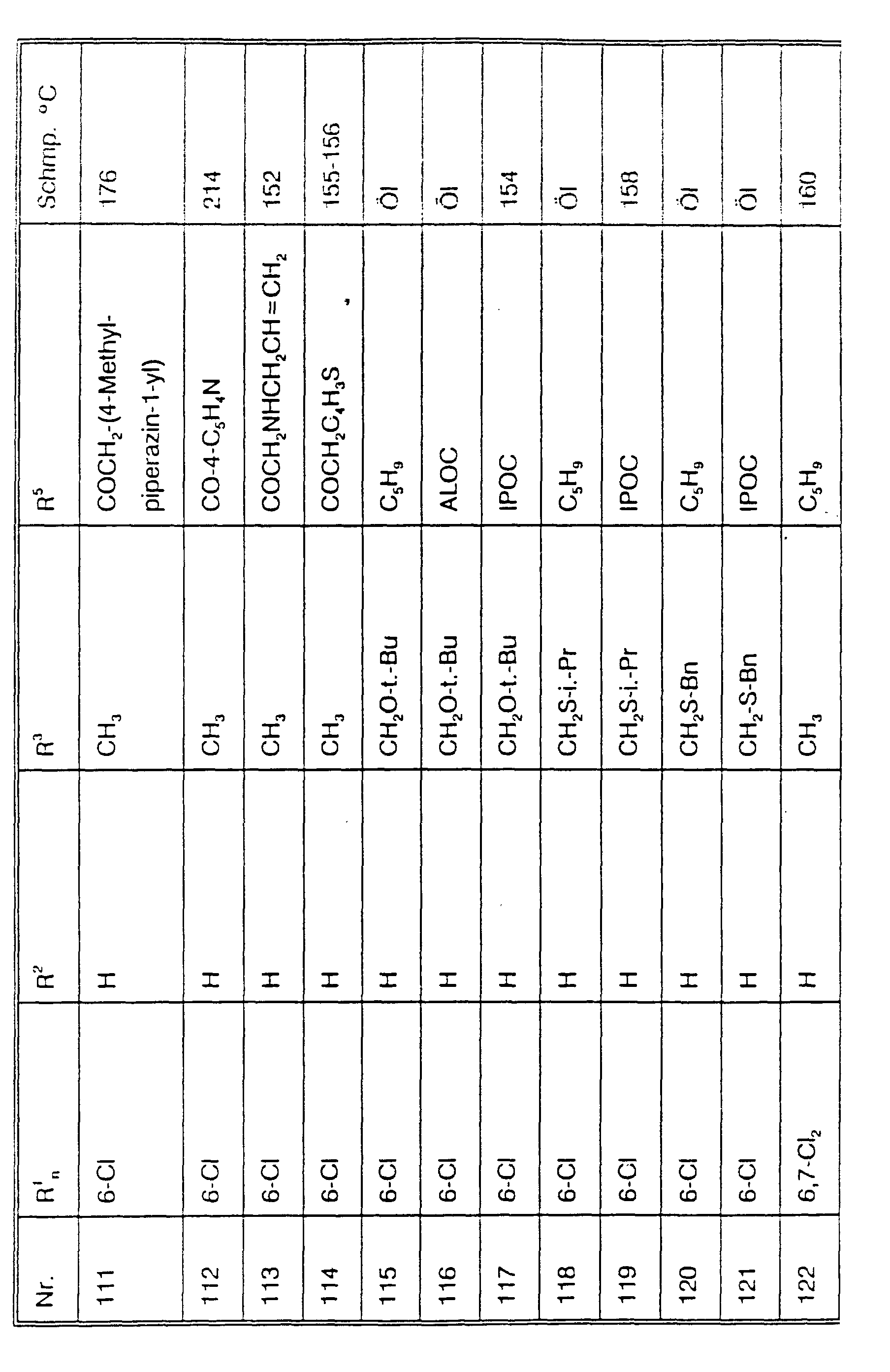

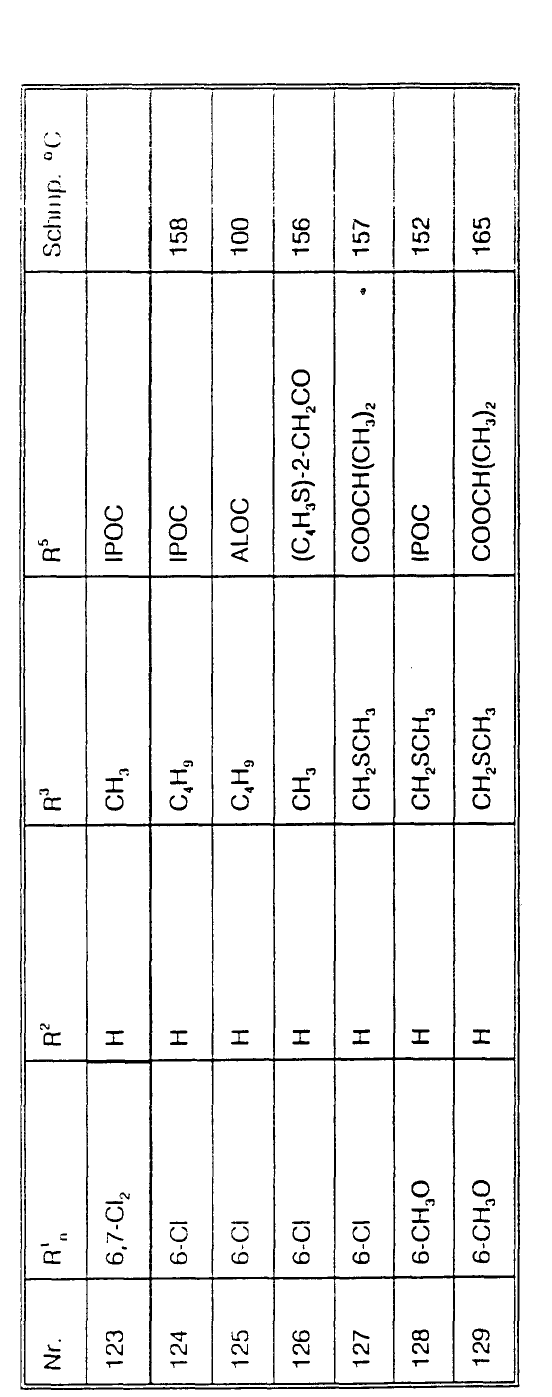

- Erläuterungen:: C₅H₉ = 3-Methyl-2-buten-1-yl
C₄H₇ = 2-Butenyl
C₅H₁₁ = 3-Methyl-1-butyl
C₆H₁₁ = 2,2-Dimethylcyclopropyl-1-methyl
sC₆H₁₁ = 4-Methyl-3-penten-2-yl
C₃H₃ = 2-Propen-1-yl
(CH₃)₂CCHCO = 3,3-Dimethylacryl
IPOC = Isopropenyloxycarbonyl
ALAC = Allylaminocarbonyl
ALOC = Allyloxycarbonyl
C₄H₃O = Furanyl
C₄H₃S = Thienyl
C₅H₄N = Pyridyl
Ph = Phenyl

### Beispiel XVII

### 6,7-Dimethoxy-3-methyl-3,4-dihydro-chinoxalin-2(1H)-on

4,5-Dimethoxy-1,2-dinitrobenzol (34.2 g, 0.15 mol) wurden in 500 ml Methanol unter Raney-Nickel-Katalyse mit 1 atm Wasserstoff hydriert. Nach Aufnahme der berechneten Menge Wasserstoff wurde abgebrochen, vom Katalysator abgesaugt und das Lösungsmittel im Vakuum abgezogen. Um alles Wasser zu entfernen, wurde noch zweimal in Methanol aufgenommen und wieder eingeengt. Das als braunes Öl verbleibende 4,5-Dimethoxy-1,2-phenylendiamin (24.0 g) wurde in 200 ml Ethanol (96 %) unter Zusatz von 21.0 ml (0.15 mol) Triethylamin mit 17.1 ml (0.15 mol) 2-Chlorpropionsäuremethylester für 48 h unter Rückfluß erhitzt. Die tiefdunkle Lösung wurde eingeengt, in Essigsäureethylester aufgenommen, zweimal mit Wasser gewaschen, getrocknet (Natriumsulfat) und das Lösungsmittel im Vakuum abgezogen.
Das Rohprodukt wurde durch Anrühren mit Diethylether kristallisiert (6.2 g, 19 %). Eine analysenreine Probe mit Schmelzpunkt 151 °C erhielt man durch Chromatographie an Kieselgel mit Essigsäureethylester als Elutionsmittel.
¹H-NMR (60 MHz d₆-DMSO): δ = 1.22 (d, J = 7 Hz, 3 H), 3.63 (s, 3 H), 3.67 (s; 1 H), 3.6 - 3.7 (m, 1 H), 5.62 (br. s, 1 H), 6.40 (s, 1 H), 6.45 (s, 1H), 9.90 ppm (br. s, 1 H).
MS: M⁺ = 222

Die folgenden Verbindungen der Formel I wurden in analoger Weise synthetisiert und gegebenenfalls weiter derivatisiert:

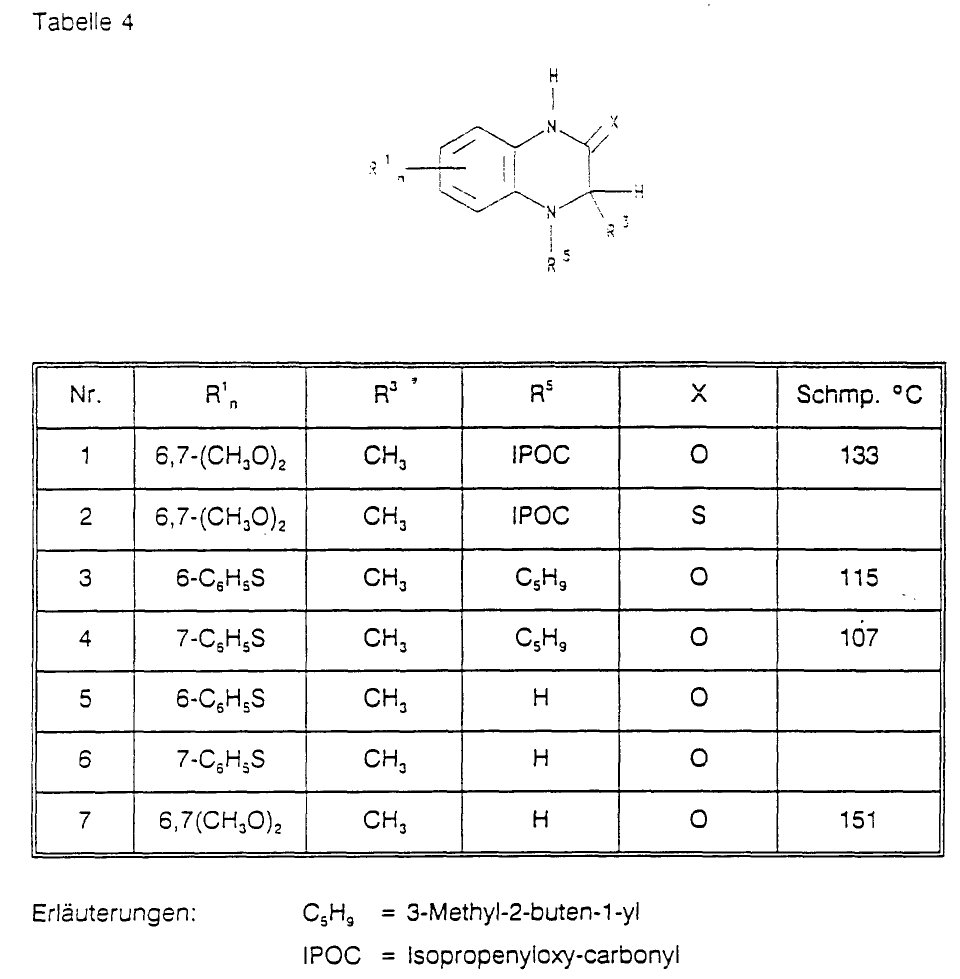

### Beispiel XVIII

### (3RS)-6-Chlor-4-N-(cyclopropyl)-3-methyl-3,4-dihydro-chinoxalin- 2(1H)-on

### A) (2RS)-N-(4-Chlor-2-cyclopropylaminophenyl)-(2-brom-propionsäureamid)

4-Chlor-2-cyclopropylamino-nitrobenzol (2.10 g, 0.01 mol) wurden in 100 ml Methanol unter Raney-Nickel-Katalyse mit 1 atm Wasserstoff hydriert. Nach Aufnahme der berechneten Menge Wasserstoff wurde abgebrochen, vom Katalysator abgesaugt und das Lösungsmittel im Vakuum abgezogen. Um alles Wasser zu entfernen, wurde noch zweimal in Methanol aufgenommen und wieder eingeengt. Das als braunes Öl verbleibende 4-Chlor-2- cyclopropylamino-anilin (1.80 g) wurde in 50 ml wasserfreiem 1,2-Dimethoxyethan gelöst und unter Rühren auf -60 °C abgekühlt. Eine Lösung von 1.1 ml (0.01 mol) 2-Brompropionsäurechlorid in 5 ml wasserfreiem 1,2-Dimethoxyethan wurde langsam zugetropft und die Reaktionsmischung 2 h bei -60 - -70 °C nachgerührt. Dann ließ man auf ca. -20 °C erwärmen und goß auf 150 ml eiskalte gesättigte wäßrige Natriumhydrogencarbonatlösung. Es wurde zweimal mit Essigsäureethylester extrahiert, die organische Phase einmal mit Wasser gewaschen, getrocknet (Natriumsulfat) und im Vakuum eingeengt. Nach Kristallisation mit Diethylether / Pentan verblieben 2.51 g (79 %) des gewünschten Produkts mit Schmp. 130 °C.
¹H-NMR (270 MHz, d₆-DMSO): δ = 0.4 - 0.5 (m, 2 H), 0.7 - 0.8 (m, 2 H), 1.75 (d, J = 7 Hz, 3 H), 2.39 (m, 1 H), 4.72 (q, J = 7 Hz, 1 H), 5.6 (br. s, 1 H), 6.66 (dd, J = 8, 2 Hz, 1 H), 6.96 (d, J = 2 Hz, 1 H), 7.21 (d, J = 8 Hz, 1 H), 9.36 ppm (br. s, 1 H).
MS: (M + H)⁺ = 319, 317

### B) (3RS)-6-Chlor-4-N-(cyclopropyl)-3-methyl-3,4-dihydro- chinoxalin-2(1H)-on

Die Verbindung des Beispiels XVIIIA (318 mg, 1.0 mmol) wurde in 20 ml Ethanol (96 %) gelöst und nach Zugabe von 0.28 ml (2.0 mmol) Triethylamin 18 h unter Rückfluß erhitzt. Das Lösungsmittel wurde unter vermindertem Druck entfernt und das Reaktionsprodukt durch Chromatographie an Kieselgel mit Essigsäureethylester / Heptan = 1 : 2 als Elutionsmittel gereinigt. Die Ausbeute betrug 200 mg (85 %) weiße Kristalle vom Schmp. 167 °C (nach Kristallisation aus Pentan).
¹H-NMR (270 MHz, d₆-DMSO): δ = 0.40 (m, 1 H), 0.63 (m, 1 H), 0.76 (m, 1 H), 0.98 (m, 1 H), 1.12 (d, J = 7 Hz, 3 H), 2.47 (m, 1 H), 3.87 (q, J = 7 Hz, 1 H), 6.78 (s, 2 H), 7.0 (s, 1 H), 10.46 ppm (br., s, 1 H),
MS: (M + H)⁺ = 237

Die folgenden Verbindungen der Formel I wurden analog der in Beispiel XVIII beschriebenen Reaktionsführung unter Verwendung der entsprechend substituierten ortho-Nitroaniline und 2- Halogencarbonsäurederivate synthetisiert und gegebenenfalls weiter derivatisiert:

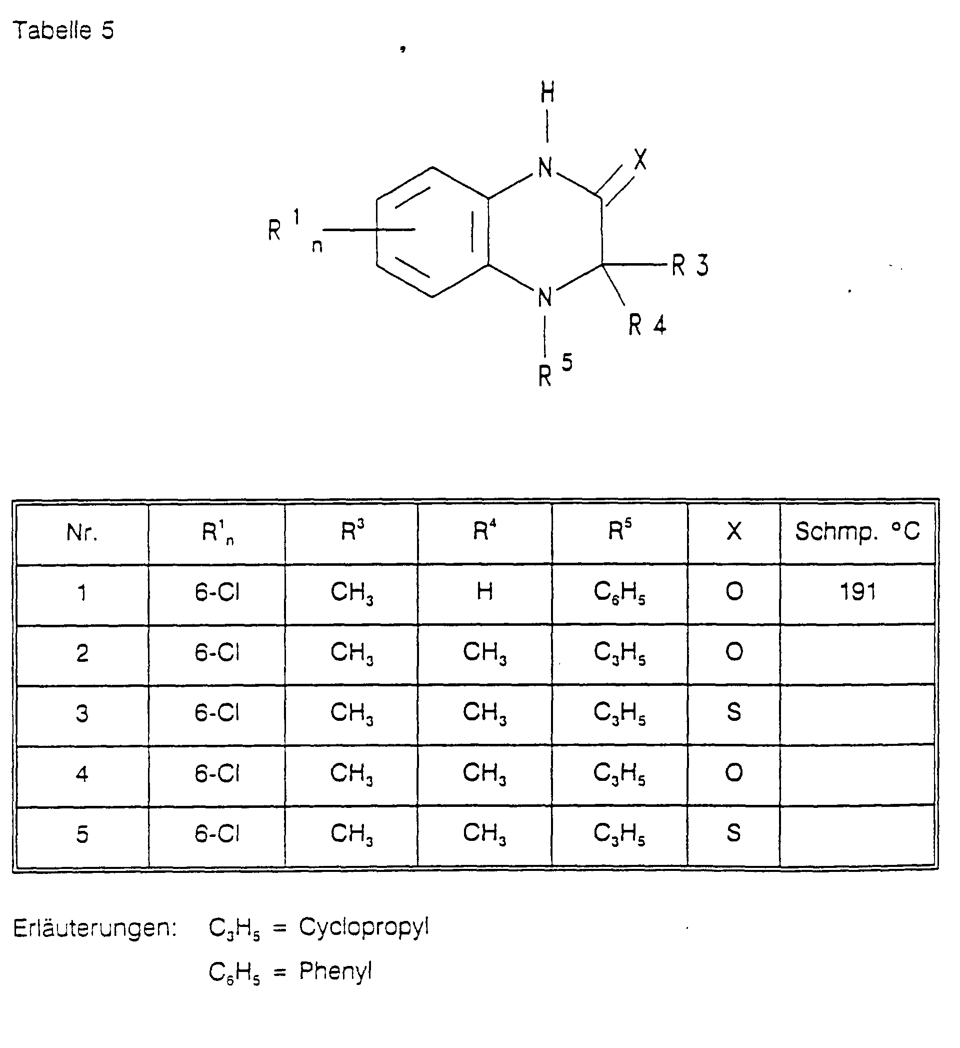

### Beispiel XIX

### 7-Chlor-1-N-(cyclopropyl)-3,3-dimethyl-3,4-dihydro-chinoxalin-2(1H)-on

4-Chlor-2-cyclopropylamino-nitrobenzol (2.0 g, 9.4 mmol) wurde wie unter Beispiel XVIIIA beschrieben hydriert. Das entstandene 4-Chlor-2-cyclopropylamino-anilin (1.70 g) wurde in 20 ml Dichlormethan aufgenommen. Es wurden 1.6 ml (2.01 mmol) Chloroform, 1.8 ml (2.45 mmol) Aceton und 0.10 g (0.4 mmol) Benzyltriethylammoniumchlorid zugesetzt und die Reaktionslösung auf 10 °C gekühlt. Langsam wurden unter kräftigem Rühren 4 ml 50 %-ige Natronlauge zugetropft, wobei die Reaktionstemperatur 10 °C nicht übersteigen sollte. Nach 5 h Rühren bei 10 °C wurden die Phasen verdünnt und getrennt. Die organische Phase wurde einmal mit Wasser gewaschen, getrocknet (Magnesiumsulfat) und im Vakuum eingedampft. Das Rohprodukt wurde durch Chromatographie an Kieselgel mit Essigsäureethylester / Heptan = 1 : 2 als Elutionsmittel gereinigt. Die Ausbeute betrug 1.0 g (42 %) weiße Kristalle vom Schmelzpunkt 132 - 133 °C (nach Umkristallisation aus Toluol / Heptan).
¹H-NMR (270 MHz, d₆-DMSO): δ = 0.45 - 0.55 (m, 2 H), 1.05 - 1.1 (m, 2 H), 1.19 (s, 6 H), 2.71 (m, 1 H), 6.09 (br. s, 1 H), 6.71 (d, J = 8 Hz, 1 H), 6.88 (dd, J = 8, 2 Hz, 1 H), 7.19 ppm (d, J = 2 Hz, 1 H).
MS: (M + H)⁺ = 251

Die folgenden Verbindungen der Formel I wurden in analoger Weise synthetisiert und gegebenenfalls weiter derivatisiert:

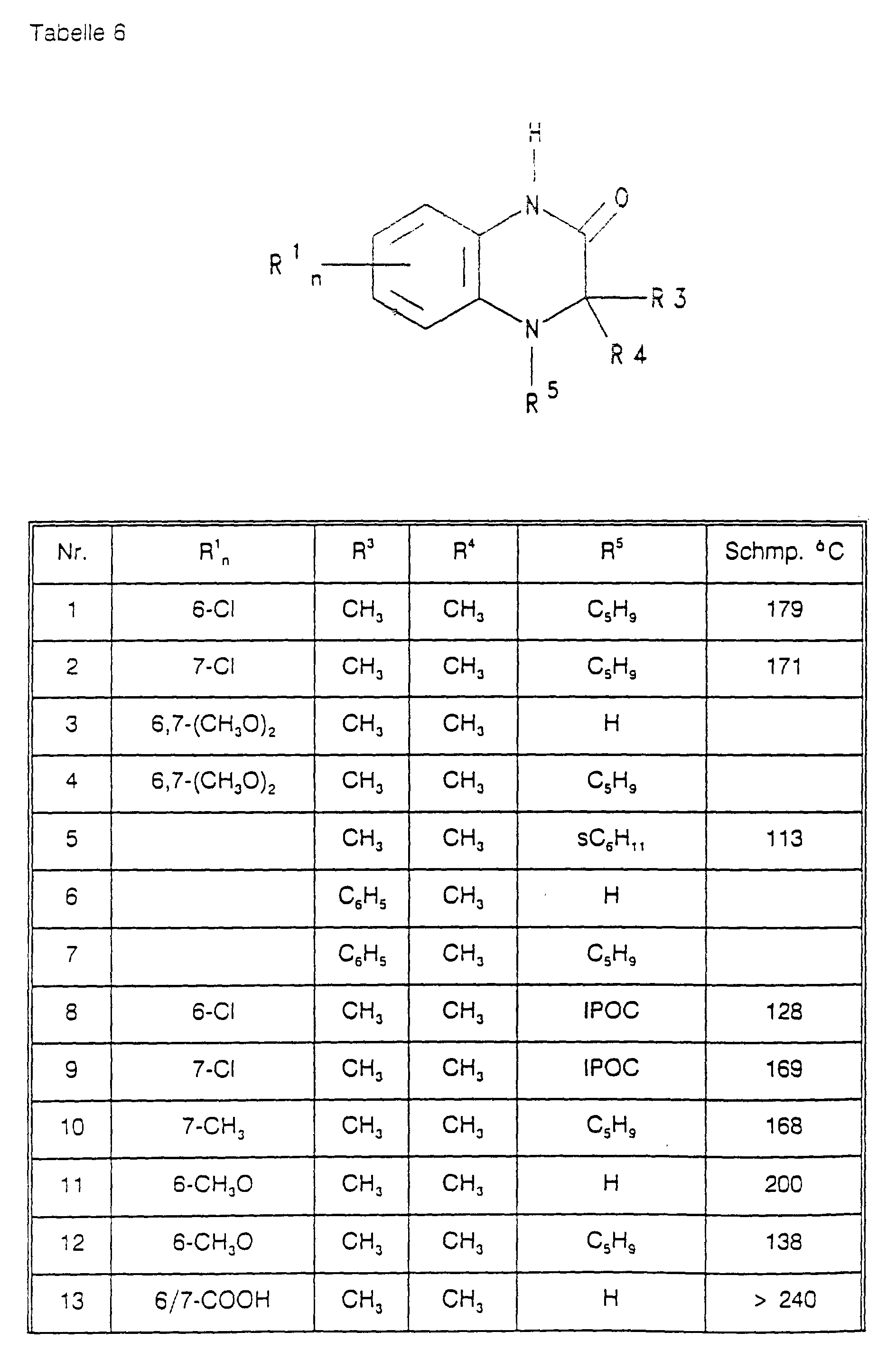

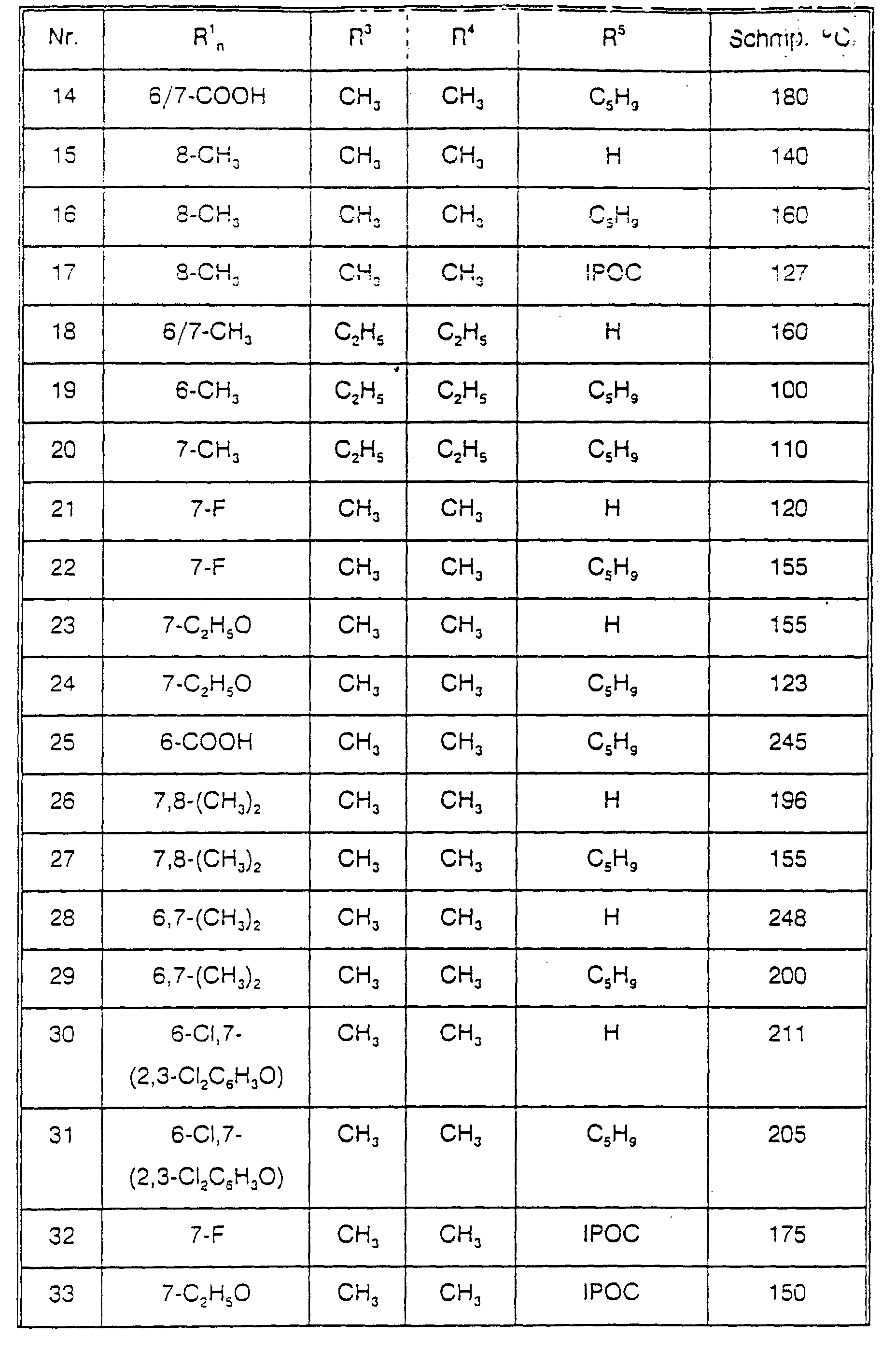

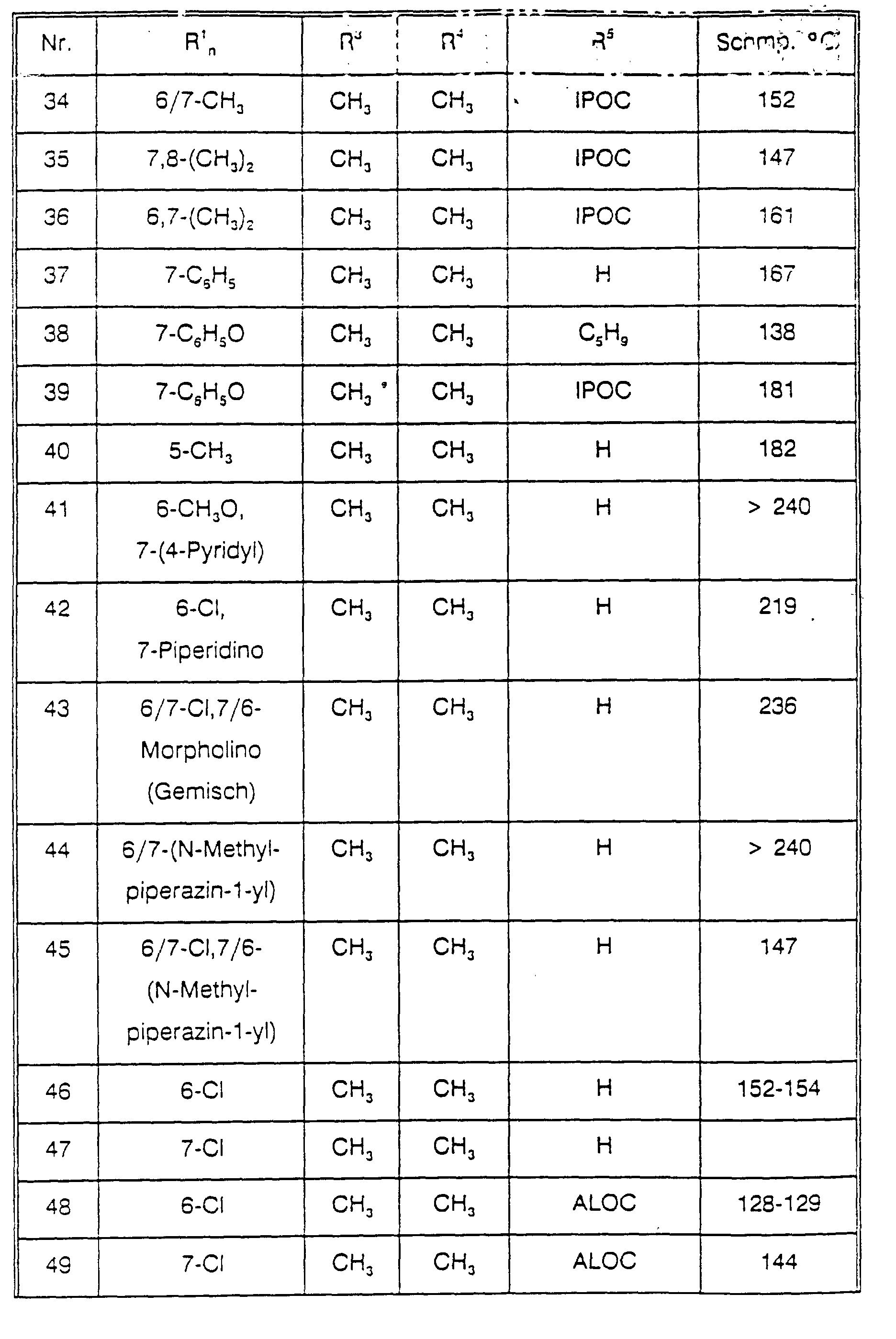

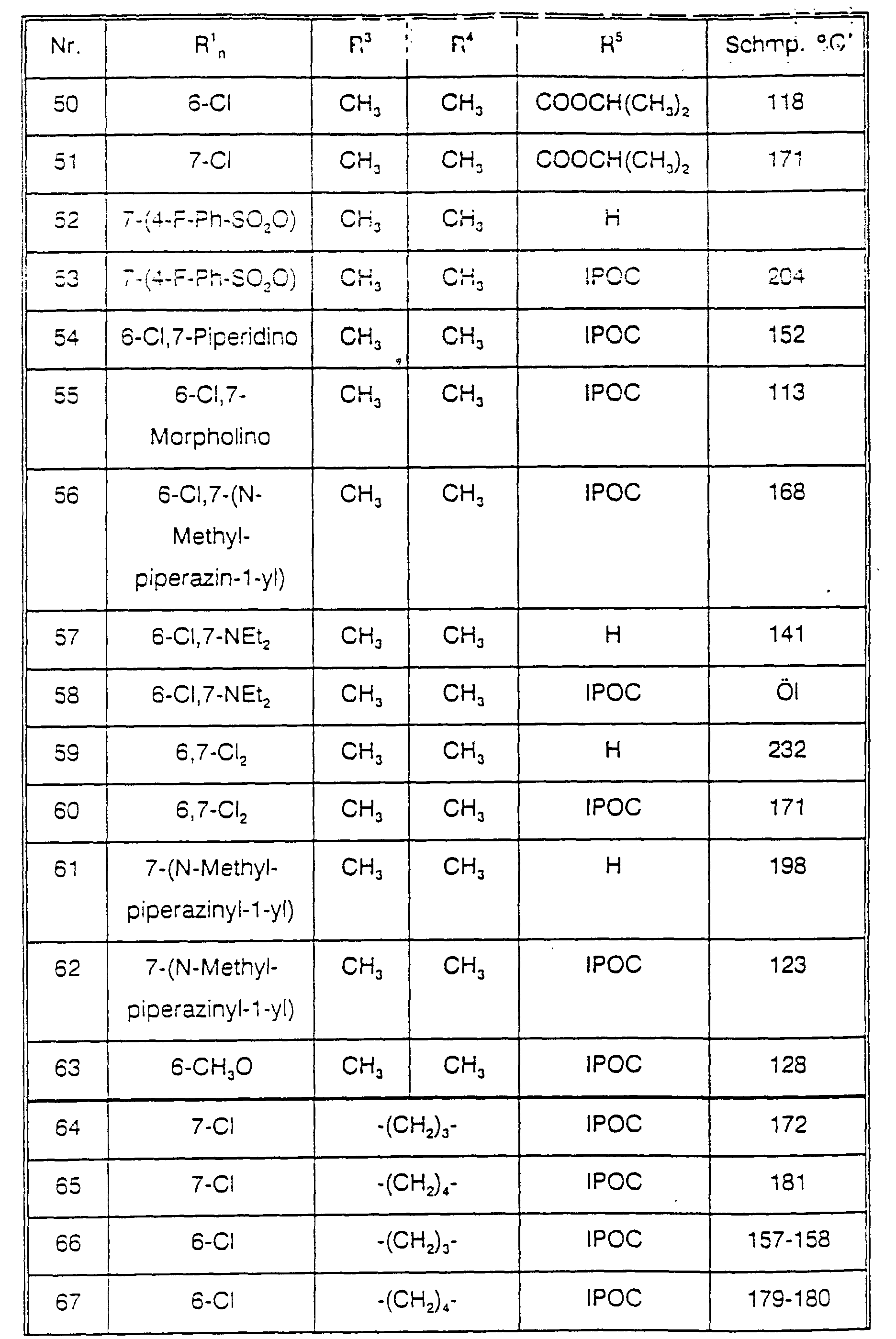

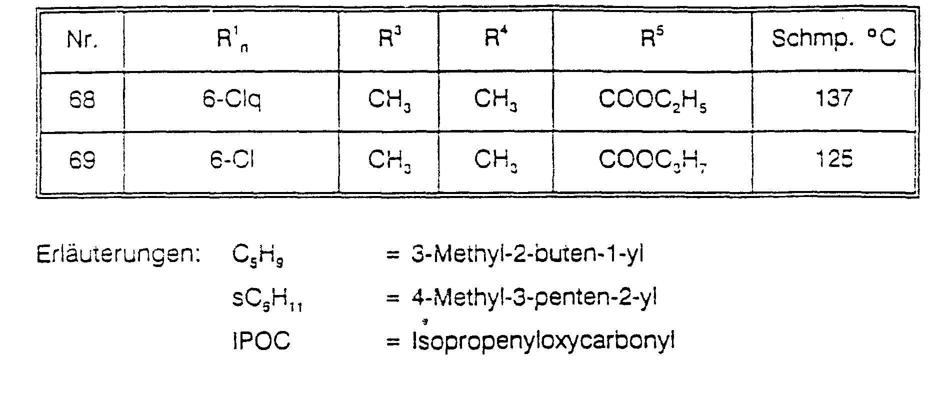

### Beispiel XX

### 3,3-Dimethyl-4-N-(3-methyl-2-buten-1-yl)-3,4-dihydro-chinoxalin- 2(1H)-on

Die Verbindung wurde analog zu der in Beispiel VIA beschriebenen Verbindung ausgehend von 3,3-Dimethyl-3,4-dihydro-chinoxalin- 2(1H)-on (J. T. Lai, Synthesis 1982, 71) hergestellt. Schmp. 146 - 147 °C (nach Kristallisation aus Methyl-tert.-butylether / Heptan)
¹H-NMR (270 MHz, d₆-DMSO): δ = 1.27 (s, 3 H), 1.68 (s, 3 H), 1.72 (s, 3 H), 3.88 (d, J = 7 Hz, 1 H), 5.15 (m, 1 H), 6.60 (d, J = 7 Hz, 1 H), 6.67 (t, J = 7 Hz, 1 H), 6.78 (d, J = 7 Hz, 1 H), 6.87 (t, J = 7 Hz, 1 H), 10.33 ppm (br. s, 1 H).
MS: (M + H)⁺ = 245

### Beispiel XXI

### 4-N-(3-Methyl-2-buten-1-yl)-3,4-dihydro-chinoxalin-2(1H)-on-3-spiro-1'-cyclohexan

Die Verbindung wurde analog zu der in Beispiel VIA beschriebenen Verbindung ausgehend von Spiro[cyclohexan-1,3'-(3',4'-dihydro- chinoxalin-(1'H)-on)] (J. T. Lai, Synthesis 1982, 71) hergestellt. Schmp. 82 - 83 °C (nach Kristallisation aus Heptan)
¹H-NMR (270 MHz, d₆-DMSO): δ = 1.25 - 1.75 (m, 10 H), 3.75 (d, J = 6 Hz, 2 H), 5.07 (m, 1 H), 6.7 - 7.0 (m, 4 H), 10.15 ppm (br. s, 1 H).
MS: (M + H)⁺ = 285

### Beispiel XXII

### 4-N-(3-Methyl-2-buten-1-yl)-3,4-dihydro-chinoxalin-2(1H)-thion- 3-spiro-1'-cyclohexan

Die Verbindung des Beispiels XXI (500 mg, 1.8 mmol) wurde unter Argon zusammen mit 370 mg (0.9 mmol) 2,4-Bis-(4-methoxyphenyl)- 1,3-dithia-2,4-diphosphetan-2,4-disulfid (Lawesson's Reagenz) in 10 ml wasserfreiem Toluol für 1.5 h unter Rückfluß erhitzt. Anschließend wurde im Vakuum eingeengt und die Produkte durch Chromatographie an Kieselgel mit Methyl-tert.-butylether / Heptan = 10 : 1 als Elutionsmittel isoliert. Die Ausbeute betrug 50 mg (9 %) gelbe Kristalle vom Schmp. 125 °C.
¹H-NMR (270 MHz, d6-DMSO): δ = 1.1 - 1.9 (m, 16 H), 3.64 (d, J = 7 Hz, 2 H), 4.99 (m, 1 H), 6.95 - 7.1 (m, 3 H), 7.18 (d, J = 7 Hz, 1 H), 12.2 ppm (br. s, 1 H).
MS: (M + H)⁺ = 301

Als weiteres Produkt wurde 3,4-Dihydro-chinoxalin-2(1H)-thion-3-spiro-1'-cyclohexan mit einer Ausbeute von 110 mg (26 %) isoliert; gelbe Kristalle vom Schmp. 178 °C. ¹H-NMR (270 MHz, CDCl₃): δ = 1.25 - 2.2 (m, 10 H), 4.18 (br. s, 1 H), 6.7 - 6.8 (m, 3 H), 6.97 (m, 1 H), 9.42 ppm (br. s, 1H). MS: (M + H)⁺ = 233.

### Beispiel XXIII

### (3S)-6-Chlor-4-N-(isopropenyloxycarbonyl)-3-methyl-3,4-dihydrochinoxalin-2(1H)-thion

Die Verbindung des Beispiels XIII (0.5 g, 1.78 mmol) gelöst in 10 ml wasserfreiem Pyridin wurde mit 0.47 g (2.12 mmol) Phosphorpentasulfid für 4 h unter Rückfluß erhitzt. Es wurde im Vakuum eingeengt und an Kieselgel mit Essigsäureethylester/Heptan = 1 : 1 als Elutionsmittel chromatographiert. Man erhielt 0.25 g (47 %) eines gelb kristallinen Feststoffs vom Schmelzpunkt 148 - 150 °C (nach Umkristallisation aus Essigsäureethylester/Heptan).
¹H-NMR (270 MHz, d₆-DMSO): δ = 1.24 (d, J = 7 Hz, 3 H), 1.96 (s, 3 H), 4.8 - 4.9 (m, 2 H), 5.28 (q, J = 7 Hz, 1 H), 7.22 (d, J = 8 Hz, 1 H), 7.30 (dd, J = 8, 2 Hz, 1 H), 7.72 (br. s, 1 H), 12.84 ppm (br. s, 1 H).
MS: (M + H)⁺ = 297.

Die folgenden Verbindungen der Formel I wurden aus den entsprechenden 3,4-Dihydrochinoxalin-2(1H)-onen in analoger Weise synthetisiert:

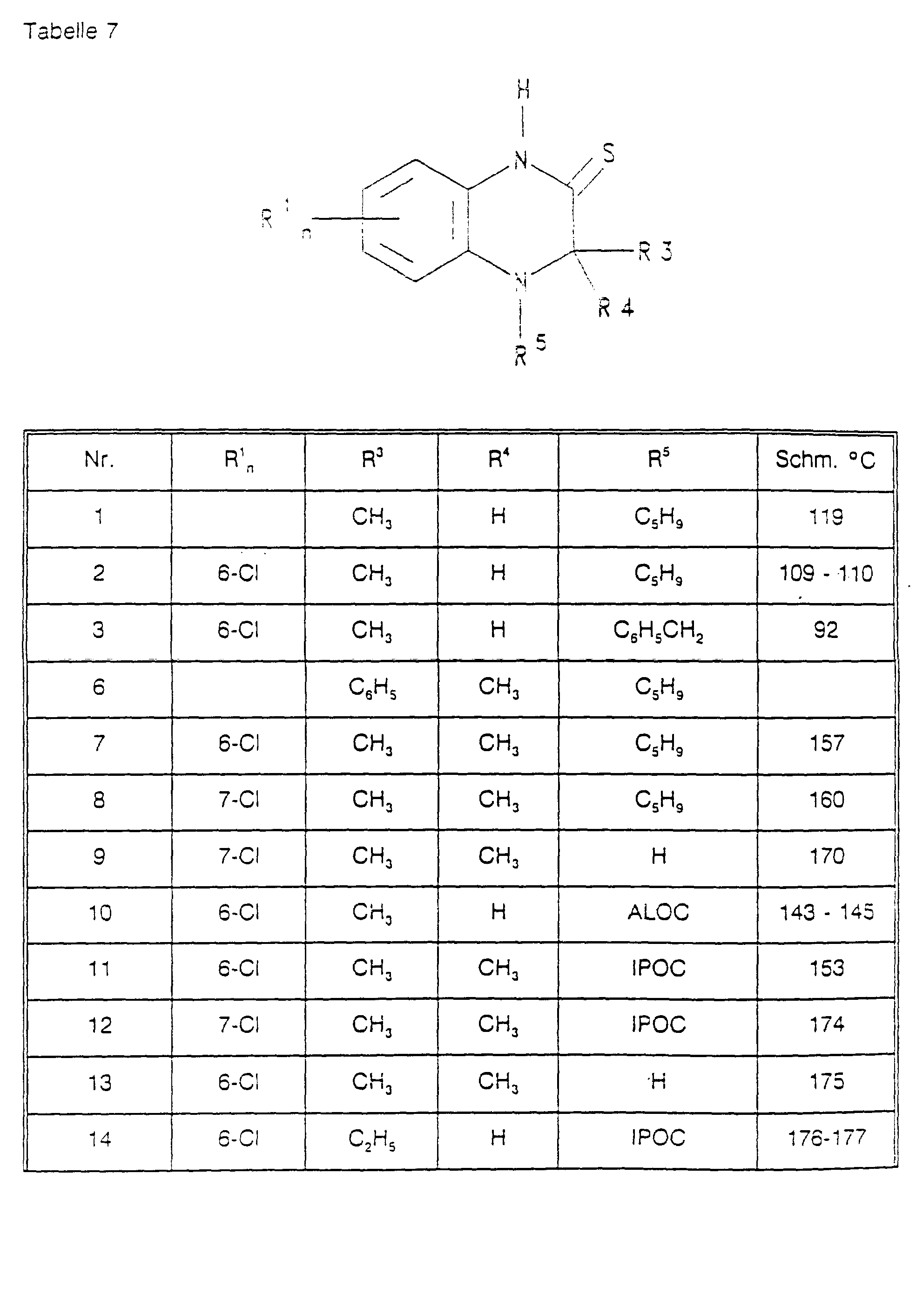

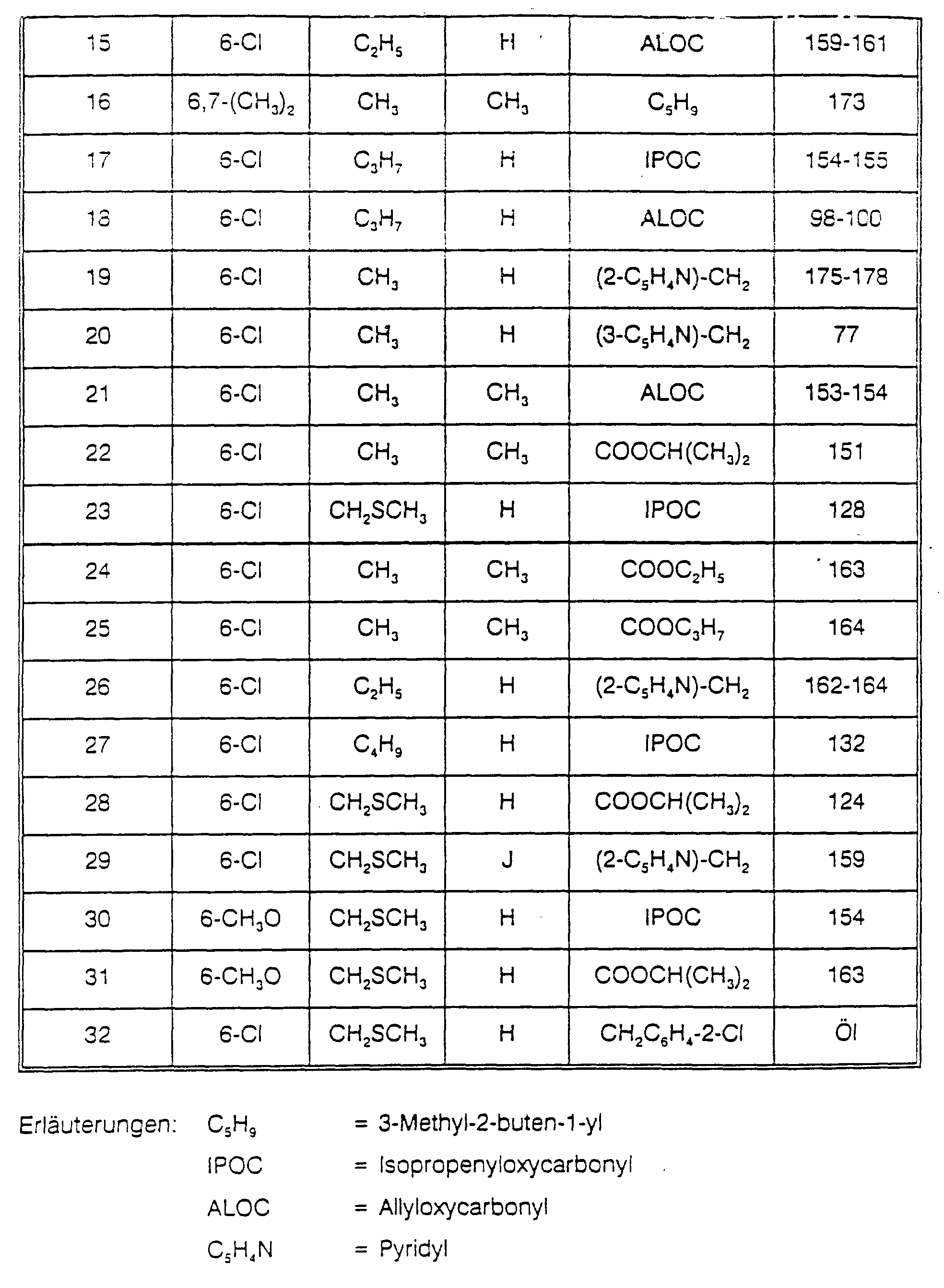

### Beispiel XXIV

### (3RS)-3-Methyl-4-N-(3-methyl-2-buten-1-yl)-2-methylthio-3,4-dihydro-chinoxalin

(3RS)-3-Methyl-4-N-(3-methyl-2-buten-1-yl)-3,4-dihydro-chinoxalin-2(1H)-thion (Tabelle 7, Nr. 1) (0.49 g, 2.0 mmol) wurden in 20 ml Ethanol (96 %) gelöst und mit 5.1 ml (2.2 mmol) einer 1 %igen Natriumethanolat-Lösung versetzt. Nach 15 min Rühren bei Raumtemperatur wurden 0.14 ml (2.2 mmol) Methyljodid zugetropft und es wurde für weitere 2 h bei Raumtemperatur gerührt. Die Reaktionslösung wurde eingeengt und der Rückstand an Kieselgel chromatographiert. Mit Essigsäureethylester / Heptan = 1 : 6 wurden 500 mg (96 %) eines gelben Öls isoliert.
¹H-NMR (d₆-DMSO): δ = 0.96 (d, J = 7 Hz, 3 H), 1.72 (s, 6 H), 2.44 (s, 3 H), 3.71 (dd, J = 15, 6 Hz, 1 H), 3.89 (dd, J = 15, 6 Hz, 1 H), 4.00 (q, J = 7 Hz, 1 H), 5.20 (m, 1 H), 6.65 - 6.75 (m, 2 H), 7.02 (t, J = 8 Hz, 1 H), 7.11 ppm (d, J = 8 Hz, 1 H).
MS: (M + H)⁺ = 261

Auf gleiche Weise wurde die folgende Verbindung der Formel I synthetisiert:

4-Isopropenyloxycarbonyl-2-(isopropenyloxycarbonyl)-thio-3,3,7,8-tetramethyl-3,4-dihydrochinoxalin.
Schmp.: 115 °C

### Beispiel XXV

### (3RS)-3-Methyl-4-N-(3-methyl-2-buten-1-yl)-3,4-dihydrochinoxalin-2(1H)-on

(3RS)-3-Methyl-3,4-dihydro-chinoxalin-2(1H)-on (4.86 g, 0.03 mol) gelöst in 50 ml N,N-Dimethylformamid wurde mit 4.2 ml (0.033 mol) 3-Methyl-2-buten-1-ylbromid (90 %-ig) in Gegenwart von 4.60 g (0.033 mol) gepulvertem Kaliumcarbonat alkyliert. Das Reaktionsgemisch wurde bei Raumtemperatur gerührt bis alles Edukt umgesetzt war. Dann wurde das Lösungsmittel im Vakuum abgezogen, der Rückstand in Essigsäureethylester und Wasser aufgenommen, die Phasen getrennt, die wäßrige zweimal mit Essigsäureethylester extrahiert und die vereinigten organischen Extrakte zweimal mit Wasser gewaschen. Trocknen über Natriumsulfat, Einengen im Vakuum und Kristallisieren aus Pentan ergab 5.80 g (84 %) weiß kristallines Produkt vom Schmp. 92 - 93 °C.
¹H-NMR (270 MHz, d₆-DMSO): δ = 0.99 (d, J = 7 Hz, 3 H), 1.72 (s, 6 H), 3.67 (dd, J = 15, 7 Hz, 1 H), 3.86 (q, J = 7 Hz, 1 H), 3.28 (dd, J = 15, 7 Hz, 1 H), 5.21 (m, 1 H), 6.65 - 6.9 (m, 4 H), 10.31 ppm (br. s, 1 H).
MS: (M + H)⁺ = 231

### Beispiel XXVI

### 3,3a-Dihydropyrrolo[1,2-a]chinoxalin-1,4(2H,5H)-dion

Man erhitzte 2-Fluornitrobenzol (14.1 g, 0.1 mol) und L- Glutaminsäure (45.0 g, 0.3 Mol) in 100 ml 2-Methoxyethanol unter Rühren auf 95 °C und tropfte 300 ml 2N Natronlauge zu. Anschließend wurde noch 3 h bei dieser Temperatur weitergerührt. Nach dem Abkühlen wurde die Lösung mit 400 ml Methanol versetzt und mit Raney-Nickel als Katalysator bei Normaldruck hydriert.
Nach beendeter Wasserstoffaufnahme saugte man den Katalysator ab und engte die Lösung unter vermindertem Druck ein.
Der Rückstand wurde mit 250 ml 2N Salzsäure angesäuert und ca. 30 Minuten auf dem Dampfbad erwärmt. Die dabei entstehende Fällung wurde abgesaugt, mit Wasser und Alkohol gewaschen und anschließend getrocknet, Schmp. 255 °C Zers..
¹H-NMR (60 MHz, d₆-DMSO): δ = 1.9 - 2.7 (m, 4 H), 4.5 (t, J = 8 Hz, 1 H), 6.8 - 7.3 (m, 3 H), 7.8 -8.2 (m, 1 H), 10.7 ppm (br. s, 1 H).
MS: M⁺ = 202

### Beispiel XXVII

### 7-Phenoxysulfonyl-3,3a-dihydropyrrolo[1,2-a]chinoxalin-1,4(2H,5H)-dion

Die Verbindung wurde in analoger Weise durch Umsetzung von 4- Chlor-3-nitrobenzolsulfonsäurephenylester mit L-Glutaminsäure erhalten, Schmp. 140 °C (Zers.).
¹H-NMR (60 MHz, d₆-DMSO): δ = 1.6 - 2.5 (m, 4 H), 4.07 (t, J = 6 Hz, 1 H), 6.7 - 7.6 (m, 8 H), 10.57 ppm (br. s, 1 H).
MS: M⁺ = 358

### Beispiel XXVIII

### 3-Carboxymethyl-3,4-dihydro-chinoxalin-2(1H)-on

Man erhitzte 2-Fluornitrobenzol (14.1 g, 0.1 mol) und L- Asparaginsäure (40.0 g, 0.3 Mol) in 100 ml 2-Methoxyethanol unter Rühren auf 95 °C und tropfte 300 ml 2N Natronlauge zu. Anschließend wurde noch 1 h bei dieser Temperatur weitergerührt. Nach dem Abkühlen wurde die Lösung mit 500 ml Methanol versetzt und mit Raney-Nickel als Katalysator bei Normaldruck hydriert.
Nach beendeter Wasserstoffaufnahme saugte man den Katalysator ab und engte die Lösung unter vermindertem Druck ein.
Der Rückstand wurde mit 500 ml 2N Salzsäure angesäuert, anschließend eingeengt, mit Natriumacetat abgestumpft und mit Essigsäureethylester extrahiert. Nach Trocknen mit Natriumsulfat und Abziehen des Lösungsmittels erhielt man einen zunächst öligen Rückstand, der beim Verrühren mit Wasser kristallisierte, Schmp. 152 -154 °C.
¹H-NMR (60 MHz, d₆-DMSO): δ = 2.5 - 2.7 (dd tw. verdeckt, 2 H), 4.1 (td, J = 6, 2 Hz, 1 H), 5.98 (br. s, 1 H), 6.5 - 6.9 (m, 4 H), 10.30 (br. s, 1 H), 12.37 ppm (br. s, 1 H).
MS: M⁺ = 206
- CHN-Analyse:: berechnet C 58.2; H 4.8; N 13.6 %,
gefunden C 58.4; H 4.7; N 13.7 %

### Beispiel XXIX

### 7-Phenoxysulfonyl-3,4-dihydro-chinoxalin-2(1H)-on

### A) N-[(2-Nitro-4-phenoxysulfonyl)phenyl]glycinmethylester

4-Chlor-3-nitrobenzolsulfonsäurephenylester (62.7 g, 0.2 mol) und Glycinmethylester Hydrochlorid (100.4 g, 0.8 mol), gelöst in 250 ml Methanol, wurden mit 200 ml Triethylamin versetzt und 15 min unter Rückfluß erhitzt. Nach dem Abkühlen wurde mit 1 I 2N Essigsäure versetzt, abgesaugt und mit Wasser gewaschen. Der Rückstand wurde aus Essigsäureethylester umkristallisiert und mit Methanol und Diisopropylether gewaschen, Schmp. 120 -123 °C.

### B) 7-Phenoxysulfonyl-3,4-dihydro-chinoxalin-2(1H)-on

N-[(2-Nitro-4-phenoxysulfonyl)phenyl]glycinmethylester (36.6 g, 0.1 mol) wurden in einem Gemisch aus 250 ml N,N- Dimethylformamid und 250 ml Methanol mit Raney-Nickel als Katalysator unter Normaldruck hydriert. Nach Beendigung der Wasserstoffaufnahme wurde der Katalysator abgesaugt und die Lösung im Vakuum vom Lösungsmittel befreit. Der Rückstand wurde in 40 ml 2-Methoxyethanol gelöst und eine Stunde auf dem Dampfbad erwärmt. Die entstehende Fällung wurde abgesaugt und mit Methanol gewaschen, Schmp. 253 - 254 °C.
¹H-NMR (60 MHz, d₆-DMSO): δ = 4.0 (d, J = 4 Hz, 2 H), 6.6 - 7.6 (m, 9 H), 10.43 ppm (br. s, 1 H).
MS: (M+H)⁺ = 305

### Beispiel XXX

### 4-(3-Methyl-2-buten-1-yl)-7-phenoxysulfonyl-3,4-dihydro-chinoxalin-2(1H)-on

7-Phenoxysulfonyl-3,4-dihydro-chinoxalin-2(1H)-on (1.52 g, 5.0 mmol) wurde in 20 ml N,N-Dimethylacetamid mit 2 ml 3-Methyl-2- buten-1-ylbromid 8 h bei 100 °C gerührt. Nach dem Abkühlen wurde mit Wasser versetzt und mit Essigsäureethylester extrahiert. Nach Trocknen mit Magnesiumsulfat wurde die Lösung eingeengt und über eine Kieselgelsäule mit Essigsäureethylester / Heptan = 1 : 1 chromatographiert. Die substanzhaltigen Fraktionen wurden einrotiert, anschließend mit Pentan verrührt und abgesaugt, Schmp. 132 °C.
¹H-NMR (270 MHz, d₆-DMSO): δ = 1.73 (s, 6 H), 3.90 (s, 2 H), 3.93 (tw. verdecktes d, J = 6 Hz, 2 H), 5.20 (br. t, J = 6 Hz, 1 H), 6.75 - 7.45 (m, 8 H), 10.66 ppm (s, 1 H).
MS: (M+H)⁺ = 373

Die folgenden Verbindungen der Formel I wurden unter Verwendung der entsprechenden Halogenaromaten und Aminosäurederivate in analoger Weise synthetisiert und gegebenenfalls am Stickstoffatom-4 weiter derivatisiert:

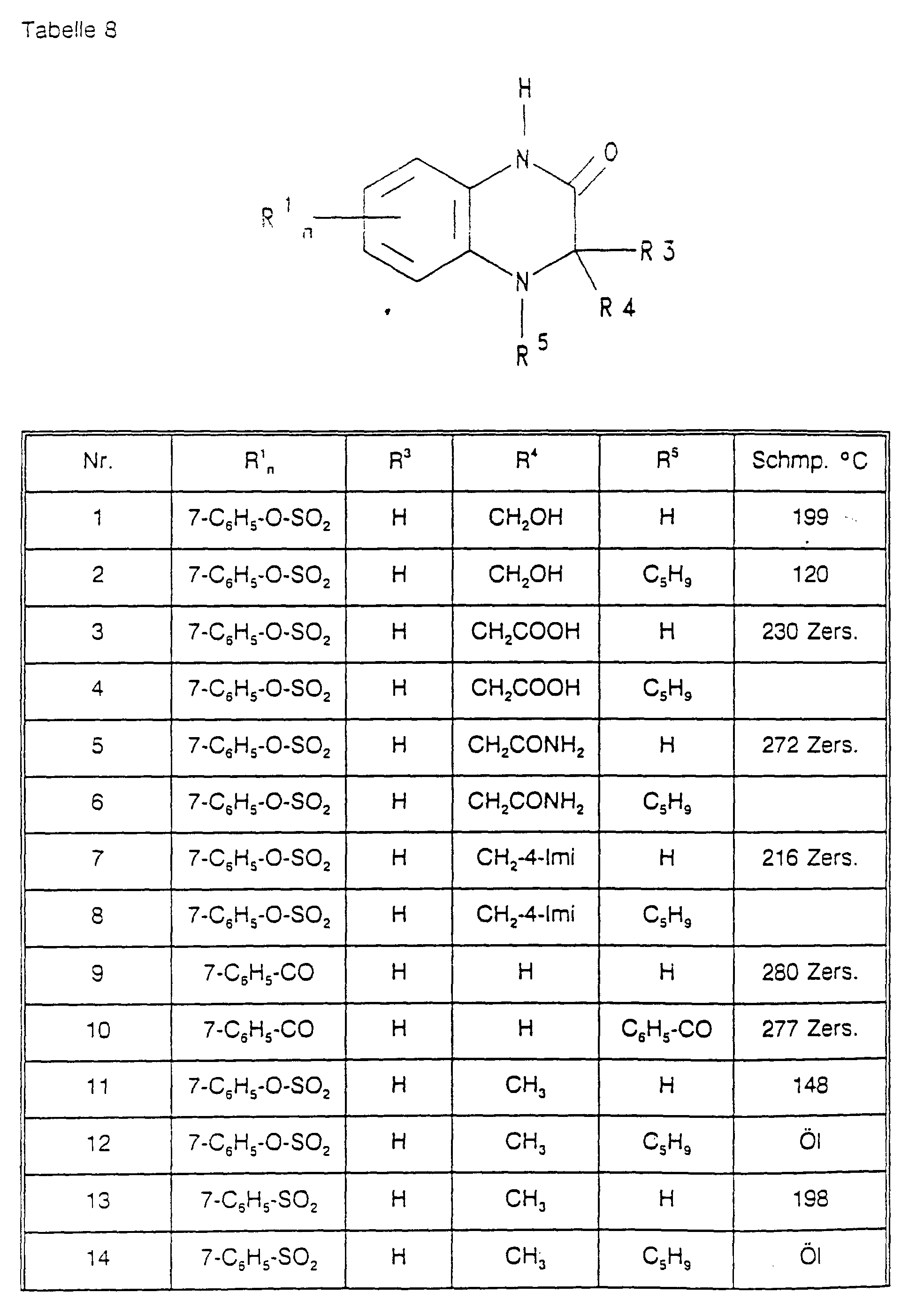

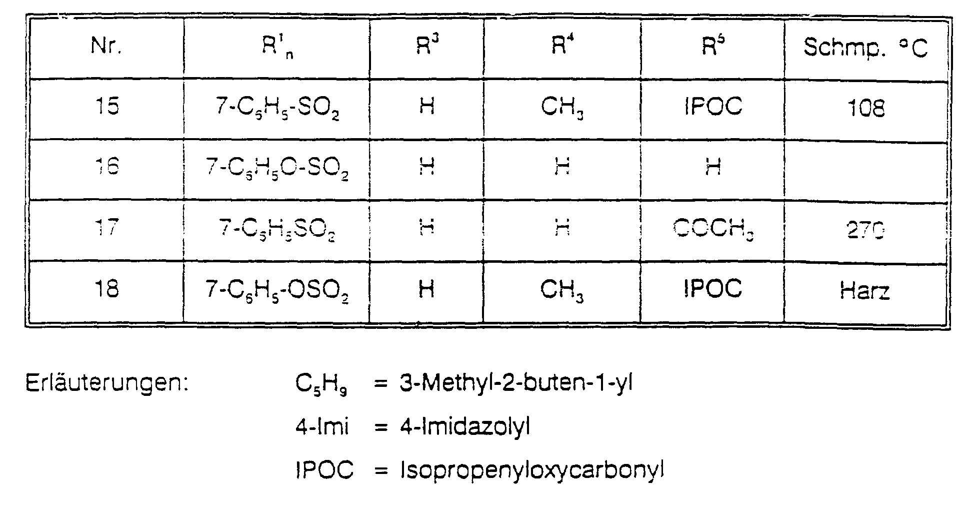

### Beispiel XXXI

### 6-Chlor-7-phenoxysulfonyl-1,2,3,3a-tetrahydropyrrolo[2,1-c]-chinoxalin-4(5H)-on

### A) 2,4-Dichlor-3-nitrobenzolsulfonsäurephenylester

2,6-Dichlornitrobenzol wurde mit einem Überschuß Chlorsulfonsäure 7 h bei 130 °C gerührt. Nach dem Abkühlen goß man auf Eis, saugte das Sulfochlorid ab, wusch neutral und trocknete über Natriumhydroxid, Schmp. 91 °C. Das erhaltene Sulfochlorid (29.05 g, 0.1 mol) und Phenol (11.5 g, 0.12 mol) wurden in 150 ml Aceton gelöst und bei 10 °C mit 14 ml Triethylamin versetzt. Man rührte 1 h unter Kühlung und weitere 4 h bei Raumtemperatur nach, versetzte dann mit 200 ml Wasser, saugte die entstandene Fällung bei 10 °C ab, wusch mit Wasser und trocknete im Vakuum bei 80 °C, Schmp. 102 °C.

### B) N-[(3-Chlor-2-nitro-4-phenoxysulfonyl)phenyl]prolin

2,4-Dichlor-3-nitrobenzolsulfonsäurephenylester (34.8 g, 0.1 mol), 69.0 g (0.6 mol) L-Prolin, 200 ml 2N Natronlauge und 200 ml 2-Methoxyethanol wurden 10 min bei 80 °C gerührt. Die klare Lösung wurde bei 50 °C mit konzentrierter Salzsäure angesäuert und auf Eis gegossen. Die Fällung wurde abgesaugt, mit Wasser neutral gewaschen und bei 80 °C getrocknet. Schmp. 148 °C (nach Umkristallisation aus Methanol)

### C) 6-Chlor-7-phenoxysulfonyl-1,2,3,3a-tetrahydropyrrolo[2,1c]-chinoxalin-4(5H)-on

N-[(3-Chlor-2-nitro-4-phenoxysulfonyl)phenyl]prolin (38.0 g, 0.075 mol) wurde in 500 ml Methanol und 25 ml conc. Ammoniaklösung mit Raney-Nickel als Katalysator unter Normaldruck hydriert.
Nach beendeter Wasserstoffaufnahme wurde der Katalysator abgesaugt und die Lösung eingeengt, mit 2N Salzsäure etwa 30 min auf dem Dampfbad erwärmt, abgekühlt, abgesaugt und mit Wasser neutral gewaschen. Schmp. 197 °C (nach Umkristallisation aus Eisessig)

### Beispiel XXXII

### 8-(4-Methyl-1-piperazinyl)-3-(2-methylpropyl)-5-phenoxysulfonyl-3,4-dihydrochinoxalin-2(1H)-on

### A) 2-Chlor-4-(4-methyl-1-piperazinyl)-3-nitrobenzolsulfonsäure- phenylester

2,4-Dichlor-3-nitrobenzolsulfonsäurephenylester (17.4 g, 0.05 mol) und 25 ml Methylpiperazin wurden in 100 ml Isopropanol 10 min unter Rückfluß erhitzt und anschließend eingeengt. Der Rückstand wurde mit 50 ml 50 %igem Methanol verrührt, abgesaugt, mit 50 %igem Methanol und zuletzt mit Wasser gewaschen. Schmp. 94 -95 °C (nach Umkristallisation aus Cyclohexan)

### B) N-[(3-(4-Methyl-1-piperazinyl)-2-nitro-6-phenoxysulfonyl)- phenyl]leucin Hydrochlorid

2-Chlor-4-(4-methyl-1-piperazinyl)-3-nitrobenzolsulfonsäure- phenylester (41.1 g, 0.1 mol) und L-Leucin (39.3 g, 0.3 mol) wurden in einem Gemisch aus 100 ml N,N-Dimethylformamid, 50 ml 2-Methoxyethanol und 100 ml 2N Natronlauge 8 h bei 95 °C gerührt. Das erkaltete Reaktionsgemisch wurde mit conc. Salzsäure angesäuert. Die Fällung wurde in Essigsäureethylester aufgenommen, mit Natriumsulfat getrocknet und im Vakuum vom Lösungsmittel befreit. Es resultierte ein orangefarbenes Öl.

### C) 8-(4-Methyl-1-piperazinyl)-3-(2-methylpropyl)-5-phenoxysulfonyl-3,4-dihydro-chinoxalin-2(1H)-on Hydrochlorid

N-[(3-(4-Methyl-1-piperazinyl)-2-nitro-6-phenoxysulfonyl)- phenyl]leucin Hydrochlorid (25.3 g, 0.05 mol) wurde in 250 ml Methanol und 25 ml Eisessig mit Raney-Nickel als Katalysator unter Normaldruck hydriert.
Nach beendeter Wasserstoffaufnahme wurde der Katalysator abgesaugt und die Lösung eingeengt, mit 2N Salzsäure etwa 10 min auf dem Dampfbad erwärmt und dann im Vakuum eingeengt. Der Rückstand wurde in Wasser gelöst, mit Ammoniak alkalisch gestellt und in Essigsäureethylester aufgenommen. Das nach dem Einengen verbleibende Öl wurde in 400 ml Diisopropylether gelöst und mit ethanolischer Salzsäure neutralisiert. Die Fällung wurde abgesaugt, mit Diisopropylether gewaschen und getrocknet, Schmp. ab 90 °C (Zers.).
MS: M⁺ = 458

Die folgenden Verbindungen der Formel I wurden unter Verwendung der entsprechenden Halogenaromaten und Aminosäurederivate in analoger Weise synthetisiert und gegebenenfalls am Stickstoffatom-4 weiter derivatisiert:

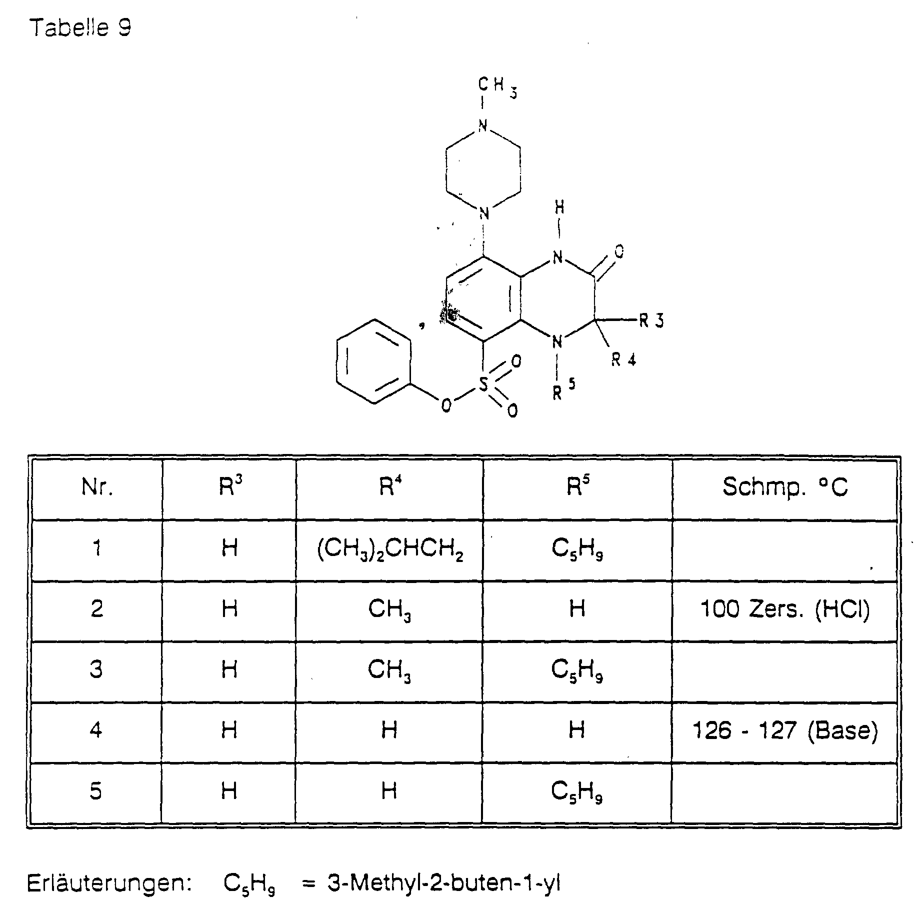

### Beispiel XXXIII

### (3RS)-4-N-Cyclohexyl-3-methyl-3,4-dihydrochinoxalin-2(H)-on

(3RS)-3-Methyl-3,4-dihydrochinoxalin-2(1H)-on (0,81 g, 0,005 mol) und 1 ml (0,1 mol) Cyclohexanon wurden in 20 ml 1,2-Dichlorethan vorgelegt. Trifluoressigsäure (1,9 ml, 0,025 mol) wurde zugetropft, wobei unter leichtem Erwärmen eine klare Lösung entstand. Nach Zugabe von 2,1 g (0,01 mol) Ntriumtriacetoxyborhydrid ließ man die exotherme Reaktion 30 min rühren und quenchte dann durch Zugabe von gesättigter wäßriger Natriumhydrogencarbonatlösung. Die Phasen wurden getrennt, die organische Phase mit gesättigter wäßriger Natriumchloridlösung nachgewaschen, getrocknet (Magnesiumsulfat) und eingeengt. Das Rohprodukt wurde an Kieselgel mit Essigsäureethylester/Heptan = 1 : 1 chromatrographiert. Man erhielt 1,15 g (94 %) des gewünschten Produkts, Schmp. 131 - 132 °C (Toluol/Heptan).
¹H-NMR (270 MHz, d₆-DMSO): δ = 0.97 (d, = 7 Hz, 3 H), 1.0 - 2.0 (m, 10 H), 3.39 (m, 1 H), 3.91 (q, J = 7 Hz, 1 H), 6.68 - 6.94 (m, 4 H), 10.27 ppm (br. s, 1 H). MS: (M + H)⁺ = 245.

Die folgenden Verbindungen der Formel I wurden in analoger Weise synthetisiert.

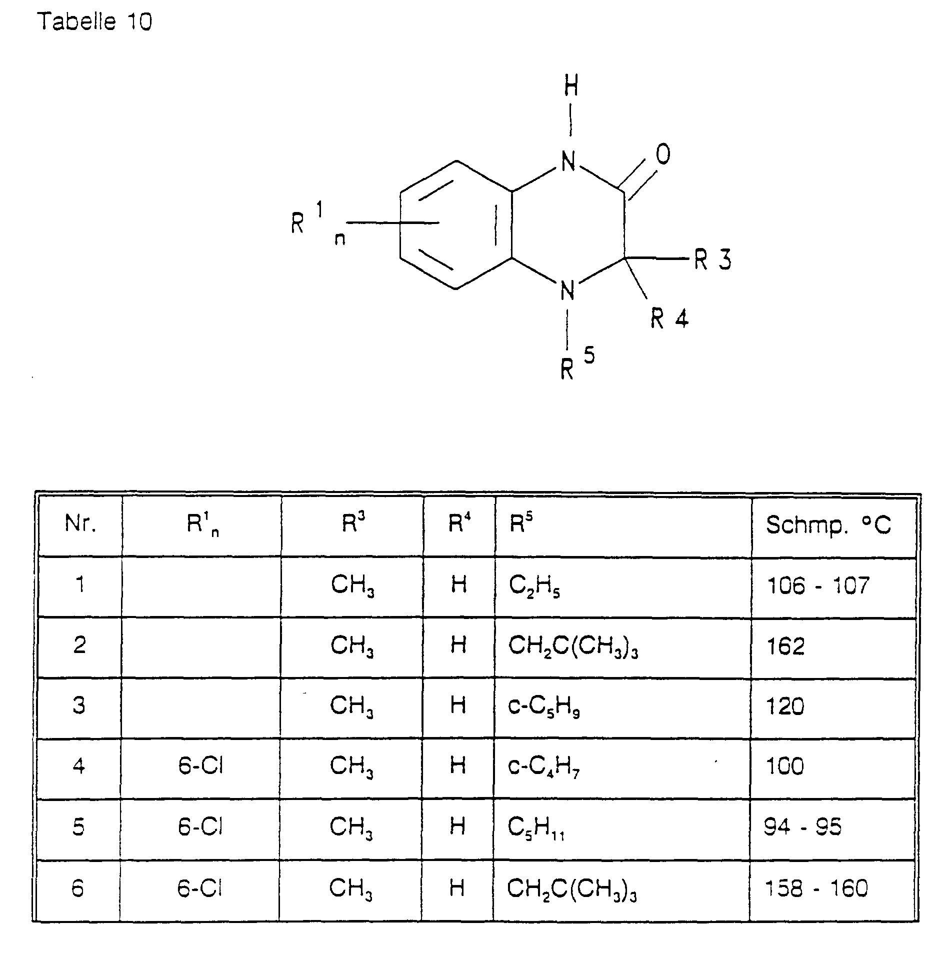

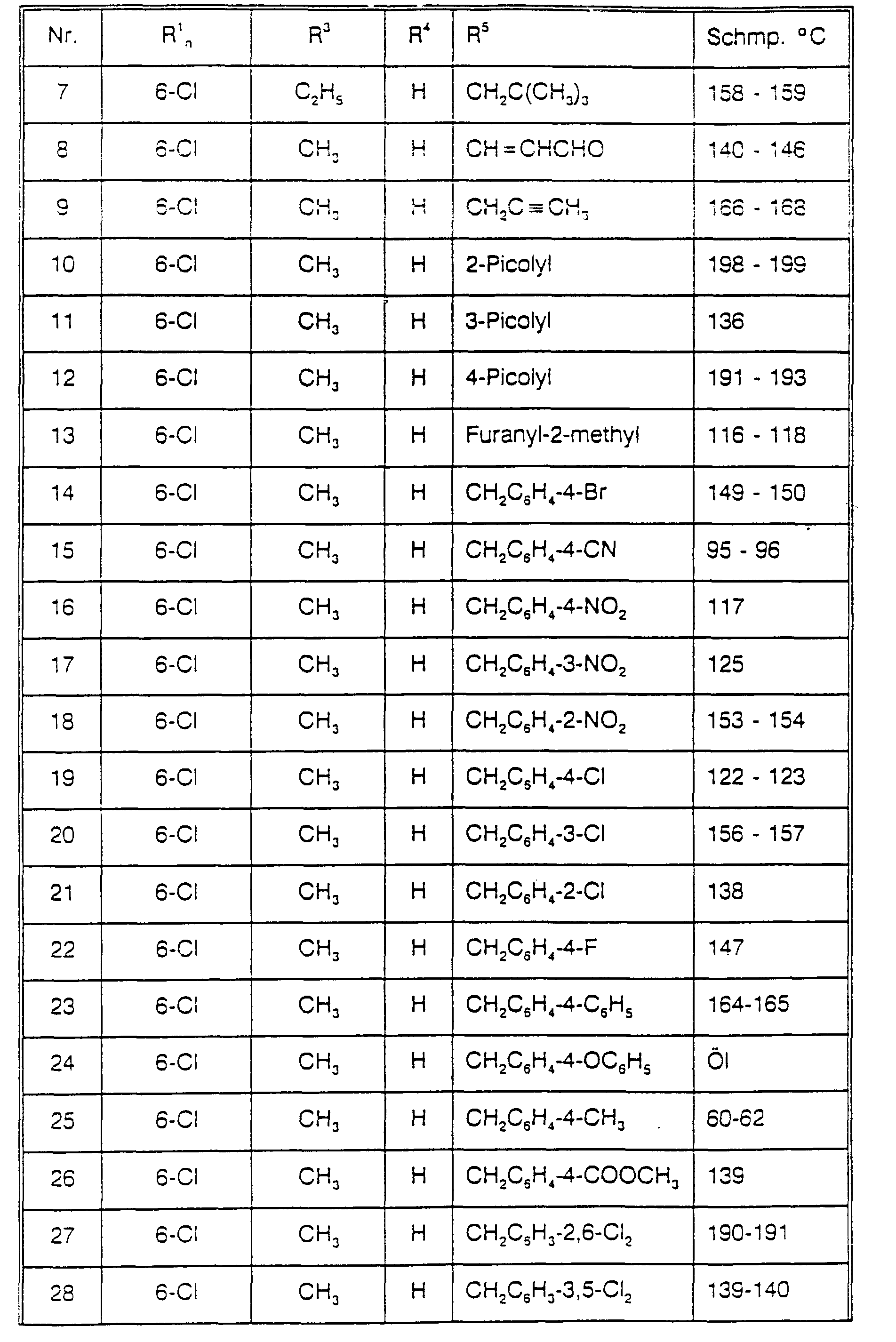

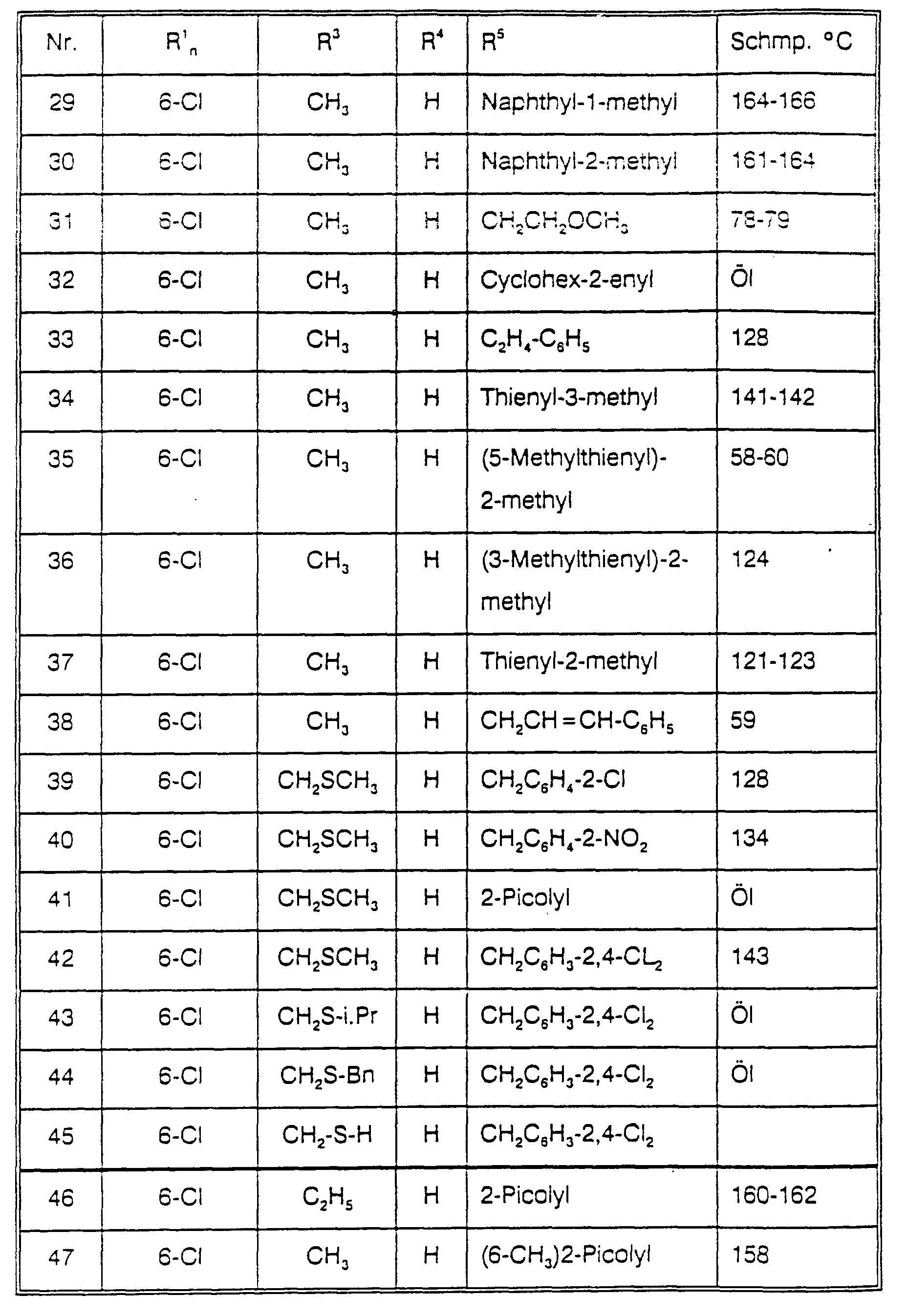

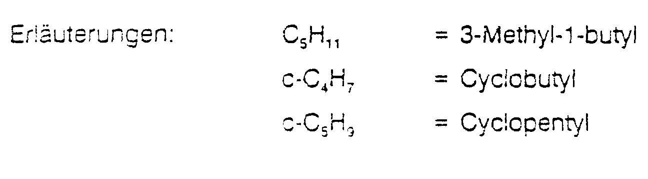

### Beispiel XXXIV

### (3RS)-3-Methyl-4-N-(3-oxo-1-butyl)-3,4-dihydrochinoxalin-2(1H)-on

3-Methyl-3,4-dihydrochinoxalin-2(1H)-on (0,5 g, 3,1 mmol) wurde zusammen mit 0,35 ml (4,3 mmol) Methylvinylketon und einer katalytischen Menge Triethylamin in 20 ml wasserfreiem Ethanol für 20 h bei Raumtemperatur gerührt. Nach Chromatographie an Kieselgel mit Methyl-tert.-butylether/Heptan = 2:1 erhielt man 620 mg (87 %) des gewünschten Produkts, Schmp. 108-109 °C (Methyl-tert.-butylether/Heptan).
¹H.NMR (270 MHz, d₆-DMSO): δ = 1,03 (d, J = 7 Hz, 3 H), 2,11 (s, 3H), 2,77 (t, J = 6 Hz, 2 H), 3,30 (m, 1 H), 3,50 (m, 1 H), 3,88 (q, J = 7 Hz, 1 H), 6,68 (m, 1 H), 6,78 (m, 1 H), 6,88 (m, 1 H), 10,31 ppm (br. s, 1 H).
MS : (M + H)⁺ = 233, M⁺ = 232

### Beispiel XXXV

### (3S)-6-Chlor-4-N-Chlorcarbonyl-3-methyl-3,4-dihydrochinoxalin-2(1H)-on

Die Verbindung des Beispiels IB (2,0 g, 0,01 mol) wurde in 100 ml wasserfreiem Toluol in Gegenwart von 2 ml (0,014 mol) Triethylamin mit Bis-(trichlormethyl)carbonat (Triphosgen) (1,5 g, 0,005 mol) für 1 h auf 80 °C erhitzt. nach dem Abkühlen wurde mit Wasser und gesättigter wäßriger Natriumchloridlösung gewaschen, getrocknet (Magnesiumsulfat) und das Lösungsmittel unter vermindertem Druck entfernt. Der Rückstand (2,5 g) kristallisierte nach Anrühren mit Heptan und war für präparative Zwecke rein genug.
Eine analysenreine Probe erhielt man durch Chromatographie an Kieselgel mit Essigsäureethylester/Heptan = 1 : 1 als Elutionsmittel. Schmp. 142-144 °C.
¹H-NMR (270 MHz, d₆-DMSO): 1,25 (d, J = 7 Hz, 3 H), 3,83 (q, J = 7 Hz, 1 H), 6,61 (dd, J = 6, 2 Hz, 1 H), 6,70 (s, 2H), 10,3 ppm (br. s, 1 H).
MS: (M + H)⁺ = 259

### Beispiel XXXVI

### (3S)-6-Chlor-4-N-(2-methoxyethoxycarbonyl)-3-methyl-3,4-dihydrochinoxalin-2(1H)-on

Zu einer Lösung von 0,24 ml (3,0 mmol) 2-Methoxyethanol in 10 ml wasserfreiem 1,2-Dimethoxyethan wurde 0,16 g einer 55 %igen Suspension von Natriumhydrid in Mineralöl zugegeben und die Reaktionsmischung bei Raumtemperatur für 30 min gerührt. Unter Eiskühlung gab man anschließend 0,50 g (1,9 mmol) der Verbindung des Beispiels XXXV zu, ließ auf Raumtemperatur erwärmen und weitere 30 min rühren. Man versetzte mit gesättigter wäßriger Natriumchloridösung, extrahierte mehrfach mit Essigsäureethylester, wusch die organische Phase einmal mit gesättigter wäßriger Natriumchloridlösung, trocknete (Magnesiumsulfat) und entfernte das Lösungsmittel im Vakuum. Nach Chromatographie an Kieselgel (Essigsäureethylester/Heptan = 1 : 1) und Kristallisation aus Ether/Heptan erhielt man 0,29 g (51 %) des gewünschten Produkts, Schmp. 93-94 °C.
¹H-NMR (200 MHz, d₆-DMSO): δ = 1,13 (d, JJ = 7,5 Hz, 3 H), 3,32 (s, 3 H), 3,6 (m, 2H), 4,24 (m, 1 H), 4,35 (m, 1 H), 4,81 (q, J = 7,5 Hz, 1 H), 6,98 (d, J = 9 Hz), 1 H), 7,2 (dd, J = 9, 3 Hz, 1 H), 7,66 (d, J = 3 Hz, 1 H), 10,81 ppm (br. 2, 1 H).
MS: (M + H)⁺ = 299

### Beispiel XXXVII

### (3S)-6-Chlor-3-methyl-4-N-[(phenylthio)carbonyl)]-3,4-dihydrochinoxalin-2(1H)-on

Zu einer Lösung von 0,31 ml (3,0 mmol) Thiophenol in 10 ml 1,2-Dimethoxyethan wurden unter Eiskühlung 0,17 g einer 55 %-igen Suspension von Natriumhydrid in Mineralöl zugegeben und 1 h bei Raumtemperatur gerührt. Wieder unter Eiskühlung wurden 0,5 g (1,9 mmol) der Verbindung des Beispiels XXXV eingetragen und anschließend 2 h bei Raumtemperatur nachgerührt. Zur Aufarbeitung wurde mit gesättigter wäßriger Natriumchloridlösung versetzt, zweimal mit Essigsäureethylester extrahiert, getrocknet (Natriumsulfat) und das Lösungsmittel abgezogen. Der feste Rückstand wurde aus Heptan/Isopropanol umkristallisiert.
0,35 g (35 %), Schmp. 194 - 195 °C.
¹H-NMR (200 MHz, d₆-DMSO): δ = 1,10 (d, J = 7 Hz, 3 H), 4,93 (q, J = 7 Hz, 1 H), 7,08 (d, J = 9 Hz, 1 H), 7,33 (dd, J = 9,3 Hz, 1 H), 7,4 - 78,6 (m, 5 H), 7,78 (d, J = 3 Hz, 1 H), 10,16 ppm (br. s, 1H).
MS: (M + H)⁺ = 333, (M - C₆H₅SH + H)⁺ 223

Die folgenden Verbindungen der Formel I wurden in analoger Weise synthetisiert.

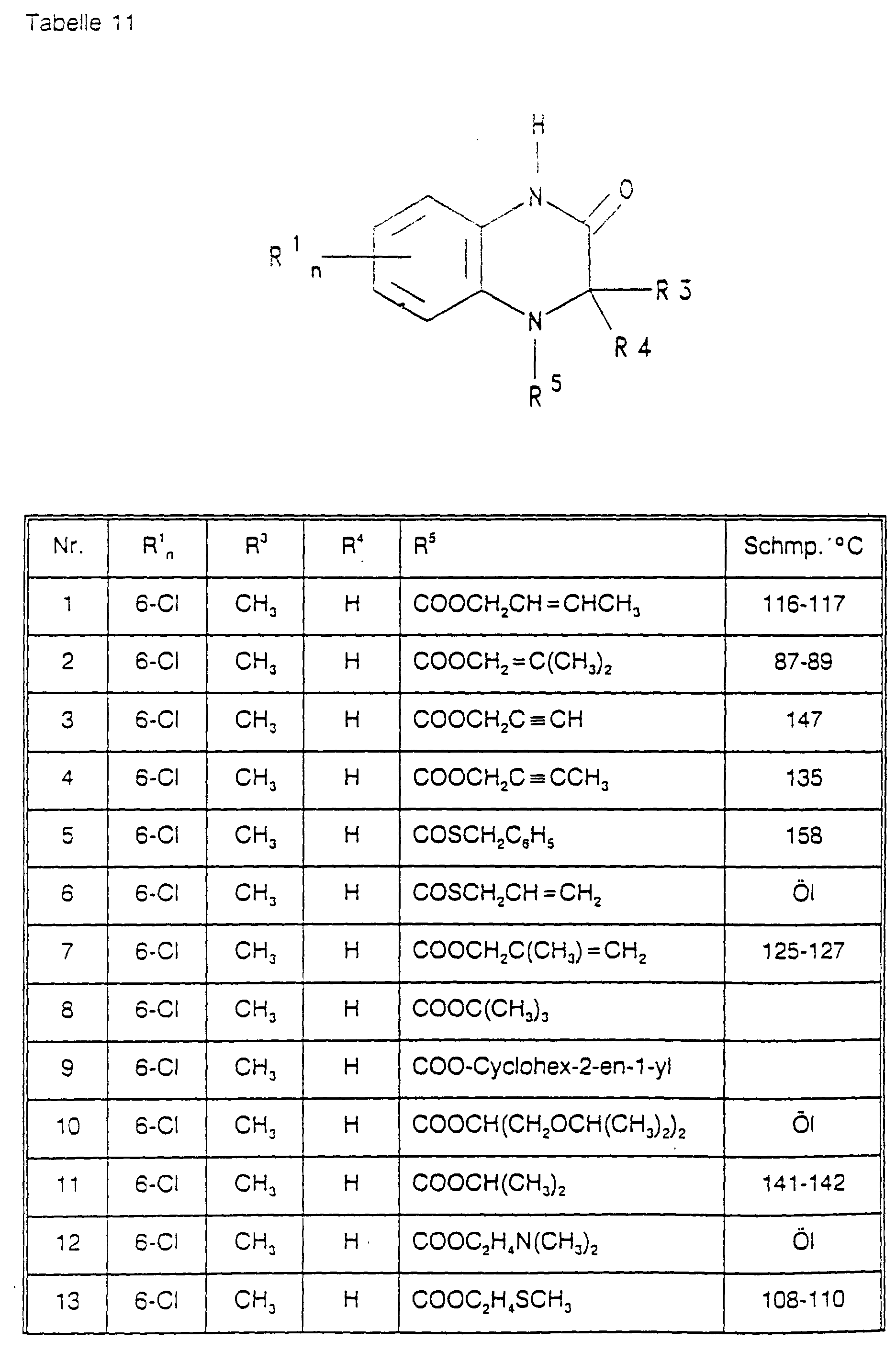

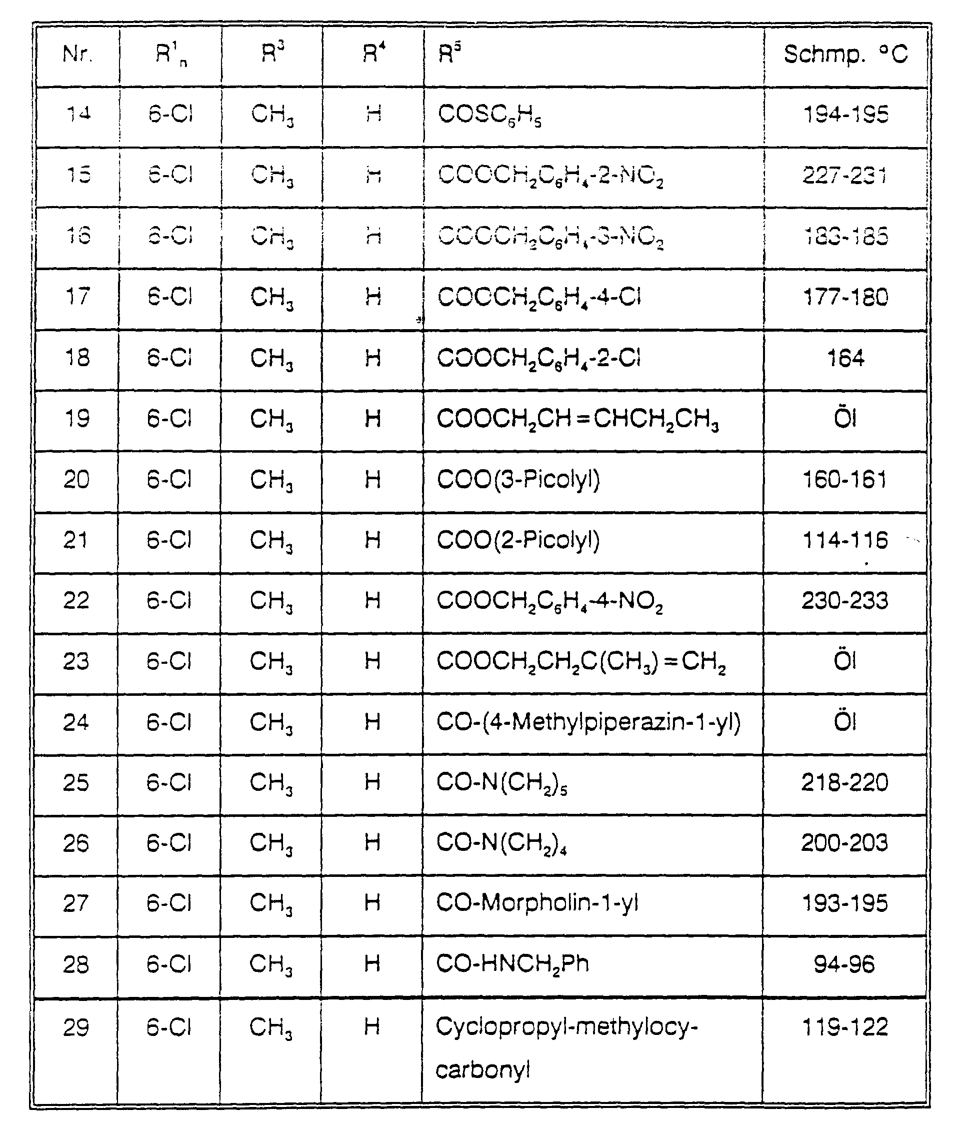

## Patentansprüche (Patentansprüche für folgende(n) Vertragsstaat(en): AT, BE, CH, DE, DK, FR, GB, IT, LI, LU, NL, PT, SE)

1. Verbindung der Formeln I und la, 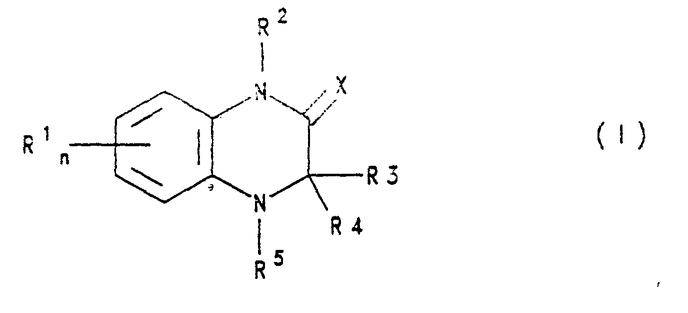 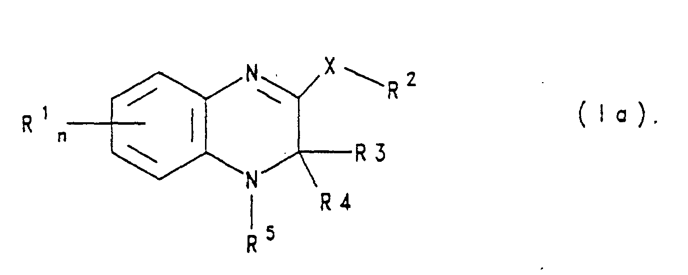 sowie deren physiologisch verträgliche Salze,
wobei in den Formeln I und la bedeuten
n null,
eins
oder zwei,
die einzelnen Substituenten R¹ unabhängig voneinander
Fluor, Chlor, Brom, Trifluormethyl, Hydroxy, C1 - C4-Alkyl, C1 - C4-Alkoxy, (C1 - C4-Alkoxy)-(C1 - C4-alkoxy), C1- C4-Alkylthio, Nitro, Amino, C1 - C4-Alkylamino, Di(C1 - C4-alkyl)amino, Piperidino, Morpholino, 1-Pyrrolidinyl, 4-Methylpiperazinyl, C1 - C4-Acyl, C1 - C4-Acyloxy, C1 - C4-Acylamino, Cyano, Carbamoyl, Carboxy, (C1 - C4-Alkyl)-oxycarbonyl, Hydroxysulfonyl, Sulfamoyl
oder
einen mit bis zu zwei
voneinander unabhängigen Resten R⁶ substituierten Phenyl-, Phenoxy-, Phenylthio-, Phenylsulfonyl-, Phenoxysulfonyl-, Benzoyl-, 2-Pyridyl-, 3-Pyridyl- oder 4-Pyridylrest,
wobei R⁶
Fluor, Chlor, Brom, Cyano, Trifluormethyl, Nitro, Amino, C1 - C4-Alkyl, C1 - C4-Alkoxy, (C1 - C4-Alkyl)-oxycarbonyl, Phenyl, Phenoxy
sein kann,
R² Wasserstoff und R⁵
C1 - C6-Alkyl,
gegebenenfalls substituiert mit Fluor,
Chlor, Hydroxy, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, Oxo, Thioxo, Carboxy, Carbamoyl;
C2 - C6-Alkenyl,
gegebenenfalls substituiert mit Fluor,
Chlor, Hydroxy, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, Oxo, Thioxo, Carboxy, Carbamoyl;
Allenyl,
C3 - C8-Alkinyl,
gegebenenfalls substituiert mit Fluor,
Chlor, Hydroxy, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio. Oxo, Thioxo, Carboxy, Carbamoyl;
C3 - C8-Cycloalkyl,
gegebenenfalls substituiert mit Fluor,
Chlor, Hydroxy, C1 - C4-Alkyl, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, Oxo, Thioxo, Carboxy, Carbamoyl;
C3 - C8-Cycloalkenyl,
gegebenenfalls substituiert mit Fluor,
Chlor, Hydroxy, C1 - C4-Alkyl, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, Oxo, Thioxo, Carboxy, Carbamoyl;
(C3 - C6-Cycloalkyl)-(C1 - C2-alkyl),
gegebenenfalls substituiert mit Fluor,
Chlor, Hydroxy, C1 - C4-Alkyl, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, Oxo, Thioxo, Carboxy, Carbamoyl;
(C3 - C6-Cycloalkenyl)-(C1 - C2-alkyl),
gegebenenfalls substituiert mit Fluor,
Chlor, Hydroxy, C1 - C4-Alkyl, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, Oxo, Thioxo, Carboxy, Carbamoyl;
C1 - C6-Alkylcarbonyl,
gegebenenfalls substituiert mit Fluor,
Chlor, Hydroxy, C1 - C4-Alkyl, C1 - C4-Acyloxy, Benzoyloxy, Benzyloxy, Phenoxy, C1 - C4-Alkoxy, C1 - C4-Alkylamino, C1 - C4-Alkenylamino, Di(C1 - C4-alkyl)amino, C1 - C4-Alkylthio, Oxo, Thioxo, Carboxy, Carbamoyl;
C2 - C6-Alkenylcarbonyl, gegebenenfalls substituiert durch Fluor, Chlor oder Hydroxy;
(C3 - C6-Cycloalkyl)carbonyl,
(C5 - C6-Cycloalkenyl)carbonyl,
(C3 - C6-Cycloalkyl)-(C1 - C2-alkyl)carbonyl,
(C5 - C6-Cycloalkenyl)-(C1 - C2-alkyl)carbonyl,
C1 - C6-Alkyloxycarbonyl, gegebenenfalls substituiert durch Fluor, Chlor, Brom, Hydroxy, C1 - C4-Alkoxy, C1 - C4-Alkylamino, Di(C1 - C4-alkyl)amino, C1 - C4-Alkylthio;
C2 - C6-Alkenyloxycarbonyl, gegebenenfalls substituiert durch Fluor, Chlor, Hydroxy, C1 - C4-Alkoxy;
C2 - C6-Alkinyloxycarbonyl, gegebenenfalls substituiert durch Fluor, Chlor, Hydroxy, C1 - C4-Alkoxy;
C1 - C6-Alkylthiocarbonyl,gegebenenfalls substituiert durch Fluor, Chlor, Hydroxy, C1 - C4-Alkoxy;
C2 - C6-Alkenylthiocarbonyl, gegebenenfalls substituiert durch Fluor, Chlor, Hydroxy, C1 - C4-Alkoxy;
C1 - C6-Alkylamino- und Di(C1 - C6-alkyl)aminocarbonyl, gegebenenfalls substituiert durch Fluor, Chlor, Hydroxy, C1 - C4-Alkoxy;
Pyrrolidin-1-yl, Morpholino-, Piperidino-, Piperazinyl-, oder 4-Methylpiperazin-1-yl-carbonyl;
C2 - C6-Alkenylamino- und Di(C1 - C6-alkenyl)aminocarbonyl, gegebenenfalls substituiert durch Fluor, Chlor, Hydroxy, C1 - C4-Alkoxy;
C1 - C4-Alkylsulfonyl, gegebenenfalls substituiert durch Fluor, Chlor, Hydroxy, C1 - C4-Alkoxy;
C1 - C4-Alkenylsulfonyl;
oder mit bis zu zwei voneinander unabhängigen Resten R⁶ substituiertes Aryl, Arylcarbonyl, (Arylthio)carbonyl, Aryloxycarbonyl, Arylaminocarbonyl, (Arylamino)thiocarbonyl, Arylsulfonyl, Arylalkylaminocarbonyl, Arylalkyl, Arylalkenyl, Arylalkoxycarbonyl, Aryl(alkylthio)carbonyl, wobei der Alkylrest jeweils 1 bis 3 C-Atome enthalten kann und R⁶ wie oben definiert ist
oder mit bis zu zwei voneinander unabhängigen Resten R⁶ substituiertes 1- oder 2-Naphthylmethyl, 2-, 3- oder 4-Picolyl, 2- oder 3-Furylmethyl, 2- oder 3-Thienylmethyl, 2- oder 3-Pyrrolylmethyl,
2-, 3- oder 4-Pyridylcarbonyl, 2- oder 3-Furylcarbonyl, 2- oder 3-Thienylcarbonyl, 2-, 3- oder 4-Picolyloxycarbonyl, 2- oder 3-Furylmethyloxycarbonyl, 2- oder 3-Thienylmethyloxycarbonyl,
und
R³ und R⁴ gleich oder verschieden, unabhängig voneinander
Wasserstoff, C1 - C4-Alkyl, gegebenenfalls substituiert mit Fluor, Chlor, Hydroxy, Amino, Mercapto, C1 - C4-Acyloxy, Benzoyloxy, Phenoxy, C1 - C4-Alkoxy, C1 - C4-Alkylamino, Di(C1 - C4-alkyl)amino, C1 - C4-Alkylthio, C1 - C4-Alkylsulfonyl, C1 - C4-Alkylsulfinyl, Carboxy, Carbamoyl;
C2 - C6-Alkenyl, gegebenenfalls substituiert mit Fluor oder Chlor;
C3 - C6-Cycloalkyl, gegebenenfalls substituiert mit Fluor, Chlor, Hydroxy, Amino, Mercapto, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, C1 - C4-Alkylsulfonyl, C1 - C4-Alkylsulfinyl, Carboxy, Carbamoyl;
C3 - C8-Cycloalkenyl, gegebenenfalls substituiert mit Fluor oder Chlor;
mit bis zu zwei voneinander unabhängigen Resten R⁶ substituiertes Aryl, Benzyl, Heteroaryl oder Heteroarylmethyl bedeuten,
R³ und R⁴ können ferner auch
Teil eines gesättigten oder ungesättigten carbo- oder heterocyclischen Ringes mit 3 bis 6 C-Atomen sein, der gegebenenfalls mit Fluor, Chlor, Hydroxy, Amino, C1 - C4-Acyloxy, Benzoyloxy, C1 - C4-Alkoxy, Oxo, Thioxo, Carboxy, Carbamoyl substituiert sein kann, und
die in den vorausgegangenen Definitionen genannten Arylgruppen sind aromatische Gruppen mit 6-14 C-Atomen, die in den vorausgegangenen Definitionen genannten heterocyclischen Ringe bzw. Heteroarylgruppen enthalten 1-13 C-Atome und 1-6 Heteroatome ausgewähit aus der Gruppe O, S und N, wobei im Falle eines an dieser Stelle gesättigten N-haltigen Ringes N - Z vorliegt, worin Z H oder R⁵ mit den jeweiligen oben beschriebenen Definitionen bedeutet,
X bedeutet Sauerstoff oder Schwefel
mit Ausnahme der Verbindungen in denen R³ und R⁴ gleichzeitig H bedeuten
und Verbindungen, in denen R⁵ COCHCl₂ bedeuten und Verbindungen, in denen n 1, R¹, R³ und R⁴ Methyl und R⁵ Acetyl bedeuten und Verbindungen, in denen n Null, R³ und R⁴ Methyl oder Phenyl und R⁵ Ethyloxycarbonyl bedeuten.

2. Verbindungen der Formel I oder la gemäß Anspruch 1, **dadurch gekennzeichnet, daß** die genannten Substituenten bedeuten:
n null,
eins
oder zwei,
die einzelnen Substituenten R¹ unabhängig voneinander
Fluor, Chlor, Brom, Trifluormethyl, Hydroxy, C1 - C4-Alkyl, C1 - C4-Alkoxy, (C1 - C4-Alkoxy)-(C1 - C2-alkoxy), C1- C4-Alkylthio, Nitro, Amino, C1 - C4-Alkylamino, Di(C1 - C4-alkyl)amino, Piperidino, Morpholino, 1-Pyrrolidinyl, 4-Methylpiperazinyl, C1 - C4-Acyl, C1 - C4-Acyloxy, C1 - C4-Acylamino, Cyano, Carbamoyl, Carboxy, (C1 - C4-Alkyl)-oxycarbonyl, Hydroxysulfonyl, Sulfamoyl
oder
einen mit bis zu zwei
voneinander unabhängigen Resten R⁶ substituierten Phenyl-, Phenoxy-, Phenylthio-, Phenylsulfonyl-, Phenoxysulfonyl-, Benzoyl-, 2-Pyridyl-, 3-Pyridyl- oder 4-Pyridylrest,
wobei R⁶
Fluor, Chlor, Brom, Cyano, Trifluormethyl, Nitro, Amino, C1 - C4-Alkyl, C1 - C4-Alkoxy, (C1 - C4-Alkyl)-oxycarbonyl, Phenyl, Phenoxy
sein kann,
R² Wasserstoff und R⁵
C1 - C6-Alkyl
gegebenenfalls substituiert mit C1 - C4-Alkoxy oder C1 - C4-Alkylthio;
C2 - C6-Alkenyl,
gegebenenfalls substituiert mit Oxo;
Allenyl;
C3 - C8-Alkinyl, insbesondere 2-Butinyl;
C3 - C6-Cycloalkyl;
C5 - C6-Cycloalkenyl;
(C3 - C6-Cycloalkyl)-(C1 - C2-alkyl), insbesondere Cyclopropylmethyl, gegebenenfalls substituiert mit C1 - C4-Alkyl;
(C3 - C6-Cycloalkenyl)-(C1 - C2-alkyl), insbesondere Cyclohexenylmethyl;
C1 - C6-Alkylcarbonyl,
gegebenenfalls substituiert mit Fluor,
Chlor, Hydroxy, Benzyloxy, Phenoxy, C1 - C4-Alkoxy, C1 - C4-Alkylamino, C1 - C4-Alkenylamino, Di(C1 - C4-alkyl)amino, C1 - C4-Alkylthio;
C2 - C6-Alkenylcarbonyl;
C1 - C6-Alkyloxycarbonyl, gegebenenfalls substituiert durch Fluor, Chlor, Brom, Hydroxy, C1 - C4-Alkoxy, C1 - C4-Alkylamino, Di(C1 - C4-alkyl)amino, C1 - C4-Alkylthio;
C2 - C6-Alkenyloxycarbonyl, insbesondere Vinyloxycarbonyl, Allyloxycarbonyl, Isopropenyloxycarbonyl, Butenyloxycarbonyl, Pentenyloxycarbonyl;
C2 - C6-Alkinyloxycarbonyl, insbesondere Propinyloxycarbonyl, Butinyloxycarbonyl;
C1 - C6-Alkylthiocarbonyl;
C2 - C6-Alkenylthiocarbonyl, insbesondere Allylthiocarbonyl;
C1 - C6-Alkylamino- und Di(C1 - C6-alkyl)aminocarbonyl;
Pyrrolidin-1-yl, Morpholino-, Piperidino-, Piperazinyl-, oder 4-Methylpiperazin-1-yl-carbonyl;
C2 - C6-Alkenylamino- und Di(C1 - C6-alkenyl)aminocarbonyl;
C1 - C4-Alkylsulfonyl;
C1 - C4-Alkenylsulfonyl;
oder mit bis zu zwei voneinander unabhängigen Resten R⁶ substituiertes Aryl, insbesondere Phenyl,
Arylcarbonyl, insbesondere Benzoyl, (Arylthio)carbonyl, Aryloxycarbonyl,
Arylaminocarbonyl, (Arylamino)thiocarbonyl, Arylalkylaminocarbonyl,
Arylsulfonyl, Arylalkyl, insbesondere Benzyl, Phenylethyl,
Arylalkenyl, Arylalkylcarbonyl, Arylalkoxycarbonyl,
Aryl(alkylthio)carbonyl, wobei der Alkylrest jeweils 1 bis 3 C-Atome enthalten kann und R⁶ wie oben definiert ist
oder mit bis zu zwei voneinander unabhängigen Resten R⁶ substituiertes 1- oder 2-Naphthylmethyl, 2-, 3- oder 4-Picolyl, 2- oder 3-Furylmethyl, 2- oder 3-Thienylmethyl, 2- oder 3-Pyrrolylmethyl,
2-, 3- oder 4-Pyridylcarbonyl, 2- oder 3-Furylcarbonyl, 2- oder 3-Thienylcarbonyl, 2-, 3- oder 4-Picolyloxycarbonyl, 2- oder 3-Furylmethyloxycarbonyl, 2- oder 3-Thienylmethyloxycarbonyl,
und
R³ und R⁴ gleich oder verschieden, unabhängig voneinander
Wasserstoff,
C1 - C4-Alkyl,
gegebenenfalls substituiert mit Hydroxy, Mercapto,
C1 - C4-Alkoxy, C1 - C4-Alkylthio, C1 - C4-Alkylsulfonyl, C1 - C4-Alkylsulfinyl, Carboxy, Carbamoyl;
C2 - C6-Alkenyl,
mit bis zu zwei voneinander unabhängigen Resten R⁶ substituiertes Aryl, Benzyl, Thienyl oder Thienylmethyl, wobei R⁶ wie oben definiert ist, bedeuten,
R³ und R⁴ können ferner auch
Teil eines gesättigten oder ungesättigten carbo- oder heterocyclischen Ringes mit 3 bis 6 C-Atomen sein, der gegebenenfalls mit Oxo oder Thioxo substituiert sein kann und
die in den vorausgegangenen Definitionen genannten Arylgruppen sind aromatische Gruppen mit 6-14 C-Atomen, die in den vorausgegangenen Definitionen genannten heterocyclischen Ringe bzw. Heteroarylgruppen enthalten 1-13 C-Atome und 1-6 Heteroatome ausgewählt aus der Gruppe O, S und N, wobei im Falle eines an dieser Stelle gesättigten N-haltigen Ringes N-Z vorliegt, worin Z H oder R⁵ mit den jeweiligen oben beschriebenen Definitionen bedeutet.
X bedeutet Sauerstoff oder Schwefel.

3. Verbindungen der Formel I oder la gemäß den Ansprüchen 1 oder 2, **dadurch gekennzeichnet, daß** die genannten Substituenten bedeuten:
n null oder
eins
die einzelnen Substituenten R¹ unabhängig voneinander
Fluor, Chlor, Brom, C1 - C2-Alkyl, C1 - C2-Alkoxy, C2 - C4-Acyl, Cyano
R² Wasserstoff und R⁵
C2-C6-Alkenyl
C3 - C8-Alkinyl, insbesondere 2-Butinyl;
(C3 - C6-Cycloalkyl)-(C1 - C2-alkyl), insbesondere Cyclopropylmethyl, gegebenenfalls substituiert mit C1 - C4-Alkyl;
(C3 - C6-Cycloalkenyl)-(C1 - C2-alkyl), insbesondere Cyclohexenylmethyl;
C2 - C6-Alkylcarbonyl,
C2 - C6-Alkenylcarbonyl;
C1 - C6-Alkyloxycarbonyl;
C2 - C6-Alkenyloxycarbonyl, insbesondere Vinyloxycarbonyl, Allyloxycarbonyl, Isopropenyloxycarbonyl, Butenyloxycarbonyl, Pentenyloxycarbonyl;
C2 - C6-Alkinyloxycarbonyl, insbesondere Propinyloxycarbonyl, Butinyloxycarbonyl;
C2 - C6-Alkenylthiocarbonyl, insbesondere Allylthiocarbonyl;
C1 - C4-Alkylsulfonyl;
C1 - C4-Alkenylsulfonyl;
oder mit bis zu zwei voneinander unabhängigen Resten R⁶ substituiertes Arylalkyl, insbesondere Benzyl oder Arylalkenyl, wobei der Alkylrest jeweils 1 bis 3 C-Atome und der Alkenylrest 2-3 C-Atome enthalten kann
oder mit bis zu zwei voneinander unabhängigen Resten R⁶ substituiertes 1-Naphthylmethyl, 2- oder 3-Picolyl, 2-Furylmethyl, 2- oder 3-Thienylmethyl,
wobei R⁶
Fluor, Chlor, Brom, Cyano, C1 - C2-Alkyl, C1 - C2-Alkoxy
und
R³ und R⁴ gleich oder verschieden, unabhängig voneinander
Wasserstoff,
C1 - C4-Alkyl,
gegebenenfalls substituiert mit Hydroxy, Mercapto, C1 - C4-Alkoxy, C1 - C2-Alkylthio und X bedeutet Sauerstoff oder Schwefel.

4. Verfahren zur Herstellung von Verbindungen der Formel I gemäß Anspruch 1,
**dadurch gekennzeichnet**,
daß A)
zur Herstellung von Verbindungen der Formel I mit X gleich Sauerstoff und den Resten R¹, R², R³, R⁴ und R⁵ wie in Anspruch 1 definiert eine Verbindung der Formel II, 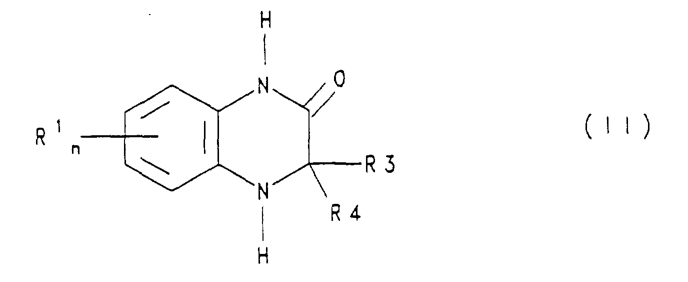 wobei für R¹, R³ und R⁴ die in Anspruch 1 genannten Definitionen gelten, mit einer Verbindung der Formel III,
R-Z (III)
wobei R die in Anspruch 1 genannten Bedeutungen für R⁵ und R² mit Ausnahme von Wasserstoff, Hydroxy, C1 - C6-Alkoxy, Aryloxy, C1 - C6-Acyloxy, Amino, C1 - C6-Alkylamino, Di(C1 - C6-Alkyl)amino, Arylamino, C1 - C6-Acylamino hat und Z eine Abgangsgruppe ist umsetzt
oder
daß B)
Verbindungen der Formel I, mit X gleich Schwefel und R¹, R², R³, R⁴ und R⁵ wie in Anspruch 1 definiert hergestellt werden durch Reaktion einer Verbindung der Formel I, wobei X Sauerstoff ist und für R¹, R², R³, R⁴ und R⁵ die in Anspruch 1 genannten Definitionen gelten, mit einem Schwefelungsreagenz behandelt werden, oder
daß C)
Verbindungen der Formel la, wobei X und die Reste R¹ bis R⁵ wie in Anspruch 1 definiert, hergestellt werden, indem man eine Verbindung der Formel IV, 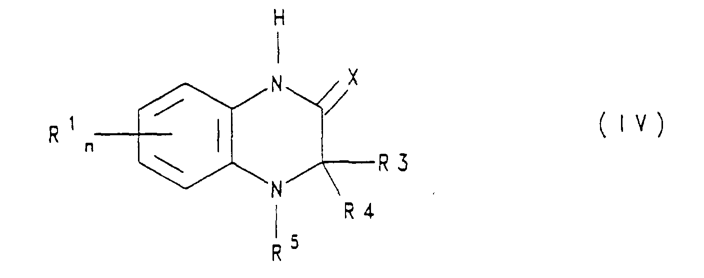 bzw. 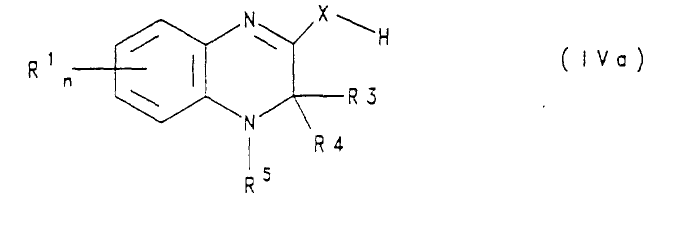 wobei für R¹, R³, R⁴ und R⁵ die in Anspruch 1 genannten Definitionen gelten, mit einer Verbindung der Formel III,
R²-Z (III)
wobei für R² die in Anspruch 1 für Formel I und la beschriebenen Definitionen mit Ausnahme von Wasserstoff, Hydroxy, C1 - C6-Alkoxy, Aryloxy, C1 - C6-Acyloxy, Amino, C1 - C6-Alkylamino, Di(C1 - C6-alkyl)amino, Arylamino, C1 - C6-Acylamino gelten und Z eine Abgangsgruppe ist umsetzt,
oder
daß D)
Verbindungen der Formel I mit X gleich Sauerstoff und den Resten R¹ bis R⁵ wie in Anspruch 1 definiert durch Cyclisierung einer Verbindung der Formel V 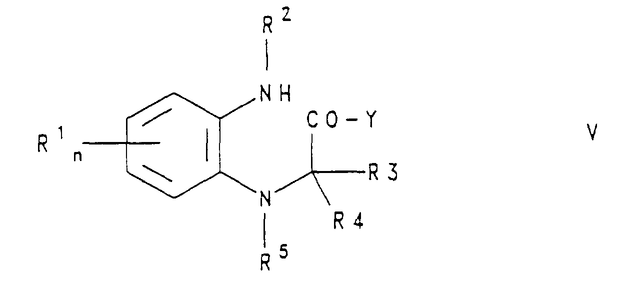 mit R¹ bis R⁵ wie in Anspruch 1 definiert und Y gleich Hydroxy, C1 - C4-Alkoxy, ggf. halogeniertes C1 - C4-Acyloxy, Chlor, Brom oder Jod, hergestellt werden, oder
daß E)
Verbindungen der Formel I, wobei X Sauerstoff ist, R⁴ und R⁵ Wasserstoff sind und für R¹ bis R³ die in Anspruch 1 genannten Definitionen gelten, aus den Chinoxalinonen der Formel XI, 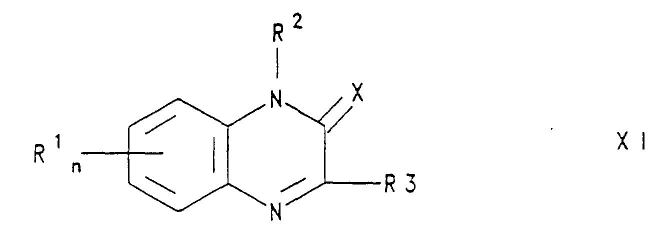 mit R¹ bis R³ wie eingangs definiert, hergestellt werden, indem man an die C =N- Bindung Wasserstoff anlagert,
oder
daß F)
Verbindungen der Formel I, wobei X Sauerstoff und R¹ bis R⁵ wie in Anspruch 1 definiert, hergestellt werden aus Verbindungen der Formel VI, 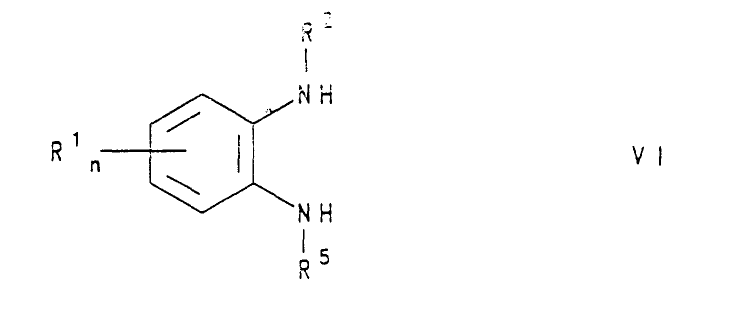 mit R¹, R² und R⁵ wie in Anspruch 1 definiert, durch Umsetzung mit Chloroform oder Bromoform und einer Carbonylverbindung der Formel XIII,
R³-CO-R⁴ (XIII)
mit R³ und R⁴ wie in Anspruch 1 definiert oder mit α-(Trihalogenmethyl)-alkanolen der Formel XIV,
Hal₃C-C(OH)-R³R⁴ (XIV)
worin Hal für Cl, Br oder J steht,
in denen R³ und R⁴ wie eingangs definiert sind,
oder
daß G)
Verbindungen der Formel I, mit X gleich Sauerstoff und R¹, R², R³, R⁴ und R⁵ wie in Anspruch 1 definiert, durch Reaktion einer Verbindung der Formel I, wobei X Sauerstoff ist, für R¹, R², R⁵ sowie für R³ und R⁴ die in Anspruch1 genannten Definitionen gelten, außer daß mindestens einer der Reste R³ oder R⁴ Wasserstoff ist, mit einem Alkylierungsreagenz der Formel XV,
R'-Z (XV)
wobei R' die oben angegebenen Bedeutungen für R³ und R⁴ mit Ausnahme von Wasserstoff hat und Z eine Abgangsgruppe ist, hergestellt werden,
oder
daß H)
Verbindungen der Formel I, mit X gleich Sauerstoff, R¹, R², R³ und R⁴ wie in Anspruch 1 definiert und R⁵ C1 - C6-Alkyl, gegebenenfalls substituiert mit Fluor, Chlor, Hydroxy, C1 - C4-Acyloxy, Benzoyloxy, Phenoxy, C1 - C4-Alkoxy, C1 - C4-Alkylamino, Di(C1 - C4-Alkyl)amino, C1 - C4-Alkylthio, Carboxy, Carbamoyl, C3 - C6-Alkenyl, gegebenenfalls substituiert mit Fluor, Chlor, Hydroxy, C1 - C4-Acyloxy, Benzoyloxy, Phenoxy, C1 - C4-Alkoxy, C1 - C4-Alkylamino, Di(C1 - C4-alkyl)amino, C1 - C4-Alkylthio, Carboxy, Carbamoyl, C3 - C8-Alkinyl, gegebenenfalls substituiert mit Fluor, Chlor, Hydroxy, C1 - C4-Acyloxy, Benzoyloxy, Phenoxy, C1 - C4-Alkoxy, C1 - C4-Alkylamino, Di(C1 - C4-alkyl)amino, C1 - C4-Alkylthio, Carboxy, Carbamoyl, C4 - C8-Cycloalkyl, gegebenenfalls substituiert mit Fluor, Chlor, Hydroxy, C1 - C4-Acyloxy, Benzoyloxy, C1 - C4-Alkoxy, C1 - C4-Alkylamino, Di(C1 - C4-alkyl)-amino, C1 - C4-Alkylthio, Carboxy, Carbamoyl, C5 - C8-Cycloalkenyl, gegebenenfalls substituiert mit Fluor, Chlor, Hydroxy, C1 -C4-Acyloxy, Benzoyloxy, Phenoxy, C1 - C4-Alkoxy, C1 - C4-Alkylamino, Di(C1 - C4-alkyl)amino, C1 - C4-Alkylthio, Carboxy, Carbamoyl, durch reduktive Alkylierung einer Verbindung der Formel I, wobei R⁵ Wasserstoff und X Sauerstoff sind und für R¹, R², R³ und R⁴ die in Anspruch 1 genannten Definitionen gelten mit einer Carbonylverbindung der Formel XVI,
R"-C(=O)-R'" (XVI),
wobei R" und R"' gleich oder verschieden, unabhängig voneinander Wasserstoff, C1 - C5-Alkyl, gegebenenfalls substituiert mit Fluor, Chlor, Hydroxy, C1 - C4-Acyloxy, Benzoyloxy, Phenoxy, C1 - C4-Alkoxy, C1 - C4-Alkylamino, Di(C1 - C4-alkyl)amino, C1 - C4-Alkylthio, Carboxy, Carbamoyl, C3 - C5-Alkenyl, gegebenenfalls substituiert mit Fluor, Chlor, Hydroxy, C1 - C4-Acyloxy, Benzoyloxy, Phenoxy, C1 - C4-Alkoxy, C1 - C4-Alkylamino, Di(C1 - C4-alkyl)amino, C1 - C4-Alkylthio, Carboxy, Carbamoyl, C3 - C7-Alkinyl, gegebenenfalls substituiert mit Fluor, Chlor, Hydroxy, C1 - C4-Acyloxy, Benzoyloxy, Phenoxy, C1 - C4-Alkoxy, C1 - C4-Alkylamino, Di(C1 - C4-alkyl)amino, C1 - C4-Alkylthio, Carboxy, Carbamoyl, C4 - C8-Cycloalkyl, gegebenenfalls substituiert mit Fluor, Chlor, Hydroxy, C1 - C4-Acyloxy, Benzoyloxy, Phenoxy, C1 - C4-Alkoxy, C1 - C4-Alkylamino, Di(C1 - C4-alkyl)amino, C1 - C4-Alkylthio, Carboxy, Carbamoyl, C6-Cycloalkenyl, gegebenenfalls substituiert mit Fluor, Chlor, Hydroxy, C1 - C4-Acyloxy, Benzoyloxy, Phenoxy, C1 - C4-Alkoxy, C1 - C4-Alkylamino, Di(C1 - C4-alkyl)amino, C1 - C4-Alkylthio, Carboxy, und wobei R" und R"' unter Bildung eines 4- bis 8-gliedrigen Ringes miteinander verknüpft sein können, hergestellt werden
oder I Verbindungen der Formel I, mit X gleich Sauerstoff und R¹, R², R³ und R⁴ wie in Anspruch 1 definiert und R⁵ C1 - C6-Alkyloxycarbonyl, C1 - C6-Alkylthiocarbonyl, C2 - C6-Alkenyloxycarbonyl, C2 - C6-Alkenylthiocarbonyl, C2 - C6-Alkinyloxycarbonyl, C1 - C6-Alkylaminocarbonyl, C3 - C6-Alkenylaminocarbonyl, Di(C1 - C6-alkyl)aminocarbonyl, Pyrrolidin-1-yl, Morpholino-, Piperidino-, Piperazinyl-, 4-Methylpiperazin-1-yl-carbonyl gegebenenfalls substituiert mit Fluor, Chlor, Hydroxy, oder mit bis zu zwei voneinander unabhängigen Resten R⁶ substituiertes Aryloxycarbonyl, Arylthio(carbonyl), Arylaminocarbonyl, (Arylalkylthio)carbonyl, Aryalkylaminocarbonyl, wobei der Alkylrest jeweils 1 bis 3 C-Atome enthalten kann, hergestellt werden, indem man eine Verbindung der Formel XVII, 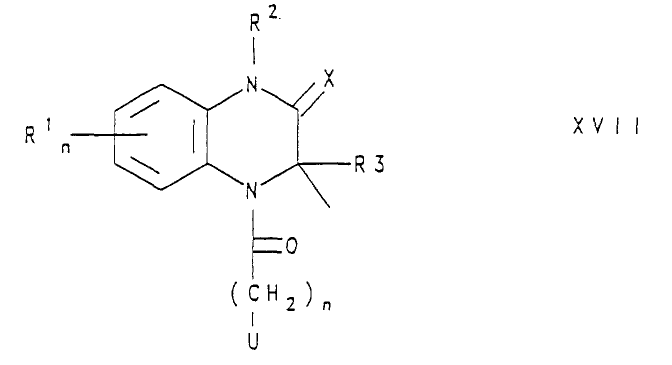 wobei für R¹, R², R³ und R⁴ die in Anspruch 1 genannten Definitionen gelten, n gleich 0, 1, 2 oder 3 ist, X gleich Sauerstoff und U eine Abgangsgruppe ist, mit einer Verbindung der Formel XVIII,
Nu - H (XVIII),
wobei Nu C1 - C6-Alkyloxy, C2 - C6-Alkenyloxy, C2 - C6-Alkinyloxy, C1 - C6-Alkylthio, C2 - C6-Alkenylthio, C1 - C6-Alkylamino- und Di(C1 - C6-Alkyl)amino, C3 - C6-Alkenylamino- und Di(C1 - C6-alkyl)amino, gegebenenfalls substituiert durch Fluor, Chlor, Hydroxy, C1 - C4-Alkoxy, Pyrrolidin-1-yl, Morpholino-, Piperidino-, Piperazinyl-, oder 4-Methylpiperazin-1-yl-, oder mit bis zu zwei voneinander unabhängigen Resten R⁶ (R⁶ ist wie eingangs definiert) substituiertes Aryloxy, Arylthio, Arylamino, Arylalkyloxy, Arylalkylthio, Aryalkylamino, 2-, 3-, oder 4-Pyridyl-, 2- oder 3-Furyl, 2- oder 3-Thienyl, 2-, 3- oder 4-Picolyloxy, 2- oder 3-Furtylmethyloxy, 2- oder 3-Thienylmethyloxy wobei der Alkylrest jeweils 1 bis 3 C-Atome enthalten kann, sein kann, zur Reaktion bringt.

5. Verbindungen der Formel I bzw. la gemäß einem oder mehreren der Ansprüche 1 - 3 zur Anwendung als Arzneimittel.

6. Arzneimittel, enthaltend eine wirksame Menge mindestens einer Verbindung der Formel la gemäß einem oder mehreren der Ansprüche 1 - 3.

7. Verfahren zur Herstellung eines Arzneimittels gemäß Anspruch 6 **dadurch gekennzeichnet, daß** die wirksame Menge einer Verbindung der Formel I bzw. la mit üblichen pharmazeutischen Hilfsstoffen in eine geeignete Darreichungsform gebracht wird.

8. Verwendung von Verbindungen der Formel I bzw. la
gemäß einem oder mehreren der Ansprüche 1 - 3 zur Herstellung von Arzneimitteln zur Behandlung von Krankheiten, die durch Viren verursacht werden, insbesondere von Krankheiten, die durch das HIV verursacht werden.

## Patentansprüche (Patentansprüche für folgende(n) Vertragsstaat(en): ES, GR)

1. Verfahren zur Herstellung von Verbindung der Formeln I und la, 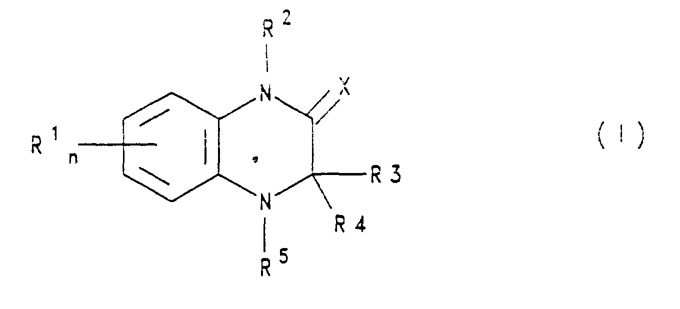 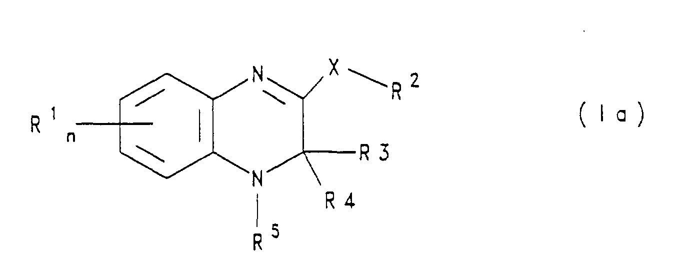 sowie deren physiologisch verträgliche Salze
wobei in den Formeln I und la bedeuten
n null,
eins
oder zwei,
die einzelnen Substituenten R¹ unabhängig voneinander
Fluor, Chlor, Brom, Trifluormethyl, Hydroxy, C1 - C4-Alkyl, C1 - C4-Alkoxy, (C1
- C4-Alkoxy)-(C1 - C4-alkoxy), C1- C4-Alkylthio, Nitro, Amino, C1 - C4-Alkylamino, Di(C1 - C4-alkyl)amino, Piperidino, Morpholino, 1-Pyrrolidinyl, 4-Methylpiperazinyl, C1 - C4-Acyl, C1 - C4-Acyloxy, C1 - C4-Acylamino, Cyano, Carbamoyl, Carboxy, (C1 - C4-Alkyl)-oxycarbonyl, Hydroxysulfonyl, Sulfamoyl
oder
einen mit bis zu zwei
voneinander unabhängigen Resten R⁶ substituierten Phenyl-, Phenoxy-, Phenylthio-, Phenylsulfonyl-, Phenoxysulfonyl-, Benzoyl-, 2-Pyridyl-, 3-Pyridyl- oder 4-Pyridylrest,
wobei R⁶
Fluor, Chlor, Brom, Cyano, Trifluormethyl, Nitro, Amino, C1 - C4-Alkyl, C1 - C4-Alkoxy, (C1 - C4-Alkyl)-oxycarbonyl, Phenyl, Phenoxy
sein kann,
R² Wasserstoff und R⁵
C1 - C6-Alkyl,
gegebenenfalls substituiert mit Fluor,
Chlor, Hydroxy, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, Oxo, Thioxo, Carboxy, Carbamoyl;
C2 - C6-Alkenyl,
gegebenenfalls substituiert mit Fluor,
Chlor, Hydroxy, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, Oxo, Thioxo, Carboxy, Carbamoyl;
Allenyl,
C3 - C8-Alkinyl,
gegebenenfalls substituiert mit Fluor,
Chlor, Hydroxy, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, Oxo, Thioxo, Carboxy, Carbamoyl;
C3 - C8-Cycloalkyl,
gegebenenfalls substituiert mit Fluor,
Chlor, Hydroxy, C1 - C4-Alkyl, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, Oxo, Thioxo, Carboxy, Carbamoyl;
C3 - C8-Cycloalkenyl,
gegebenenfalls substituiert mit Fluor,
Chlor, Hydroxy, C1 - C4-Alkyl, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, Oxo, Thioxo, Carboxy, Carbamoyl;
(C3 - C6-Cycloalkyl)-(C1 - C2-alkyl),
gegebenenfalls substituiert mit Fluor,
Chlor, Hydroxy, C1 - C4-Alkyl, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, Oxo, Thioxo, Carboxy, Carbamoyl;
(C3 - C6-Cycloalkenyl)-(C1 - C2-alkyl),
gegebenenfalls substituiert mit Fluor,
Chlor, Hydroxy, C1 - C4-Alkyl, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, Oxo, Thioxo, Carboxy, Carbamoyl;
C1 - C6-Alkylcarbonyl,
gegebenenfalls substituiert mit Fluor,
Chlor, Hydroxy, C1 - C4-Alkyl, C1 - C4-Acyloxy, Benzoyloxy, Benzyloxy, Phenoxy, C1 - C4-Alkoxy, C1 - C4-Alkylamino, C1 - C4-Alkenylamino, Di(C1 - C4-alkyl)amino, C1 - C4-Alkylthio, Oxo, Thioxo, Carboxy, Carbamoyl;
C2 - C6-Alkenylcarbonyl, gegebenenfalls substituiert durch Fluor, Chlor oder Hydroxy;
(C3 - C6-Cycloalkyl)carbonyl,
(C5 - C6-Cycloalkenyl)carbonyl,
(C3 - C6-Cycloalkyl)-(C1 - C2-alkyl)carbonyl,
(C5 - C6-Cycloalkenyl)-(C1 - C2-alkyl)carbonyl,
C1 - C6-Alkyloxycarbonyl, gegebenenfalls substituiert durch Fluor, Chlor, Brom, Hydroxy, C1 - C4-Alkoxy, C1 - C4-Alkylamino, Di(C1 - C4-alkyl)amino, C1 - C4-Alkylthio;
C2 - C6-Alkenyloxycarbonyl, gegebenenfalls substituiert durch Fluor, Chlor, Hydroxy, C1 - C4-Alkoxy;
C2 - C6-Alkinyloxycarbonyl, gegebenenfalls substituiert durch Fluor, Chlor, Hydroxy, C1 - C4-Alkoxy;
C1 - C6-Alkylthiocarbonyl,gegebenenfalls substituiert durch Fluor, Chlor, Hydroxy, C1 - C4-Alkoxy;
C2 - C6-Alkenylthiocarbonyl, gegebenenfalls substituiert durch Fluor, Chlor, Hydroxy, C1 - C4-Alkoxy;
C1 - C6-Alkylamino- und Di(C1 - C6-alkyl)aminocarbonyl, gegebenenfalls substituiert durch Fluor, Chlor, Hydroxy, C1 - C4-Alkoxy;
Pyrrolidin-1-yl, Morpholino-, Piperidino-, Piperazinyl-, oder
4-Methylpiperazin-1-yl-carbonyl;
C2 - C6-Alkenylamino- und Di(C1 - C6-alkenyl)aminocarbonyl, gegebenenfalls substituiert durch Fluor, Chlor, Hydroxy, C1 - C4-Alkoxy;
C1 - C4-Alkylsulfonyl, gegebenenfalls substituiert durch Fluor, Chlor, Hydroxy, C1 - C4-Alkoxy;
C1 - C4-Alkenylsulfonyl;
oder mit bis zu zwei voneinander unabhängigen Resten R⁶ substituiertes Aryl, Arylcarbonyl, (Arylthio)carbonyl, Aryloxycarbonyl, Arylaminocarbonyl, (Arylamino)thiocarbonyl, Arylsulfonyl, Arylalkylaminocarbonyl, Arylalkyl, Arylalkenyl, Arylalkoxycarbonyl, Aryl(alkylthio)carbonyl, wobei der Alkylrest jeweils 1 bis 3 C-Atome enthalten kann und R⁶ wie oben definiert ist
oder mit bis zu zwei voneinander unabhängigen Resten R⁶ substituiertes
1- oder 2-Naphthylmethyl, 2-, 3- oder 4-Picolyl, 2- oder 3-Furylmethyl, 2- oder 3-Thienylmethyl, 2- oder 3-Pyrrolylmethyl,
2-, 3- oder 4-Pyridylcarbonyl, 2- oder 3-Furylcarbonyl, 2- oder
3-Thienylcarbonyl, 2-, 3- oder 4-Picolyloxycarbonyl, 2-oder 3-Furylmethyloxycarbonyl, 2- oder 3-Thienylmethyloxycarbonyl,
und
R³ und R⁴ gleich oder verschieden, unabhängig voneinander
Wasserstoff, C1 - C4-Alkyl, gegebenenfalls substituiert mit Fluor, Chlor, Hydroxy, Amino, Mercapto, C1 - C4-Acyloxy, Benzoyloxy, Phenoxy, C1 - C4-Alkoxy, C1 - C4-Alkylamino, Di(C1 - C4-alkyl)amino, C1 - C4-Alkylthio, C1 - C4-Alkylsulfonyl, C1 - C4-Alkylsulfinyl, Carboxy, Carbamoyl;
C2 - C6-Alkenyl, gegebenenfalls substituiert mit Fluor oder Chlor;
C3 - C6-Cycloalkyl, gegebenenfalls substituiert mit Fluor, Chlor, Hydroxy, Amino, Mercapto, C1 - C4-Acyloxy, Benzoyloxy, Benzyloxy, Phenoxy, C1 - C4-Alkoxy, C1 - C4-Alkylamino, Di(C1 - C4-alkyl)amino, C1 - C4-Alkylthio, C1 - C4-Alkylsulfonyl, C1 - C4-Alkylsulfinyl, Carboxy, Carbamoyl;
C3 - C8-Cycloalkenyl, gegebenenfalls substituiert mit Fluor oder Chlor;
mit bis zu zwei voneinander unabhängigen Resten R⁶ substituiertes Aryl, Benzyl, Heteroaryl oder Heteroarylmethyl bedeuten,
R³ und R⁴ können ferner auch
Teil eines gesättigten oder ungesättigten carbo- oder heterocyclischen Ringes mit 3 bis 6 C-Atomen sein, der gegebenenfalls mit Fluor, Chlor, Hydroxy, Amino, C1 - C4-Acyloxy, Benzoyloxy, C1 - C4-Alkoxy, Oxo, Thioxo, Carboxy, Carbamoyl substituiert sein kann, und
die in den vorausgegangenen Definitionen genannten Arylgruppen sind aromatische Gruppen mit 6-14 C-Atomen, die in den vorausgegangenen Definitionen genannten heterocyclischen Ringe bzw. Heteroarylgruppen enthalten 1-13 C-Atome und 1-6 Heteroatome ausgewählt aus der Gruppe O, S und N, wobei im Falle eines an dieser Stelle gesättigten N-haltigen Ringes N - Z vorliegt, worin Z H oder R⁵ mit den jeweiligen oben beschriebenen Definitionen bedeutet,
X bedeutet Sauerstoff oder Schwefel
mit Ausnahme der Verbindungen in denen R³ und R⁴ gleichzeitig H bedeuten
und Verbindungen, in denen R⁵ COCHCl₂ bedeuten und Verbindungen, in denen n 1, R¹, R³ und R⁴ Methyl und R⁵ Acetyl bedeuten und Verbindungen, in denen n Null, R³ und R⁴ Methyl oder Phenyl und R⁵ Ethyloxycarbonyl bedeuten,
**dadurch gekennzeichnet**,
daß A)
zur Herstellung von Verbindungen der Formel I mit X gleich Sauerstoff und den Resten R¹, R², R³, R⁴ und R⁵ wie oben definiert eine Verbindung der Formel II, 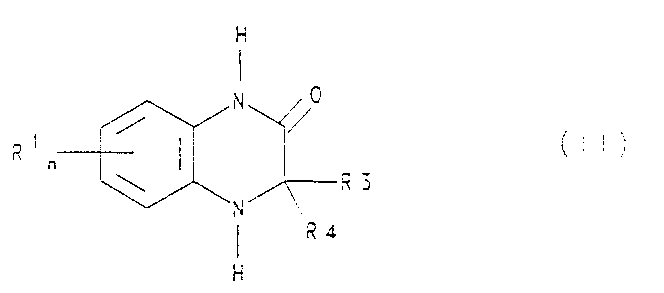 wobei für R¹, R³ und R⁴ die oben genannten Definitionen gelten, mit einer Verbindung der Formel III,
R-Z (III)
wobei R die in Anspruch 1 genannten Bedeutungen für R⁵ und R² mit Ausnahme von Wasserstoff, Hydroxy, C1 - C6-Alkoxy, Aryloxy, C1 - C6-Acyloxy, Amino, C1 - C6-Alkylamino, Di(C1 - C6-Alkyl)amino, Arylamino, C1 - C6-Acylamino hat und Z eine Abgangsgruppe ist umsetzt
oder
daß B)
Verbindungen der Formel I, mit X gleich Schwefel und R¹, R², R³, R⁴ und R⁵ wie in Anspruch 1 definiert hergestellt werden durch Reaktion einer Verbindung der Formel I, wobei X Sauerstoff ist und für R¹, R², R³, R⁴ und R⁵ die in Anspruch 1 genannten Definitionen gelten, mit einem Schwefelungsreagenz behandelt werden, oder
daß C)
Verbindungen der Formel la, wobei X und die Reste R¹ bis R⁵ wie in Anspruch 1 definiert, hergestellt werden, indem man eine Verbindung der Formel IV, 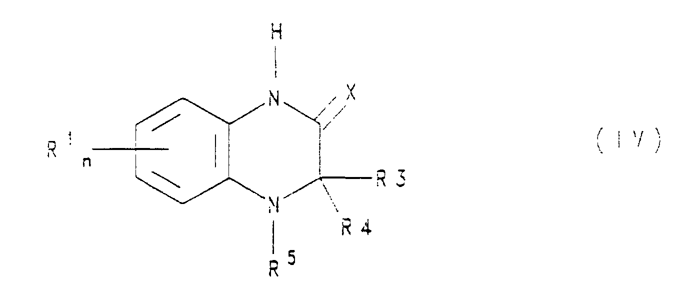 bzw. 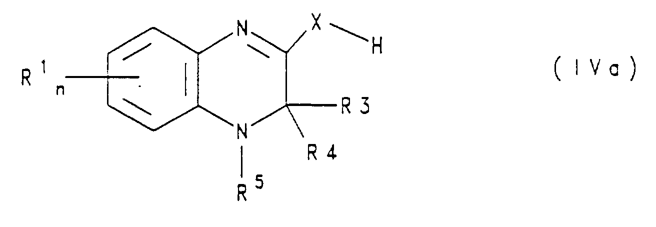 wobei für R¹, R³, R⁴ und R⁵ die in Anspruch 1 genannten Definitionen gelten, mit einer Verbindung der Formel III,
R²-Z (III)
wobei für R² die in Anspruch 1 für Formel I und la beschriebenen Definitionen mit Ausnahme von Wasserstoff Hydroxy, C1 - C6-Alkoxy, Aryloxy, C1 - C6-Acyloxy, Amino, C1 - C6-Alkylamino, Di(C1 - C6-alkyl)amino, Arylamino, C1 - C6-Acylamino gelten und Z eine Abgangsgruppe ist umsetzt,
oder
daß D)
Verbindungen der Formel I mit X gleich Sauerstoff und den Resten R¹ bis R⁵ wie in Anspruch 1 definiert durch Cyclisierung einer Verbindung der Formel V 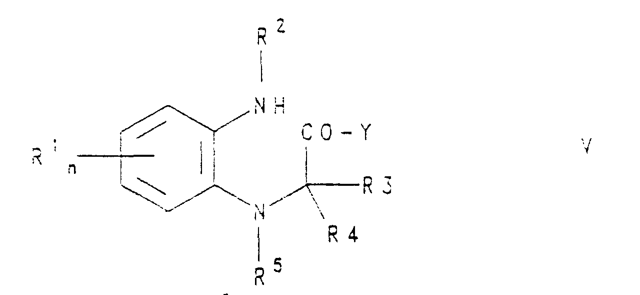 mit R¹ bis R⁵ wie in Anspruch 1 definiert und Y gleich Hydroxy, C1 - C4-Alkoxy, ggf. halogeniertes C1 - C4-Acyloxy, Chlor, Brom oder Jod, hergestellt werden, oder
daß E)
Verbindungen der Formel I, wobei X Sauerstoff ist, R⁴ und R⁵ Wasserstoff sind und für R¹ bis R³ die in Anspruch 1 genannten Definitionen gelten, aus den Chinoxalinonen der Formel XI, 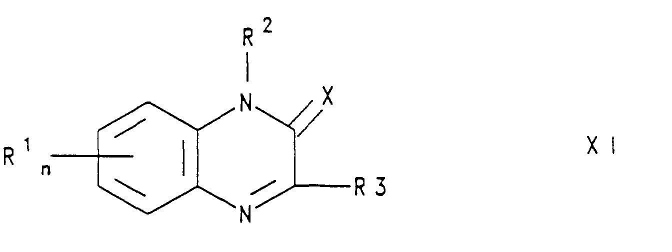 mit R¹ bis R³ wie eingangs definiert, hergestellt werden, indem man an die C=N- Bindung Wasserstoff anlagert,
oder
daß F)
Verbindungen der Formel I, wobei X Sauerstoff und R¹ bis R⁵ wie in Anspruch 1 definiert, hergestellt werden aus Verbindungen der Formel VI, 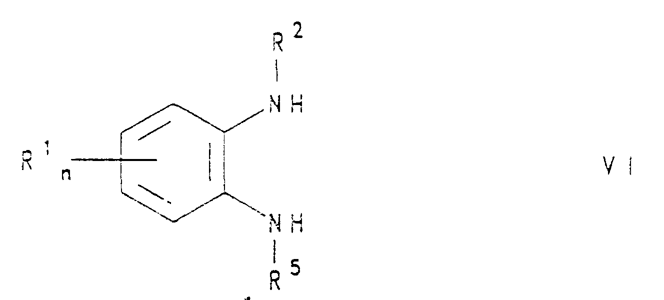 mit R¹, R² und R⁵ wie in Anspruch 1 definiert, durch Umsetzung mit Chloroform oder Bromoform und einer Carbonylverbindung der Formel XIII,
R³-CO-R⁴ (XIII)
mit R³ und R⁴ wie in Anspruch 1 definiert oder mit α-(Trihalogenmethyl)-alkanolen der Formel XIV,
Hal₃C-C(OH)-R³R⁴ (XIV)
worin Hal für Cl, Br oder J steht,
in denen R³ und R⁴ wie eingangs definiert sind,
oder
daß G)
Verbindungen der Formel I, mit X gleich Sauerstoff und R¹, R², R³, R⁴ und R⁵ wie in Anspruch 1 definiert, durch Reaktion einer Verbindung der Formel I, wobei X Sauerstoff ist, für R¹, R², R⁵ sowie für R³ und R⁴ die in Anspruch1 genannten Definitionen gelten, außer daß mindestens einer der Reste R³ oder R⁴ Wasserstoff ist, mit einem Alkylierungsreagenz der Formel XV,
R'-Z (XV)
wobei R' die oben angegebenen Bedeutungen für R³ und R⁴ mit Ausnahme von Wasserstoff hat und Z eine Abgangsgruppe ist, hergestellt werden, oder
daß H)
Verbindungen der Formel I, mit X gleich Sauerstoff, R¹, R², R³ und R⁴ wie in Anspruch 1 definiert und R⁵ C1 - C6-Alkyl, gegebenenfalls substituiert mit Fluor, Chlor, Hydroxy, C1 - C4 -Acyloxy, Benzoyloxy, Phenoxy, C1 - C4-Alkoxy, C1 - C4-Alkylamino, Di(C1 - C4-Alkyl)amino, C1 - C4-Alkylthio, Carboxy, Carbamoyl, C3 - C6-Alkenyl, gegebenenfalls substituiert mit Fluor, Chlor, Hydroxy, C1 - C4-Acyloxy, Benzoyloxy, Phenoxy, C1 - C4-Alkoxy, C1 - C4-Alkylamino, Di(C1 - C4-alkyl)amino, C1 - C4-Alkylthio, Carboxy, Carbamoyl, C3 - C8-Alkinyl, gegebenenfalls substituiert mit Fluor, Chlor, Hydroxy, C1 - C4-Acyloxy, Benzoyloxy, Phenoxy, C1 - C4-Alkoxy, C1 - C4-Alkylamino, Di(C1 - C4-alkyl)amino, C1 - C4-Alkylthio, Carboxy, Carbamoyl, C4 - C8-Cycloalkyl, gegebenenfalls substituiert mit Fluor, Chlor, Hydroxy, C1 - C4-Acyloxy, Benzoyloxy, C1 - C4-Alkoxy, C1 - C4-Alkylamino, Di(C1 - C4-alkyl)-amino, C1 - C4-Alkylthio, Carboxy, Carbamoyl, C5 - C8-Cycloalkenyl, gegebenenfalls substituiert mit Fluor, Chlor, Hydroxy, C1 -C4-Acyloxy, Benzoyloxy, Phenoxy, C1 - C4-Alkoxy, C1 - C4-Alkylamino, Di(C1 - C4-alkyl)amino, C1 - C4-Alkylthio, Carboxy, Carbamoyl,
durch reduktive Alkylierung einer Verbindung der Formel I, wobei R⁵ Wasserstoff und X Sauerstoff sind und für R¹, R², R³ und R⁴ die in Anspruch 1 genannten Definitionen gelten mit einer Carbonylverbindung der Formel XVI,
R"-C(=O)-R"' (XVI),
wobei R" und R"' gleich oder verschieden, unabhängig voneinander Wasserstoff, C1 - C5-Alkyl, gegebenenfalls substituiert mit Fluor, Chlor, Hydroxy, C1 - C4-Acyloxy, Benzoyloxy, Phenoxy, C1 - C4-Alkoxy, C1 - C4-Alkylamino, Di(C1 - C4-alkyl)amino, C1 - C4-Alkylthio, Carboxy, Carbamoyl, C3 - C5-Alkenyl, gegebenenfalls substituiert mit Fluor, Chlor, Hydroxy, C1 - C4-Acyloxy, Benzoyloxy, Phenoxy, C1 - C4-Alkoxy, C1 - C4-Alkylamino, Di(C1 - C4-alkyl)amino, C1 - C4-Alkylthio, Carboxy, Carbamoyl, C3 - C7-Alkinyl, gegebenenfalls substituiert mit Fluor, Chlor, Hydroxy, C1 - C4-Acyloxy, Benzoyloxy, Phenoxy, C1 - C4-Alkoxy, C1 - C4-Alkylamino, Di(C1 - C4-alkyl)amino, C1 - C4-Alkylthio, Carboxy, Carbamoyl, C4 - C8-Cycloalkyl, gegebenenfalls substituiert mit Fluor, Chlor, Hydroxy, C1 - C4-Acyloxy, Benzoyloxy, Phenoxy, C1 - C4-Alkoxy, C1 - C4-Alkylamino, Di(C1 - C4-alkyl)amino, C1 - C4-Alkylthio, Carboxy, Carbamoyl, C6-Cycloalkenyl, gegebenenfalls substituiert mit Fluor, Chlor, Hydroxy, C1 - C4-Acyloxy, Benzoyloxy, Phenoxy, C1 - C4-Alkoxy, C1 - C4-Alkylamino, Di(C1 - C4-alkyl)amino, C1 - C4-Alkylthio, Carboxy,
und wobei R" und R"' unter Bildung eines 4- bis 8-gliedrigen Ringes miteinander verknüpft sein können, hergestellt werden
oder I Verbindungen der Formel I, mit X gleich Sauerstoff und R¹, R², R³ und R⁴ wie in Anspruch 1 definiert und R⁵ C1 - C6-Alkyloxycarbonyl, C1 - C6-Alkylthiocarbonyl, C2 - C6-Alkenyloxycarbonyl, C2 - C6-Alkenylthiocarbonyl, C2 - C6-Alkinyloxycarbonyl, C1 - C6-Alkylaminocarbonyl, C3 - C6-Alkenylaminocarbonyl, Di(C1 - C6-alkyl)aminocarbonyl, Pyrrolidin-1-yl, Morpholino-, Piperidino-, Piperazinyl-, 4-Methylpiperazin-1-yl-carbonyl gegebenenfalls substituiert mit Fluor, Chlor, Hydroxy,
oder mit bis zu zwei voneinander unabhängigen Resten R⁶ substituiertes Aryloxycarbonyl, Arylthio(carbonyl), Arylaminocarbonyl, (Arylalkylthio)carbonyl, Aryalkylaminocarbonyl, wobei der Alkylrest jeweils 1 bis 3 C-Atome enthalten kann, hergestellt werden, indem man eine Verbindung der Formel XVII, 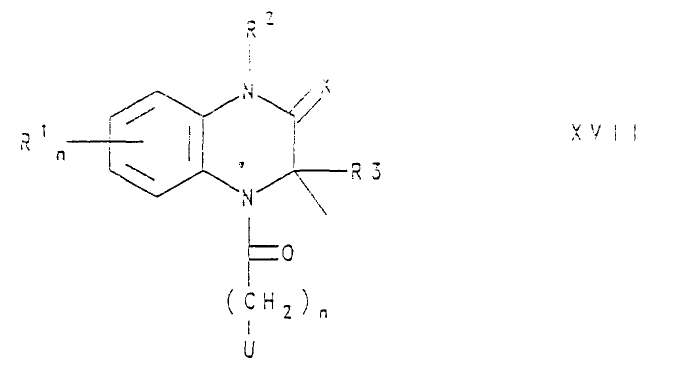 wobei für R¹, R², R³ und R⁴ die in Anspruch 1 genannten Definitionen gelten, n gleich 0, 1, 2 oder 3 ist, X gleich Sauerstoff und U eine Abgangsgruppe ist, mit einer Verbindung der Formel XVIII,
Nu - H (XVIII),
wobei Nu C1 - C6-Alkyloxy, C2 - C6-Alkenyloxy, C2 - C6-Alkinyloxy, C1 - C6-Alkylthio, C2 - C6-Alkenylthio, C1 - C6-Alkylamino- und Di(C1 - C6-Alkyl)amino, C3 - C6-Alkenylamino- und Di(C1 - C6-alkyl)amino, gegebenenfalls substituiert durch Fluor, Chlor, Hydroxy, C1 - C4-Alkoxy, Pyrrolidin-1-yl, Morpholino-, Piperidino-, Piperazinyl-, oder 4-Methylpiperazin-1-yl-,
oder mit bis zu zwei voneinander unabhängigen Resten R⁶ (R⁶ ist wie eingangs definiert) substituiertes Aryloxy, Arylthio, Arylamino, Arylalkyloxy, Arylalkylthio, Aryalkylamino, 2-, 3-, oder 4-Pyridyl-, 2- oder 3-Furyl, 2- oder 3-Thienyl, 2-, 3- oder 4-Picolyloxy, 2- oder 3-Furtylmethyloxy, 2- oder 3-Thienylmethyloxy
wobei der Alkylrest jeweils 1 bis 3 C-Atome enthalten kann, sein kann, zur Reaktion bringt.

2. Verfahren zur Herstellung von Verbindungen der Formel I oder la gemäß Anspruch 1, **dadurch gekennzeichnet, daß** die genannten Substituenten bedeuten:
n null,
eins
oder zwei,
die einzelnen Substituenten R¹ unabhängig voneinander
Fluor, Chlor, Brom, Trifluormethyl, Hydroxy, C1 - C4-Alkyl, C1 - C4-Alkoxy, (C1 - C4-Alkoxy)-(C1 - C2-alkoxy), C1- C4-Alkylthio, Nitro, Amino, C1 - C4-Alkylamino, Di(C1 - C4-alkyl)amino, Piperidino, Morpholino, 1-Pyrrolidinyl, 4-Methylpiperazinyl, C1 - C4-Acyl, C1 - C4-Acyloxy, C1 - C4-Acylamino, Cyano, Carbamoyl, Carboxy, (C1 - C4-Alkyl)-oxycarbonyl, Hydroxysulfonyl, Sulfamoyl
oder
einen mit bis zu zwei
voneinander unabhängigen Resten R⁶ substituierten Phenyl-, Phenoxy-, Phenylthio-, Phenylsulfonyl-, Phenoxysulfonyl-, Benzoyl-, 2-Pyridyl-, 3-Pyridyl- oder 4-Pyridylrest,
wobei R⁶
Fluor, Chlor, Brom, Cyano, Trifluormethyl, Nitro, Amino, C1 - C4-Alkyl, C1 - C4-Alkoxy, (C1 - C4-Alkyl)-oxycarbonyl, Phenyl, Phenoxy
sein kann,
R² Wasserstoff und R⁵
C1 - C6-Alkyl
gegebenenfalls substituiert mit C1 - C4-Alkoxy oder C1 - C4-Alkylthio;
C2 - C6-Alkenyl,
gegebenenfalls substituiert mit Oxo;
Allenyl;
C3 - C8-Alkinyl, insbesondere 2-Butinyl;
C3 - C6-Cycloalkyl;
C5 - C6-Cycloalkenyl;
(C3 - C6-Cycloalkyl)-(C1 - C2-alkyl), insbesondere Cyclopropylmethyl, gegebenenfalls substituiert mit C1 - C4-Alkyl;
(C3 - C6-Cycloalkenyl)-(C1 - C2-alkyl), insbesondere Cyclohexenylmethyl;
C1 - C6-Alkylcarbonyl,
gegebenenfalls substituiert mit Fluor,
Chlor, Hydroxy, Benzyloxy, Phenoxy, C1 - C4-Alkoxy, C1 - C4-Alkylamino, C1 - C4-Alkenylamino, Di(C1 - C4-alkyl)amino, C1 - C4-Alkylthio;
C2 - C6-Alkenylcarbonyl;
C1 - C6-Alkyloxycarbonyl, gegebenenfalls substituiert durch Fluor, Chlor, Brom, Hydroxy, C1 - C4-Alkoxy, C1 - C4-Alkylamino, Di(C1 - C4-alkyl)amino, C1 - C4-Alkylthio;
C2 - C6-Alkenyloxycarbonyl, insbesondere Vinyloxycarbonyl, Allyloxycarbonyl, Isopropenyloxycarbonyl, Butenyloxycarbonyl, Pentenyloxycarbonyl;
C2 - C6-Alkinyloxycarbonyl, insbesondere Propinyloxycarbonyl, Butinyloxycarbonyl;
C1 - C6-Alkylthiocarbonyl;
C2 - C6-Alkenylthiocarbonyl, insbesondere Allylthiocarbonyl;
C1 - C6-Alkylamino- und Di(C1 - C6-alkyl)aminocarbonyl;
Pyrrolidin-1-yl, Morpholino-, Piperidino-, Piperazinyl-, oder
4-Methylpiperazin-1-yl-carbonyl;
C2 - C6-Alkenylamino- und Di(C1 - C6-alkenyl)aminocarbonyl;
C1 - C4-Alkylsulfonyl;
C1 - C4-Alkenylsulfonyl;
oder mit bis zu zwei voneinander unabhängigen Resten R⁶ substituiertes Aryl, insbesondere Phenyl,
Arylcarbonyl, insbesondere Benzoyl, (Arylthio)carbonyl, Aryloxycarbonyl, Arylaminocarbonyl, (Arylamino)thiocarbonyl, Arylalkylaminocarbonyl, Arylsulfonyl, Arylalkyl, insbesondere Benzyl, Phenylethyl, Arylalkenyl, Arylalkylcarbonyl, Arylalkoxycarbonyl, Aryl(alkylthio)carbonyl, wobei der Alkylrest jeweils 1 bis 3 C-Atome enthalten kann und R⁶ wie oben definiert ist oder mit bis zu zwei voneinander unabhängigen Resten R⁶ substituiertes 1- oder 2-Naphthylmethyl, 2-, 3- oder 4-Picolyl, 2- oder 3-Furylmethyl, 2- oder 3-Thienylmethyl, 2- oder 3-Pyrrolylmethyl,
2-, 3- oder 4-Pyridylcarbonyl, 2- oder 3-Furylcarbonyl, 2- oder 3-Thienylcarbonyl, 2-, 3- oder 4-Picolyloxycarbonyl, 2- oder 3-Furylmethyloxycarbonyl, 2- oder 3-Thienylmethyloxycarbonyl,
und
R³ und R⁴ gleich oder verschieden, unabhängig voneinander
Wasserstoff,
C1 - C4-Alkyl,
gegebenenfalls substituiert mit Hydroxy, Mercapto,
C1 - C4-Alkoxy, C1 - C4-Alkylthio, C1 - C4-Alkylsulfonyl, C1 - C4-Alkylsulfinyl, Carboxy, Carbamoyl;
C2 - C6-Alkenyl,
mit bis zu zwei voneinander unabhängigen Resten R⁶ substituiertes Aryl, Benzyl, Thienyl oder Thienylmethyl wobei R⁶ wie oben definiert ist, bedeuten,
R³ und R⁴ können ferner auch
Teil eines gesättigten oder ungesättigten carbo- oder heterocyclischen Ringes mit 3 bis 6 C-Atomen sein, der gegebenenfalls mit Oxo oder Thioxo substituiert sein kann und
die in den vorausgegangenen Definitionen genannten Arylgruppen sind aromatische Gruppen mit 6-14 C-Atomen, die in den vorausgegangenen Definitionen genannten heterocyclischen Ringe bzw. Heteroarylgruppen enthalten 1-13 C-Atome und 1-6 Heteroatome ausgewählt aus der Gruppe O, S und N, wobei im Falle eines an dieser Stelle gesättigten N-haltigen Ringes N - Z vorliegt, worin Z H oder R⁵ mit den jeweiligen oben beschriebenen Definitionen bedeutet,
X bedeutet Sauerstoff oder Schwefel

3. Verfahren zur Herstellung von Verbindungen der Formel I oder la gemäß den Ansprüchen 1 oder 2, **dadurch gekennzeichnet, daß** die genannten Substituenten bedeuten: n null oder 1,
die einzelnen Substituenten R¹ unabhängig voneinander
Fluor, Chlor, Brom, C1 - C2-Alkyl, C1 - C2-Alkoxy, C2 - C4-Acyl, Cyano
R² Wasserstoff und R⁵
C2-C6-Alkenyl
C3 - C8-Alkinyl, insbesondere 2-Butinyl;
(C3 - C6-Cycloalkyl)-(C1 - C2-alkyl), insbesondere Cyclopropylmethyl, gegebenenfalls substituiert mit C1 - C4-Alkyl;
(C3 - C6-Cycloalkenyl)-(C1 - C2-alkyl), insbesondere Cyclohexenylmethyl;
C2 - C6-Alkylcarbonyl,
C2 - C6-Alkenylcarbonyl;
C1 - C6-Alkyloxycarbonyl;
C2 - C6-Alkenyloxycarbonyl, insbesondere Vinyloxycarbonyl, Allyloxycarbonyl, Isopropenyloxycarbonyl, Butenyloxycarbonyl, Pentenyloxycarbonyl;
C2 - C6-Alkinyloxycarbonyl, insbesondere Propinyloxycarbonyl, Butinyloxycarbonyl;
C2 - C6-Alkenylthiocarbonyl, insbesondere Allylthiocarbonyl;
C1 - C4-Alkylsulfonyl;
C1 - C4-Alkenylsulfonyl;
oder mit bis zu zwei voneinander unabhängigen Resten R⁶ substituiertes Arylalkyl, insbesondere Benzyl oder Arylalkenyl, wobei der Alkylrest jeweils 1 bis 3 C-Atome und der Alkenylrest 2-3 C-Atome enthalten kann
oder mit bis zu zwei voneinander unabhängigen Resten R⁶ substituiertes 1-Naphthylmethyl, 2- oder 3-Picolyl, 2-Furylmethyl, 2- oder 3-Thienylmethyl,
wobei R⁶
Fluor, Chlor, Brom, Cyano, C1 - C2-Alkyl, C1 - C2-Alkoxy
und
R³ und R⁴ gleich oder verschieden, unabhängig voneinander
Wasserstoff,
C1 - C4-Alkyl,
gegebenenfalls substituiert mit Hydroxy, Mercapto, C1 - C4-Alkoxy, C1 - C2-Alkylthio und X bedeutet Sauerstoff oder Schwefel.

4. Verbindungen der Formel I bzw. la gemäß einem oder mehreren der Ansprüche 1 - 3 zur Anwendung als Arzneimittel.

5. Arzneimittel, enthaltend eine wirksame Menge mindestens einer Verbindung der Formel la gemäß einem oder mehreren der Ansprüche 1 - 3.

6. Verfahren zur Herstellung eines Arzneimittels gemäß Anspruch 6, **dadurch gekennzeichnet**. daß die wirksame Menge einer Verbindung der Formel I bzw. la mit üblichen pharmazeutischen Hilfsstoffen in eine geeignete Darreichungsform gebracht wird.

7. Verwendung von Verbindungen der Formel I oder la gemäß einem oder mehreren der Ansprüche 1 - 3 zur Herstellung von Arzneimitteln zur Behandlung von Krankheiten, die durch Viren verursacht werden, insbesondere von Krankheiten, die durch das HIV verursacht werden.

## Claims (Claims for the following Contracting State(s): AT, BE, CH, DE, DK, FR, GB, IT, LI, LU, NL, PT, SE)

1. A compound of the formula I or Ia, 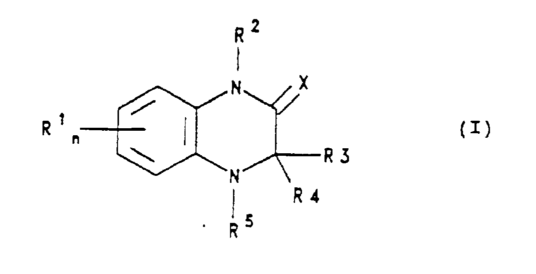 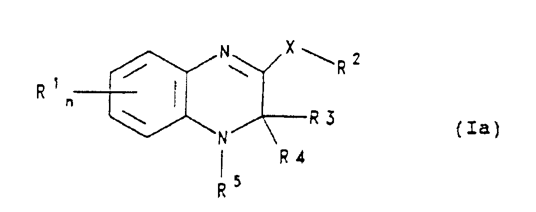 and physiologically acceptable salts thereof, where, in the formulae I and Ia,
n is zero,
one
or two,
the individual substituents R¹ independently of one another are
fluorine, chlorine, bromine, trifluoromethyl, hydroxyl, C₁-C₄-alkyl, C₁-C₄-alkoxy, (C₁-C₄-alkoxy)-(C₁-C₄-alkoxy), C₁-C₄-alkylthio, nitro, amino, C₁-C₄-alkylamino, di (C₁-C₄-alkyl)amino, piperidino, morpholino, 1-pyrrolidinyl, 4-methylpiperazinyl, C₁-C₄-acyl, C₁-C₄-acyloxy, C₁-C₄-acrylamino, cyano, carbamoyl, carboxyl, (C₁-C₄-alkyl)oxycarbonyl, hydroxysulfonyl, sulfamoyl
or
a phenyl, phenoxy, phenylthio, phenylsulfonyl, phenoxysulfonyl, benzoyl, 2-pyridyl, 3-pyridyl or 4-pyridyl radical which is substituted by up to two radicals R⁶ which are independent of one another,
where R⁶ can be
fluorine, chlorine, bromine, cyano, trifluoromethyl, nitro, amino, C₁-C₄-alkyl, C₁-C₄-alkoxy, (C₁-C₄-alkyl)oxycarbonyl, phenyl, phenoxy,
R² is hydrogen and R⁵ is
C₁-C₆-alkyl,
optionally substituted by fluorine, chlorine, hydroxyl, C₁-C₄-acyloxy, benzoyloxy, benzyloxy, phenoxy, C₁-C₄-alkoxy, C₁-C₄-alkylamino, di (C₁-C₄-alkyl)amino, C₁-C₄-alkylthio, oxo, thioxo, carboxyl or carbamoyl;
C₂-C₆-alkenyl,
optionally substituted by fluorine, chlorine, hydroxyl, C₁-C₄-acyloxy, benzoyloxy, benzyloxy, phenoxy, C₁-C₄-alkoxy, C₁-C₄-alkylamino, di (C₁-C₄-alkyl) amino, C₁-C₄-alkylthio, oxo, thioxo, carboxyl or carbamoyl;
allenyl,
C₃-C₈-alkynyl,
optionally substituted by fluorine, chlorine, hydroxyl, C₁-C₄-acyloxy, benzoyloxy, benzyloxy, phenoxy, C₁-C₄-alkoxy, C₁-C₄-alkylamino, di (C₁-C₄-alkyl) amino, C₁-C₄-alkylthio, oxo, thioxo, carboxyl or carbamoyl;
C₃-C₈-cycloalkyl,
optionally substituted by fluorine, chlorine, hydroxyl, C₁-C₄-alkyl, C₁-C₄-acyloxy, benzoyloxy, benzyloxy, phenoxy, C₁-C₄-alkoxy, C₁-C₄-alkylamino, di (C₁-C₄-alkyl) amino, C₁-C₄-alkylthio, oxo, thioxo, carboxyl or carbamoyl;
C₃-C₈-cycloalkenyl,
optionally substituted by fluorine, chlorine, hydroxyl, C₁-C₄-alkyl, C₁-C₄-acyloxy, benzoyloxy, benzyloxy, phenoxy, C₁-C₄-alkoxy, C₁-C₄-alkylamino, di(C₁-C₄-alkyl) amino, C₁-C₄-alkylthio, oxo, thioxo, carboxyl or carbamoyl;
(C₃-C₆-cycloalkyl)-(C₁-C₂-alkyl),
optionally substituted by fluorine, chlorine, hydroxyl, C₁-C₄-alkyl, C₁-C₄-acyloxy, benzoyloxy, benzyloxy, phenoxy, C₁-C₄-alkoxy, C₁-C₄-alkylamino, di (C₁-C₄-alkyl) amino, C₁-C₄-alkylthio, oxo, thioxo, carboxyl or carbamoyl;
(C₃-C₆-cycloalkenyl) - (C₁-C₂-alkyl) ,
optionally substituted by fluorine, chlorine, hydroxyl, C₁-C₄-alkyl, C₁-C₄-acyloxy, benzoyloxy, benzyloxy, phenoxy, C₁-C₄-alkoxy, C₁-C₄-alkylamino, di(C₁-C₄-alkyl)amino, C₁-C₄-alkylthio, oxo, thioxo, carboxyl or carbamoyl;
C₁-C₆-alkylcarbonyl,
optionally substituted by fluorine, chlorine, hydroxyl, C₁-C₄-alkyl, C₁-C₄-acyloxy, benzoyloxy, benzyloxy, phenoxy, C₁-C₄-alkoxy, C₁-C₄-alkylamino, C₁-C₄-alkenylamino, di(C₁-C₄-alkyl)amino, C₁-C₄-alkylthio, oxo, thioxo, carboxyl or carbamoyl;
C₂-C₆-alkenylcarbonyl, optionally substituted by fluorine, chlorine or hydroxyl;
(C₃-C₆-cycloalkyl)carbonyl,
(C₅-C₆-cycloalkenyl)carbonyl,
(C₃-C₆-cycloalkyl)-(C₁-C₂-alkyl)carbonyl,
(C₅-C₆-cycloalkenyl)- (C₁-C₂-alkyl) carbonyl,
C₁-C₆-alkyloxycarbonyl, optionally substituted by fluorine, chlorine, bromine, hydroxyl, C₁-C₄-alkoxy, C₁-C₄-alkylamino, di (C₁-C₄-alkyl) amino, C₁-C₄-alkylthio;
C₂-C₆-alkenyloxycarbonyl, optionally substituted by fluorine, chlorine, hydroxyl, C₁-C₄-alkoxy;
C₂-C₆-alkynyloxycarbonyl, optionally substituted by fluorine, chlorine, hydroxyl, C₁-C₄-alkoxy;
C₁-C₆-alkylthiocarbonyl, optionally substituted by fluorine, chlorine, hydroxyl, C₁-C₄-alkoxy;
C₂-C₆-alkenylthiocarbonyl, optionally substituted by fluorine, chlorine, hydroxyl, C₁-C₄-alkoxy;
C₁-C₆-alkylamino- and di(C₁-C₆-alkyl)aminocarbonyl, in each case optionally substituted by fluorine, chlorine, hydroxyl, C₁-C₄-alkoxy;
pyrrolidin-1-yl, morpholino-, piperidino-, piperazinyl- or 4-methylpiperazin-1-ylcarbonyl;
C₂-C₆-alkenylamino- and di(C₁-C₆-alkenyl)aminocarbonyl, in each case optionally substituted by fluorine, chlorine, hydroxyl, C₁-C₄-alkoxy;
C₁-C₄-alkylsulfonyl, optionally substituted by fluorine, chlorine, hydroxyl, C₁-C₄-alkoxy;
C₁-C₄-alkenylsulfonyl;
or
aryl, arylcarbonyl, (arylthio)carbonyl, aryloxycarbonyl, arylaminocarbonyl, (arylamino)thiocarbonyl, arylsulfonyl, arylalkylaminocarbonyl, arylalkyl, arylalkenyl, arylalkoxycarbonyl or aryl(alkylthio)carbonyl, each of which is substituted by up to two radicals R⁶ which are independent of one another, it being possible for the alkyl radical to contain in each case 1 to 3 carbon atoms, and R⁶ being as defined above,
or 1- or 2-naphthylmethyl, 2-, 3- or 4-picolyl, 2- or 3-furylmethyl, 2- or 3-thienylmethyl, 2-or 3-pyrrolylmethyl, 2-, 3- or 4-pyridylcarbonyl, 2- or 3-furylcarbonyl, 2- or 3-thienylcarbonyl, 2-, 3- or 4-picolyloxycarbonyl, 2- or 3-furylmethyloxycarbonyl or 2- or 3-thienylmethyloxycarbonyl, each of which is substituted by up to two radicals R⁶ which are independent of one another,
and
R³ and R⁴ are identical or different and, independently of one another, are hydrogen, C₁-C₄-alkyl which is optionally substituted by fluorine, chlorine, hydroxyl, amino, mercapto, C₁-C₄-acyloxy, benzoyloxy, phenoxy, C₁-C₄-alkoxy, C₁-C₄-alkylamino, di (C₁-C₄-alkyl) amino, C₁-C₄-alkylthio, C₁-C₄-alkylsulfonyl, C₁-C₄-alkylsulfinyl, carboxyl or carbamoyl;
C₂-C₆-alkenyl, optionally substituted by fluorine or chlorine;
C₃-C₆-cycloalkyl, optionally substituted by fluorine, chlorine, hydroxyl, amino, mercapto, C₁-C₄-acyloxy, benzoyloxy, benzyloxy, phenoxy, C₁-C₄-alkoxy, C₁-C₄-alkylamino, di(C₁-C₄-alkyl)amino, C₁-C₄-alkylthio, C₁-C₄-alkylsulfonyl, C₁-C₄-alkylsulfinyl, carboxyl or carbamoyl;
C₃-C₈-cycloalkenyl, optionally substituted by fluorine or chlorine;
aryl, benzyl, heteroaryl or heteroarylmethyl, each of which is substituted by up to two radicals R⁶ which are independent of one another,
R₃ and R₄ can furthermore also be
part of a saturated or unsaturated carbo- or heterocyclic ring which has 3 to 6 carbon atoms and which can optionally be substituted by fluorine, chlorine, hydroxyl, amino, C₁-C₄-acyloxy, benzoyloxy, C₁-C₄-alkoxy, oxo, thioxo, carboxyl or carbamoyl, and
the aryl groups mentioned in the preceding definitions are aromatic groups having 6-14 carbon atoms, the heterocyclic rings or heteroaryl groups mentioned in the preceding definitions contain 1-13 carbon atoms and 1-6 heteroatoms selected from the group consisting of O, S and N, where in the case of an N-containing ring saturated in this position, N-Z is present, in which ring Z is H or R⁵ having the respective definitions described above,
X is oxygen or sulfur,
with the exception of those compounds in which R³ and R⁴ are both hydrogen, and compounds in which R⁵ is COCHCl₂, and compounds in which n is 1, R¹, R³ and R⁴ are methyl and R⁵ is acetyl, and compounds in which n is zero, R³ and R⁴ are methyl or phenyl and R⁵ is ethoxycarbonyl.

2. A compound of the formula I or Ia as claimed in claim 1, wherein the abovementioned substituents have the following meaning:
n is zero,
one
or two,
the individual substituents R¹ independently of one another are
fluorine, chlorine, bromine, trifluoromethyl, hydroxyl, C₁-C₄-alkyl, C₁-C₄-alkoxy, (C₁-C₄-alkoxy)-(C₁-C₂-alkyloxy), C₁-C₄-alkylthio, nitro, amino, C₁-C₄-alkylamino, di(C₁-C₄-alkyl)amino, piperidino, morpholino, 1-pyrrolidinyl, 4-methylpiperazinyl, C₁-C₄-acyl, C₁-C₄-acyloxy, C₁-C₄-acylamino, cyano, carbamoyl, carboxyl, (C₁-C₄-alkyl)oxycarbonyl, hydroxysulfonyl or sulfamoyl
or
a phenyl, phenoxy, phenylthio, phenylsulfonyl, phenoxysulfonyl, benzoyl, 2-pyridyl, 3-pyridyl or 4-pyridyl radical which is substituted by up to two radicals R⁶ which are independent of one another,
where R⁶ can be
fluorine, chlorine, bromine, cyano, trifluoromethyl, nitro, amino, C₁-C₄-alkyl, C₁-C₄-alkoxy, (C₁-C₄-alkyl)oxycarbonyl, phenyl or phenoxy,
R² is hydrogen and R⁵ is
C₁-C₆-alkyl,
optionally substituted by C₁-C₄-alkoxy or C₁-C₄-alkylthio;
C₂-C₆-alkenyl,
optionally substituted by oxo;
allenyl;
C₃-C₈-alkynyl, in particular 2-butynyl;
C₃-C₆-cycloalkyl;
C₅-C₆-cycloalkenyl;
(C₃-C₆-cycloalkyl)-(C₁-C₂-alkyl), in particular cyclopropylmethyl,
optionally substituted by C₁-C₄-alkyl;
(C₃-C₆-cycloalkenyl)-(C₁-C₂-alkyl), in particular cyclohexenylmethyl;
C₁-C₆-alkylcarbonyl,
optionally substituted by fluorine, chlorine, hydroxyl, benzyloxy, phenoxy, C₁-C₄-alkoxy, C₁-C₄-alkylamino, C₁-C₄-alkenylamino, di(C₁-C₄-alkyl)amino or C₁-C₄-alkylthio;
C₂-C₆-alkenylcarbonyl;
C₁-C₆-alkyloxycarbonyl, optionally substituted by fluorine, chlorine, bromine, hydroxyl, C₁-C₄-alkoxy, C₁-C₄-alkylamino, di (C₁-C₄-alkyl) amino, C₁-C₄-alkylthio;
C₂-C₆-alkenyloxycarbonyl, in particular vinyloxycarbonyl, allyloxycarbonyl, isopropenyloxycarbonyl, butenyloxycarbonyl or pentenyloxycarbonyl;
C₂-C₆-alkynyloxycarbonyl, in particular propynyloxycarbonyl or butynyloxycarbonyl;
C₁-C₆-alkylthiocarbonyl;
C₂-C₆-alkenylthiocarbonyl, in particular allylthiocarbonyl;
C₁-C₆-alkylamino- and di(C₁-C₆-alkyl)aminocarbonyl;
pyrrolidin-1-yl, morpholino-, piperidino-, piperazinyl- or 4-methylpiperazin-1-ylcarbonyl;
C₂-C₆-alkenylamino- and di(C₁-C₆-alkenyl)aminocarbonyl;
C₁-C₄-alkylsulfonyl;
C₁-C₄-alkenylsulfonyl;
or aryl which is substituted by up to two radicals R⁶ which are independent of one another, in particular phenyl, arylcarbonyl, in particular benzoyl, (arylthio)carbonyl, aryloxycarbonyl, arylaminocarbonyl, (arylamino)thiocarbonyl, arylalkylamino-carbonyl, arylsulfonyl, arylalkyl, in particular benzyl, phenylethyl, arylalkenyl, arylalkylcarbonyl, arylalkoxycarbonyl, aryl(alkylthio)carbonyl, it being possible for the alkyl radical in each case to contain 1 to 3 carbon atoms and R⁶ being as defined above,
or 1- or 2-naphthylmethyl, 2-, 3- or 4-picolyl, 2- or 3-furylmethyl, 2- or 3-thienylmethyl, 2- or 3-pyrrolylmethyl, 2-, 3- or 4-pyridylcarbonyl, 2- or 3-furylcarbonyl, 2- or 3-thienylcarbonyl, 2-, 3- or 4-picolyloxycarbonyl, 2- or 3-furylmethyloxycarbonyl or 2- or 3-thienylmethyloxycarbonyl, each of which is substituted by up to two radicals R⁶ which are independent of one another,
and
R³ and R⁴ are identical or different and independently of one another are
hydrogen,
C₁-C₄-alkyl,
optionally substituted by hydroxyl, mercapto, C₁-C₄-alkoxy, C₁-C₄-alkylthio, C₁-C₄-alkylsulfonyl, C₁-C₄-alkylsulfinyl, carboxyl or carbamoyl ;
C₂-C₆-alkenyl,
aryl, benzyl, thienyl or thienylmethyl, each of which is substituted by up to two radicals R⁶ which are independent of one another, R⁶ being as defined above,
R³ and R⁴ can furthermore also be
part of a saturated or unsaturated carbo- or heterocyclic ring which has 3 to 6 carbon atoms and which can optionally be substituted by oxo or thioxo, and
the aryl groups mentioned in the preceding definitions are aromatic groups having 6-14 carbon atoms, the heterocyclic rings or heteroaryl groups mentioned in the preceding definitions contain 1-13 carbon atoms and 1-6 heteroatoms selected from the group consisting of O, S and N, where in the case of an N-containing ring saturated in this position, N-Z is present, in which ring Z is H or R⁵ having the respective definitions described above,
X is oxygen or sulfur.

3. A compound of the formula I or Ia as claimed in claim 1 or 2, wherein the abovementioned substituents have the following meanings:
n is zero or
one,
the individual substituents R¹ independently of one another are
fluorine, chlorine, bromine, C₁-C₂-alkyl, C₁-C₂-alkoxy, C₂-C₄-acyl, cyano;
R² is hydrogen and R⁵ is
C₂-C₆-alkenyl;
C₃-C₈-alkynyl, in particular 2-butynyl;
(C₃-C₆-cycloalkyl) - (C₁-C₂-alkyl) , in particular cyclopropylmethyl,
optionally substituted by C₁-C₄-alkyl;
(C₃-C₆-cycloalkenyl)- (C₁-C₂-alkyl), in particular cyclohexenylmethyl;
C₂-C₆-alkylcarbonyl;
C₂-C₆-alkenylcarbonyl;
C₁-C₆-alkyloxycarbonyl;
C₂-C₆-alkenyloxycarbonyl, in particular vinyloxycarbonyl, allyloxycarbonyl, isopropenyloxycarbonyl, butenyloxycarbonyl or pentenyloxycarbonyl;
C₂-C₆-alkynyloxycarbonyl, in particular propynyloxycarbonyl or butynyloxycarbonyl;
C₂-C₆-alkenylthiocarbonyl, in particular allylthiocarbonyl;
C₁-C₄-alkylsulfonyl;
C₁-C₄-alkenylsulfonyl;
or which is substituted by up to two radicals R⁶ which are independent of one another, in particular benzyl or arylalkenyl, it being possible for the alkyl radical to contain in each case 1 to 3 carbon atoms and for the alkenyl radical to contain 2-3 carbon atoms,
or 1-naphthylmethyl, 2- or 3-picolyl, 2-furylmethyl or 2- or 3-thienylmethyl, each of which is substituted by up to two radicals R⁶ which are independent of one another,
where R⁶ is
fluorine, chlorine, bromine, cyano, C₁-C₂-alkyl or C₁-C₂-alkoxy,
and
R³ and R⁴ are identical or different and independently of one another are
hydrogen,
C₁-C₄-alkyl,
optionally substituted by hydroxyl, mercapto, C₁-C₄-alkoxy or C₁-C₂-alkylthio, and
X is oxygen or sulfur.

4. A process for the preparation of a compound of the formula I as claimed in claim 1, which comprises
A) for preparing compounds of the formula I where X is oxygen and the radicals R¹, R², R³, R⁴ and R⁵ are as defined in claim 1, reacting a compound of the formula II 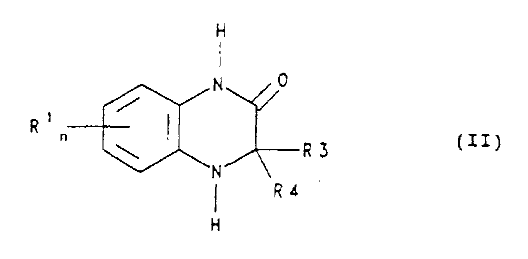 with the definitions mentioned in claim 1 applying to R¹, R³ and R⁴, with a compound of the formula III
R-Z (III)
where R has the meanings for R⁵ and R² which have been mentioned in claim 1 with the exception of hydrogen, hydroxyl, C₁-C₆-alkoxy, aryloxy, C₁-C₆-acyloxy, amino, C₁-C₆-alkylamino, di(C₁-C₆-alkyl)amino, arylamino and C₁-C₆-acylamino, and Z is a leaving group,
or
B) preparing compounds of the formula I where X is sulfur and R¹, R², R³, R⁴ and R⁵ are as defined in claim 1 by reacting a compound of the formula I where X is oxygen and the definitions mentioned in claim 1 apply to R¹, R², R³, R⁴ and R⁵, with a sulfurizing reagent,
or
C) preparing compounds of the formula Ia where X and the radicals R¹ to R⁵ are as defined in claim 1, by reacting a compound of the formula IV 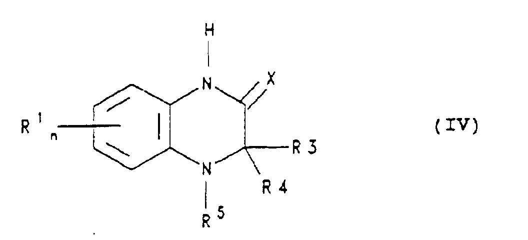 or 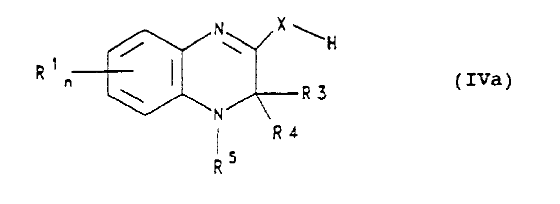 where the definitions mentioned in claim 1 apply to R¹, R³, R⁴ and R⁵, with a compound of the formula III
R²-Z (III)
where the definitions described in claim 1 for formula I and Ia apply to R² with the exception of hydrogen, hydroxyl, C₁-C₆-alkoxy, aryloxy, C₁-C₆-acyloxy, amino, C₁-C₆-alkylamino, di(C₁-C₆-alkyl)amino, arylamino and C₁-C₆-acylamino, and Z is a leaving group,
or
D) preparing compounds of the formula I where X is oxygen and the radicals R¹ to R⁵ are as defined in claim 1 by cyclizing a compound of the formula V 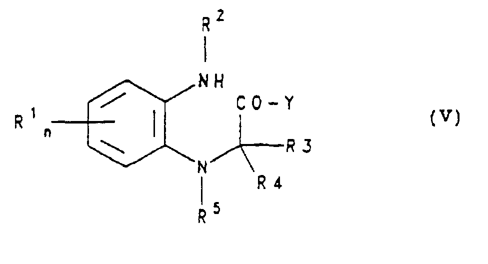 where R¹ to R⁵ are as defined in claim 1 and Y is hydroxyl, C₁-C₄-alkoxy, optionally halogenated, C₁-C₄-acyloxy, chlorine, bromine or iodine,
or
E) preparing compounds of the formula I where X is oxygen, R⁴ and R⁵ are hydrogen and the definitions mentioned in claim 1 apply to R¹ to R³, from the quinoxalinones of the formula XI 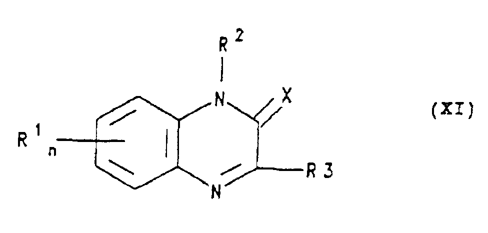 where R¹ to R³ are as defined at the outset, by addition of hydrogen onto the C=N bond,
or
F) preparing compounds of the formula I where X is oxygen and R¹ to R⁵ are as defined in claim 1, from compounds of the formula VI 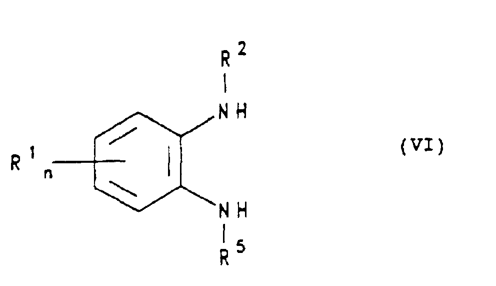 where R¹, R² and R⁵ are as defined in claim 1, by reacting them with chloroform or bromoform and a carbonyl compound of the formula XIII
R³-CO-R⁴ (XIII)
where R³ and R⁴ are as defined in claim 1, or with α-(trihalomethyl)alkanols of the formula XIV
Hal₃C-C (OH) -R³R⁴ (XIV)
where Hal is Cl, Br or I,
in which R³ and R⁴ are as defined at the outset,
or
G) preparing compounds of the formula I where X is oxygen and R¹, R², R³, R⁴ and R⁵ are as defined in claim 1, by reacting a compound of the formula I where X is oxygen and the definitions mentioned in claim 1 apply to R¹, R², R⁵ and to R³ and R⁴, with the exception that at least one of the radicals R³ or R⁴ is hydrogen, with an alkylating reagent of the formula XV
R'-Z (XV)
where R' has the meanings mentioned above for R³ and R⁴ with the exception of hydrogen and Z is a leaving group,
or
H) preparing compounds of the formula I where X is oxygen, R¹, R², R³ and R⁴ are as defined in claim 1 and R⁵ is C₁-C₆-alkyl, optionally substituted by fluorine, chlorine, hydroxyl, C₁-C₄-acyloxy, benzoyloxy, phenoxy, C₁-C₄-alkoxy, C₁-C₄-alkylamino, di (C₁-C₄-alkyl)amino, C₁-C₄-alkylthio, carboxyl, carbamoyl, C₃-C₆-alkenyl, optionally substituted by fluorine, chlorine, hydroxyl, C₁-C₄-acyloxy, benzoyloxy, phenoxy, C₁-C₄-alkoxy, C₁-C₄-alkylamino, di (C₁-C₄-alkyl) amino, C₁-C₄-alkylthio, carboxyl, carbamoyl, C₃-C₈-alkynyl, optionally substituted by fluorine, chlorine, hydroxyl, C₁-C₄-acyloxy, benzoyloxy, phenoxy, C₁-C₄-alkoxy, C₁-C₄-alkylamino, di (C₁-C₄-alkyl)amino, C₁-C₄-alkylthio, carboxyl, carbamoyl, C₄-C₈-cycloalkyl, optionally substituted by fluorine, chlorine, hydroxyl, C₁-C₄-acyloxy, benzoyloxy, C₁-C₄-alkoxy, C₁-C₄-alkylamino, di(C₁-C₄-alkyl)amino, C₁-C₄-alkylthio, carboxyl, carbamoyl, C₅-C₈-cycloalkenyl, optionally substituted by fluorine, chlorine, hydroxyl, C₁-C₄-acyloxy, benzoyloxy, phenoxy, C₁-C₄-alkoxy, C₁-C₄-alkylamino, di (C₁-C₄-alkyl) amino, C₁-C₄-alkylthio, carboxyl or carbamoyl, by reductive alkylation of a compound of the formula I where R⁵ is hydrogen and X is oxygen and the definitions mentioned in claim 1 apply to R¹, R², R³ and R⁴, with a carbonyl compound of the formula XVI,
R"-C(=O)-R''' (XVI)
where R" and R''' are identical or different and independently of one another are hydrogen, C₁-C₅-alkyl, optionally substituted by fluorine, chlorine, hydroxyl, C₁-C₄-acyloxy, benzoyloxy, phenoxy, C₁-C₄-alkoxy, C₁-C₄-alkylamino, di (C₁-C₄-alkyl)amino, C₁-C₄-alkylthio, carboxyl, carbamoyl, C₃-C₅-alkenyl, optionally substituted by fluorine, chlorine, hydroxyl, C₁-C₄-acyloxy, benzoyloxy, phenoxy, C₁-C₄-alkoxy, C₁-C₄-alkylamino, di (C₁-C₄-alkyl)amino, C₁-C₄-alkylthio, carboxyl, carbamoyl, C₃-C₇-alkynyl, optionally substituted by fluorine, chlorine, hydroxyl, C₁-C₄-acyloxy, benzoyloxy, phenoxy, C₁-C₄-alkoxy, C₁-C₄-alkylamino, di (C₁-C₄-alkyl)amino, C₁-C₄-alkylthio, carboxyl, carbamoyl, C₄-C₈-cycloalkyl, optionally substituted by fluorine, chlorine, hydroxyl, C₁-C₄-acyloxy, benzoyloxy, phenoxy, C₁-C₄-alkoxy, C₁-C₄-alkylamino, di (C₁-C₄-alkyl)amino, C₁-C₄-alkylthio, carboxyl, carbamoyl, C₆-cycloalkenyl, optionally substituted by fluorine, chlorine, hydroxyl, C₁-C₄-acyloxy, benzoyloxy, phenoxy, C₁-C₄-alkoxy, C₁-C₄-alkylamino, di(C₁-C₄-alkyl)amino, C₁-C₄-alkylthio or carboxyl, and where R" and R''' can be linked to each other to form a 4- to 8-membered ring,
or
I preparing compounds of the formula I where X is oxygen and R¹, R², R³ and R⁴ are as defined in claim 1 and R⁵ is C₁-C₆-alkyloxycarbonyl, C₁-C₆-alkylthiocarbonyl, C₂-C₆-alkenyloxycarbonyl, C₂-C₆-alkenylthiocarbonyl, C₂-C₆-alkynyloxycarbonyl, C₁-C₆-alkylaminocarbonyl, C₃-C₆-alkenylaminocarbonyl, di(C₁-C₆-alkyl)aminocarbonyl, pyrrolidin-1-yl, morpholino-, piperidino-, piperazinyl-, 4-methyl-piperazin-1-ylcarbonyl, optionally substituted by fluorine or chlorine, or aryloxycarbonyl, arylthio(carbonyl), arylaminocarbonyl, (arylalkylthio)carbonyl or arylalkylaminocarbonyl, each of which is substituted by up to two radicals R⁶ which are independent of one another, it being possible for the alkyl radical to contain in each case 1 to 3 carbon atoms, by reacting a compound of the formula XVII 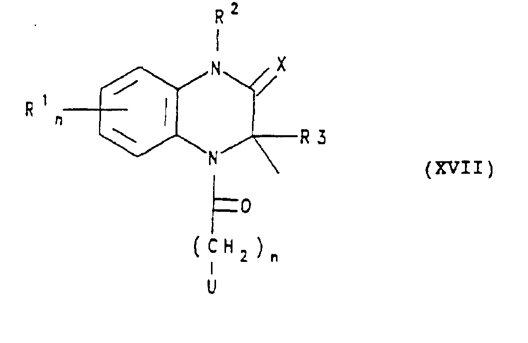 where the definitions mentioned in claim 1 apply to R¹, R², R³ and R⁴, n is 0, 1, 2 or 3, X is oxygen and U is a leaving group, with a compound of the formula XVIII
Nu-H (XVIII)
where Nu is C₁-C₆-alkoxy, C₂-C₆-alkenyloxy, C₂-C₆-alkynyloxy, C₁-C₆-alkylthio, C₂-C₆-alkenylthio, C₁-C₆-alkylamino and di (C1-C6-alkyl) amino, C₃-C₆-alkenylamino- and di(C₁-C₆-alkyl)amino, optionally substituted by fluorine, chlorine, hydroxyl, C₁-C₄-alkoxy, pyrrolidin-1-yl, morpholino-, piperidino-, piperazinyl- or 4-methylpiperazin-1-ylcarbonyl, or aryloxy, arylthio, arylamino, arylalkyloxy, arylalkylthio, arylalkylamino, 2-, 3- or 4-pyridyl, 2- or 3-furyl, 2- or 3-thienyl, 2-, 3- or 4-picolyloxy, 2- or 3-furylmethyloxy or 2- or 3-thienylmethyloxy, each of which is substituted by up to two radicals R⁶ (R⁶ is as defined at the outset) which are independent of one another, it being possible for the alkyl radical to contain in each case 1 to 3 carbon atoms.

5. A compound of the formula I or Ia as claimed in one or more of claims 1-3 for use as a pharmaceutical.

6. A pharmaceutical comprising an effective amount of at least one compound of the formula Ia as claimed in one or more of claims 1-3.

7. A process for the preparation of a pharmaceutical as claimed in claim 6, which comprises formulating an effective amount of a compound of the formula I or Ia together with customary pharmaceutical auxiliaries to give a suitable dosage form.

8. The use of a compound of the formula I or Ia as claimed in one or more of claims 1-3 for the preparation of pharmaceuticals for the treatment of diseases caused by viruses, in particular diseases caused by HIV.

## Claims (Claims for the following Contracting State(s): ES, GR)

1. A process for the preparation of a compound of the formula I or Ia, 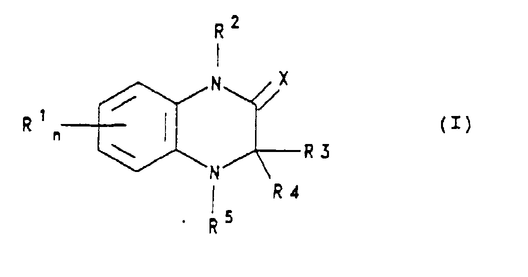 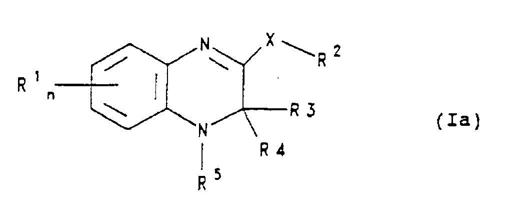 and physiologically acceptable salts thereof, where, in the formulae I and Ia,
n is zero,
one
or two,
the individual substituents R¹ independently of one another are
fluorine, chlorine, bromine, trifluoromethyl, hydroxyl, C₁-C₄-alkyl, C₁-C₄-alkoxy, (C₁-C₄-alkoxy)-(C₁-C₄-alkoxy), C₁-C₄-alkylthio, nitro, amino, C₁-C₄-alkylamino, di(C₁-C₄-alkyl) amino, piperidino, morpholino, 1-pyrrolidinyl, 4-methylpiperazinyl, C₁-C₄-acyl, C₁-C₄-acyloxy, C₁-C₄-acrylamino, cyano, carbamoyl, carboxyl, (C₁-C₄-alkyl)oxycarbonyl, hydroxysulfonyl, sulfamoyl
or
a phenyl, phenoxy, phenylthio, phenylsulfonyl, phenoxysulfonyl, benzoyl, 2-pyridyl, 3-pyridyl or 4-pyridyl radical which is substituted by up to two radicals R⁶ which are independent of one another,
where R⁶ can be
fluorine, chlorine, bromine, cyano, trifluoromethyl, nitro, amino, C₁-C₄-alkyl, C₁-C₄-alkoxy, (C₁-C₄-alkyl)oxycarbonyl, phenyl, phenoxy,
R² is hydrogen and R⁵ is
C₁-C₆-alkyl,
optionally substituted by fluorine, chlorine, hydroxyl, C₁-C₄-acyloxy, benzoyloxy, benzyloxy, phenoxy, C₁-C₄-alkoxy, C₁-C₄-alkylamino, di(C₁-C₄-alkyl)amino, C₁-C₄-alkylthio, oxo, thioxo, carboxyl or carbamoyl;
C₂-C₆-alkenyl,
optionally substituted by fluorine, chlorine, hydroxyl, C₁-C₄-acyloxy, benzoyloxy, benzyloxy, phenoxy, C₁-C₄-alkoxy, C₁-C₄-alkylamino, di(C₁-C₄-alkyl)amino, C₁-C₄-alkylthio, oxo, thioxo, carboxyl or carbamoyl;
allenyl,
C₃-C₈-alkynyl,
optionally substituted by fluorine, chlorine, hydroxyl, C₁-C₄-acyloxy, benzoyloxy, benzyloxy, phenoxy, C₁-C₄-alkoxy, C₁-C₄-alkylamino, di(C₁-C₄-alkyl)amino, C₁-C₄-alkylthio, oxo, thioxo, carboxyl or carbamoyl;
C₃-C₈-cycloalkyl,
optionally substituted by fluorine, chlorine, hydroxyl, C₁-C₄-alkyl, C₁-C₄-acyloxy, benzoyloxy, benzyloxy, phenoxy, C₁-C₄-alkoxy, C₁-C₄-alkylamino, di(C₁-C₄-alkyl)amino, C₁-C₄-alkylthio, oxo, thioxo, carboxyl or carbamoyl;
C₃-C₈-cycloalkenyl,
optionally substituted by fluorine, chlorine, hydroxyl, C₁-C₄-alkyl, C₁-C₄-acyloxy, benzoyloxy, benzyloxy, phenoxy, C₁-C₄-alkoxy, C₁-C₄-alkylamino, di(C₁-C₄-alkyl)amino, C₁-C₄-alkylthio, oxo, thioxo, carboxyl or carbamoyl;
(C₃-C₆-cycloalkyl) - (C₁-C₂-alkyl),
optionally substituted by fluorine, chlorine, hydroxyl, C₁-C₄-alkyl, C₁-C₄-acyloxy, benzoyloxy, benzyloxy, phenoxy, C₁-C₄-alkoxy, C₁-C₄-alkylamino, di(C₁-C₄-alkyl) amino, C₁-C₄-alkylthio, oxo, thioxo, carboxyl or carbamoyl;
(C₃-C₆-cycloalkenyl)-(C₁-C₂-alkyl),
optionally substituted by fluorine, chlorine, hydroxyl, C₁-C₄-alkyl, C₁-C₄-acyloxy, benzoyloxy, benzyloxy, phenoxy, C₁-C₄-alkoxy, C₁-C₄-alkylamino, di(C₁-C₄-alkyl)amino, C₁-C₄-alkylthio, oxo, thioxo, carboxyl or carbamoyl;
C₁-C₆-alkylcarbonyl,
optionally substituted by fluorine, chlorine, hydroxyl, C₁-C₄-alkyl, C₁-C₄-acyloxy, benzoyloxy, benzyloxy, phenoxy, C₁-C₄-alkoxy, C₁-C₄-alkylamino, C₁-C₄-alkenylamino, di(C₁-C₄-alkyl)amino, C₁-C₄-alkylthio, oxo, thioxo, carboxyl or carbamoyl;
C₂-C₆-alkenylcarbonyl, optionally substituted by fluorine, chlorine or hydroxyl;
(C₃-C₆-cycloalkyl)carbonyl,
(C₅-C₆-cycloalkenyl)carbonyl,
(C₃-C₆-cycloalkyl) - (C₁-C₂-alkyl) carbonyl,
(C₅-C₆-cycloalkenyl)-(C₁-C₂-alkyl) carbonyl,
C₁-C₆-alkyloxycarbonyl, optionally substituted by fluorine, chlorine, bromine, hydroxyl, C₁-C₄-alkoxy, C₁-C₄-alkylamino, di(C₁-C₄-alkyl)amino, C₁-C₄-alkylthio;
C₂-C₆-alkenyloxycarbonyl, optionally substituted by fluorine, chlorine, hydroxyl, C₁-C₄-alkoxy;
C₂-C₆-alkynyloxycarbonyl, optionally substituted by fluorine, chlorine, hydroxyl, C₁-C₄-alkoxy;
C₁-C₆-alkylthiocarbonyl, optionally substituted by fluorine, chlorine, hydroxyl, C₁-C₄-alkoxy;
C₂-C₆-alkenylthiocarbonyl, optionally substituted by fluorine, chlorine, hydroxyl, C₁-C₄-alkoxy;
C₁-C₆-alkylamino- and di(C₁-C₆-alkyl)aminocarbonyl, in each case optionally substituted by fluorine, chlorine, hydroxyl, C₁-C₄-alkoxy;
pyrrolidin-1-yl, morpholino-, piperidino-, piperazinyl- or 4-methylpiperazin-1-ylcarbonyl ;
C₂-C₆-alkenylamino- and di (C₁-C₆-alkenyl)aminocarbonyl, in each case optionally substituted by fluorine, chlorine, hydroxyl, C₁-C₄-alkoxy;
C₁-C₄-alkylsulfonyl, optionally substituted by fluorine, chlorine, hydroxyl, C₁-C₄-alkoxy;
C₁-C₄-alkenylsulfonyl;
or
aryl, arylcarbonyl, (arylthio)carbonyl, aryloxycarbonyl, arylaminocarbonyl, (arylamino)thiocarbonyl, arylsulfonyl, arylalkylaminocarbonyl, arylalkyl, arylalkenyl, arylalkoxycarbonyl or aryl(alkylthio)carbonyl, each of which is substituted by up to two radicals R⁶ which are independent of one another, it being possible for the alkyl radical to contain in each case 1 to 3 carbon atoms, and R⁶ being as defined above,
or 1- or 2-naphthylmethyl, 2-, 3- or 4-picolyl, 2- or 3-furylmethyl, 2- or 3-thienylmethyl, 2- or 3-pyrrolylmethyl, 2-, 3- or 4-pyridylcarbonyl, 2- or 3-furylcarbonyl, 2- or 3-thienylcarbonyl, 2-, 3- or 4-picolyloxycarbonyl, 2- or 3-furylmethyloxycarbonyl or 2- or 3-thienylmethyloxycarbonyl, each of which is substituted by up to two radicals R⁶ which are independent of one another,
and
R³ and R⁴ are identical or different and, independently of one another, are hydrogen, C₁-C₄-alkyl which is optionally substituted by fluorine, chlorine, hydroxyl, amino, mercapto, C₁-C₄-acyloxy, benzoyloxy, phenoxy, C₁-C₄-alkoxy, C₁-C₄-alkylamino, di(C₁-C₄-alkyl)amino, C₁-C₄-alkylthio, C₁-C₄-alkylsulfonyl, C₁-C₄-alkylsulfinyl, carboxyl or carbamoyl;
C₂-C₆-alkenyl, optionally substituted by fluorine or chlorine;
C₃-C₆-cycloalkyl, optionally substituted by fluorine, chlorine, hydroxyl, amino, mercapto, C₁-C₄-acyloxy, benzoyloxy, benzyloxy, phenoxy, C₁-C₄-alkoxy, C₁-C₄-alkylamino, di(C₁-C₄-alkyl)amino, C₁-C₄-alkylthio, C₁-C₄-alkylsulfonyl, C₁-C₄-alkylsulfinyl, carboxyl or carbamoyl;
C₃-C₈-cycloalkenyl, optionally substituted by fluorine or chlorine;
aryl, benzyl, heteroaryl or heteroarylmethyl, each of which is substituted by up to two radicals R⁶ which are independent of one another,
R₃ and R₄ can furthermore also be
part of a saturated or unsaturated carbo- or heterocyclic ring which has 3 to 6 carbon atoms and which can optionally be substituted by fluorine, chlorine, hydroxyl, amino, C₁-C₄-acyloxy, benzoyloxy, C₁-C₄-alkoxy, oxo, thioxo, carboxyl or carbamoyl, and
the aryl groups mentioned in the preceding definitions are aromatic groups having 6-14 carbon atoms, the heterocyclic rings or heteroaryl groups mentioned in the preceding definitions contain 1-13 carbon atoms and 1-6 heteroatoms selected from the group consisting of 0, S and N, where in the case of an N-containing ring saturated in this position, N-Z is present, in which ring Z is H or R⁵ having the respective definitions described above,
X is oxygen or sulfur,
with the exception of those compounds in which R³ and R⁴ are both hydrogen, and compounds in which R⁵ is COCHCl₂, and compounds in which n is 1, R¹, R³ and R⁴ are methyl and R⁵ is acetyl, and compounds in which n is zero, R³ and R⁴ are methyl or phenyl and R⁵ is ethoxycarbonyl, which comprises
A) for preparing compounds of the formula I where X is oxygen and the radicals R¹, R², R³, R⁴ and R⁵ are as defined in claim 1, reacting a compound of the formula II 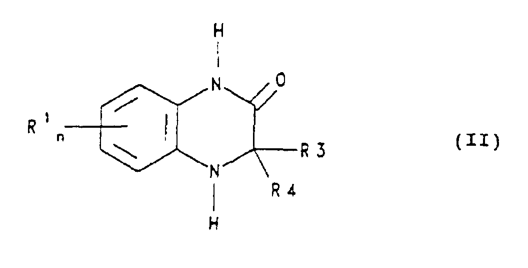 with the definitions mentioned above applying to R¹, R³ and R⁴, with a compound of the formula III
R-Z (III)
where R has the meanings for R⁵ and R² mentioned above with the exception of hydrogen, hydroxyl, C₁-C₆-alkoxy, aryloxy, C₁-C₆-acyloxy, amino, C₁-C₆-alkylamino, di(C₁-C₆-alkyl)amino, arylamino and C₁-C₆-acylamino, and Z is a leaving group,
or
B) preparing compounds of the formula I where X is sulfur and R¹, R², R³, R⁴ and R⁵ are as defined above by reacting a compound of the formula I where X is oxygen and the definitions mentioned above apply to R¹, R², R³, R⁴ and R⁵, with a sulfurizing reagent,
or
C) preparing compounds of the formula Ia where X and the radicals R¹ to R⁵ are as defined above, by reacting a compound of the formula IV 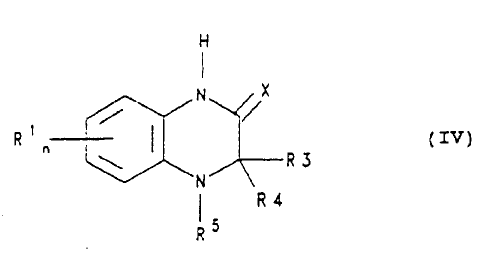 or 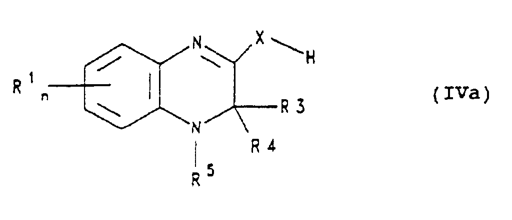 where the definitions mentioned above apply to R¹, R³, R⁴ and R⁵, with a compound of the formula III
R²-Z (III)
where the definitions described above for formula I and Ia apply to R² with the exception of hydrogen, hydroxyl, C₁-C₆-alkoxy, aryloxy, C₁-C₆-acyloxy, amino, C₁-C₆-alkylamino, di(C₁-C₆-alkyl)amino, arylamino and C₁-C₆-acylamino, and Z is a leaving group,
or
D) preparing compounds of the formula I where X is oxygen and the radicals R¹ to R⁵ are as defined above by cyclizing a compound of the formula V 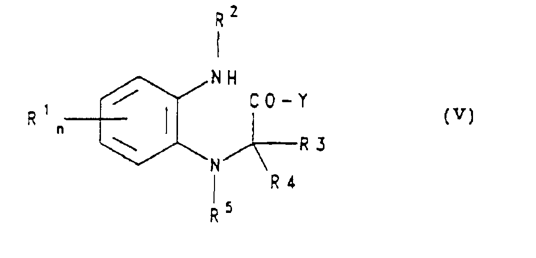 where R¹ to R⁵ are as defined above and Y is hydroxyl, C₁-C₄-alkoxy, optionally halogenated, C₁-C₄-acyloxy, chlorine, bromine or iodine,
or
E) preparing compounds of the formula I where X is oxygen, R⁴ and R⁵ are hydrogen and the definitions mentioned in claim 1 apply to R¹ to R³, from the quinoxalinones of the formula XI 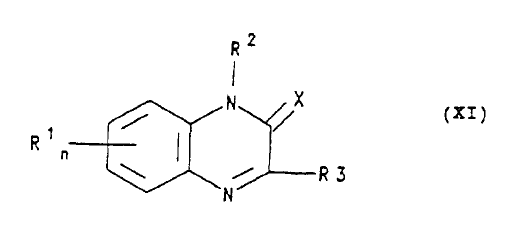 where R¹ to R³ are as defined at the outset, by addition of hydrogen onto the C=N bond,
or
F) preparing compounds of the formula I where X is oxygen and R¹ to R⁵ are as defined above, from compounds of the formula VI 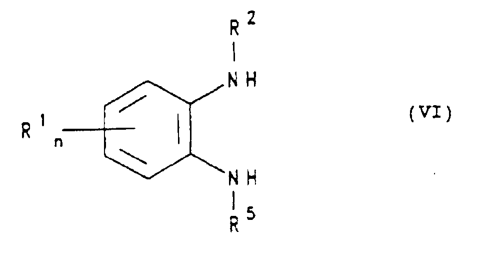 where R¹, R² and R⁵ are as defined above, by reacting them with chloroform or bromoform and a carbonyl compound of the formula XIII
R³-CO-R⁴ (XIII)
where R³ and R⁴ are as defined above, or with α-(trihalomethyl)alkanols of the formula XIV
Hal₃C-C(OH)-R³R⁴ (XIV)
where Hal is Cl, Br or I,
in which R³ and R⁴ are as defined at the outset,
or
G) preparing compounds of the formula I where X is oxygen and R¹, R², R³, R⁴ and R⁵ are as defined above, by reacting a compound of the formula I where X is oxygen and the definitions mentioned above apply to R¹, R², R⁵ and to R³ and R⁴, with the exception that at least one of the radicals R³ or R⁴ is hydrogen, with an alkylating reagent of the formula XV
R'-Z (XV)
where R' has the meanings mentioned above for R³ and R⁴ with the exception of hydrogen and Z is a leaving group,
or
H) preparing compounds of the formula I where X is oxygen, R¹, R², R³ and R⁴ are as defined above and R⁵ is C₁-C₆-alkyl, optionally substituted by fluorine, chlorine, hydroxyl, C₁-C₄-acyloxy, benzoyloxy, phenoxy, C₁-C₄-alkoxy, C₁-C₄-alkylamino, di(C₁-C₄-alkyl)amino, C₁-C₄-alkylthio, carboxyl, carbamoyl, C₃-C₆-alkenyl, optionally substituted by fluorine, chlorine, hydroxyl, C₁-C₄-acyloxy, benzoyloxy, phenoxy, C₁-C₄-alkoxy, C₁-C₄-alkylamino, di(C₁-C₄-alkyl)amino, C₁-C₄-alkylthio, carboxyl, carbamoyl, C₃-C₈-alkynyl, optionally substituted by fluorine, chlorine, hydroxyl, C₁-C₄-acyloxy, benzoyloxy, phenoxy, C₁-C₄-alkoxy, C₁-C₄-alkylamino, di(C₁-C₄-alkyl)amino, C₁-C₄-alkylthio, carboxyl, carbamoyl, C₄-C₈-cycloalkyl, optionally substituted by fluorine, chlorine, hydroxyl, C₁-C₄-acyloxy, benzoyloxy, C₁-C₄-alkoxy, C₁-C₄-alkylamino, di(C₁-C₄-alkyl)amino, C₁-C₄-alkylthio, carboxyl, carbamoyl, C₅-C₈-cycloalkenyl, optionally substituted by fluorine, chlorine, hydroxyl, C₁-C₄-acyloxy, benzoyloxy, phenoxy, C₁-C₄-alkoxy, C₁-C₄-alkylamino, di(C₁-C₄-alkyl)amino, C₁-C₄-alkylthio, carboxyl or carbamoyl,
by reductive alkylation of a compound of the formula I where R⁵ is hydrogen and X is oxygen and the definitions mentioned above apply to R¹, R², R³ and R⁴, with a carbonyl compound of the formula XVI,
R"-C(=O)-R''' (XVI)
where R" and R''' are identical or different and independently of one another are hydrogen, C₁-C₅-alkyl, optionally substituted by fluorine, chlorine, hydroxyl, C₁-C₄-acyloxy, benzoyloxy, phenoxy, C₁-C₄-alkoxy, C₁-C₄-alkylamino, di(C₁-C₄-alkyl)amino, C₁-C₄-alkylthio, carboxyl, carbamoyl, C₃-C₅-alkenyl, optionally substituted by fluorine, chlorine, hydroxyl, C₁-C₄-acyloxy, benzoyloxy, phenoxy, C₁-C₄-alkoxy, C₁-C₄-alkylamino, di(C₁-C₄-alkyl)amino, C₁-C₄-alkylthio, carboxyl, carbamoyl, C₃-C₇-alkynyl, optionally substituted by fluorine, chlorine, hydroxyl, C₁-C₄-acyloxy, benzoyloxy, phenoxy, C₁-C₄-alkoxy, C₁-C₄-alkylamino, di(C₁-C₄-alkyl)amino, C₁-C₄-alkylthio, carboxyl, carbamoyl, C₄-C₈-cycloalkyl, optionally substituted by fluorine, chlorine, hydroxyl, C₁-C₄-acyloxy, benzoyloxy, phenoxy, C₁-C₄-alkoxy, C₁-C₄-alkylamino, di(C₁-C₄-alkyl)amino, C₁-C₄-alkylthio, carboxyl, carbamoyl, C₆-cycloalkenyl, optionally substituted by fluorine, chlorine, hydroxyl, C₁-C₄-acyloxy, benzoyloxy, phenoxy, C₁-C₄-alkoxy, C₁-C₄-alkylamino, di(C₁-C₄-alkyl)amino, C₁-C₄-alkylthio or carboxyl, and where R" and R''' can be linked to each other to form a 4- to 8-membered ring,
or
I preparing compounds of the formula I where X is oxygen and R¹, R², R³ and R⁴ are as defined above and R⁵ is C₁-C₆-alkyloxycarbonyl, C₁-C₆-alkylthiocarbonyl, C₂-C₆-alkenyloxycarbonyl, C₂-C₆-alkenylthiocarbonyl, C₂-C₆-alkynyloxycarbonyl, C₁-C₆-alkylaminocarbonyl, C₃-C₆-alkenylaminocarbonyl, di(C₁-C₆-alkyl)aminocarbonyl, pyrrolidin-1-yl, morpholino-, piperidino-, piperazinyl-, 4-methyl-piperazin-1-ylcarbonyl, optionally substituted by fluorine or chlorine, or aryloxycarbonyl, arylthio(carbonyl), arylaminocarbonyl, (arylalkylthio)carbonyl or arylalkylaminocarbonyl, each of which is substituted by up to two radicals R⁶ which are independent of one another, it being possible for the alkyl radical to contain in each case 1 to 3 carbon atoms, by reacting a compound of the formula XVII 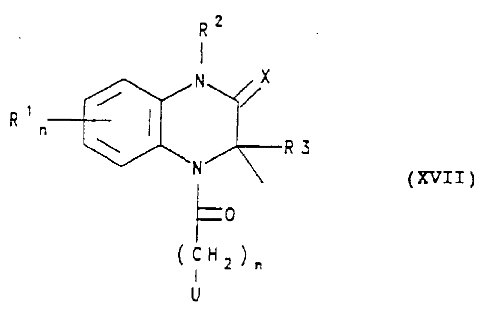 where the definitions mentioned above apply to R¹, R², R³ and R⁴, n is 0, 1, 2 or 3, X is oxygen and U is a leaving group, with a compound of the formula XVIII
Nu-H (XVIII)
where Nu is C₁-C₆-alkoxy, C₂-C₆-alkenyloxy, C₂-C₆-alkynyloxy, C₁-C₆-alkylthio, C₂-C₆-alkenylthio, C₁-C₆-alkylamino and di(C₁-C₆-alkyl)amino, C₃-C₆-alkenylamino- and di (C₁-C₆-alkyl)amino, optionally substituted by fluorine, chlorine, hydroxyl, C₁-C₄-alkoxy, pyrrolidin-1-yl, morpholino-, piperidino-, piperazinyl- or 4-methylpiperazin-1-ylcarbonyl, or aryloxy, arylthio, arylamino, arylalkyloxy, arylalkylthio, arylalkylamino, 2-, 3- or 4-pyridyl, 2- or 3-furyl, 2- or 3-thienyl, 2-, 3- or 4-picolyloxy, 2- or 3-furylmethyloxy or 2- or 3-thienylmethyloxy, each of which is substituted by up to two radicals R⁶ (R⁶ is as defined at the outset) which are independent of one another, it being possible for the alkyl radical to contain in each case 1 to 3 carbon atoms.

2. A process for the preparation of a compound of the formula I or Ia as claimed in claim 1, wherein the abovementioned substituents have the following meaning:
n is zero,
one
or two,
the individual substituents R¹ independently of one another are
fluorine, chlorine, bromine, trifluoromethyl, hydroxyl, C₁-C₄-alkyl, C₁-C₄-alkoxy, (C₁-C₄-alkoxy)-(C₁-C₂-alkyloxy), C₁-C₄-alkylthio, nitro, amino, C₁-C₄-alkylamino, di (C₁-C₄-alkyl)amino, piperidino, morpholino, 1-pyrrolidinyl, 4-methylpiperazinyl, C₁-C₄-acyl, C₁-C₄-acyloxy, C₁-C₄-acylamino, cyano, carbamoyl, carboxyl, (C₁-C₄-alkyl)oxycarbonyl, hydroxysulfonyl or sulfamoyl
or
a phenyl, phenoxy, phenylthio, phenylsulfonyl, phenoxysulfonyl, benzoyl, 2-pyridyl, 3-pyridyl or 4-pyridyl radical which is substituted by up to two radicals R⁶ which are independent of one another,
where R⁶ can be
fluorine, chlorine, bromine, cyano, trifluoromethyl, nitro, amino, C₁-C₄-alkyl, C₁-C₄-alkoxy, (C₁-C₄-alkyl)oxycarbonyl, phenyl or phenoxy,
R² is hydrogen and R⁵ is
C₁-C₆-alkyl,
optionally substituted by C₁-C₄-alkoxy or C₁-C₄-alkylthio;
C₂-C₆-alkenyl,
optionally substituted by oxo;
allenyl;
C₃-C₈-alkynyl, in particular 2-butynyl;
C₃-C₆-cycloalkyl;
C₅-C₆-cycloalkenyl;
(C₃-C₆-cycloalkyl) - (C₁-C₂-alkyl), in particular cyclopropylmethyl,
optionally substituted by C₁-C₄-alkyl;
(C₃-C₆-cycloalkenyl)- (C₁-C₂-alkyl), in particular cyclohexenylmethyl;
C₁-C₆-alkylcarbonyl,
optionally substituted by fluorine, chlorine, hydroxyl, benzyloxy, phenoxy, C₁-C₄-alkoxy, C₁-C₄-alkylamino, C₁-C₄-alkenylamino, di(C₁-C₄-alkyl)amino or C₁-C₄-alkylthio;
C₂-C₆-alkenylcarbonyl;
C₁-C₆-alkyloxycarbonyl, optionally substituted by fluorine, chlorine, bromine, hydroxyl, C₁-C₄-alkoxy, C₁-C₄-alkylamino, di(C₁-C₄-alkyl)amino, C₁-C₄-alkylthio;
C₂-C₆-alkenyloxycarbonyl, in particular vinyloxycarbonyl, allyloxycarbonyl, isopropenyloxycarbonyl, butenyloxycarbonyl or pentenyloxycarbonyl;
C₂-C₆-alkynyloxycarbonyl, in particular propynyloxycarbonyl or butynyloxycarbonyl;
C₁-C₆-alkylthiocarbonyl;
C₂-C₆-alkenylthiocarbonyl, in particular allylthiocarbonyl;
C₁-C₆-alkylamino- and di (C₁-C₆-alkyl)aminocarbonyl;
pyrrolidin-1-yl, morpholino-, piperidino-, piperazinyl- or 4-methylpiperazin-1-ylcarbonyl;
C₂-C₆-alkenylamino- and di(C₁-C₆-alkenyl)aminocarbonyl;
C₁-C₄-alkylsulfonyl;
C₁-C₄-alkenylsulfonyl;
or aryl which is substituted by up to two radicals R⁶ which are independent of one another, in particular phenyl, arylcarbonyl, in particular benzoyl, (arylthio)carbonyl, aryloxycarbonyl, arylaminocarbonyl, (arylamino)thiocarbonyl, arylalkylamino-carbonyl, arylsulfonyl, arylalkyl, in particular benzyl, phenylethyl, arylalkenyl, arylalkylcarbonyl, arylalkoxycarbonyl, aryl(alkylthio)carbonyl, it being possible for the alkyl radical in each case to contain 1 to 3 carbon atoms and R⁶ being as defined above,
or 1- or 2-naphthylmethyl, 2-, 3- or 4-picolyl, 2- or 3-furylmethyl, 2- or 3-thienylmethyl, 2- or 3-pyrrolylmethyl, 2-, 3- or 4-pyridylcarbonyl, 2- or 3-furylcarbonyl, 2- or 3-thienylcarbonyl, 2-, 3- or 4-picolyloxycarbonyl, 2- or 3-furylmethyloxycarbonyl or 2- or 3-thienylmethyloxycarbonyl, each of which is substituted by up to two radicals R⁶ which are independent of one another,
and
R³ and R⁴ are identical or different and independently of one another are
hydrogen,
C₁-C₄-alkyl,
optionally substituted by hydroxyl, mercapto, C₁-C₄-alkoxy, C₁-C₄-alkylthio, C₁-C₄-alkylsulfonyl, C₁-C₄-alkylsulfinyl, carboxyl or carbamoyl;
C₂-C₆-alkenyl,
aryl, benzyl, thienyl or thienylmethyl, each of which is substituted by up to two radicals R⁶ which are independent of one another, R⁶ being as defined above,
R³ and R⁴ can furthermore also be
part of a saturated or unsaturated carbo- or heterocyclic ring which has 3 to 6 carbon atoms and which can optionally be substituted by oxo or thioxo, and
the aryl groups mentioned in the preceding definitions are aromatic groups having 6-14 carbon atoms, the heterocyclic rings or heteroaryl groups mentioned in the preceding definitions contain 1-13 carbon atoms and 1-6 heteroatoms selected from the group consisting of O, S and N, where in the case of an N-containing ring saturated in this position, N-Z is present, in which ring Z is H or R⁵ having the respective definitions described above,
X is oxygen or sulfur.

3. A process for the preparation of a compound of the formula I or Ia as claimed in claim 1 or 2, wherein the abovementioned substituents have the following meanings:
n is zero or
1,
the individual substituents R¹ independently of one another are
fluorine, chlorine, bromine, C₁-C₂-alkyl, C₁-C₂-alkoxy, C₂-C₄-acyl, cyano;
R² is hydrogen and R⁵ is
C₂-C₆-alkenyl;
C₃-C₈-alkynyl, in particular 2-butynyl;
(C₃-C₆-cycloalkyl)-(C₁-C₂-alkyl), in particular cyclopropylmethyl,
optionally substituted by C₁-C₄-alkyl;
(C₃-C₆-cycloalkenyl)-(C₁-C₂-alkyl), in particular cyclohexenylmethyl;
C₂-C₆-alkylcarbonyl;
C₂-C₆-alkenylcarbonyl;
C₁-C₆-alkyloxycarbonyl;
C₂-C₆-alkenyloxycarbonyl, in particular vinyloxycarbonyl, allyloxycarbonyl, isopropenyloxycarbonyl, butenyloxycarbonyl or pentenyloxycarbonyl;
C₂-C₆-alkynyloxycarbonyl, in particular propynyloxycarbonyl or butynyloxycarbonyl;
C₂-C₆-alkenylthiocarbonyl, in particular allylthiocarbonyl;
C₁-C₄-alkylsulfonyl;
C₁-C₄-alkenylsulfonyl;
or which is substituted by up to two radicals R⁶ which are independent of one another, in particular benzyl or arylalkenyl, it being possible for the alkyl radical to contain in each case 1 to 3 carbon atoms and for the alkenyl radical to contain 2-3 carbon atoms,
or 1-naphthylmethyl, 2- or 3-picolyl, 2-furylmethyl or 2- or 3-thienylmethyl, each of which is substituted by up to two radicals R⁶ which are independent of one another,
where R⁶ is
fluorine, chlorine, bromine, cyano, C₁-C₂-alkyl or C₁-C₂-alkoxy,
and
R³ and R⁴ are identical or different and independently of one another are
hydrogen,
C₁-C₄-alkyl,
optionally substituted by hydroxyl, mercapto, C₁-C₄-alkoxy or C₁-C₂-alkylthio, and
X is oxygen or sulfur.

4. A compound of the formula I or Ia as claimed in one or more of claims 1-3 for use as a pharmaceutical.

5. A pharmaceutical comprising an effective amount of at least one compound of the formula Ia as claimed in one or more of claims 1-3.

6. A process for the preparation of a pharmaceutical as claimed in claim 5, which comprises formulating an effective amount of a compound of the formula I or Ia together with customary pharmaceutical auxiliaries to give a suitable dosage form.

7. The use of a compound of the formula I or Ia as claimed in one or more of claims 1-3 for the preparation of pharmaceuticals for the treatment of diseases caused by viruses, in particular diseases caused by HIV.

## Revendications (Revendications pour l'(les) Etat(s) contractant(s) suivant(s): AT, BE, CH, DE, DK, FR, GB, IT, LI, LU, NL, PT, SE)

1. Composé des formules I et Ia, 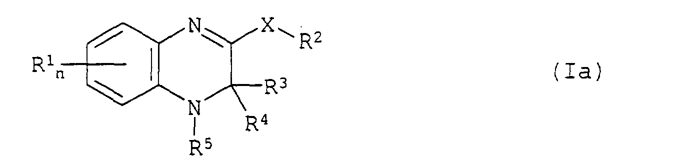 ainsi que leurs sels physiologiquement compatibles,
les symboles dans les formules I et Ia ayant les significations suivantes :
n vaut zéro, un ou deux ;
les différents substituants R¹ sont indépendamment les uns des autres fluoro, chloro, bromo, trifluorométhyle, hydroxy, alkyle en C₁-C₄, alcoxy en C₁-C₄, (alcoxy en C₁-C₄)-(alcoxy en C₁-C₄), alkylthio en C₁-C₄, nitro, amino, alkylamino en C₁-C₄, dialkylamino en C₁-C₄, pipéridino, morpholino, 1-pyrrolidinyle, 4-méthylpipérazinyle, acyle en C₁-C₄, acyloxy en C₁-C₄, acylamino en C₁-C₄, cyano, carbamoyle, carboxy, (alkyle en C₁-C₄)oxycarbonyle, hydroxysulfonyle, sulfamoyle,
ou
un résidu phényle, phénoxy, phénylthio, phénylsulfonyle, phénoxysulfonyle, benzoyle, 2-pyridyle, 3-pyridyle ou 4-pyridyle substitué par jusqu'à deux résidus R⁶ indépendants les uns des autres,
dans lesquels R⁶ peut être fluoro, chloro, bromo, cyano, trifluorométhyle, nitro, amino, alkyle en C₁-C₄, alcoxy en C₁-C₄, (alkyle en C₁-C₄)-oxycarbonyle, phényle, phénoxy,
R² est hydrogène et R⁵ est
alkyle en C₁-C₆ le cas échéant substitué par fluoro, chloro, hydroxy, acyloxy en C₁-C₄, benzoyloxy, benzyloxy, phénoxy, alcoxy en C₁-C₄, alkylamino en C₁-C₄, dialkylamino en C₁-C₄, alkylthio en C₁-C₄, oxo, thioxo, carboxy, carbamoyle ;
alcényle en C₂-C₆ le cas échéant substitué par fluoro, chloro, hydroxy, acyloxy en C₁-C₄, benzoyloxy, benzyloxy, phénoxy, alcoxy en C₁-C₄, alkylamino en C₁-C₄, dialkylamino en C₁-C₄, alkylthio en C₁-C₄, oxo, thioxo, carboxy, carbamoyle ;
alcynyle en C₃-C₈ le cas échéant substitué par fluoro, chloro, hydroxy, acyloxy en C₁-C₄, benzoyloxy, benzyloxy, phénoxy, alcoxy en C₁-C₄, alkylamino en C₁-C₄, dialkylamino en C₁-C₄, alkylthio en C₁-C₄, oxo, thioxo, carboxy, carbamoyle ;
cycloalkyle en C₃-C₈ le cas échéant substitué par fluoro, chloro, hydroxy, alkyle en C₁-C₄, acyloxy en C₁-C₄, benzoyloxy, benzyloxy, phénoxy, alcoxy en C₁-C₄, alkylamino en C₁-C₄, dialkylamino en C₁-C₄, alkylthio en C₁-C₄, oxo, thioxo, carboxy, carbamoyle ;
cycloalcényle en C₃-C₈ le cas échéant substitué par fluoro, chloro, hydroxy, alkyle en C₁-C₄, acyloxy en C₁-C₄, benzoyloxy, benzyloxy, phénoxy, alcoxy en C₁-C₄, alkylamino en C₁-C₄, dialkylamino en C₁-C₄, alkylthio en C₁-C₄, oxo, thioxo, carboxy, carbamoyle ;
(cycloalkyle en C₃-C₆) - (alkyle en C₁-C₂), le cas échéant substitué par fluoro, chloro, hydroxy, alkyle en C₁-C₄, acyloxy en C₁-C₄, benzoyloxy, benzyloxy, phénoxy, alcoxy en C₁-C₄, alkylamino en C₁-C₄, dialkylamino en C₁-C₄, alkylthio en C₁-C₄, oxo, thioxo, carboxy, carbamoyle ;
(Cycloalcényle en C₃-C₆)-(alkyle en C₁-C₂) , le cas échéant substitué par fluoro, chloro, hydroxy, alkyle en C₁-C₄, acyloxy en C₁-C₄, benzoyloxy, benzyloxy, phénoxy, alcoxy en C₁-C₄, alkylamino en C₁-C₄, dialkylamino en C₁-C₄, alkylthio en C₁-C₄, oxo, thioxo, carboxy, carbamoyle ;
alkylcarbonyle en C₁-C₆ le cas échéant substitué par fluoro, chloro, hydroxy, alkyle en C₁-C₄, acyloxy en C₁-C₄, benzoyloxy, benzyloxy, phénoxy, alcoxy en C₁-C₄, alkylamino en C₁-C₄, alcénylamino en C₁-C₄, dialkylamino en C₁-C₄, alkylthio en C₁-C₄, oxo, thioxo, carboxy, carbamoyle ;
alcénylcarbonyle en C₂-C₆ le cas échéant substitué par fluoro, chloro ou hydroxy ;
(cycloalkyle en C₃-C₆)carbonyle ;
(cycloalcényle en C₅-C₆)carbonyle ;
(cycloalkyle en C₃-C₆)-(alkyle en C₁-C₂)carbonyle,
(cycloalcényle en C₅-C₆)-(alkyle en C₁-C₂)carbonyle, alkyloxycarbonyle en C₁-C₆ le cas échéant substitué par fluoro, chloro, bromo, hydroxy, alcoxy en C₁-C₄, alkylamino en C₁-C₄, dialkylamino en C₁-C₄, alkylthio en C₁-C₄ ;
alcényloxycarbonyle en C₂-C₆ le cas échéant substitué par fluoro, chloro, hydroxy, alcoxy en C₁-C₄ ;
alcynyloxycarbonyle en C₂-C₆ le cas échéant substitué par fluoro, chloro, hydroxy, alcoxy en C₁-C₄ ;
alkylthiocarbonyle en C₁-C₆ le cas échéant substitué par fluoro, chloro, hydroxy, alcoxy en C₁-C₄ ;
alcénylthiocarbonyle en C₂-C₆ le cas échéant substitué par fluoro, chloro, hydroxy, alcoxy en C₁-C₄ ;
alkylaminocarbonyle en C₁-C₆ et dialkylaminocarbonyle en C₁-C₆ le cas échéant substitués par fluoro, chloro, hydroxy, alcoxy en C₁-C₄ ;
pyrrolidin-1-yle, morpholino, pipéridino, pipérazinyle ou 4-méthylpipérazin-1-ylcarbonyle;
alcénylaminocarbonyle en C₂-C₆ et dialcénylaminocarbonyle en C₁-C₆ le cas échéant substitués par fluoro, chloro, hydroxy, alcoxy en C₁-C₄ ;
alkylsulfonyle en C₁-C₄ le cas échéant substitué par fluoro, chloro, hydroxy, alcoxy en C₁-C₄ ;
alcénylsulfonyle en C₁-C₄ ;
ou aryle, arylcarbonyle, (arylthio)carbonyle, aryloxycarbonyle arylaminocarbonyle, (arylamino)thiocarbonyle, arylsulfonyle, arylalkylaminocarbonyle, arylalkyle, arylalcényle, arylalcoxycarbonyle, aryl(alkylthio)carbonyle substitués par jusqu'à deux résidus R⁶ indépendants les uns des autres, le résidu alkyle pouvant contenir respectivement de 1 à 3 atomes de carbone et R⁶ étant tel que défini plus haut
ou 1- ou 2-naphthylméthyle, 2-, 3- ou 4-picolyle, 2- ou 3-furylméthyle, 2- ou 3-thiénylméthyle, 2- ou 3-pyrrolylméthyle, 2-, 3- ou 4-pyridylcarbonyle, 2- ou 3-furylcarbonyle, 2- ou 3-thiénylcarbonyle, 2-, 3- ou 4-picolyloxycarbonyle, 2- ou 3-furylméthyloxycarbonyle, 2- ou 3-thiénylméthyloxycarbonyle substitués par jusqu'à deux résidus R⁶ indépendants les uns des autres,
et
R³ et R⁴ sont identiques ou différents et indépendamment les uns des autres hydrogène, alkyle en C₁-C₄ le cas échéant substitué par fluoro, chloro, hydroxy, amino, mercapto, acyloxy en C₁-C₄, benzoyloxy, phénoxy, alcoxy en C₁-C₄, alkylamino en C₁-C₄, dialkylamino en C₁-C₄, alkylthio en C₁-C₄, alkylsulfonyle en C₁-C₄, alkylsulfinyle en C₁-C₄, carboxy, carbamoyle ;
alcényle en C₂-C₆ le cas échéant substitué par fluoro ou chloro ;
cycloalkyle en C₃-C₆ le cas échéant substitué par fluoro, chloro, hydroxy, amino, mercapto, acyloxy en C₁-C₄, benzoyloxy, benzyloxy, phénoxy, alcoxy en C₁-C₄, alkylamino en C₁-C₄, dialkylamino en C₁-C₄, alkylthio en C₁-C₄, alkylsulfonyle en C₁-C₄, alkylsulfinyle en C₁-C₄, carboxy, carbamoyle ;
cycloalcényle en C₃-C₈ le cas échéant substitué par fluoro ou chloro ;
aryle, benzyle, hétéroaryle ou hétéroarylméthyle substitués par jusqu'à deux résidus R⁶ indépendants les uns des autres,
R³ et R⁴ peuvent également être
une partie d'un noyau carbo- ou hétérocyclique saturé ou insaturé ayant de 3 à 6 atomes de carbone, qui peut le cas échéant être substitué par fluoro, chloro, hydroxy, amino, acyloxy en C₁-C₄, benzoyloxy, alcoxy en C₁-C₄, oxo, thioxo, carboxy, carbamoyle, et
les groupes aryles mentionnés dans les définitions précédentes sont des groupes aromatiques ayant de 6 à 14 atomes de carbone, les noyaux hétérocycliques ou groupes hétéroaryles mentionnés dans les définitions précédentes contiennent 1 à 13 atomes de carbone et 1 à 6 hétéroatomes choisis dans le groupe comprenant O, S et N, N-Z étant présent dans le cas d'un noyau contenant N saturé dans cette position, Z, H ou R⁵ ayant les définitions respectives décrites plus haut,
X est oxygène ou soufre, à l'exception des composés dans lesquels R³ et R⁴ sont en même temps H,
et des composés dans lesquels R⁵ est COCHCl₂ et des composés dans lesquels n vaut 1, R¹, R³ et R⁴ sont méthyle et R⁵ est acétyle, et des composés dans lesquels n vaut zéro, R³ et R⁴ sont méthyle ou phényle et R⁵ est éthyloxycarbonyle.

2. Composés des formules I ou Ia selon la revendication 1, **caractérisés en ce que** les substituants mentionnés ont les significations suivantes :
n vaut zéro, un ou deux ;
les différents substituants R¹ sont indépendamment les uns des autres fluoro, chloro, bromo, trifluorométhyle hydroxy, alkyle en C₁-C₄, alcoxy en C₁-C₄, (alcoxy en C₁-C₄)-(alcoxy en C₁-C₄), alkylthio en C₁-C₄, nitro, amino, alkylamino en C₁-C₄, dialkylamino en C₁-C₄, pipéridino, morpholino, 1-pyrrolidinyle, 4-méthylpipérazinyle, acyle en C₁-C₄, acyloxy en C₁-C₄, acylamino en C₁-C₄, cyano, carbamoyle, carboxy, alcoxycarbonyle en C₁-C₄, hydroxysulfonyle, sulfamoyle,
ou
un résidu phényle, phénoxy, phénylthio, phénylsulfonyle, phénoxysulfonyle, benzoyle, 2-pyridyle, 3-pyridyle ou 4-pyridyle substitué par jusqu'à deux résidus R⁶ indépendants les uns des autres,
dans lesquels R⁶ peut être fluoro, chloro, bromo, cyano, trifluorométhyle, nitro, amino, alkyle en C₁-C₄, alcoxy en C₁-C₄, (alkyle en C₁-C₄)-oxycarbonyle, phényle, phénoxy,
R² est hydrogène et R⁵ est
alkyle en C₁-C₆ le cas échéant substitué par alcoxy en C₁-C₄ ou alkylthio en C₁-C₄ ;
alcényle en C₂-C₆ le cas échéant substitué par oxo ;
allényle ;
alcynyle en C₃-C₈, en particulier 2-butynyle ;
cycloalkyle en C₃-C₆ ;
cycloalcényle en C₅-C₆ ;
(cycloalkyle en C₃-C₆) - (alkyle en C₁-C₂), en particulier cyclopropylméthyle, le cas échéant substitué par un alkyle en C₁-C₄ ;
(cycloalcényle en C₃-C₆) - (alkyle en C₁-C₂), en
particulier cyclohéxénylméthyle ;
alkylcarbonyle en C₁-C₆ le cas échéant substitué par fluoro, chloro, hydroxy, benzyloxy, phénoxy, alcoxy en C₁-C₄, alkylamino en C₁-C₄, alcénylamino en C₁-C₄, dialkylamino en C₁-C₄, alkylthio en C₁-C₄ ;
alcénylcarbonyle en C₂-C₆ ;
alkyloxycarbonyle en C₁-C₆ le cas échéant substitué par fluoro, chloro, bromo, hydroxy, alcoxy en C₁-C₄, alkylamino en C₁-C₄, dialkylamino en C₁-C₄, alkylthio en C₁-C₄ ;
alcényloxycarbonyle en C₂-C₆, en particulier vinyloxycarbonyle, allyloxycarbonyle, isopropényloxycarbonyle, butényloxycarbonyle, pentényloxycarbonyl ;
alcynyloxycarbonyle en C₂-C₆, en particulier propynyloxycarbonyle, butynyloxycarbonyle ;
alkylthiocarbonyle en C₁-C₆ ;
alcénylthiocarbonyle en C₂-C₈, en particulier allylthiocarbonyle ;
alkylaminocarbonyle en C₁-C₆ et dialkylaminocarbonyle en C₁-C₆ ;
pyrrolidin-1-yle, morpholino-, pipéridino-, pipérazinyle ou 4-méthylpipérazin-1-ylcarbonyle;
alcénylaminocarbonyle en C₂-C₆ et dialcénylaminocarbonyle en C₁-C₆ ;
alkylsulfonyle en C₁-C₄ ;
alcénylsulfonyle en C₁-C₄ ;
ou aryle substitué par jusqu'à deux résidus R⁶ indépendants les uns des autres, en particulier phényle, arylcarbonyle, en particulier benzoyle, (arylthio)carbonyle, aryloxycarbonyle, arylaminocarbonyle (arylamino)thiocarbonyle, arylalkylaminocarbonyle, arylsulfonyle, arylalkyle, en particulier benzyle, phényléthyle, arylalcényle, arylalkylcarbonyle, arylalcoxycarbonyle, aryl(alkylthio)carbonyle, le résidu alkyle pouvant contenir respectivement de 1 à 3 atomes de carbone et R⁶ étant tel que défini plus haut,
ou 1- ou 2-naphthylméthyle, 2-, 3- ou 4-picolyle, 2- ou 3-furylméthyle, 2- ou 3-thiénylméthyle, 2- ou 3-pyrrolylméthyle, 1-, 2-, 3- ou 4-pyridylcarbonyle, 2- ou 3-furylcarbonyle, 2- ou 3-thiénylcarbonyle, 2-, 3- ou 4-picolyloxycarbonyle, 2- ou 3-furylméthyloxycarbonyle, 2-ou 3-thiénylméthyloxycarbonyle substitués par jusqu'à deux résidus R⁶ indépendants les uns des autres ;
et
R³ et R⁴ sont identiques ou différents et indépendamment les uns des autres hydrogène, alkyle en C₁-C₄ le cas échéant substitué par hydroxy, mercapto, alcoxy en C₁-C₄, alkylthio en C₁-C₄, alkylsulfonyle en C₁-C₄, alkylsulfinyle en C₁-C₄, carboxy, carbamoyle ;
alcényle en C₂-C₆ ;
aryle, benzyle, thiényle ou thiénylméthyle substitués par jusqu'à deux résidus R⁶ indépendants les uns des autres, dans lesquels R⁶ est tel que défini plus haut,
R³ et R⁴ peuvent également être
une partie d'un noyau carbo- ou hétérocyclique saturé ou insaturé ayant de 3 à 6 atomes de carbone, qui peut être substitué le cas échéant par oxo ou thioxo, et
X est oxygène ou soufre.

3. Composés des formules I ou Ia selon la revendication 1 ou la revendication 2, **caractérisés en ce que** les substituants mentionnés ont les significations suivantes :
n vaut zéro ou un ;
les différents substituants R¹ sont indépendamment les uns des autres fluoro, chloro, bromo, alkyle en C₁-C₂, alcoxy en C₁-C₂, acyle en C₂-C₄, cyano,
R² est hydrogène et R⁵ est
alcényle en C₂-C₆ ;
alcynyle en C₃-C₈, en particulier 2-butynyle ;
(cycloalkyle en C₃-C₆)-(alkyle en C₁-C₂), en particulier cyclopropylméthyle, le cas échéant substitué par un alkyle en C₁-C₄ ;
(cycloalcényle en C₃-C₆)-(alkyle en C₁-C₂), en
particulier cyclohéxénylméthyle ;
alkylcarbonyle en C₂-C₆ ;
alcénylcarbonyle en C₂-C₆ ;
alkyloxycarbonyle en C₁-C₆ ;
alcényloxycarbonyle en C₂-C₆, en particulier vinyloxycarbonyle, allyloxycarbonyle, isopropényloxycarbonyle, butényloxycarbonyle, pentényloxycarbonyle ;
alcynyloxycarbonyle en C₂-C₆, en particulier propynyloxycarbonyle, butynyloxycarbonyle ;
alcénylthiocarbonyle en C₂-C₆, en particulier allylthiocarbonyle ;
alkylsulfonyle en C₁-C₄ ;
alcénylsulfonyle en C₁-C₄ ;
ou arylalkyle substitué par jusqu'à deux résidus R⁶ indépendants les uns des autres, en particulier benzyle ou arylalcényle, le résidu alkyle pouvant contenir respectivement de 1 à 3 atomes de carbone et le résidu alcényle 2 à 3 atomes de carbone,
ou 1-naphthylméthyle, 2- ou 3-picolyle, 2-furylméthyle, 2- ou 3-thiénylméthyle substitués par jusqu'à deux résidus R⁶ indépendants les uns des autres,
dans lesquels R⁶ est fluoro, chloro, bromo, cyano, alkyle en C₁-C₂, alcoxy en C₁-C₂,
et
R³ et R⁴ sont identiques ou différents et indépendamment les uns des autres hydrogène, alkyle en C₁-C₄ le cas échéant substitué par hydroxy, mercapto, alcoxy en C₁-C₄, alkylthio en C₁-C₂ et X est oxygène ou soufre.

4. Procédé pour la fabrication de composés de la formule I selon la revendication 1, **caractérisé en ce que**
A) pour la fabrication de composés de la formule I dans laquelle X est oxygène et les résidus R¹, R², R³, R⁴ et R⁵ sont tels que définis en 1), on fait réagir un composé de la formule II 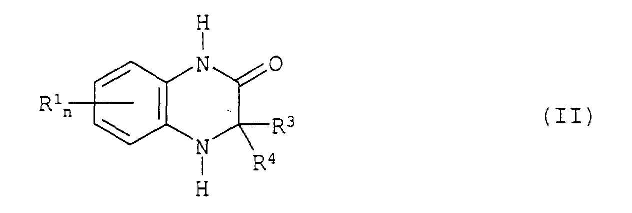 dans laquelle R¹, R³ et R⁴ ont les définitions mentionnées en 1), avec un composé de la formule III,
R-Z (III)
dans laquelle R a les significations mentionnées en 1) pour R⁵ et R² à l'exception de hydrogène, hydroxy, alcoxy en C₁-C₆, aryloxy, acyloxy en C₁-C₆, amino, alkylamino en C₁-C₆, dialkylamino en C₁-C₆, arylamino, acylamino en C₁-C₆ et Z est un groupe partant,
ou en ce que
B) on prépare des composés de la formule I, dans laquelle X est soufre et R¹, R², R³, R⁴ et R⁵ sont tels que définis en 1), par réaction d'un composé de la formule I, dans laquelle X est oxygène et R¹, R², R³, R⁴ et R⁵ ont les définitions mentionnées en 1), avec un réactif de sulfuration,
ou en ce que
C) on prépare des composés de la formule Ia, dans laquelle X et les résidus R¹ à R⁵ sont tels que- définis en 1), en faisant réagir un composé de la formule IV, 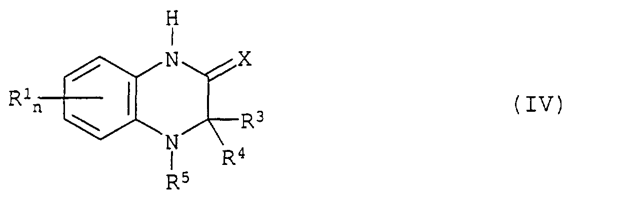 ou 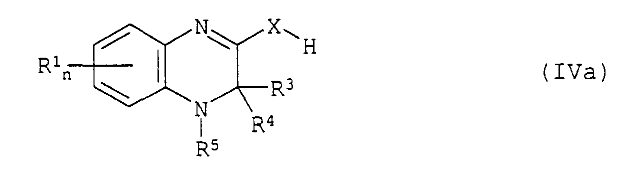 dans laquelle R¹, R³, R⁴ et R⁵ ont les définitions mentionnées en 1), avec un composé de la formule III,
R²-Z (III)
dans laquelle R² a les définitions décrites en 1) pour les formules I et Ia à l'exception de hydrogène, hydroxy, alcoxy en C₁-C₆, aryloxy, acyloxy en C₁-C₆, amino, alkylamino en C₁-C₆, dialkylamino en C₁-C₆, arylamino, acylamino en C₁-C₆ et Z est un groupe partant,
ou en ce que
D) on prépare des composés de la formule I, dans laquelle X est oxygène et les résidus R¹ à R⁵ sont tels que définis en 1), par cyclisation d'un composé de la formule V 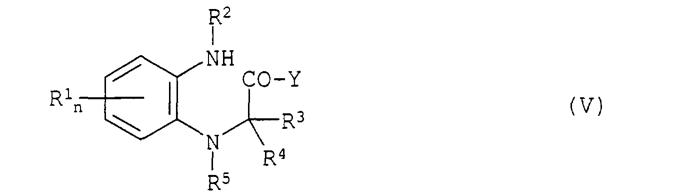 dans laquelle R¹ à R⁵ sont tels que définis en 1) et Y est hydroxy, alcoxy en C₁-C₄, le cas échéant acyloxy en C₁-C₄ halogéné, chloro, bromo ou iodo,
ou en ce que
E) on prépare des composés de la formule I, dans laquelle X est oxygène, R⁴ et R⁵ sont hydrogène et R¹ à R³ ont les définitions mentionnées en 1), à partir des quinoxalinones de la formule XI, 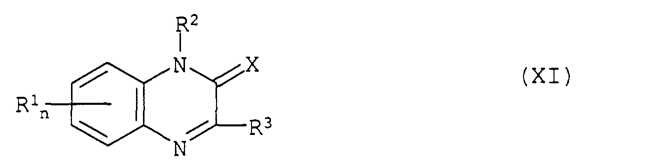 dans laquelle R¹ à R³ sont tels que définis en 1), en fixant par addition un hydrogène au niveau de la liaison C=N,
ou en ce que
F) on prépare des composés de la formule I, dans laquelle X est oxygène et R¹ à R⁵ sont tels que définis en 1), à partir de composés de la formule VI, 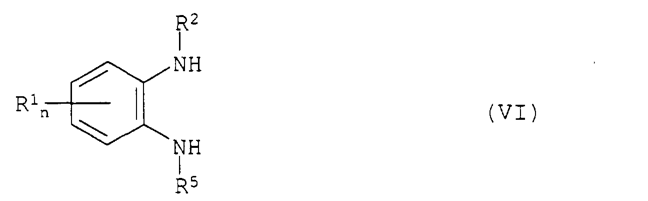 dans laquelle R¹, R² et R⁵ sont tels que définis en 1), par réaction avec du chloroforme ou du bromoforme et un composé carbonyle de la formule XIII,
R³-CO-R⁴ (XIII)
dans laquelle R³ et R⁴ sont tels que définis en 1) ou avec des α-(trihalogénométhyl)-alcanols de la formule XIV,
Hal₃C-C(OH)-R³R⁴ (XIV)
dans laquelle Hal est mis pour Cl, Br ou I et R³ et R⁴ sont tels que définis en 1),
ou en ce que
G) on prépare des composés de la formule I, dans laquelle X est oxygène et R¹, R², R³, R⁴ et R⁵ sont tels que définis en 1), par réaction d'un composé de la formule I, dans laquelle X est oxygène et R¹, R², R⁵ ainsi que R³ et R⁴ ont les définitions mentionnées en 1), sauf que l'un au moins des résidus R³ ou R⁴ est hydrogène, avec un réactif d'alkylation de la formule XV,
R'-Z (XV)
dans laquelle R' a les significations indiquées plus haut pour R³ et R⁴ à l'exception d'hydrogène et Z est un groupe partant,
ou en ce que
H) on prépare des composés de la formule I, dans laquelle X est oxygène, R¹, R², R³ et R⁴ sont tels que définis dans la revendication 1 et R⁵ est alkyle en C₁-C₆ le cas échéant substitué par fluoro, chloro, hydroxy, acyloxy en C₁-C₄, benzoyloxy, phénoxy, alcoxy en C₁-C₄, alkylamino en C₁-C₄, dialkylamino en C₁-C₄, alkylthio en C₁-C₄, carboxy, carbamoyle, alcényle en C₃-C₆ le cas échéant substitué par fluoro, chloro, hydroxy, acyloxy en C₁-C₄, benzoyloxy, phénoxy, alcoxy en C₁-C₄, alkylamino en C₁-C₄, dialkylamino en C₁-C₄, alkylthio en C₁-C₄, carboxy, carbamoyle, alcynyle en C₃-C₈ le cas échéant substitué par fluoro, chloro, hydroxy, acyloxy en C₁-C₄, benzoyloxy, phénoxy, alcoxy en C₁-C₄, alkylamino en C₁-C₄, dialkylamino en C₁-C₄, alkylthio en C₁-C₄, carboxy, carbamoyle, cycloalkyle en C₄-C₈ le cas échéant substitué par fluoro, chloro, hydroxy, acyloxy en C₁-C₄, benzoyloxy, alcoxy en C₁-C₄, alkylamino en C₁-C₄, dialkylamino en C₁-C₄, alkylthio en C₁-C₄, carboxy, carbamoyle, cycloalcényle en C₅-C₈ le cas échéant substitué par fluoro, chloro, hydroxy, acyloxy en C₁-C₄, benzoyloxy, phénoxy, alcoxy en C₁-C₄, alkylamino en C₁-C₄, dialkylamino en C₁-C₄, alkylthio en C₁-C₄, carboxy, carbamoyle, par alkylation réductrice d'un composé de la formule I, dans laquelle R⁵ est hydrogène et X est oxygène et R¹, R², R³ et R⁴ ont les définitions mentionnées en 1), avec un composé carbonyle de la formule XVI,
R"-C(=O)-R"' (XVI)
dans laquelle R" et R"' sont identiques ou différents et indépendamment les uns des autres hydrogène, alkyle en C₁-C₅ le cas échéant substitué par fluoro, chloro, hydroxy, acyloxy en C₁-C₄, benzoyloxy, phénoxy, alcoxy en C₁-C₄, alkylamino en C₁-C₄, dialkylamino en C₁-C₄, alkylthio en C₁-C₄, carboxy, carbamoyle, alcényle en C₃-C₅ le cas échéant substitué par fluoro, chloro, hydroxy, acyloxy en C₁-C₄, benzoyloxy, phénoxy, alcoxy en C₁-C₄, alkylamino en C₁-C₄, carboxy, carbamoyle, alcynyle en C₃-C₇ le cas échéant substitué par fluoro, chloro, hydroxy, acyloxy en C₁-C₄, benzoyloxy, phénoxy, alcoxy en C₁-C₄, alkylamino en C₁-C₄, dialkylamino en C₁-C₄, alkylthio en C₁-C₄, carboxy, carbamoyle, cycloalkyle en C₄-C₈ le cas échéant substitué par fluoro, chloro, hydroxy, acyloxy en C₁-C₄, benzoyloxy, phénoxy, alcoxy en C₁-C₄, alkylamino en C₁-C₄, dialkylamino en C₁-C₄, alkylthio en C₁-C₄, carboxy, carbamoyle, cycloalcényle en C₆ le cas échéant substitué par fluoro, chloro, hydroxy, acyloxy en C₁-C₄, benzoyloxy, phénoxy, alcoxy en C₁-C₄, alkylamino en C₁-C₄, dialkylamino en C₁-C₄, alkylthio en C₁-C₄, carboxy, et dans laquelle R" et R"' peuvent être reliés entre eux avec formation d'un noyau de 4 à 8 termes,
ou en ce que
I) on prépare des composés de la formule I, dans laquelle X est oxygène et R¹, R², R³ et R⁴ sont tels que définis en 1) et R⁵ est alkyloxycarbonyle en C₁-C₆, alkylthiocarbonyle en C₁-C₆, alcényloxycarbonyle en C₂-C₆, alcénylthiocarbonyle en C₂-C₆, alcynyloxycarbonyle en C₂-C₈, alkylaminocarbonyle en C₁-C₆, alcénylaminocarbonyle en C₃-C₆, dialkylaminocarbonyle en C₁-C₆, pyrrolidin-1-yle, morpholino, pipéridino, pipérazinyle, 4-méthylpipérazin-1-ylcarbonyle le cas échéant substitué par fluoro, chloro, ou aryloxycarbonyle, arylthiocarbonyle, arylaminocarbonyle, arylalkylthiocarbonyle, arylalkylaminocarbonyle substitués par jusqu'à deux résidus R⁶ indépendants les uns des autres, le résidu alkyle pouvant contenir respectivement de 1 à 3 atomes de carbone, en faisant réagir un composé de la formule XVII, 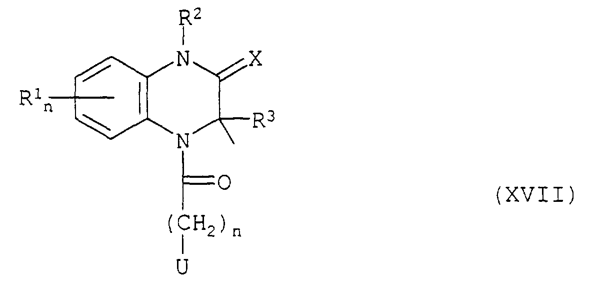 dans laquelle R¹, R², R³ et R⁴ ont les définitions mentionnées en 1), n vaut 0, 1, 2 ou 3, X est oxygène et U est un groupe partant, avec un composé de la formule XVIII,
Nu-H (XVIII)
dans laquelle Nu est alkyloxy en C₁-C₆, alcényloxy en C₂-C₆, alcynyloxy en C₂-C₆, alkylthio en C₁-C₆, alcénylthio en C₂-C₆, alkylamino en C₁-C₆ et dialkylamino en C₁-C₆, alcénylamino en C₃-C₆ et dialkylamino en C₁-C₆, le cas échéant substitué par fluoro, chloro, hydroxy, alcoxy en C₁-C₄, pyrrolidin-1-yle, morpholino-, pipéridino, pipérazinyle ou 4-méthylpipérazin-1-yle, ou aryloxy, arylthio, arylamino, arylalkyloxy, arylalkylthio, arylalkylamino, 2-, 3-, ou 4-pyridyle, 2- ou 3-furyle, 2- ou 3-thiényle, 2-, 3- ou 4-picolyloxy, 2- ou 3-furylméthyloxy, 2- ou 3-thiénylméthyloxy substitués par jusqu'à deux résidus R⁶ indépendants les uns des autres (R⁶ étant tel que défini plus haut), le résidu alkyle pouvant contenir respectivement de 1 à 3 atomes de carbone.

5. Composés des formules I ou Ia selon l'une ou plusieurs des revendications 1 à 3, destinés à être utilisés comme médicaments.

6. Médicament contenant une quantité efficace d'au moins un composé de la formule Ia selon l'une ou plusieurs des revendications 1 à 3.

7. Procédé pour la fabrication d'un médicament selon la revendication 6, **caractérisé en ce que** la quantité efficace d'un composé de la formule I ou Ia est mise sous une forme de présentation appropriée avec des adjuvants pharmaceutiques usuels.

8. Utilisation de composés de la formule I ou Ia selon l'une ou plusieurs des revendications 1 à 3 pour la fabrication de médicaments destinés au traitement de maladies provoquées par des virus, en particulier des maladies provoquées par le virus HIV.

## Revendications (Revendications pour l'(les) Etat(s) contractant(s) suivant(s): ES, GR)

1. Procédé pour la fabrication d'un composé des formules I et Ia, 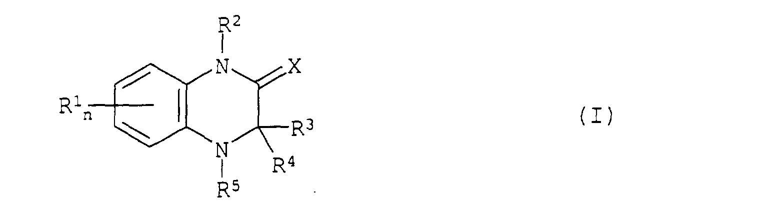 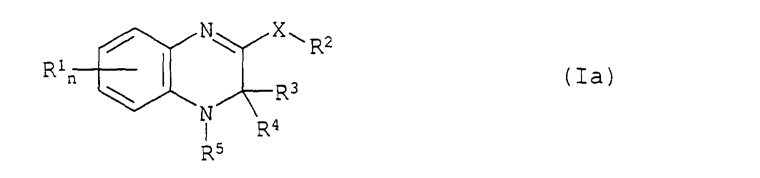 ainsi que leurs sels physiologiquement compatibles,
les symboles dans les formules I et Ia ayant les significations suivantes :
n vaut zéro, un ou deux ;
les différents substituants R¹ sont indépendamment les uns des autres fluoro, chloro, bromo, trifluorométhyle, hydroxy, alkyle en C₁-C₄, alcoxy en C₁-C₄, (alcoxy en C₁-C₄)-(alcoxy en C₁-C₄), alkylthio en C₁-C₄, nitro, amino, alkylamino en C₁-C₄, dialkylamino en C₁-C₄, pipéridino, morpholino, 1-pyrrolidinyle, 4-méthylpipérazinyle, acyle en C₁-C₄, acyloxy en C₁-C₄, acylamino en C₁-C₄, cyano, carbamoyle, carboxy, (alkyle en C₁-C₄)oxycarbonyle, hydroxysulfonyle, sulfamoyle,
ou
un résidu phényle, phénoxy, phénylthio, phénylsulfonyle, phénoxysulfonyle, benzoyle, 2-pyridyle, 3-pyridyle ou 4-pyridyle substitué par jusqu'à deux résidus R⁶ indépendants les uns des autres,
dans lesquels R⁶ peut être fluoro, chloro, bromo, cyano, trifluorométhyle, nitro, amino, alkyle en C₁-C₄, alcoxy en C₁-C₄, (alkyle en C₁-C₄)-oxycarbonyle, phényle, phénoxy,
R² est hydrogène et R⁵ est
alkyle en C₁-C₆ le cas échéant substitué par fluoro, chloro, hydroxy, acyloxy en C₁-C₄, benzoyloxy, benzyloxy, phénoxy, alcoxy en C₁-C₄, alkylamino en C₁-C₄, dialkylamino en C₁-C₄, alkylthio en C₁-C₄, oxo, thioxo, carboxy, carbamoyle ;
alcényle en C₂-C₆ le cas échéant substitué par fluoro, chloro, hydroxy, acyloxy en C₁-C₄, benzoyloxy, benzyloxy, phénoxy, alcoxy en C₁-C₄, alkylamino en C₁-C₄, dialkylamino en C₁-C₄, alkylthio en C₁-C₄, oxo, thioxo, carboxy, carbamoyle ;
alcynyle en C₃-C₈ le cas échéant substitué par fluoro, chloro, hydroxy, acyloxy en C₁-C₄, benzoyloxy, benzyloxy, phénoxy, alcoxy en C₁-C₄, alkylamino en C₁-C₄, dialkylamino en C₁-C₄, alkylthio en C₁-C₄, oxo, thioxo, carboxy, carbamoyle ;
cycloalkyle en C₃-C₈ le cas échéant substitué par fluoro, chloro, hydroxy, alkyle en C₁-C₄, acyloxy en C₁-C₄, benzoyloxy, benzyloxy, phénoxy, alcoxy en C₁-C₄, alkylamino en C₁-C₄, dialkylamino en C₁-C₄, alkylthio en C₁-C₄, oxo, thioxo, carboxy, carbamoyle ;
cycloalcényle en C₃-C₈ le cas échéant substitué par fluoro, chloro, hydroxy, alkyle en C₁-C₄, acyloxy en C₁-C₄, benzoyloxy, benzyloxy, phénoxy, alcoxy en C₁-C₄, alkylamino en C₁-C₄, dialkylamino en C₁-C₄, alkylthio en C₁-C₄, oxo, thioxo, carboxy, carbamoyle ;
(cycloalkyle en C₃-C₆)-(alkyle en C₁-C₂) , le cas échéant substitué par fluoro, chloro, hydroxy, alkyle en C₁-C₄, acyloxy en C₁-C₄, benzoyloxy, benzyloxy, phénoxy, alcoxy en C₁-C₄, alkylamino en C₁-C₄, dialkylamino en C₁-C₄, alkylthio en C₁-C₄, oxo, thioxo, carboxy, carbamoyle ;
(Cycloalcényle en C₃-C₆)-(alkyle en C₁-C₂), le cas échéant substitué par fluoro, chloro, hydroxy, alkyle en C₁-C₄, acyloxy en C₁-C₄, benzoyloxy, benzyloxy, phénoxy, alcoxy en C₁-C₄, alkylamino en C₁-C₄, dialkylamino en C₁-C₄, alkylthio en C₁-C₄, oxo, thioxo, carboxy, carbamoyle ;
alkylcarbonyle en C₁-C₆ le cas échéant substitué par fluoro, chloro, hydroxy, alkyle en C₁-C₄, acyloxy en C₁-C₄, benzoyloxy, benzyloxy, phénoxy, alcoxy en C₁-C₄, alkylamino en C₁-C₄, alcénylamino en C₁-C₄, dialkylamino en C₁-C₄, alkylthio en C₁-C₄, oxo, thioxo, carboxy, carbamoyle ;
alcénylcarbonyle en C₂-C₆ le cas échéant substitué par fluoro, chloro ou hydroxy ;
(cycloalkyle en C₃-C₆)carbonyle ;
(cycloalcényle en C₅-C₆)carbonyle ;
(cycloalkyle en C₃-C₆)-(alkyle en C₁-C₂)carbonyle,
(cycloalcényle en C₅-C₆)-(alkyle en C₁-C₂)carbonyle, alkyloxycarbonyle en C₁-C₆ le cas échéant substitué par fluoro, chloro, bromo, hydroxy, alcoxy en C₁-C₄, alkylamino en C₁-C₄, dialkylamino en C₁-C₄, alkylthio en C₁-C₄ ;
alcényloxycarbonyle en C₂-C₆ le cas échéant substitué par fluoro, chloro, hydroxy, alcoxy en C₁-C₄ ;
alcynyloxycarbonyle en C₂-C₆ le cas échéant substitué par fluoro, chloro, hydroxy, alcoxy en C₁-C₄ ;
alkylthiocarbonyle en C₁-C₆ le cas échéant substitué par fluoro, chloro, hydroxy, alcoxy en C₁-C₄ ;
alcénylthiocarbonyle en C₂-C₆ le cas échéant substitué par fluoro, chloro, hydroxy, alcoxy en C₁-C₄ ;
alkylaminocarbonyle en C₁-C₆ et dialkylaminocarbonyle en C₁-C₆ le cas échéant substitués par fluoro, chloro, hydroxy, alcoxy en C₁-C₄ ;
pyrrolidin-1-yle, morpholino, pipéridino, pipérazinyle ou 4-méthylpipérazin-1-ylcarbonyle;
alcénylaminocarbonyle en C₂-C₆ et dialcénylaminocarbonyle en C₁-C₆ le cas échéant substitués par fluoro, chloro, hydroxy, alcoxy en C₁-C₄ ;
alkylsulfonyle en C₁-C₄ le cas échéant substitué par fluoro, chloro, hydroxy, alcoxy en C₁-C₄ ;
alcénylsulfonyle en C₁-C₄ ;
ou aryle, arylcarbonyle, (arylthio)carbonyle, aryloxycarbonyle arylaminocarbonyle, (arylamino)thiocarbonyle, arylsulfonyle, arylalkylaminocarbonyle, arylalkyle, arylalcényle, arylalcoxycarbonyle, aryl(alkylthio)carbonyle substitués par jusqu'à deux résidus R⁶ indépendants les uns des autres, le résidu alkyle pouvant contenir respectivement de 1 à 3 atomes de carbone et R⁶ étant tel que défini plus haut
ou 1- ou 2-naphthylméthyle, 2-, 3- ou 4-picolyle, 2-ou 3-furylméthyle, 2- ou 3-thiénylméthyle, 2- ou 3-pyrrolylméthyle, 2-, 3- ou 4-pyridylcarbonyle, 2- ou 3-furylcarbonyle, 2- ou 3-thiénylcarbonyle, 2-, 3- ou 4-picolyloxycarbonyle, 2- ou 3-furylméthyloxycarbonyle, 2- ou 3-thiénylméthyloxycarbonyle substitués par jusqu'à deux résidus R⁶ indépendants les uns des autres,
et
R³ et R⁴ sont identiques ou différents et indépendamment les uns des autres hydrogène, alkyle en C₁-C₄ le cas échéant substitué par fluoro, chloro, hydroxy, amino, mercapto, acyloxy en C₁-C₄, benzoyloxy, phénoxy, alcoxy en C₁-C₄, alkylamino en C₁-C₄, dialkylamino en C₁-C₄, alkylthio en C₁-C₄, alkylsulfonyle en C₁-C₄, alkylsulfinyle en C₁-C₄, carboxy, carbamoyle ;
alcényle en C₂-C₆ le cas échéant substitué par fluoro ou chloro ;
cycloalkyle en C₃-C₆ le cas échéant substitué par fluoro, chloro, hydroxy, amino, mercapto, acyloxy en C₁C₄, benzoyloxy, benzyloxy, phénoxy, alcoxy en C₁-C₄, alkylamino en C₁-C₄, dialkylamino en C₁-C₄, alkylthio en C₁-C₄, alkylsulfonyle en C₁-C₄, alkylsulfinyle en C₁-C₄, carboxy, carbamoyle ;
cycloalcényle en C₃-C₈ le cas échéant substitué par fluoro ou chloro ;
aryle, benzyle, hétéroaryle ou hétéroarylméthyle substitués par jusqu'à deux résidus R⁶ indépendants les uns des autres,
R³ et R⁴ peuvent également être
Une partie d'un noyau carbo- ou hétérocyclique saturé ou insaturé ayant de 3 à 6 atomes de carbone, qui peut le cas échéant être substitué par fluoro, chloro, hydroxy, amino, acyloxy en C₁-C₄, benzoyloxy, alcoxy en C₁-C₄, oxo, thioxo, carboxy, carbamoyle, et
les groupes aryles mentionnés dans les définitions précédentes sont des groupes aromatiques ayant de 6 à 14 atomes de carbone, les noyaux hétérocycliques ou groupes hétéroaryles mentionnés dans les définitions précédentes contiennent 1 à 13 atomes de carbone et 1 à 6 hétéroatomes choisis dans le groupe comprenant O, S et N, N-Z étant présent dans le cas d'un noyau contenant N saturé dans cette position, Z, H ou R⁵ ayant les définitions respectives décrites plus haut,
X est oxygène ou soufre, à l'exception des composés dans lesquels R³ et R⁴ sont en même temps H,
et des composés dans lesquels R⁵ est COCHCl₂ et des composés dans lesquels n vaut 1, R¹, R³ et R⁴ sont méthyle et R⁵ est acétyle, et des composés dans lesquels n vaut zéro, R³ et R⁴ sont méthyle ou phényle et R⁵ est éthyloxycarbonyle,
**caractérisé en ce que**
A) pour la fabrication de composés de la formule I dans laquelle X est oxygène et les résidus R¹, R², R³, R⁴ et R⁵ sont tels que définis en 1), on fait réagir un composé de la formule II 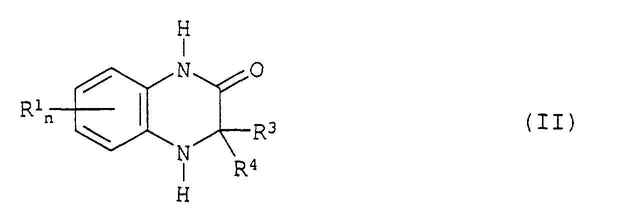 dans laquelle R¹, R³ et R⁴ ont les définitions mentionnées en 1), avec un composé de la formule III,
R-Z (III)
dans laquelle R a les significations mentionnées en 1) pour R⁵ et R² à l'exception de hydrogène, hydroxy, alcoxy en C₁-C₆, aryloxy, acyloxy en C₁-C₆, amino, alkylamino en C₁-C₆, dialkylamino en C₁-C₆, arylamino, acylamino en C₁-C₆ et Z est un groupe partant,
ou en ce que
B) on prépare des composés de la formule I, dans laquelle X est soufre et R¹, R², R³, R⁴ et R⁵ sont tels que définis en 1), par réaction d'un composé de la formule I, dans laquelle X est oxygène et R¹, R², R³, R⁴ et R⁵ ont les définitions mentionnées en 1), avec un réactif de sulfuration,
ou en ce que
C) on prépare des composés de la formule Ia, dans laquelle X et les résidus R¹ à R⁵ sont tels que définis en 1), en faisant réagir un composé de la formule IV, 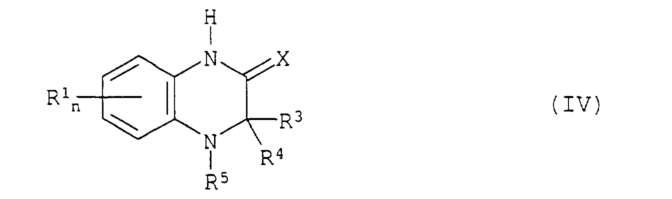 ou 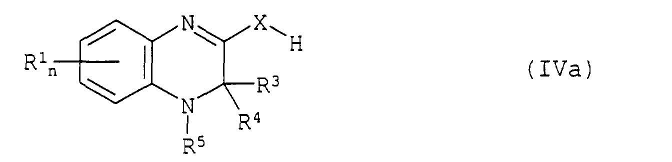 dans laquelle R¹, R³, R⁴ et R⁵ ont les définitions mentionnées en 1), avec un composé de la formule III,
R²-Z (III)
dans laquelle R² a les définitions décrites en 1) pour les formules I et Ia à l'exception de hydrogène, hydroxy, alcoxy en C₁-C₆, aryloxy, acyloxy en C₁-C₆, amino, alkylamino en C₁-C₆, dialkylamino en C₁-C₆, arylamino, acylamino en C₁-C₆ et Z est un groupe partant,
ou en ce que
D) on prépare des composés de la formule I, dans laquelle X est oxygène et les résidus R¹ à R⁵ sont tels que définis en 1), par cyclisation d'un composé de la formule V 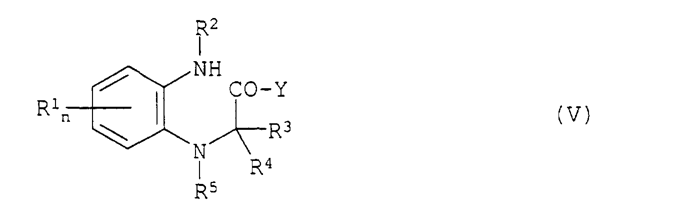 dans laquelle R¹ à R⁵ sont tels que définis en 1) et Y est hydroxy, alcoxy en C₁-C₄, le cas échéant acyloxy en C₁-C₄ halogéné, chloro, bromo ou iodo,
ou en ce que
E) on prépare des composés de la formule I, dans laquelle X est oxygène, R⁴ et R⁵ sont hydrogène et R¹ à R³ ont les définitions mentionnées en 1), à partir des quinoxalinones de la formule XI, 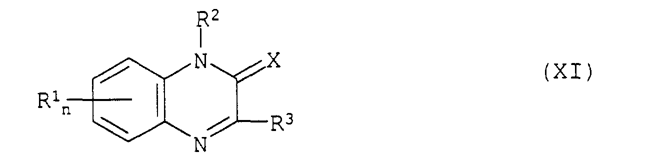 dans laquelle R¹ à R³ sont tels que définis en 1), en fixant par addition un hydrogène au niveau de la liaison C=N,
ou en ce que
F) on prépare des composés de la formule I, dans laquelle X est oxygène et R¹ à R⁵ sont tels que définis en 1), à partir de composés de la formule VI, 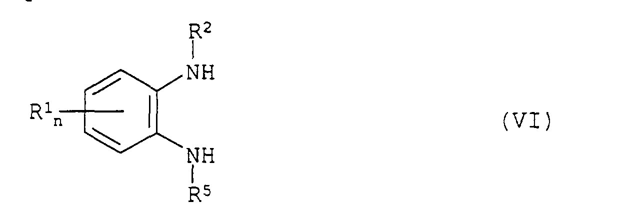 dans laquelle R¹, R² et R⁵ sont tels que définis en 1) , par réaction avec du chloroforme ou du bromoforme et un composé carbonyle de la formule XIII,
R³-CO-R⁴ (XIII)
dans laquelle R³ et R⁴ sont tels que définis en 1) ou avec des α-(trihalogénométhyl)-alcanols de la formule XIV,
Hal₃C-C(OH)-R³R⁴ (XIV)
dans laquelle Hal est mis pour Cl, Br ou I et R³ et R⁴ sont tels que définis en 1),
ou en ce que
G) on prépare des composés de la formule I, dans laquelle X est oxygène et R¹, R², R³, R⁴ et R⁵ sont tels que définis en 1), par réaction d'un composé de la formule I, dans laquelle X est oxygène et R¹, R², R⁵ ainsi que R³ et R⁴ ont les définitions mentionnées en 1), sauf que l'un au moins des résidus R³ ou R⁴ est hydrogène, avec un réactif d'alkylation de la formule XV,
R'-Z (XV)
dans laquelle R' a les significations indiquées plus haut pour R³ et R⁴ à l'exception d'hydrogène et Z est un groupe partant,
ou en ce que
H) on prépare des composés de la formule I, dans laquelle X est oxygène, R¹, R², R³ et R⁴ sont tels que définis dans la revendication 1 et R⁵ est alkyle en C₁-C₆ le cas échéant substitué par fluoro, chloro, hydroxy, acyloxy en C₁-C₄, benzoyloxy, phénoxy, alcoxy en C₁-C₄, alkylamino en C₁-C₄, dialkylamino en C₁-C₄, alkylthio en C₁-C₄, carboxy, carbamoyle, alcényle en C₃-C₆ le cas échéant substitué par fluoro, chloro, hydroxy, acyloxy en C₁-C₄, benzoyloxy, phénoxy, alcoxy en C₁-C₄, alkylamino en C₁-C₄, dialkylamino en C₁-C₄, alkylthio en C₁-C₄, carboxy, carbamoyle, alcynyle en C₃-C₈ le cas échéant substitué par fluoro, chloro, hydroxy, acyloxy en C₁-C₄, benzoyloxy, phénoxy, alcoxy en C₁-C₄, alkylamino en C₁-C₄, dialkylamino en C₁-C₄, alkylthio en C₁-C₄, carboxy, carbamoyle, cycloalkyle en C₄-C₈ le cas échéant substitué par fluoro, chloro, hydroxy, acyloxy en C₁-C₄, benzoyloxy, alcoxy en C₁-C₄, alkylamino en C₁-C₄, dialkylamino en C₁-C₄, alkylthio en C₁-C₄, carboxy, carbamoyle, cycloalcényle en C₅-C₈ le cas échéant substitué par fluoro, chloro, hydroxy, acyloxy en C₁-C₄, benzoyloxy, phénoxy, alcoxy en C₁-C₄, alkylamino en C₁-C₄, dialkylamino en C₁-C₄, alkylthio en C₁-C₄, carboxy, carbamoyle, par alkylation réductrice d'un composé de la formule I, dans laquelle R⁵ est hydrogène et X est oxygène et R¹, R², R³ et R⁴ ont les définitions mentionnées en 1), avec un composé carbonyle de la formule XVI,
R"-C(=O)-R"' (XVI)
dans laquelle R" et R"' sont identiques ou différents et indépendamment les uns des autres hydrogène, alkyle en C₁-C₅ le cas échéant substitué par fluoro, chloro, hydroxy, acyloxy en C₁-C₄, benzoyloxy, phénoxy, alcoxy en C₁-C₄, alkylamino en C₁-C₄, dialkylamino en C₁-C₄, alkylthio en C₁-C₄, carboxy, carbamoyle, alcényle en C₃-C₅ le cas échéant substitué par fluoro, chloro, hydroxy, acyloxy en C₁-C₄, benzoyloxy, phénoxy, alcoxy en C₁-C₄, alkylamino en C₁-C₄, carboxy, carbamoyle, alcynyle en C₃-C₇ le cas échéant substitué par fluoro, chloro, hydroxy, acyloxy en C₁-C₄, benzoyloxy, phénoxy, alcoxy en C₁-C₄, alkylamino en C₁-C₄, dialkylamino en C₁-C₄, alkylthio en C₁-C₄, carboxy, carbamoyle, cycloalkyle en C₄-C₈ le cas échéant substitué par fluoro, chloro, hydroxy, acyloxy en C₁-C₄, benzoyloxy, phénoxy, alcoxy en C₁-C₄, alkylamino en C₁-C₄, dialkylamino en C₁-C₄, alkylthio en C₁-C₄, carboxy, carbamoyle, cycloalcényle en C₆ le cas échéant substitué par fluoro, chloro, hydroxy, acyloxy en C₁-C₄, benzoyloxy, phénoxy, alcoxy en C₁-C₄, alkylamino en C₁-C₄, dialkylamino en C₁-C₄, alkylthio en C₁-C₄, carboxy, et dans laquelle R" et R"' peuvent être reliés entre eux avec formation d'un noyau de 4 à 8 termes,
ou en ce que
I) on prépare des composés de la formule I, dans laquelle X est oxygène et R¹, R², R³ et R⁴ sont tels que définis en 1) et R⁵ est alkyloxycarbonyle en C₁-C₆, alkylthiocarbonyle en C₁-C₆, alcényloxycarbonyle en C₂-C₆, alcénylthiocarbonyle en C₂-C₆, alcynyloxycarbonyle en C₂-C₈, alkylaminocarbonyle en C₁-C₆, alcénylaminocarbonyle en C₃-C₆, dialkylaminocarbonyle en C₁-C₆, pyrrolidin-1-yle, morpholino, pipéridino, pipérazinyle, 4-méthylpipérazin-1-ylcarbonyle le cas échéant substitué par fluoro, chloro, ou aryloxycarbonyle, arylthiocarbonyle, arylaminocarbonyle, arylalkylthiocarbonyle, arylalkylaminocarbonyle substitués par jusqu'à deux résidus R⁶ indépendants les uns des autres, le résidu alkyle pouvant contenir respectivement de 1 à 3 atomes de carbone, en faisant réagir un composé de la formule XVII, 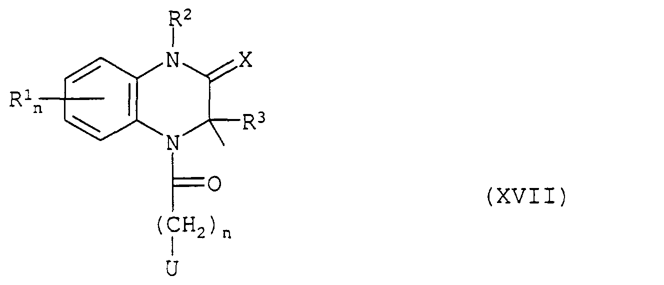 dans laquelle R¹, R², R³ et R⁴ ont les définitions mentionnées en 1), n vaut 0, 1, 2 ou 3, X est oxygène et U est un groupe partant, avec un composé de la formule XVIII,
Nu-H (XVIII)
dans laquelle Nu est alkyloxy en C₁-C₆, alcényloxy en C₂-C₆, alcynyloxy en C₂-C₆, alkylthio en C₁-C₆, alcénylthio en C₂-C₆, alkylamino en C₁-C₆ et dialkylamino en C₁-C₆, alcénylamino en C₃-C₆ et dialkylamino en C₁-C₆, le cas échéant substitué par fluoro, chloro, hydroxy, alcoxy en C₁-C₄, pyrrolidin-1-yle, morpholino-, pipéridino, pipérazinyle ou 4-méthylpipérazin-1-yle, ou aryloxy, arylthio, arylamino, arylalkyloxy, arylalkylthio, arylalkylamino, 2-, 3-, ou 4-pyridyle, 2- ou 3-furyle, 2- ou 3-thiényle, 2-, 3- ou 4-picolyloxy, 2- ou 3-furylméthyloxy, 2- ou 3-thiénylméthyloxy substitués par jusqu'à deux résidus R⁶ indépendants les uns des autres (R⁶ étant tel que défini plus haut), le résidu alkyle pouvant contenir respectivement de 1 à 3 atomes de carbone.

2. Procédé pour la fabrication de composés de la formule I ou Ia selon la revendication 1, **caractérisé en ce que** les substituants mentionnés ont les significations suivantes :
n vaut zéro, un ou deux ;
les différents substituants R¹ sont indépendamment les uns des autres fluoro, chloro, bromo, trifluorométhyle hydroxy, alkyle en C₁-C₄, alcoxy en C₁-C₄, (alcoxy en C₁-C₄)-(alcoxy en C₁-C₄), alkylthio en C₁-C₄, nitro, amino, alkylamino en C₁-C₄, dialkylamino en C₁-C₄, pipéridino, morpholino, 1-pyrrolidinyle, 4-méthylpipérazinyle, acyle en C₁-C₄, acyloxy en C₁-C₄, acylamino en C₁-C₄, cyano, carbamoyle, carboxy, alcoxycarbonyle en C₁-C₄, hydroxysulfonyle, sulfamoyle,
ou
un résidu phényle, phénoxy, phénylthio, phénylsulfonyle, phénoxysulfonyle, benzoyle, 2-pyridyle, 3-pyridyle ou 4-pyridyle substitué par jusqu'à deux résidus R⁶ indépendants les uns des autres,
dans lesquels R⁶ peut être fluoro, chloro, bromo, cyano, trifluorométhyle, nitro, amino, alkyle en C₁-C₄, alcoxy en C₁-C₄, (alkyle en C₁-C₄)-oxycarbonyle, phényle, phénoxy,
R² est hydrogène et R⁵ est
alkyle en C₁-C₆ le cas échéant substitué par alcoxy en C₁-C₄ ou alkylthio en C₁-C₄ ;
alcényle en C₂-C₆ le cas échéant substitué par oxo ;
allényle ;
alcynyle en C₃-C₈, en particulier 2-butynyle ;
cycloalkyle en C₃-C₆ ;
cycloalcényle en C₅-C₆ ;
(cycloalkyle en C₃-C₆)-(alkyle en C₁-C₂), en particulier cyclopropylméthyle, le cas échéant substitué par un alkyle en C₁-C₄ ;
(cycloalcényle en C₃-C₆)-(alkyle en C₁-C₂), en particulier cyclohéxénylméthyle ;
alkylcarbonyle en C₁-C₆ le cas échéant substitué par fluoro, chloro, hydroxy, benzyloxy, phénoxy, alcoxy en C₁-C₄, alkylamino en C₁-C₄, alcénylamino en C₁-C₄, dialkylamino en C₁-C₄, alkylthio en C₁-C₄ ;
alcénylcarbonyle en C₂-C₆ ;
alkyloxycarbonyle en C₁-C₆ le cas échéant substitué par fluoro, chloro, bromo, hydroxy, alcoxy en C₁-C₄, alkylamino en C₁-C₄, dialkylamino en C₁-C₄, alkylthio en C₁-C₄ ;
alcényloxycarbonyle en C₂-C₆, en particulier vinyloxycarbonyle, allyloxycarbonyle, isopropényloxycarbonyle, butényloxycarbonyle, pentényloxycarbonyl ;
alcynyloxycarbonyle en C₂-C₆, en particulier propynyloxycarbonyle, butynyloxycarbonyle ;
alkylthiocarbonyle en C₁-C₆ ;
alcénylthiocarbonyle en C₂-C₈, en particulier allylthiocarbonyle ;
alkylaminocarbonyle en C₁-C₆ et dialkylaminocarbonyle en C₁-C₆ ;
pyrrolidin-1-yle, morpholino-, pipéridino-, pipérazinyle ou 4-méthylpipérazin-1-ylcarbonyle;
alcénylaminocarbonyle en C₂-C₆ et dialcénylaminocarbonyle en C₁-C₆ ;
alkylsulfonyle en C₁-C₄ ;
alcénylsulfonyle en C₁-C₄ ;
ou aryle substitué par jusqu'à deux résidus R⁶ indépendants les uns des autres, en particulier phényle, arylcarbonyle, en particulier benzoyle, (arylthio)carbonyle, aryloxycarbonyle, arylaminocarbonyle (arylamino)thiocarbonyle, arylalkylaminocarbonyle, arylsulfonyle, arylalkyle, en particulier benzyle, phényléthyle, arylalcényle, arylalkylcarbonyle, arylalcoxycarbonyle, aryl(alkylthio)carbonyle, le résidu alkyle pouvant contenir respectivement de 1 à 3 atomes de carbone et R⁶ étant tel que défini plus haut,
ou 1- ou 2-naphthylméthyle, 2-, 3- ou 4-picolyle, 2- ou 3-furylméthyle, 2- ou 3-thiénylméthyle, 2- ou 3-pyrrolylméthyle, 1-, 2-, 3- ou 4-pyridylcarbonyle, 2- ou 3-furylcarbonyle, 2- ou 3-thiénylcarbonyle, 2-, 3- ou 4-picolyloxycarbonyle, 2- ou 3-furylméthyloxycarbonyle, 2- ou 3-thiénylméthyloxycarbonyle substitués par jusqu'à deux résidus R⁶ indépendants les uns des autres ;
et
R³ et R⁴ sont identiques ou différents et indépendamment les uns des autres hydrogène, alkyle en C₁-C₄ le cas échéant substitué par hydroxy, mercapto, alcoxy en C₁-C₄, alkylthio en C₁-C₄, alkylsulfonyle en C₁-C₄, alkylsulfinyle en C₁-C₄, carboxy, carbamoyle ;
alcényle en C₂-C₆ ;
aryle, benzyle, thiényle ou thiénylméthyle substitués par jusqu'à deux résidus R⁶ indépendants les uns des autres, dans lesquels R⁶ est tel que défini plus haut,
R³ et R⁴ peuvent également être
une partie d'un noyau carbo- ou hétérocyclique saturé ou insaturé ayant de 3 à 6 atomes de carbone, qui peut être substitué le cas échéant par oxo ou thioxo, et
X est oxygène ou soufre.

3. Procédé pour la fabrication de composés de la formule I ou Ia selon la revendication 1 ou la revendication 2, **caractérisé en ce que** les substituants mentionnés ont les significations suivantes :
n vaut zéro ou un ;
les différents substituants R¹ sont indépendamment les uns des autres fluoro, chloro, bromo, alkyle en C₁-C₂, alcoxy en C₁-C₂, acyle en C₂-C₄, cyano,
R² est hydrogène et R⁵ est
alcényle en C₂-C₆ ;
alcynyle en C₃-C₈, en particulier 2-butynyle ;
(cycloalkyle en C₃-C₆)-(alkyle en C₁-C₂), en particulier cyclopropylméthyle, le cas échéant substitué par un alkyle en C₁-C₄ ;
(cycloalcényle en C₃-C₆) - (alkyle en C₁-C₂), en particulier cyclohéxénylméthyle ;
alkylcarbonyle en C₂-C₆ ;
alcénylcarbonyle en C₂-C₆ ;
alkyloxycarbonyle en C₁-C₆ ;
alcényloxycarbonyle en C₂-C₆, en particulier vinyloxycarbonyle, allyloxycarbonyle, isopropényloxycarbonyle, butényloxycarbonyle, pentényloxycarbonyle ;
alcynyloxycarbonyle en C₂-C₆, en particulier propynyloxycarbonyle, butynyloxycarbonyle ;
alcénylthiocarbonyle en C₂-C₆, en particulier allylthiocarbonyle ;
alkylsulfonyle en C₁-C₄ ;
alcénylsulfonyle en C₁-C₄ ;
ou arylalkyle substitué par jusqu'à deux résidus R⁶ indépendants les uns des autres, en particulier benzyle ou arylalcényle, le résidu alkyle pouvant contenir respectivement de 1 à 3 atomes de carbone et le résidu alcényle 2 à 3 atomes de carbone,
ou 1-naphthylméthyle, 2- ou 3-picolyle, 2-furylméthyle, 2- ou 3-thiénylméthyle substitués par jusqu'à deux résidus R⁶ indépendants les uns des autres,
dans lesquels R⁶ est fluoro, chloro, bromo, cyano, alkyle en C₁-C₂, alcoxy en C₁-C₂,
et
R³ et R⁴ sont identiques ou différents et indépendamment les uns des autres hydrogène, alkyle en C₁-C₄ le cas échéant substitué par hydroxy, mercapto, alcoxy en C₁-C₄, alkylthio en C₁-C₂ et X est oxygène ou soufre.

4. Composés de la formule I ou Ia selon l'un ou plusieurs des revendications 1 à 3, destinés à être utilisés comme médicaments.

5. Médicament contenant une quantité efficace d'au moins un composé de la formule Ia selon l'une ou plusieurs des revendications 1 à 3.

6. Procédé pour la fabrication d'un médicament selon la revendication 5, **caractérisé en ce que** la quantité efficace d'un composé de la formule I ou Ia est mise sous une forme de présentation appropriée avec des adjuvants pharmaceutiques usuels.

7. Utilisation de composés de la formule I ou Ia selon l'une ou plusieurs des revendications 1 à 3 pour la fabrication de médicaments destinés au traitement de maladies provoquées par des virus, en particulier des maladies provoquées par le virus HIV.
